# EUROPEAN PATENT SPECIFICATION

(11) **EP 2 368 561 B1**
(45) Date of publication and mention of the grant of the patent: **04.12.2013**
(21) Application number: 11155927.4
(22) Date of filing: 18.04.2006
(51) Int. Cl.: A61K 38/00, C07K 5/06, C07K 5/08, C07K 5/10, C07K 14/775, C07K 7/02, C07K 7/08

(54) **Peptides and peptide mimetics to treat pathologies characterized by an inflammatory response**
Peptide und Peptidmimetika zur Behandlung von Pathologien, die durch eine Entzündungsreaktion gekennzeichnet sind
Peptides et mimétiques de peptides pour traiter des pathologies caractérisées par une réponse inflammatoire

(30) Priority: 29.04.2005 US 676431 P; 07.07.2005 US 697495 P
(43) Date of publication of application: 28.09.2011
(62) Divisional of application: 06750791.3
(73) Proprietor: The Regents of The University of California, Oakland, CA 94607-5200 (US)
(72) Inventor: Fogelman, Alan, Beverly Hills, CA 90212-4107 (US); Navab, Mohamad, Los Angeles, CA 90049 (US)
(74) Representative: Turner, Rhiannon Rosalind

(56) References cited:
- WO-A2-2004/034977
- US-A1- 2003 171 277
- US-A1- 2004 266 671
- GARG A ET AL: "Nuclear transcription factor-[kappa]B as a target for cancer drug development", LEUKEMIA, MACMILLAN PRESS LTD, US, vol. 16, no. 6, 1 January 2002 (2002-01-01), pages 1053-1068, XP002510847, ISSN: 0887-6924, DOI: 10.1038/SJ.LEU.2402482
- SU FENG ET AL: "Apolipoprotein A-I (apoA-I) and apoA-I mimetic peptides inhibit tumor development in a mouse model of ovarian cancer.", PROCEEDINGS OF THE NATIONAL ACADEMY OF SCIENCES OF THE UNITED STATES OF AMERICA 16 NOV 2010, vol. 107, no. 46, 16 November 2010 (2010-11-16), pages 19997-20002, ISSN: 1091-6490

### SEQUENCE LISTING

<110> THE REGENTS OF THE UNIVERSITY OF CALIFORNIA ET AL.
<120> PEPTIDES AND PEPTIDE MIMETICS TO TREAT PATHOLOGIES CHARACTERIZED BY AN INFLAMMATORY RESPONSE
<130> MAR/P1414EP00
<140> EP 06750791
   <141> 2006-04-18
<150> US 60/697,495
   <151> 2005-07-07
<150> US 60/676,431
   <151> 2005-04-29
<160> 1146
<170> PatentIn version 3.3
<210> 1
   <211> 18
   <212> PRT
   <213> Artificial
<220>
   <223> Synthetic peptide optionally bearing protecting groups.
<400> 1
   Asp Trp Leu Lys Ala Phe Tyr Asp Lys Val Ala Glu Lys Leu Lys Glu
   1 5 10 15
   Ala Phe
<210> 2
   <211> 18
   <212> PRT
   <213> Artificial
<220>
   <223> Synthetic peptide optionally bearing protecting groups.
<400> 2
   Asp Trp Leu Lys Ala Phe Tyr Asp Lys Val Ala Glu Lys Leu Lys Glu
   1 5 10 15
   Ala Phe
<210> 3
   <211> 18
   <212> PRT
   <213> Artificial
<220>
   <223> Synthetic peptide optionally bearing protecting groups.
<400> 3
   Asp Trp Phe Lys Ala Phe Tyr Asp Lys Val Ala Glu Lys Leu Lys Glu
   1 5 10 15
   Ala Phe
<210> 4
   <211> 18
   <212> PRT
   <213> Artificial
<220>
   <223> Synthetic peptide optionally bearing protecting groups.
<400> 4
   Asp Trp Leu Lys Ala Phe Tyr Asp Lys Val Ala Glu Lys Phe Lys Glu
   1 5 10 15
   Ala Phe
<210> 5
   <211> 18
   <212> PRT
   <213> Artificial
<220>
   <223> Synthetic peptide optionally bearing protecting groups.
<400> 5
   Asp Trp Phe Lys Ala Phe Tyr Asp Lys Val Ala Glu Lys Phe Lys Glu
   1               5                   10                  15
   Ala Phe
<210> 6
   <211> 18
   <212> PRT
   <213> Artificial
<220>
   <223> Synthetic peptide optionally bearing protecting groups.
<400> 6
   Asp Trp Leu Lys Ala Phe Tyr Asp Lys Val Phe Glu Lys Phe Lys Glu
   1               5                   10                  15
   Phe Phe
<210> 7
   <211> 18
   <212> PRT
   <213> Artificial
<220>
   <223> Synthetic peptide optionally bearing protecting groups.
<400> 7
   Asp Trp Leu Lys Ala Phe Tyr Asp Lys Phe Phe Glu Lys Phe Lys Glu
   1               5                   10                  15
   Phe Phe
<210> 8
   <211> 18
   <212> PRT
   <213> Artificial
<220>
   <223> Synthetic peptide optionally bearing protecting groups.
<400> 8
   Asp Trp Phe Lys Ala Phe Tyr Asp Lys Phe Phe Glu Lys Phe Lys Glu
   1               5                   10                  15
   Phe Phe
<210> 9
   <211> 18
   <212> PRT
   <213> Artificial
<220>
   <223> Synthetic peptide optionally bearing protecting groups.
<400> 9
   Asp Trp Leu Lys Ala Phe Tyr Asp Lys Val Ala Glu Lys Leu Lys Glu
   1               5                   10                  15
   Phe Phe
<210> 10
   <211> 18
   <212> PRT
   <213> Artificial
<220>
   <223> Synthetic peptide optionally bearing protecting groups.
<400> 10
   Asp Trp Leu Lys Ala Phe Tyr Asp Lys Val Phe Glu Lys Phe Lys Glu
   1               5                   10                  15
   Ala Phe
<210> 11
   <211> 18
   <212> PRT
   <213> Artificial
<220>
   <223> Synthetic peptide optionally bearing protecting groups.
<400> 11
   Asp Trp Leu Lys Ala Phe Tyr Asp Lys Val Phe Glu Lys Leu Lys Glu
   1               5                   10                  15
   Phe Phe
<210> 12
   <211> 18
   <212> PRT
   <213> Artificial
<220>
   <223> Synthetic peptide optionally bearing protecting groups.
<400> 12
   Asp Trp Leu Lys Ala Phe Tyr Asp Lys Val Ala Glu Lys Phe Lys Glu
   1 5 10 15
   Phe Phe
<210> 13
   <211> 18
   <212> PRT
   <213> Artificial
<220>
   <223> Synthetic peptide optionally bearing protecting groups.
<400> 13
   Asp Trp Leu Lys Ala Phe Tyr Asp Lys Val Phe Glu Lys Phe Lys Glu
   1 5 10 15
   Phe Phe
<210> 14
   <211> 18
   <212> PRT
   <213> Artificial
<220>
   <223> Synthetic peptide optionally bearing protecting groups.
<400> 14
   Glu Trp Leu Lys Leu Phe Tyr Glu Lys Val Leu Glu Lys Phe Lys Glu
   1 5 10 15
   Ala Phe
<210> 15
   <211> 18
   <212> PRT
   <213> Artificial
<220>
   <223> Synthetic peptide optionally bearing protecting groups.
<400> 15
   Glu Trp Leu Lys Ala Phe Tyr Asp Lys Val Ala Glu Lys Phe Lys Glu
   1 5 10 15
   Ala Phe
<210> 16
   <211> 18
   <212> PRT
   <213> Artificial
<220>
   <223> Synthetic peptide optionally bearing protecting groups.
<400> 16
   Glu Trp Leu Lys Ala Phe Tyr Asp Lys Val Ala Glu Lys Leu Lys Glu
   1 5 10 15
   Phe Phe
<210> 17
   <211> 18
   <212> PRT
   <213> Artificial
<220>
   <223> Synthetic peptide optionally bearing protecting groups.
<400> 17
   Glu Trp Leu Lys Ala Phe Tyr Asp Lys Val Phe Glu Lys Phe Lys Glu
   1 5 10 15
   Ala Phe
<210> 18
   <211> 18
   <212> PRT
   <213> Artificial
<220>
   <223> Synthetic peptide optionally bearing protecting groups.
<400> 18
   Glu Trp Leu Lys Ala Phe Tyr Asp Lys Val Phe Glu Lys Leu Lys Glu
   1 5 10 15
   Phe Phe
<210> 19
   <211> 18
   <212> PRT
   <213> Artificial
<220>
   <223> Synthetic peptide optionally bearing protecting groups.
<400> 19
   Glu Trp Leu Lys Ala Phe Tyr Asp Lys Val Ala Glu Lys Phe Lys Glu
   1 5 10 15
   Phe Phe
<210> 20
   <211> 18
   <212> PRT
   <213> Artificial
<220>
   <223> Synthetic peptide optionally bearing protecting groups.
<400> 20
   Glu Trp Leu Lys Ala Phe Tyr Asp Lys Val Phe Glu Lys Phe Lys Glu
   1 5 10 15
   Phe Phe
<210> 21
   <211> 14
   <212> PRT
   <213> Artificial
<220>
   <223> Synthetic peptide optionally bearing protecting groups.
<400> 21
   Ala Phe Tyr Asp Lys Val Ala Glu Lys Leu Lys Glu Ala Phe
   1 5 10
<210> 22
   <211> 14
   <212> PRT
   <213> Artificial
<220>
   <223> Synthetic peptide optionally bearing protecting groups.
<400> 22
   Ala Phe Tyr Asp Lys Val Ala Glu Lys Phe Lys Glu Ala Phe
   1 5 10
<210> 23
   <211> 14
   <212> PRT
   <213> Artificial
<220>
   <223> Synthetic peptide optionally bearing protecting groups.
<400> 23
   Ala Phe Tyr Asp Lys Val Ala Glu Lys Phe Lys Glu Ala Phe
   1 5 10
<210> 24
   <211> 14
   <212> PRT
   <213> Artificial
<220>
   <223> Synthetic peptide optionally bearing protecting groups.
<400> 24
   Ala Phe Tyr Asp Lys Phe Phe Glu Lys Phe Lys Glu Phe Phe
   1 5 10
<210> 25
   <211> 14
   <212> PRT
   <213> Artificial
<220>
   <223> Synthetic peptide optionally bearing protecting groups.
<400> 25
   Ala Phe Tyr Asp Lys Phe Phe Glu Lys Phe Lys Glu Phe Phe
   1 5 10
<210> 26
   <211> 14
   <212> PRT
   <213> Artificial
<220>
   <223> Synthetic peptide optionally bearing protecting groups.
<400> 26
   Ala Phe Tyr Asp Lys Val Ala Glu Lys Phe Lys Glu Ala Phe
   1 5 10
<210> 27
   <211> 14
   <212> PRT
   <213> Artificial
<220>
   <223> Synthetic peptide optionally bearing protecting groups.
<400> 27
   Ala Phe Tyr Asp Lys Val Ala Glu Lys Leu Lys Glu Phe Phe
   1 5 10
<210> 28
   <211> 14
   <212> PRT
   <213> Artificial
<220>
   <223> Synthetic peptide optionally bearing protecting groups.
<400> 28
   Ala Phe Tyr Asp Lys Val Phe Glu Lys Phe Lys Glu Ala Phe
   1               5                   10
<210> 29
   <211> 14
   <212> PRT
   <213> Artificial
<220>
   <223> Synthetic peptide optionally bearing protecting groups.
<400> 29
   Ala Phe Tyr Asp Lys Val Phe Glu Lys Leu Lys Glu Phe Phe
   1               5                   10
<210> 30
   <211> 14
   <212> PRT
   <213> Artificial
<220>
   <223> Synthetic peptide optionally bearing protecting groups.
<400> 30
   Ala Phe Tyr Asp Lys Val Ala Glu Lys Phe Lys Glu Phe Phe
   1               5                   10
<210> 31
   <211> 14
   <212> PRT
   <213> Artificial
<220>
   <223> Synthetic peptide optionally bearing protecting groups.
<400> 31
   Lys Ala Phe Tyr Asp Lys Val Phe Glu Lys Phe Lys Glu Phe
   1               5                   10
<210> 32
   <211> 14
   <212> PRT
   <213> Artificial
<220>
   <223> Synthetic peptide optionally bearing protecting groups.
<400> 32
   Leu Phe Tyr Glu Lys Val Leu Glu Lys Phe Lys Glu Ala Phe
   1 5 10
<210> 33
   <211> 14
   <212> PRT
   <213> Artificial
<220>
   <223> Synthetic peptide optionally bearing protecting groups.
<400> 33
   Ala Phe Tyr Asp Lys Val Ala Glu Lys Phe Lys Glu Ala Phe
   1 5 10
<210> 34
   <211> 14
   <212> PRT
   <213> Artificial
<220>
   <223> Synthetic peptide optionally bearing protecting groups.
<400> 34
   Ala Phe Tyr Asp Lys Val Ala Glu Lys Leu Lys Glu Phe Phe
   1 5 10
<210> 35
   <211> 14
   <212> PRT
   <213> Artificial
<220>
   <223> Synthetic peptide optionally bearing protecting groups.
<400> 35
   Ala Phe Tyr Asp Lys Val Phe Glu Lys Phe Lys Glu Ala Phe
   1 5 10
<210> 36
   <211> 14
   <212> PRT
   <213> Artificial
<220>
   <223> Synthetic peptide optionally bearing protecting groups.
<400> 36
   Ala Phe Tyr Asp Lys Val Phe Glu Lys Leu Lys Glu Phe Phe
   1 5 10
<210> 37
   <211> 14
   <212> PRT
   <213> Artificial
<220>
   <223> Synthetic peptide optionally bearing protecting groups.
<400> 37
   Ala Phe Tyr Asp Lys Val Ala Glu Lys Phe Lys Glu Phe Phe
   1 5 10
<210> 38
   <211> 14
   <212> PRT
   <213> Artificial
<220>
   <223> Synthetic peptide optionally bearing protecting groups.
<400> 38
   Ala Phe Tyr Asp Lys Val Phe Glu Lys Phe Lys Glu Phe Phe
   1 5 10
<210> 39
   <211> 18
   <212> PRT
   <213> Artificial
<220>
   <223> Synthetic peptide optionally bearing protecting groups.
<400> 39
   Asp Trp Leu Lys Ala Leu Tyr Asp Lys Val Ala Glu Lys Leu Lys Glu
   1 5 10 15
   Ala Leu
<210> 40
   <211> 18
   <212> PRT
   <213> Artificial
<220>
   <223> Synthetic peptide optionally bearing protecting groups.
<400> 40
   Asp Trp Phe Lys Ala Phe Tyr Glu Lys Val Ala Glu Lys Leu Lys Glu
   1 5 10 15
   Phe Phe
<210> 41
   <211> 18
   <212> PRT
   <213> Artificial
<220>
   <223> Synthetic peptide optionally bearing protecting groups.
<400> 41
   Asp Trp Phe Lys Ala Phe Tyr Glu Lys Phe Phe Glu Lys Phe Lys Glu
   1 5 10 15
   Phe Phe
<210> 42
   <211> 18
   <212> PRT
   <213> Artificial
<220>
   <223> Synthetic peptide optionally bearing protecting groups.
<400> 42
   Glu Trp Leu Lys Ala Leu Tyr Glu Lys Val Ala Glu Lys Leu Lys Glu
   1 5 10 15
   Ala Leu
<210> 43
   <211> 18
   <212> PRT
   <213> Artificial
<220>
   <223> Synthetic peptide optionally bearing protecting groups.
<400> 43
   Glu Trp Leu Lys Ala Phe Tyr Glu Lys Val Ala Glu Lys Leu Lys Glu
   1 5 10 15
   Ala Phe
<210> 44
   <211> 18
   <212> PRT
   <213> Artificial
<220>
   <223> Synthetic peptide optionally bearing protecting groups.
<400> 44
   Glu Trp Phe Lys Ala Phe Tyr Glu Lys Val Ala Glu Lys Leu Lys Glu
   1               5                   10                  15
   Phe Phe
<210> 45
   <211> 18
   <212> PRT
   <213> Artificial
<220>
   <223> Synthetic peptide optionally bearing protecting groups.
<400> 45
   Glu Trp Leu Lys Ala Phe Tyr Glu Lys Val Phe Glu Lys Phe Lys Glu
   1               5                   10                  15
   Phe Phe
<210> 46
   <211> 18
   <212> PRT
   <213> Artificial
<220>
   <223> Synthetic peptide optionally bearing protecting groups.
<400> 46
   Glu Trp Leu Lys Ala Phe Tyr Glu Lys Phe Phe Glu Lys Phe Lys Glu
   1               5                   10                  15
   Phe Phe
<210> 47
   <211> 18
   <212> PRT
   <213> Artificial
<220>
   <223> Synthetic peptide optionally bearing protecting groups.
<400> 47
   Glu Trp Phe Lys Ala Phe Tyr Glu Lys Phe Phe Glu Lys Phe Lys Glu
   1               5                   10                  15
   Phe Phe
<210> 48
   <211> 18
   <212> PRT
   <213> Artificial
<220>
   <223> Synthetic peptide optionally bearing protecting groups.
<400> 48
   Asp Phe Leu Lys Ala Trp Tyr Asp Lys Val Ala Glu Lys Leu Lys Glu
   1 5 10 15
   Ala Trp
<210> 49
   <211> 18
   <212> PRT
   <213> Artificial
<220>
   <223> Synthetic peptide optionally bearing protecting groups.
<400> 49
   Glu Phe Leu Lys Ala Trp Tyr Glu Lys Val Ala Glu Lys Leu Lys Glu
   1 5 10 15
   Ala Trp
<210> 50
   <211> 18
   <212> PRT
   <213> Artificial
<220>
   <223> Synthetic peptide optionally bearing protecting groups.
<400> 50
   Asp Phe Trp Lys Ala Trp Tyr Asp Lys Val Ala Glu Lys Leu Lys Glu
   1 5 10 15
   Trp Trp
<210> 51
   <211> 18
   <212> PRT
   <213> Artificial
<220>
   <223> Synthetic peptide optionally bearing protecting groups.
<400> 51
   Glu Phe Trp Lys Ala Trp Tyr Glu Lys Val Ala Glu Lys Leu Lys Glu
   1 5 10 15
   Trp Trp
<210> 52
   <211> 18
   <212> PRT
   <213> Artificial
<220>
   <223> Synthetic peptide optionally bearing protecting groups.
<400> 52
   Asp Lys Leu Lys Ala Phe Tyr Asp Lys Val Phe Glu Trp Ala Lys Glu
   1 5 10 15
   Ala Phe
<210> 53
   <211> 18
   <212> PRT
   <213> Artificial
<220>
   <223> Synthetic peptide optionally bearing protecting groups.
<400> 53
   Asp Lys Trp Lys Ala Val Tyr Asp Lys Phe Ala Glu Ala Phe Lys Glu
   1 5 10 15
   Phe Leu
<210> 54
   <211> 18
   <212> PRT
   <213> Artificial
<220>
   <223> Synthetic peptide optionally bearing protecting groups.
<400> 54
   Glu Lys Leu Lys Ala Phe Tyr Glu Lys Val Phe Glu Trp Ala Lys Glu
   1 5 10 15
   Ala Phe
<210> 55
   <211> 18
   <212> PRT
   <213> Artificial
<220>
   <223> Synthetic peptide optionally bearing protecting groups.
<400> 55
   Glu Lys Trp Lys Ala Val Tyr Glu Lys Phe Ala Glu Ala Phe Lys Glu
   1               5                   10                  15
   Phe Leu
<210> 56
   <211> 18
   <212> PRT
   <213> Artificial
<220>
   <223> Synthetic peptide optionally bearing protecting groups.
<400> 56
   Asp Trp Leu Lys Ala Phe Val Asp Lys Phe Ala Glu Lys Phe Lys Glu
   1               5                   10                  15
   Ala Tyr
<210> 57
   <211> 18
   <212> PRT
   <213> Artificial
<220>
   <223> Synthetic peptide optionally bearing protecting groups.
<400> 57
   Glu Lys Trp Lys Ala Val Tyr Glu Lys Phe Ala Glu Ala Phe Lys Glu
   1               5                   10                  15
   Phe Leu
<210> 58
   <211> 18
   <212> PRT
   <213> Artificial
<220>
   <223> Synthetic peptide optionally bearing protecting groups.
<400> 58
   Asp Trp Leu Lys Ala Phe Val Tyr Asp Lys Val Phe Lys Leu Lys Glu
   1 5 10 15
   Phe Phe
<210> 59
   <211> 18
   <212> PRT
   <213> Artificial
<220>
   <223> Synthetic peptide optionally bearing protecting groups.
<400> 59
   Glu Trp Leu Lys Ala Phe Val Tyr Glu Lys Val Phe Lys Leu Lys Glu
   1 5 10 15
   Phe Phe
<210> 60
   <211> 18
   <212> PRT
   <213> Artificial
<220>
   <223> Synthetic peptide optionally bearing protecting groups.
<400> 60
   Asp Trp Leu Arg Ala Phe Tyr Asp Lys Val Ala Glu Lys Leu Lys Glu
   1 5 10 15
   Ala Phe
<210> 61
   <211> 18
   <212> PRT
   <213> Artificial
<220>
   <223> Synthetic peptide optionally bearing protecting groups.
<400> 61
   Glu Trp Leu Arg Ala Phe Tyr Glu Lys Val Ala Glu Lys Leu Lys Glu
   1 5 10 15
   Ala Phe
<210> 62
   <211> 18
   <212> PRT
   <213> Artificial
<220>
   <223> Synthetic peptide optionally bearing protecting groups.
<400> 62
   Asp Trp Leu Lys Ala Phe Tyr Asp Arg Val Ala Glu Lys Leu Lys Glu
   1 5 10 15
   Ala Phe
<210> 63
   <211> 18
   <212> PRT
   <213> Artificial
<220>
   <223> Synthetic peptide optionally bearing protecting groups.
<400> 63
   Glu Trp Leu Lys Ala Phe Tyr Glu Arg Val Ala Glu Lys Leu Lys Glu
   1 5 10 15
   Ala Phe
<210> 64
   <211> 18
   <212> PRT
   <213> Artificial
<220>
   <223> Synthetic peptide optionally bearing protecting groups.
<400> 64
   Asp Trp Leu Lys Ala Phe Tyr Asp Lys Val Ala Glu Arg Leu Lys Glu
   1 5 10 15
   Ala Phe
<210> 65
   <211> 18
   <212> PRT
   <213> Artificial
<220>
   <223> Synthetic peptide optionally bearing protecting groups.
<400> 65
   Glu Trp Leu Lys Ala Phe Tyr Glu Lys Val Ala Glu Arg Leu Lys Glu
   1 5 10 15
   Ala Phe
<210> 66
   <211> 18
   <212> PRT
   <213> Artificial
<220>
   <223> Synthetic peptide optionally bearing protecting groups.
<400> 66
   Asp Trp Leu Lys Ala Phe Tyr Asp Lys Val Ala Glu Lys Leu Arg Glu
   1               5                   10                  15
   Ala Phe
<210> 67
   <211> 18
   <212> PRT
   <213> Artificial
<220>
   <223> Synthetic peptide optionally bearing protecting groups.
<400> 67
   Glu Trp Leu Lys Ala Phe Tyr Glu Lys Val Ala Glu Lys Leu Arg Glu
   1               5                   10                  15
   Ala Phe
<210> 68
   <211> 18
   <212> PRT
   <213> Artificial
<220>
   <223> Synthetic peptide optionally bearing protecting groups.
<400> 68
   Asp Trp Leu Lys Ala Phe Tyr Asp Arg Val Ala Glu Arg Leu Lys Glu
   1               5                   10                  15
   Ala Phe
<210> 69
   <211> 18
   <212> PRT
   <213> Artificial
<220>
   <223> Synthetic peptide optionally bearing protecting groups.
<400> 69
   Glu Trp Leu Lys Ala Phe Tyr Glu Arg Val Ala Glu Arg Leu Lys Glu
   1 5 10 15
   Ala Phe
<210> 70
   <211> 18
   <212> PRT
   <213> Artificial
<220>
   <223> Synthetic peptide optionally bearing protecting groups.
<400> 70
   Asp Trp Leu Arg Ala Phe Tyr Asp Lys Val Ala Glu Lys Leu Arg Glu
   1 5 10 15
   Ala Phe
<210> 71
   <211> 18
   <212> PRT
   <213> Artificial
<220>
   <223> Synthetic peptide optionally bearing protecting groups.
<400> 71
   Glu Trp Leu Arg Ala Phe Tyr Glu Lys Val Ala Glu Lys Leu Arg Glu
   1 5 10 15
   Ala Phe
<210> 72
   <211> 18
   <212> PRT
   <213> Artificial
<220>
   <223> Synthetic peptide optionally bearing protecting groups.
<400> 72
   Asp Trp Leu Arg Ala Phe Tyr Asp Arg Val Ala Glu Lys Leu Lys Glu
   1 5 10 15
   Ala Phe
<210> 73
   <211> 18
   <212> PRT
   <213> Artificial
<220>
   <223> Synthetic peptide optionally bearing protecting groups.
<400> 73
   Glu Trp Leu Arg Ala Phe Tyr Glu Arg Val Ala Glu Lys Leu Lys Glu
   1 5 10 15
   Ala Phe
<210> 74
   <211> 18
   <212> PRT
   <213> Artificial
<220>
   <223> Synthetic peptide optionally bearing protecting groups.
<400> 74
   Asp Trp Leu Lys Ala Phe Tyr Asp Lys Val Ala Glu Arg Leu Arg Glu
   1 5 10 15
   Ala Phe
<210> 75
   <211> 18
   <212> PRT
   <213> Artificial
<220>
   <223> Synthetic peptide optionally bearing protecting groups.
<400> 75
   Glu Trp Leu Lys Ala Phe Tyr Glu Lys Val Ala Glu Arg Leu Arg Glu
   1 5 10 15
   Ala Phe
<210> 76
   <211> 18
   <212> PRT
   <213> Artificial
<220>
   <223> Synthetic peptide optionally bearing protecting groups.
<400> 76
   Asp Trp Leu Arg Ala Phe Tyr Asp Lys Val Ala Glu Arg Leu Lys Glu
   1 5 10 15
   Ala Phe
<210> 77
   <211> 18
   <212> PRT
   <213> Artificial
<220>.
   <223> Synthetic peptide optionally bearing protecting groups.
<400> 77
   Glu Trp Leu Arg Ala Phe Tyr Glu Lys Val Ala Glu Arg Leu Lys Glu
   1 5 10 15
   Ala Phe
<210> 78
   <211> 37
   <212> PRT
   <213> Artificial
<220>
   <223> Synthetic peptide optionally bearing protecting groups.
<400> 78
   Asp Trp Leu Lys Ala Phe Tyr Asp Lys Val Ala Glu Lys Leu Lys Glu
   1 5 10 15
   Ala Phe Pro Asp Trp Leu Lys Ala Phe Tyr Asp Lys Val Ala Glu Lys
   20 25 30
   Leu Lys Glu Ala Phe
   35
<210> 79
   <211> 37
   <212> PRT
   <213> Artificial
<220>
   <223> Synthetic peptide optionally bearing protecting groups.
<400> 79
   Asp Trp Leu Lys Ala Phe Tyr Asp Lys Val Ala Glu Lys Leu Lys Glu
   1               5                   10                  15
   Phe Phe Pro Asp Trp Leu Lys Ala Phe Tyr Asp Lys Val Ala Glu Lys
               20                  25                  30
   Leu Lys Glu Phe Phe
           35

<210> 80
   <211> 37
   <212> PRT
   <213> Artificial
<220>
   <223> Synthetic peptide optionally bearing protecting groups.
<400> 80
   Asp Trp Phe Lys Ala Phe Tyr Asp Lys Val Ala Glu Lys Leu Lys Glu
   1               5                   10                  15
   Ala Phe Pro Asp Trp Phe Lys Ala Phe Tyr Asp Lys Val Ala Glu Lys
               20                  25                  30
   Leu Lys Glu Ala Phe
           35
<210> 81
   <211> 37
   <212> PRT
   <213> Artificial
<220>
   <223> Synthetic peptide optionally bearing protecting groups.
<400> 81
   Asp Lys Leu Lys Ala Phe Tyr Asp Lys Val Phe Glu Trp Ala Lys Glu
   1               5                   10                  15
   Ala Phe Pro Asp Lys Leu Lys Ala Phe Tyr Asp Lys Val Phe Glu Trp
               20                  25                  30
   Leu Lys Glu Ala Phe
           35
<210> 82
   <211> 37
   <212> PRT
   <213> Artificial
<220>
   <223> Synthetic peptide optionally bearing protecting groups.
<400> 82
   Asp Lys Trp Lys Ala Val Tyr Asp Lys Phe Ala Glu Ala Phe Lys Glu
   1 5 10 15
   Phe Leu Pro Asp Lys Trp Lys Ala Val Tyr Asp Lys Phe Ala Glu Ala
   20 25 30
   Phe Lys Glu Phe Leu
   35
<210> 83
   <211> 37
   <212> PRT
   <213> Artificial
<220>
   <223> Synthetic peptide optionally bearing protecting groups.
<400> 83
   Asp Trp Phe Lys Ala Phe Tyr Asp Lys Val Ala Glu Lys Phe Lys Glu
   1 5 10 15
   Ala Phe Pro Asp Trp Phe Lys Ala Phe Tyr Asp Lys Val Ala Glu Lys
   20 25 30
   Phe Lys Glu Ala Phe
   35
<210> 84
   <211> 37
   <212> PRT
   <213> Artificial
<220>
   <223> Synthetic peptide optionally bearing protecting groups.
<400> 84
   Asp Trp Leu Lys Ala Phe Val Tyr Asp Lys Val Phe Lys Leu Lys Glu
   1 5 10 15
   Phe Phe Pro Asp Trp Leu Lys Ala Phe Val Tyr Asp Lys Val Phe Lys
   20 25 30
   Leu Lys Glu Phe Phe
   35
<210> 85
   <211> 37
   <212> PRT
   <213> Artificial
<220>
   <223> Synthetic peptide optionally bearing protecting groups.
<400> 85
   Asp Trp Leu Lys Ala Phe Tyr Asp Lys Phe Ala Glu Lys Phe Lys Glu
   1               5                   10                  15
   Phe Phe Pro Asp Trp Leu Lys Ala Phe Tyr Asp Lys Phe Ala Glu Lys
               20                  25                  30
   Phe Lys Glu Phe Phe
           35
<210> 86
   <211> 18
   <212> PRT
   <213> Artificial
<220>
   <223> Synthetic peptide optionally bearing protecting groups.
<400> 86
   Glu Trp Phe Lys Ala Phe Tyr Glu Lys Val Ala Glu Lys Phe Lys Glu
   1               5                   10                  15
   Ala Phe
<210> 87
   <211> 14
   <212> PRT
   <213> Artificial
<220>
   <223> synthetic peptide optionally bearing protecting groups.
<400> 87
   Asp Trp Phe Lys Ala Phe Tyr Asp Lys Val Ala Glu Lys Phe
   1               5                   10
<210> 88
   <211> 14
   <212> PRT
   <213> Artificial
<220>
   <223> Synthetic peptide optionally bearing protecting groups.
<400> 88 - -
<210> 89
   <211> 14
   <212> PRT
   <213> Artificial
<220>
   <223> Synthetic peptide optionally bearing protecting groups.
<400> 89
   Phe Lys Ala Phe Tyr Glu Lys Val Ala Glu Lys Phe Lys Glu
   1               5                   10
<210> 90
   <211> 17
   <212> PRT
   <213> Artificial
<220>
   <223> Synthetic peptide optionally bearing protecting groups.
<400> 90
   Asn Met Ala Phe Lys Ala Phe Tyr Asp Lys Val Ala Glu Lys Phe Lys
   1               5                   10                  15
   Glu
<210> 91
   <211> 17
   <212> PRT
   <213> Artificial
<220>
   <223> Synthetic peptide optionally bearing protecting groups.
<400> 91
   Asn Met Ala Phe Lys Ala Phe Tyr Glu Lys Val Ala Glu Lys Phe Lys
   1               5                   10                  15
   Glu
<210> 92
   <211> 21
   <212> PRT
   <213> Artificial
<220>
   <223> Synthetic peptide optionally bearing protecting groups.
<400> 92
   Asn Met Ala Asp Trp Phe Lys Ala Phe Tyr Asp Lys Val Ala Glu Lys
   1 5 10 15
   Phe Lys Glu Ala Phe
   20
<210> 93
   <211> 21
   <212> PRT
   <213> Artificial
<220>
   <223> Synthetic peptide optionally bearing protecting groups.
<400> 93
   Asn Met Ala Glu Trp Phe Lys Ala Phe Tyr Glu Lys Val Ala Glu Lys
   1 5 10 15
   Phe Lys Glu Ala Phe
   20
<210> 94
   <211> 17
   <212> PRT
   <213> Artificial
<220>
   <223> Synthetic peptide optionally bearing protecting groups.
<400> 94
   Asn Met Ala Ala Phe Tyr Asp Lys Val Ala Glu Lys Phe Lys Glu Ala
   1 5 10 15
   Phe
<210> 95
   <211> 17
   <212> PRT
   <213> Artificial
<220>
   <223> Synthetic peptide optionally bearing protecting groups.
<400> 95
   Asn Met Ala Asp Trp Phe Lys Ala Phe Tyr Asp Lys Val Ala Glu Lys
   1 5 10 15
   Phe
<210> 96
   <211> 18
   <212> PRT
   <213> Artificial
<220>
   <223> Synthetic peptide optionally bearing protecting groups.
<400> 96
   Asp Trp Leu Lys Ala Phe Tyr Asp Lys Val Phe Glu Lys Phe Lys Glu
   1               5                   10                  15
   Phe Phe
<210> 97
   <211> 18
   <212> PRT
   <213> Artificial
<220>
   <223> Synthetic peptide optionally bearing protecting groups.
<400> 97
   Glu Trp Leu Lys Ala Phe Tyr Glu Lys Val Phe Glu Lys Phe Lys Glu
   1               5                   10                  15
   Phe Phe
<210> 98
   <211> 14
   <212> PRT
   <213> Artificial
<220>
   <223> Synthetic peptide optionally bearing protecting groups.
<400> 98
   Ala Phe Tyr Asp Lys Val Phe Glu Lys Phe Lys Glu Phe Phe
   1               5                   10
<210> 99
   <211> 14
   <212> PRT
   <213> Artificial
<220>
   <223> Synthetic peptide optionally bearing protecting groups.
<400> 99
   Ala Phe Tyr Glu Lys Val Phe Glu Lys Phe Lys Glu Phe Phe
   1               5                   10
<210> 100
   <211> 14
   <212> PRT
   <213> Artificial
<220>
   <223> Synthetic peptide optionally bearing protecting groups.
<400> 100
   Asp Trp Leu Lys Ala Phe Tyr Asp Lys Val Phe Glu Lys Phe
   1               5                   10
<210> 101
   <211> 14
   <212> PRT
   <213> Artificial
<220>
   <223> Synthetic peptide optionally bearing protecting groups.
<400> 101
   Glu Trp Leu Lys Ala Phe Tyr Glu Lys Val Phe Glu Lys Phe
   1               5                   10
<210> 102
   <211> 14
   <212> PRT
   <213> Artificial
<220>
   <223> Synthetic peptide optionally bearing protecting groups.
<400> 102
   Leu Lys Ala Phe Tyr Asp Lys Val Phe Glu Lys Phe Lys Glu
   1               5                   10
<210> 103
   <211> 14
   <212> PRT
   <213> Artificial
<220>
   <223> Synthetic peptide optionally bearing protecting groups.
<400> 103
   Leu Lys Ala Phe Tyr Glu Lys Val Phe Glu Lys Phe Lys Glu
   1               5                   10
<210> 104
   <211> 18
   <212> PRT
   <213> Artificial
<220>
   <223> Synthetic peptide optionally bearing protecting groups.
<400> 104
   Phe Ala Glu Lys Phe Lys Glu Ala Val Lys Asp Tyr Phe Ala Lys Phe
   1 5 10 15
   Trp Asp
<210> 105
   <211> 18
   <212> PRT
   <213> Artificial
<220>
   <223> Synthetic peptide optionally bearing protecting groups.
<400> 105
   Asp Lys Trp Lys Ala Val Tyr Asp Lys Phe Ala Glu Ala Phe Lys Glu
   1 5 10 15
   Phe Leu
<210> 106
   <211> 18
   <212> PRT
   <213> Artificial
<220>
   <223> Synthetic peptide optionally bearing protecting groups.
<400> 106
   Asp Lys Leu Lys Ala Phe Tyr Asp Lys Val Phe Glu Trp Ala Lys Glu
   1 5 10 15
   Ala Phe
<210> 107
   <211> 18
   <212> PRT
   <213> Artificial
<220>
   <223> Synthetic peptide optionally bearing protecting groups.
<400> 107
   Glu Trp Phe Lys Ala Phe Tyr Glu Lys Val Ala Asp Lys Phe Lys Asp
   1 5 10 15
   Ala Phe
<210> 108
   <211> 18
   <212>- PRT
   <213> Artificial
<220>
   <223> Synthetic peptide optionally bearing protecting groups.
<400> 108
   Glu Trp Phe Lys Ala Phe Tyr Asp Lys Val Ala Asp Lys Phe Lys Glu
   1               5                   10                  15
   Ala Phe
<210> 109
   <211> 18
   <212> PRT
   <213> Artificial
<220>
   <223> Synthetic peptide optionally bearing protecting groups.
<400> 109
   Asp Trp Phe Lys Ala Phe Tyr Glu Lys Val Ala Asp Lys Phe Lys Glu
   1               5                   10                  15
   Ala Phe
<210> 110
   <211> 18
   <212> PRT
   <213> Artificial
<220>
   <223> Synthetic peptide optionally bearing protecting groups.
<400> 110
   Asp Trp Phe Lys Ala Phe Tyr Glu Lys Val Ala Glu Lys Phe Lys Asp
   1               5                   10                  15
   Ala Phe
<210> 111
   <211> 18
   <212> PRT
   <213> Artificial
<220>
   <223> Synthetic peptide optionally bearing protecting groups.
<400> 111
   Asp Phe Trp Lys Ala Phe Tyr Asp Lys Val Ala Glu Lys Phe Lys Glu
   1 5 10 15
   Ala Phe
<210> 112
   <211> 18
   <212> PRT
   <213> Artificial
<220>
   <223> Synthetic peptide optionally bearing protecting groups.
<400> 112
   Glu Phe Trp Lys Ala Phe Tyr Glu Lys Val Ala Asp Lys Phe Lys Asp
   1 5 10 15
   Ala Phe
<210> 113
   <211> 18
   <212> PRT
   <213> Artificial
<220>
   <223> Synthetic peptide optionally bearing protecting groups.
<400> 113
   Glu Phe Trp Lys Ala Phe Tyr Asp Lys Val Ala Asp Lys Phe Lys Glu
   1 5 10 15
   Ala Phe
<210> 114
   <211> 18
   <212> PRT
   <213> Artificial
<220>
   <223> Synthetic peptide optionally bearing protecting groups.
<400> 114
   Asp Phe Trp Lys Ala Phe Tyr Glu Lys Val Ala Asp Lys Phe Lys Glu
   1 5 10 15
   Ala Phe
<210> 115
   <211> 18
   <212> PRT
   <213> Artificial
<220>
   <223> Synthetic peptide optionally bearing protecting groups.
<400> 115
   Asp Phe Trp Lys Ala Phe Tyr Glu Lys Val Ala Glu Lys Phe Lys Asp
   1 5 10 15
   Ala Phe
<210> 116
   <211> 18
   <212> PRT
   <213> Artificial
<220>
   <223> Synthetic peptide optionally bearing protecting groups.
<400> 116
   Asp Trp Phe Lys Ala Tyr Phe Asp Lys Val Ala Glu Lys Phe Lys Glu
   1 5 10 15
   Ala Phe
<210> 117
   <211> 18
   <212> PRT
   <213> Artificial
<220>
   <223> Synthetic peptide optionally bearing protecting groups.
<400> 117
   Glu Trp Phe Lys Ala Tyr Phe Glu Lys Val Ala Asp Lys Phe Lys Asp
   1 5 10 15
   Ala Phe
<210> 118
   <211> 18
   <212> PRT
   <213> Artificial
<220>
   <223> Synthetic peptide optionally bearing protecting groups.
<400> 118
   Glu Trp Phe Lys Ala Tyr Phe Asp Lys Val Ala Asp Lys Phe Lys Glu
   1 5 10 15
   Ala Phe
<210> 119
   <211> 18
   <212> PRT
   <213> Artificial
<220>
   <223> Synthetic peptide optionally bearing protecting groups.
<400> 119
   Asp Trp Phe Lys Ala Tyr Phe Glu Lys Val Ala Asp Lys Phe Lys Glu
   1               5                   10                  15
   Ala Phe
<210> 120
   <211> 18
   <212> PRT
   <213> Artificial
<220>
   <223> Synthetic peptide optionally bearing protecting groups.
<400> 120
   Asp Trp Phe Lys Ala Tyr Phe Glu Lys Val Ala Glu Lys Phe Lys Asp
   1               5                   10                  15
   Ala Phe
<210> 121
   <211> 18
   <212> PRT
   <213> Artificial
<220>
   <223> Synthetic peptide optionally bearing protecting groups.
<400> 121
   Asp Trp Phe Lys Ala Phe Val Asp Lys Tyr Ala Glu Lys Phe Lys Glu
   1               5                   10                  15
   Ala Phe
<210> 122
   <211> 18
   <212> PRT
   <213> Artificial
<220>
   <223> Synthetic peptide optionally bearing protecting groups.
<400> 122
   Glu Trp Phe Lys Ala Phe Val Glu Lys Tyr Ala Asp Lys Phe Lys Asp
   1 5 10 15
   Ala Phe
<210> 123
   <211> 18
   <212> PRT
   <213> Artificial
<220>
   <223> Synthetic peptide optionally bearing protecting groups.
<400> 123
   Glu Trp Phe Lys Ala Phe Val Asp Lys Tyr Ala Asp Lys Phe Lys Glu
   1 5 10 15
   Ala Phe
<210> 124
   <211> 18
   <212> PRT
   <213> Artificial
<220>
   <223> Synthetic peptide optionally bearing protecting groups.
<400> 124
   Asp Trp Phe Lys Ala Phe Val Glu Lys Tyr Ala Asp Lys Phe Lys Glu
   1 5 10 15
   Ala Phe
<210> 125
   <211> 18
   <212> PRT
   <213> Artificial
<220>
   <223> Synthetic peptide optionally bearing protecting groups.
<400> 125
   Asp Trp Phe Lys Ala Phe Val Glu Lys Tyr Ala Glu Lys Phe Lys Asp
   1 5 10 15
   Ala Phe
<210> 126
   <211> 18
   <212> PRT
   <213> Artificial
<220>
   <223> Synthetic peptide optionally bearing protecting groups.
<400> 126
   Asp Trp Phe Lys Ala Phe Tyr Asp Lys Ala Val Glu Lys Phe Lys Glu
   1 5 10 15
   Ala Phe
<210> 127
   <211> 18
   <212> PRT
   <213> Artificial
<220>
   <223> Synthetic peptide optionally bearing protecting groups.
<400> 127
   Glu Trp Phe Lys Ala Phe Tyr Glu Lys Ala Val Asp Lys Phe Lys Asp
   1 5 10 15
   Ala Phe
<210> 128
   <211> 18
   <212> PRT
   <213> Artificial
<220>
   <223> Synthetic peptide optionally bearing protecting groups.
<400> 128
   Glu Trp Phe Lys Ala Phe Tyr Asp Lys Ala Val Asp Lys Phe Lys Glu
   1 5 10 15
   Ala Phe
<210> 129
   <211> 18
   <212> PRT
   <213> Artificial
<220>
   <223> Synthetic peptide optionally bearing protecting groups.
<400> 129
   Asp Trp Phe Lys Ala Phe Tyr Glu Lys Ala Val Asp Lys Phe Lys Glu
   1 5 10 15
   Ala Phe
<210> 130
   <211> 18
   <212> PRT
   <213> Artificial
<220>
   <223> Synthetic peptide optionally bearing protecting groups.
<400> 130
   Asp Trp Phe Lys Ala Phe Tyr Glu Lys Ala Val Glu Lys Phe Lys Asp
   1 5 10 15
   Ala Phe
<210> 131
   <211> 18
   <212> PRT
   <213> Artificial
<220>
   <223> Synthetic peptide optionally bearing protecting groups.
<400> 131
   Asp Trp Phe Lys Ala Phe Tyr Asp Lys Val Phe Glu Lys Ala Lys Glu
   1 5 10 15
   Ala Phe
<210> 132
   <211> 18
   <212> PRT
   <213> Artificial
<220>
   <223> Synthetic peptide optionally bearing protecting groups.
<400> 132
   Glu Trp Phe Lys Ala Phe Tyr Glu Lys Val Phe Asp Lys Ala Lys Asp
   1 5 10 15
   Ala Phe
<210> 133
   <211> 18
   <212> PRT
   <213> Artificial
<220>
   <223> Synthetic peptide optionally bearing protecting groups.
<400> 133
   Glu Trp Phe Lys Ala Phe Tyr Asp Lys Val Phe Asp Lys Ala Lys Glu
   1 5 10 15
   Ala Phe
<210> 134
   <211> 18
   <212> PRT
   <213> Artificial
<220>
   <223> Synthetic peptide optionally bearing protecting groups.
<400> 134
   Asp Trp Phe Lys Ala Phe Tyr Glu Lys Val Phe Asp Lys Ala Lys Glu
   1 5 10 15
   Ala Phe
<210> 135
   <211> 18
   <212> PRT
   <213> Artificial
<220>
   <223> Synthetic peptide optionally bearing protecting groups.
<400> 135
   Asp Trp Phe Lys Ala Phe Tyr Glu Lys Val Phe Glu Lys Ala Lys Asp
   1 5 10 15
   Ala Phe
<210> 136
   <211> 18
   <212> PRT
   <213> Artificial
<220>
   <223> Synthetic peptide optionally bearing protecting groups.
<400> 136
   Asp Trp Phe Lys Ala Phe Tyr Asp Lys Val Ala Glu Lys Ala Lys Glu
   1 5 10 15
   Phe Phe
<210> 137
   <211> 18
   <212> PRT
   <213> Artificial
<220>
   <223> Synthetic peptide optionally bearing protecting groups.
<400> 137
   Glu Trp Phe Lys Ala Phe Tyr Glu Lys Val Ala Asp Lys Ala Lys Asp
   1               5                   10                  15
   Phe Phe
<210> 138
   <211> 18
   <212> PRT
   <213> Artificial
<220>
   <223> Synthetic peptide optionally bearing protecting groups.
<400> 138
   Glu Trp Phe Lys Ala Phe Tyr Asp Lys Val Ala Asp Lys Ala Lys Glu
   1               5                   10                  15
   Phe Phe
<210> 139
   <211> 18
   <212> PRT
   <213> Artificial
<220>
   <223> Synthetic peptide optionally bearing protecting groups.
<400> 139
   Asp Trp Phe Lys Ala Phe Tyr Glu Lys Val Ala Asp Lys Ala Lys Glu
   1               5                   10                  15
   Phe Phe
<210> 140
   <211> 18
   <212> PRT
   <213> Artificial
<220>
   <223> Synthetic peptide optionally bearing protecting groups.
<400> 140
   Asp Trp Phe Lys Ala Phe Tyr Glu Lys Val Ala Glu Lys Ala Lys Asp
   1               5                   10                  15
   Phe Phe
<210> 141
   <211> 18
   <212> PRT
   <213> Artificial
<220>
   <223> Synthetic peptide optionally bearing protecting groups.
<400> 141
   Asp Trp Phe Lys Ala Phe Tyr Asp Lys Val Ala Glu Lys Phe Lys Glu
   1               5                   10                  15
   Phe Ala
<210> 142
   <211> 18
   <212> PRT
   <213> Artificial
<220>
   <223> Synthetic peptide optionally bearing protecting groups.
<400> 142
   Glu Trp Phe Lys Ala Phe Tyr Glu Lys Val Ala Asp Lys Phe Lys Asp
   1               5                   10                  15
   Phe Ala
<210> 143
   <211> 18
   <212> PRT
   <213> Artificial
<220>
   <223> Synthetic peptide optionally bearing protecting groups.
<400> 143
   Glu Trp Phe Lys Ala Phe Tyr Asp Lys Val Ala Asp Lys Phe Lys Glu
   1               5                   10                  15
   Phe Ala
<210> 144
   <211> 18
   <212> PRT
   <213> Artificial
<220>
   <223> Synthetic peptide optionally bearing protecting groups.
<400> 144
   Asp Trp Phe Lys Ala Phe Tyr Glu Lys Val Ala Asp Lys Phe Lys Glu
   1 5 10 15
   Phe Ala
<210> 145
   <211> 18
   <212> PRT
   <213> Artificial
<220>
   <223> Synthetic peptide optionally bearing protecting groups.
<400> 145
   Asp Trp Phe Lys Ala Phe Tyr Glu Lys Val Ala Glu Lys Phe Lys Asp
   1 5 10 15
   Phe Ala
<210> 146
   <211> 18
   <212> PRT
   <213> Artificial
<220>
   <223> Synthetic peptide optionally bearing protecting groups.
<400> 146
   Asp Ala Phe Lys Ala Phe Tyr Asp Lys Val Ala Glu Lys Phe Lys Glu
   1 5 10 15
   Trp Phe
<210> 147
   <211> 18
   <212> PRT
   <213> Artificial
<220>
   <223> Synthetic peptide optionally bearing protecting groups.
<400> 147
   Glu Ala Phe Lys Ala Phe Tyr Glu Lys Val Ala Asp Lys Phe Lys Asp
   1 5 10 15
   Trp Phe
<210> 148
   <211> 18
   <212> PRT
   <213> Artificial
<220>
   <223> Synthetic peptide optionally bearing protecting groups.
<400> 148
   Glu Ala Phe Lys Ala Phe Tyr Asp Lys Val Ala Asp Lys Phe Lys Glu
   1               5                   10                  15
   Trp Phe
<210> 149
   <211> 18
   <212> PRT
   <213> Artificial
<220>
   <223> Synthetic peptide optionally bearing protecting groups.
<400> 149
   Asp Ala Phe Lys Ala Phe Tyr Glu Lys Val Ala Asp Lys Phe Lys Glu
   1               5                   10                  15
   Trp Phe
<210> 150
   <211> 18
   <212> PRT
   <213> Artificial
<220>
   <223> Synthetic peptide optionally bearing protecting groups.
<400> 150
   Asp Ala Phe Lys Ala Phe Tyr Glu Lys Val Ala Glu Lys Phe Lys Asp
   1               5                   10                  15
   Trp Phe
<210> 151
   <211> 18
   <212> PRT
   <213> Artificial
<220>
   <223> Synthetic peptide optionally bearing protecting groups.
<400> 151
   Asp Ala Phe Lys Ala Phe Tyr Asp Lys Val Trp Glu Lys Phe Lys Glu
   1               5                   10                  15
   Ala Phe
<210> 152
   <211> 18
   <212> PRT
   <213> Artificial
<220>
   <223> Synthetic peptide optionally bearing protecting groups.
<400> 152
   Glu Ala Phe Lys Ala Phe Tyr Glu Lys Val Trp Asp Lys Phe Lys Asp
   1               5                   10                  15
   Ala Phe
<210> 153
   <211> 18
   <212> PRT
   <213> Artificial
<220>
   <223> Synthetic peptide optionally bearing protecting groups.
<400> 153
   Glu Ala Phe Lys Ala Phe Tyr Asp Lys Val Trp Asp Lys Phe Lys Glu
   1               5                   10                  15
   Ala Phe
<210> 154
   <211> 18
   <212> PRT
   <213> Artificial
<220>
   <223> Synthetic peptide optionally bearing protecting groups.
<400> 154
   Asp Ala Phe Lys Ala Phe Tyr Glu Lys Val Trp Asp Lys Phe Lys Glu
   1               5                   10                  15
   Ala Phe
<210> 155
   <211> 18
   <212> PRT
   <213> Artificial
<220>
   <223> Synthetic peptide optionally bearing protecting groups.
<400> 155
   Asp Ala Phe Lys Ala Phe Tyr Glu Lys Val Trp Glu Lys Phe Lys Asp
   1 5 10 15
   Ala Phe
<210> 156
   <211> 18
   <212> PRT
   <213> Artificial
<220>
   <223> Synthetic peptide optionally bearing protecting groups.
<400> 156
   Asp Tyr Phe Lys Ala Phe Trp Asp Lys Val Ala Glu Lys Phe Lys Glu
   1 5 10 15
   Ala Phe
<210> 157
   <211> 18
   <212> PRT
   <213> Artificial
<220>
   <223> Synthetic peptide optionally bearing protecting groups.

<400> 157
   Glu Tyr Phe Lys Ala Phe Trp Glu Lys Val Ala Asp Lys Phe Lys Asp
   1 5 10 15
   Ala Phe
<210> 158
   <211> 18
   <212> PRT
   <213> Artificial
<220>
   <223> Synthetic peptide optionally bearing protecting groups.
<400> 158
   Glu Tyr Phe Lys Ala Phe Trp Asp Lys Val Ala Asp Lys Phe Lys Glu
   1 5 10 15
   Ala Phe
<210> 159
   <211> 18
   <212> PRT
   <213> Artificial
<220>
   <223> Synthetic peptide optionally bearing protecting groups.
<400> 159
   Asp Tyr Phe Lys Ala Phe Trp Glu Lys Val Ala Asp Lys Phe Lys Glu
   1 5 10 15
   Ala Phe
<210> 160
   <211> 18
   <212> PRT
   <213> Artificial
<220>
   <223> Synthetic peptide optionally bearing protecting groups.
<400> 160
   Asp Tyr Phe Lys Ala Phe Trp Glu Lys Val Ala Glu Lys Phe Lys Asp
   1 5 10 15
   Ala Phe
<210> 161
   <211> 18
   <212> PRT
   <213> Artificial
<220>
   <223> Synthetic peptide optionally bearing protecting groups.
<400> 161
   Asp Trp Ala Lys Ala Phe Tyr Asp Lys Val Ala Glu Lys Phe Lys Glu
   1 5 10 15
   Phe Phe
<210> 162
   <211> 18
   <212> PRT
   <213> Artificial
<220>
   <223> Synthetic peptide optionally bearing protecting groups.
<400> 162
   Glu Trp Ala Lys Ala Phe Tyr Glu Lys Val Ala Asp Lys Phe Lys Asp
   1 5 10 15
   Phe Phe
<210> 163
   <211> 18
   <212> PRT
   <213> Artificial
<220>
   <223> Synthetic peptide optionally bearing protecting groups.
<400> 163
   Glu Trp Ala Lys Ala Phe Tyr Asp Lys Val Ala Asp Lys Phe Lys Glu
   1 5 10 15
   Phe Phe
<210> 164
   <211> 18
   <212> PRT
   <213> Artificial
<220>
   <223> Synthetic peptide optionally bearing protecting groups.
<400> 164
   Asp Trp Ala Lys Ala Phe Tyr Glu Lys Val Ala Asp Lys Phe Lys Glu
   1 5 10 15
   Phe Phe
<210> 165
   <211> 18
   <212> PRT
   <213> Artificial
<220>
   <223> Synthetic peptide optionally bearing protecting groups.
<400> 165
   Asp Trp Ala Lys Ala Phe Tyr Glu Lys Val Ala Glu Lys Phe Lys Asp
   1 5 10 15
   Phe Phe
<210> 166
   <211> 18
   <212> PRT
   <213> Artificial
<220>
   <223> Synthetic peptide optionally bearing protecting groups.
<400> 166
   Asp Trp Phe Lys Ala Ala Tyr Asp Lys Val Ala Glu Lys Phe Lys Glu
   1 5 10 15
   Phe Phe
<210> 167
   <211> 18
   <212> PRT
   <213> Artificial
<220>
   <223> Synthetic peptide optionally bearing protecting groups.
<400> 167
   Glu Trp Phe Lys Ala Ala Tyr Glu Lys Val Ala Asp Lys Phe Lys Asp
   1 5 10 15
   Phe Phe
<210> 168
   <211> 18
   <212> PRT
   <213> Artificial
<220>
   <223> Synthetic peptide optionally bearing protecting groups.
<400> 168
   Glu Trp Phe Lys Ala Ala Tyr Asp Lys Val Ala Asp Lys Phe Lys Glu
   1 5 10 15
   Phe Phe
<210> 169
   <211> 18
   <212> PRT
   <213> Artificial
<220>
   <223> Synthetic peptide optionally bearing protecting groups.
<400> 169
   Asp Trp Phe Lys Ala Ala Tyr Glu Lys Val Ala Asp Lys Phe Lys Glu
   1 5 10 15
   Phe Phe
<210> 170
   <211> 18
   <212> PRT
   <213> Artificial
<220>
   <223> Synthetic peptide optionally bearing protecting groups.
<400> 170
   Asp Trp Phe Lys Ala Ala Tyr Glu Lys Val Ala Glu Lys Phe Lys Asp
   1 5 10 15
   Phe Phe
<210> 171
   <211> 18
   <212> PRT
   <213> Artificial
<220>
   <223> Synthetic peptide optionally bearing protecting groups.
<400> 171
   Asp Trp Phe Lys Ala Phe Ala Asp Lys Val Ala Glu Lys Phe Lys Glu
   1 5 10 15
   Tyr Phe
<210> 172
   <211> 18
   <212> PRT
   <213> Artificial
<220>
   <223> Synthetic peptide optionally bearing protecting groups.
<400> 172
   Glu Trp Phe Lys Ala Phe Ala Glu Lys Val Ala Asp Lys Phe Lys Asp
   1 5 10 15
   Tyr Phe
<210> 173
   <211> 18
   <212> PRT
   <213> Artificial
<220>
   <223> Synthetic peptide optionally bearing protecting groups.
<400> 173
   Glu Trp Phe Lys Ala Phe Ala Asp Lys Val Ala Asp Lys Phe Lys Glu
   1 5 10 15
   Tyr Phe
<210> 174
   <211> 18
   <212> PRT
   <213> Artificial
<220>
   <223> Synthetic peptide optionally bearing protecting groups.
<400> 174
   Asp Trp Phe Lys Ala Phe Ala Glu Lys Val Ala Asp Lys Phe Lys Glu
   1               5                   10                  15
   Tyr Phe
<210> 175
   <211> 18
   <212> PRT
   <213> Artificial
<220>
   <223> Synthetic peptide optionally bearing protecting groups.
<400> 175
   Asp Trp Phe Lys Ala Phe Ala Glu Lys Val Ala Glu Lys Phe Lys Asp
   1               5                   10                  15
   Tyr Phe
<210> 176
   <211> 18
   <212> PRT
   <213> Artificial
<220>
   <223> Synthetic peptide optionally bearing protecting groups.
<400> 176
   Asp Trp Phe Lys Ala Phe Tyr Asp Lys Ala Ala Glu Lys Phe Lys Glu
   1               5                   10                  15
   Val Phe
<210> 177
   <211> 18
   <212> PRT
   <213> Artificial
<220>
   <223> Synthetic peptide optionally bearing protecting groups.
<400> 177
   Glu Trp Phe Lys Ala Phe Tyr Glu Lys Ala Ala Asp Lys Phe Lys Asp
   1 5 10 15
   Val Phe
<210> 178
   <211> 18
   <212> PRT
   <213> Artificial
<220>
   <223> Synthetic peptide optionally bearing protecting groups.
<400> 178
   Glu Trp Phe Lys Ala Phe Tyr Asp Lys Ala Ala Asp Lys Phe Lys Glu
   1 5 10 15
   Val Phe
<210> 179
   <211> 18
   <212> PRT
   <213> Artificial
<220>
   <223> synthetic peptide optionally bearing protecting groups.
<400> 179
   Asp Trp Phe Lys Ala Phe Tyr Glu Lys Ala Ala Asp Lys Phe Lys Glu
   1 5 10 15
   Val Phe
<210> 180
   <211> 18
   <212> PRT
   <213> Artificial
<220>
   <223> Synthetic peptide optionally bearing protecting groups.
<400> 180
   Asp Trp Phe Lys Ala Phe Tyr Glu Lys Ala Ala Glu Lys Phe Lys Asp
   1 5 10 15
   Val Phe
<210> 181
   <211> 18
   <212> PRT
   <213> Artificial
<220>
   <223> Synthetic peptide optionally bearing protecting groups.
<400> 181
   Asp Trp Tyr Lys Ala Phe Phe Asp Lys Val Ala Glu Lys Phe Lys Glu
   1 5 10 15
   Ala Phe
<210> 182
   <211> 18
   <212> PRT
   <213> Artificial
<220>
   <223> Synthetic peptide optionally bearing protecting groups.
<400> 182
   Glu Trp Tyr Lys Ala Phe Phe Glu Lys Val Ala Asp Lys Phe Lys Asp
   1 5 10 15
   Ala Phe
<210> 183
   <211> 18
   <212> PRT
   <213> Artificial
<220>
   <223> Synthetic peptide optionally bearing protecting groups.
<400> 183
   Glu Trp Tyr Lys Ala Phe Phe Asp Lys Val Ala Asp Lys Phe Lys Glu
   1 5 10 15
   Ala Phe
<210> 184
   <211> 18
   <212> PRT
   <213> Artificial
<220>
   <223> Synthetic peptide optionally bearing protecting groups.
<400> 184
   Asp Trp Tyr Lys Ala Phe Phe Glu Lys Val Ala Asp Lys Phe Lys Glu
   1 5 10 15
   Ala Phe
<210> 185
   <211> 18
   <212> PRT
   <213> Artificial
<220>
   <223> Synthetic peptide optionally bearing protecting groups.
<400> 185
   Asp Trp Tyr Lys Ala Phe Phe Glu Lys Val Ala Glu Lys Phe Lys Asp
   1               5                   10                  15
   Ala Phe
<210> 186
   <211> 18
   <212> PRT
   <213> Artificial
<220>
   <223> Synthetic peptide optionally bearing protecting groups.
<400> 186
   Asp Trp Val Lys Ala Phe Tyr Asp Lys Phe Ala Glu Lys Phe Lys Glu
   1               5                   10                  15
   Ala Phe
<210> 187
   <211> 18
   <212> PRT
   <213> Artificial
<220>
   <223> Synthetic peptide optionally bearing protecting groups.
<400> 187
   Glu Trp Val Lys Ala Phe Tyr Glu Lys Phe Ala Asp Lys Phe Lys Asp
   1               5                   10                  15
   Ala Phe
<210> 188
   <211> 18
   <212> PRT
   <213> Artificial
<220>
   <223> Synthetic peptide optionally bearing protecting groups.
<400> 188
   Glu Trp Val Lys Ala Phe Tyr Asp Lys Phe Ala Asp Lys Phe Lys Glu
   1 5 10 15
   Ala Phe
<210> 189
   <211> 18
   <212> PRT
   <213> Artificial
<220>
   <223> Synthetic peptide optionally bearing protecting groups.
<400> 189
   Asp Trp Val Lys Ala Phe Tyr Glu Lys Phe Ala Asp Lys Phe Lys Glu
   1 5 10 15
   Ala Phe
<210> 190
   <211> 18
   <212> PRT
   <213> Artificial
<220>
   <223> Synthetic peptide optionally bearing protecting groups.
<400> 190
   Asp Trp Val Lys Ala Phe Tyr Glu Lys Phe Ala Glu Lys Phe Lys Asp
   1 5 10 15
   Ala Phe
<210> 191
   <211> 18
   <212> PRT
   <213> Artificial
<220>
   <223> Synthetic peptide optionally bearing protecting groups.
<400> 191
   Asp Trp Phe Lys Ala Phe Phe Asp Lys Val Ala Glu Lys Tyr Lys Glu
   1 5 10 15
   Ala Phe
<210> 192
   <211> 18
   <212> PRT
   <213> Artificial
<220>
   <223> Synthetic peptide optionally bearing protecting groups.
<400> 192
   Glu Trp Phe Lys Ala Phe Phe Glu Lys Val Ala Asp Lys Tyr Lys Asp
   1 5 10 15
   Ala Phe
<210> 193
   <211> 18
   <212> PRT
   <213> Artificial
<220>
   <223> Synthetic peptide optionally bearing protecting groups.
<400> 193
   Glu Trp Phe Lys Ala Phe Phe Asp Lys Val Ala Asp Lys Tyr Lys Glu
   1 5 10 15
   Ala Phe
<210> 194
   <211> 18
   <212> PRT
   <213> Artificial
<220>
   <223> Synthetic peptide optionally bearing protecting groups.
<400> 194
   Asp Trp Phe Lys Ala Phe Phe Glu Lys Val Ala Asp Lys Tyr Lys Glu
   1 5 10 15
   Ala Phe
<210> 195
   <211> 18
   <212> PRT
   <213> Artificial
<220>
   <223> Synthetic peptide optionally bearing protecting groups.
<400> 195
   Asp Trp Phe Lys Ala Phe Phe Glu Lys Val Ala Asp Lys Tyr Lys Glu
   1 5 10 15
   Ala Phe
<210> 196
   <211> 18
   <212> PRT
   <213> Artificial
<220>
   <223> Synthetic peptide optionally bearing protecting groups.
<400> 196
   Asp Trp Phe Lys Ala Phe Phe Asp Lys Val Ala Glu Lys Phe Lys Glu
   1               5                   10                  15
   Ala Tyr
<210> 197
   <211> 18
   <212> PRT
   <213> Artificial
<220>
   <223> Synthetic peptide optionally bearing protecting groups.
<400> 197
   Glu Trp Phe Lys Ala Phe Phe Glu Lys Val Ala Asp Lys Phe Lys Asp
   1               5                   10                  15
   Ala Tyr
<210> 198
   <211> 18
   <212> PRT
   <213> Artificial
<220>
   <223> Synthetic peptide optionally bearing protecting groups.
<400> 198
   Glu Trp Phe Lys Ala Phe Phe Asp Lys Val Ala Asp Lys Phe Lys Glu
   1               5                   10                  15
   Ala Tyr
<210> 199
   <211> 18
   <212> PRT
   <213> Artificial
<220>
   <223> Synthetic peptide optionally bearing protecting groups.
<400> 199
   Asp Trp Phe Lys Ala Phe Phe Glu Lys Val Ala Asp Lys Phe Lys Glu
   1 5 10 15
   Ala Tyr
<210> 200
   <211> 18
   <212> PRT
   <213> Artificial
<220>
   <223> Synthetic peptide optionally bearing protecting groups.
<400> 200
   Asp Trp Phe Lys Ala Phe Phe Glu Lys Val Ala Glu Lys Phe Lys Asp
   1 5 10 15
   Ala Tyr
<210> 201
   <211> 18
   <212> PRT
   <213> Artificial
<220>
   <223> Synthetic peptide optionally bearing protecting groups.
<400> 201
   Asp Trp Phe Lys Ala Phe Tyr Asp Lys Phe Ala Glu Lys Phe Lys Glu
   1 5 10 15
   Ala Val
<210> 202
   <211> 18
   <212> PRT
   <213> Artificial
<220>
   <223> Synthetic peptide optionally bearing protecting groups.
<400> 202
   Glu Trp Phe Lys Ala Phe Tyr Glu Lys Phe Ala Asp Lys Phe Lys Asp
   1 5 10 15
   Ala Val
<210> 203
   <211> 18
   <212> PRT
   <213> Artificial
<220>
   <223> Synthetic peptide optionally bearing protecting groups.
<400> 203
   Glu Trp Phe Lys Ala Phe Tyr Asp Lys Phe Ala Asp Lys Phe Lys Glu
   1 5 10 15
   Ala Val
<210> 204
   <211> 18
   <212> PRT
   <213> Artificial
<220>
   <223> Synthetic peptide optionally bearing protecting groups.
<400> 204
   Asp Trp Phe Lys Ala Phe Tyr Glu Lys Phe Ala Asp Lys Phe Lys Glu
   1 5 10 15
   Ala Val
<210> 205
   <211> 18
   <212> PRT
   <213> Artificial
<220>
   <223> Synthetic peptide optionally bearing protecting groups.
<400> 205
   Asp Trp Phe Lys Ala Phe Tyr Glu Lys Phe Ala Glu Lys Phe Lys Asp
   1 5 10 15
   Ala Val
<210> 206
   <211> 18
   <212> PRT
   <213> Artificial
<220>
   <223> Synthetic peptide optionally bearing protecting groups.
<400> 206
   Asp Lys Phe Lys Ala Phe Tyr Asp Lys Val Ala Glu Lys Phe Trp Glu
   1 5 10 15
   Ala Phe
<210> 207
   <211> 18
   <212> PRT
   <213> Artificial
<220>
   <223> Synthetic peptide optionally bearing protecting groups.
<400> 207
   Glu Lys Phe Lys Ala Phe Tyr Glu Lys Val Ala Asp Lys Phe Trp Asp
   1 5 10 15
   Ala Phe
<210> 208
   <211> 18
   <212> PRT
   <213> Artificial
<220>
   <223> Synthetic peptide optionally bearing protecting groups.
<400> 208
   Glu Lys Phe Lys Ala Phe Tyr Asp Lys Val Ala Asp Lys Phe Trp Glu
   1 5 10 15
   Ala Phe
<210> 209
   <211> 18
   <212> PRT
   <213> Artificial
<220>
   <223> Synthetic peptide optionally bearing protecting groups.
<400> 209
   Asp Lys Phe Lys Ala Phe Tyr Glu Lys Val Ala Asp Lys Phe Trp Glu
   1 5 10 15
   Ala Phe
<210> 210
   <211> 18
   <212> PRT
   <213> Artificial
<220>
   <223> Synthetic peptide optionally bearing protecting groups.
<400> 210
   Asp Lys Phe Lys Ala Phe Tyr Glu Lys Val Ala Glu Lys Phe Trp Asp
   1 5 10 15
   Ala Phe
<210> 211
   <211> 18
   <212> PRT
   <213> Artificial
<220>
   <223> Synthetic peptide optionally bearing protecting groups.
<400> 211
   Asp Lys Trp Lys Ala Phe Tyr Asp Lys Val Ala Glu Lys Phe Phe Glu
   1 5 10 15
   Ala Phe
<210> 212
   <211> 18
   <212> PRT
   <213> Artificial
<220>
   <223> Synthetic peptide optionally bearing protecting groups.
<400> 212
   Glu Lys Trp Lys Ala Phe Tyr Glu Lys Val Ala Asp Lys Phe Phe Asp
   1 5 10 15
   Ala Phe
<210> 213
   <211> 18
   <212> PRT
   <213> Artificial
<220>
   <223> Synthetic peptide optionally bearing protecting groups.
<400> 213
   Glu Lys Trp Lys Ala Phe Tyr Asp Lys Val Ala Asp Lys Phe Phe Glu
   1 5 10 15
   Ala Phe
<210> 214
   <211> 18
   <212> PRT
   <213> Artificial
<220>
   <223> Synthetic peptide optionally bearing protecting groups.
<400> 214
   Asp Lys Trp Lys Ala Phe Tyr Glu Lys Val Ala Asp Lys Phe Phe Glu
   1 5 10 15
   Ala Phe
<210> 215
   <211> 18
   <212> PRT
   <213> Artificial
<220>
   <223> Synthetic peptide optionally bearing protecting groups.
<400> 215
   Asp Lys Trp Lys Ala Phe Tyr Glu Lys Val Ala Glu Lys Phe Phe Asp
   1 5 10 15
   Ala Phe
<210> 216
   <211> 18
   <212> PRT
   <213> Artificial
<220>
   <223> Synthetic peptide optionally bearing protecting groups.
<400> 216
   Asp Lys Phe Lys Ala Phe Tyr Asp Lys Trp Ala Glu Val Phe Lys Glu
   1 5 10 15
   Ala Phe
<210> 217
   <211> 18
   <212> PRT
   <213> Artificial
<220>
   <223> Synthetic peptide optionally bearing protecting groups.
<400> 217
   Glu Lys Phe Lys Ala Phe Tyr Glu Lys Trp Ala Asp Val Phe Lys Asp
   1 5 10 15
   Ala Phe
<210> 218
   <211> 18
   <212> PRT
   <213> Artificial
<220>
   <223> Synthetic peptide optionally bearing protecting groups.
<400> 218
   Glu Lys Phe Lys Ala Phe Tyr Asp Lys Trp Ala Asp Val Phe Lys Glu
   1               5                   10                  15
   Ala Phe
<210> 219
   <211> 18
   <212> PRT
   <213> Artificial
<220>
   <223> Synthetic peptide optionally bearing protecting groups.
<400> 219
   Asp Lys Phe Lys Ala Phe Tyr Glu Lys Trp Ala Asp Val Phe Lys Glu
   1               5                   10                  15
   Ala Phe
<210> 220
   <211> 18
   <212> PRT
   <213> Artificial
<220>
   <223> Synthetic peptide optionally bearing protecting groups.
<400> 220
   Asp Lys Phe Lys Ala Phe Tyr Glu Lys Trp Ala Glu Val Phe Lys Asp
   1               5                   10                  15
   Ala Phe
<210> 221
   <211> 18
   <212> PRT
   <213> Artificial
<220>
   <223> Synthetic peptide optionally bearing protecting groups.
<400> 221
   Asp Lys Phe Lys Ala Phe Tyr Asp Lys Val Ala Glu Phe Trp Lys Glu
   1 5 10 15
   Ala Phe
<210> 222
   <211> 18
   <212> PRT
   <213> Artificial
<220>
   <223> Synthetic peptide optionally bearing protecting groups.
<400> 222
   Glu Lys Phe Lys Ala Phe Tyr Glu Lys Val Ala Asp Phe Trp Lys Asp
   1 5 10 15
   Ala Phe
<210> 223
   <211> 18
   <212> PRT
   <213> Artificial
<220>
   <223> Synthetic peptide optionally bearing protecting groups.
<400> 223
   Glu Lys Phe Lys Ala Phe Tyr Asp Lys Val Ala Asp Phe Trp Lys Glu
   1 5 10 15
   Ala Phe
<210> 224
   <211> 18
   <212> PRT
   <213> Artificial
<220>
   <223> Synthetic peptide optionally bearing protecting groups.
<400> 224
   Asp Lys Phe Lys Ala Phe Tyr Glu Lys Val Ala Asp Phe Trp Lys Glu
   1 5 10 15
   Ala Phe
<210> 225
   <211> 18
   <212> PRT
   <213> Artificial
<220>
   <223> Synthetic peptide optionally bearing protecting groups.
<400> 225
   Asp Lys Phe Lys Ala Phe Tyr Glu Lys Val Ala Glu Phe Trp Lys Asp
   1 5 10 15
   Ala Phe
<210> 226
   <211> 18
   <212> PRT
   <213> Artificial
<220>
   <223> Synthetic peptide optionally bearing protecting groups.
<400> 226
   Phe Ala Glu Lys Phe Lys Glu Ala Val Lys Asp Tyr Phe Ala Lys Phe
   1 5 10 15
   Trp Asp
<210> 227
   <211> 18
   <212> PRT
   <213> Artificial
<220>
   <223> Synthetic peptide optionally bearing protecting groups.
<400> 227
   Phe Ala Asp Lys Phe Lys Asp Ala Val Lys Glu Tyr Phe Ala Lys Phe
   1 5 10 15
   Trp Glu
<210> 228
   <211> 18
   <212> PRT
   <213> Artificial
<220>
   <223> Synthetic peptide optionally bearing protecting groups.
<400> 228
   Phe Ala Asp Lys Phe Lys Glu Ala Val Lys Asp Tyr Phe Ala Lys Phe
   1 5 10 15
   Trp Glu
<210> 229
   <211> 18
   <212> PRT
   <213> Artificial
<220>
   <223> Synthetic peptide optionally bearing protecting groups.
<400> 229
   Phe Ala Glu Lys Phe Lys Asp Ala Val Lys Glu Tyr Phe Ala Lys Phe
   1               5                   10                  15
   Trp Asp
<210> 230
   <211> 18
   <212> PRT
   <213> Artificial
<220>
   <223> Synthetic peptide optionally bearing protecting groups.
<400> 230
   Phe Ala Glu Lys Phe Lys Asp Ala Val Lys Asp Tyr Phe Ala Lys Phe
   1               5                   10                  15
   Trp Glu
<210> 231
   <211> 18
   <212> PRT
   <213> Artificial
<220>
   <223> Synthetic peptide optionally bearing protecting groups.
<400> 231
   Phe Trp Glu Lys Phe Lys Glu Ala Val Lys Asp Tyr Phe Ala Lys Phe
   1               5                   10                  15
   Ala Asp
<210> 232
   <211> 18
   <212> PRT
   <213> Artificial
<220>
   <223> Synthetic peptide optionally bearing protecting groups.
<400> 232
   Phe Trp Asp Lys Phe Lys Asp Ala Val Lys Glu Tyr Phe Ala Lys Phe
   1 5 10 15
   Ala Glu
<210> 233
   <211> 18
   <212> PRT
   <213> Artificial
<220>
   <223> Synthetic peptide optionally bearing protecting groups.
<400> 233
   Phe Ala Asp Lys Phe Lys Glu Ala Val Lys Asp Tyr Phe Ala Lys Phe
   1 5 10 15
   Trp Glu

<210> 234
   <211> 18
   <212> PRT
   <213> Artificial
<220>
   <223> Synthetic peptide optionally bearing protecting groups.
<400> 234
   Phe Ala Glu Lys Phe Lys Asp Ala Val Lys Glu Tyr Phe Ala Lys Phe
   1 5 10 15
   Trp Asp
<210> 235
   <211> 18
   <212> PRT
   <213> Artificial
<220>
   <223> Synthetic peptide optionally bearing protecting groups.
<400> 235
   Phe Ala Glu Lys Phe Lys Asp Ala Val Lys Asp Tyr Phe Ala Lys Phe
   1 5 10 15
   Trp Glu
<210> 236
   <211> 18
   <212> PRT
   <213> Artificial
<220>
   <223> Synthetic peptide optionally bearing protecting groups.
<400> 236
   Phe Phe Glu Lys Phe Lys Glu Ala Val Lys Asp Tyr Phe Ala Lys Ala
   1               5                   10                  15
   Trp Asp
<210> 237
   <211> 18
   <212> PRT
   <213> Artificial
<220>
   <223> Synthetic peptide optionally bearing protecting groups.
<400> 237
   Phe Phe Asp Lys Phe Lys Asp Ala Val Lys Glu Tyr Phe Ala Lys Ala
   1               5                   10                  15
   Trp Glu
<210> 238
   <211> 18
   <212> PRT
   <213> Artificial
<220>
   <223> Synthetic peptide optionally bearing protecting groups.
<400> 238
   Phe Phe Asp Lys Phe Lys Glu Ala Val Lys Asp Tyr Phe Ala Lys Ala
   1               5                   10                  15
   Trp Glu
<210> 239
   <211> 18
   <212> PRT
   <213> Artificial
<220>
   <223> Synthetic peptide optionally bearing protecting groups.
<400> 239
   Phe Phe Glu Lys Phe Lys Asp Ala Val Lys Glu Tyr Phe Ala Lys Ala
   1               5                   10                  15
   Trp Asp
<210> 240
   <211> 18
   <212> PRT
   <213> Artificial
<220>
   <223> Synthetic peptide optionally bearing protecting groups.
<400> 240
   Phe Phe Glu Lys Phe Lys Asp Ala Val Lys Asp Tyr Phe Ala Lys Ala
   1               5                   10                  15
   Trp Glu
<210> 241
   <211> 18
   <212> PRT
   <213> Artificial
<220>
   <223> Synthetic peptide optionally bearing protecting groups.
<400> 241
   Phe Ala Glu Lys Ala Lys Glu Phe Val Lys Asp Tyr Phe Ala Lys Phe
   1               5                   10                  15
   Trp Asp
<210> 242
   <211> 18
   <212> PRT
   <213> Artificial
<220>
   <223> Synthetic peptide optionally bearing protecting groups.
<400> 242
   Phe Ala Asp Lys Ala Lys Asp Phe Val Lys Glu Tyr Phe Ala Lys Phe
   1               5                   10                  15
   Trp Glu
<210> 243
   <211> 18
   <212> PRT
   <213> Artificial
<220>
   <223> Synthetic peptide optionally bearing protecting groups.
<400> 243
   Phe Ala Asp Lys Ala Lys Glu Phe Val Lys Asp Tyr Phe Ala Lys Phe
   1               5                   10                  15
   Trp Glu
<210> 244
   <211> 18
   <212> PRT
   <213> Artificial
<220>
   <223> Synthetic peptide optionally bearing protecting groups.
<400> 244
   Phe Ala Glu Lys Ala Lys Asp Phe Val Lys Glu Tyr Phe Ala Lys Phe
   1               5                   10                  15
   Trp Asp
<210> 245
   <211> 18
   <212> PRT
   <213> Artificial
<220>
   <223> Synthetic peptide optionally bearing protecting groups.
<400> 245
   Phe Ala Glu Lys Ala Lys Asp Phe Val Lys Asp Tyr Phe Ala Lys Phe
   1               5                   10                  15
   Trp Glu
<210> 246
   <211> 18
   <212> PRT
   <213> Artificial
<220>
   <223> Synthetic peptide optionally bearing protecting groups.
<400> 246
   Phe Ala Glu Lys Phe Lys Glu Val Ala Lys Asp Tyr Phe Ala Lys Phe
   1               5                   10                  15
   Trp Asp
<210> 247
   <211> 18
   <212> PRT
   <213> Artificial
<220>
   <223> Synthetic peptide optionally bearing protecting groups.
<400> 247
   Phe Ala Asp Lys Phe Lys Asp Val Ala Lys Glu Tyr Phe Ala Lys Phe
   1               5                   10                  15
   Trp Glu
<210> 248
   <211> 18
   <212> PRT
   <213> Artificial
<220>
   <223> Synthetic peptide optionally bearing protecting groups.
<400> 248
   Phe Ala Asp Lys Phe Lys Glu Val Ala Lys Asp Tyr Phe Ala Lys Phe
   1               5                   10                  15
   Trp Glu
<210> 249
   <211> 18
   <212> PRT
   <213> Artificial
<220>
   <223> Synthetic peptide optionally bearing protecting groups.
<400> 249
   Phe Ala Glu Lys Phe Lys Asp Val Ala Lys Glu Tyr Phe Ala Lys Phe
   1               5                   10                  15
   Trp Asp
<210> 250
   <211> 18
   <212> PRT
   <213> Artificial
<220>
   <223> Synthetic peptide optionally bearing protecting groups.
<400> 250
   Phe Ala Glu Lys Phe Lys Asp Val Ala Lys Asp Tyr Phe Ala Lys Phe
   1               5                   10                  15
   Trp Glu
<210> 251
   <211> 18
   <212> PRT
   <213> Artificial
<220>
   <223> Synthetic peptide optionally bearing protecting groups.
<400> 251
   Phe Ala Glu Lys Phe Lys Glu Ala Tyr Lys Asp Val Phe Ala Lys Phe
   1               5                   10                  15
   Trp Asp
<210> 252
   <211> 18
   <212> PRT
   <213> Artificial
<220>
   <223> Synthetic peptide optionally bearing protecting groups.
<400> 252
   Phe Ala Asp Lys Phe Lys Asp Ala Tyr Lys Glu Val Phe Ala Lys Phe
   1               5                   10                  15
   Trp Glu
<210> 253
   <211> 18
   <212> PRT
   <213> Artificial
<220>
   <223> Synthetic peptide optionally bearing protecting groups.
<400> 253
   Phe Ala Asp Lys Phe Lys Glu Ala Tyr Lys Asp Val Phe Ala Lys Phe
   1               5                   10                  15
   Trp Glu
<210> 254
   <211> 18
   <212> PRT
   <213> Artificial
<220>
   <223> Synthetic peptide optionally bearing protecting groups.
<400> 254
   Phe Ala Glu Lys Phe Lys Asp Ala Tyr Lys Glu Val Phe Ala Lys Phe
   1               5                   10                  15
   Trp Asp
<210> 255
   <211> 18
   <212> PRT
   <213> Artificial
<220>
   <223> Synthetic peptide optionally bearing protecting groups.
<400> 255
   Phe Ala Glu Lys Phe Lys Asp Ala Tyr Lys Asp Val Phe Ala Lys Phe
   1               5                   10                  15
   Trp Glu
<210> 256
   <211> 18
   <212> PRT
   <213> Artificial
<220>
   <223> Synthetic peptide optionally bearing protecting groups.
<400> 256
   Phe Ala Glu Lys Phe Lys Glu Ala Val Lys Asp Phe Tyr Ala Lys Phe
   1               5                   10                  15
   Trp Asp
<210> 257
   <211> 18
   <212> PRT
   <213> Artificial
<220>
   <223> Synthetic peptide optionally bearing protecting groups.
<400> 257
   Phe Ala Asp Lys Phe Lys Asp Ala Val Lys Glu Phe Tyr Ala Lys Phe
   1               5                   10                  15
   Trp Glu
<210> 258
   <211> 18
   <212> PRT
   <213> Artificial
<220>
   <223> Synthetic peptide optionally bearing protecting groups.
<400> 258
   Phe Ala Asp Lys Phe Lys Glu Ala Val Lys Asp Phe Tyr Ala Lys Phe
   1               5                   10                  15
   Trp Glu
<210> 259
   <211> 18
   <212> PRT
   <213> Artificial
<220>
   <223> Synthetic peptide optionally bearing protecting groups.
<400> 259
   Phe Ala Glu Lys Phe Lys Asp Ala Val Lys Glu Phe Tyr Ala Lys Phe
   1               5                   10                  15
   Trp Asp
<210> 260
   <211> 18
   <212> PRT
   <213> Artificial
<220>
   <223> Synthetic peptide optionally bearing protecting groups.
<400> 260
   Phe Ala Glu Lys Phe Lys Asp Ala Val Lys Asp Phe Tyr Ala Lys Phe
   1               5                   10                  15
   Trp Glu
<210> 261
   <211> 18
   <212> PRT
   <213> Artificial
<220>
   <223> Synthetic peptide optionally bearing protecting groups.
<400> 261
   Phe Ala Glu Lys Phe Trp Glu Ala Val Lys Asp Tyr Phe Ala Lys Phe
   1               5                   10                  15
   Lys Asp
<210> 262
   <211> 18
   <212> PRT
   <213> Artificial
<220>
   <223> Synthetic peptide optionally bearing protecting groups.
<400> 262
   Phe Ala Asp Lys Phe Trp Asp Ala Val Lys Glu Tyr Phe Ala Lys Phe
   1               5                   10                  15
   Lys Glu
<210> 263
   <211> 18
   <212> PRT
   <213> Artificial
<220>
   <223> Synthetic peptide optionally bearing protecting groups.
<400> 263
   Phe Ala Asp Lys Phe Trp Glu Ala Val Lys Asp Tyr Phe Ala Lys Phe
   1               5                   10                  15
   Lys Glu
<210> 264
   <211> 18
   <212> PRT
   <213> Artificial
<220>
   <223> Synthetic peptide optionally bearing protecting groups.
<400> 264
   Phe Ala Glu Lys Phe Trp Asp Ala Val Lys Glu Tyr Phe Ala Lys Phe
   1               5                   10                  15
   Lys Asp
<210> 265
   <211> 18
   <212> PRT
   <213> Artificial
<220>
   <223> Synthetic peptide optionally bearing protecting groups.
<400> 265
   Phe Ala Glu Lys Phe Trp Asp Ala Val Lys Asp Tyr Phe Ala Lys Phe
   1               5                   10                  15
   Lys Glu
<210> 266
   <211> 18
   <212> PRT
   <213> Artificial
<220>
   <223> Synthetic peptide optionally bearing protecting groups.
<400> 266
   Ala Phe Glu Lys Phe Lys Glu Ala Val Lys Asp Tyr Phe Ala Lys Phe
   1               5                   10                  15
   Trp Asp
<210> 267
   <211> 18
   <212> PRT
   <213> Artificial
<220>
   <223> Synthetic peptide optionally bearing protecting groups.
<400> 267
   Ala Phe Asp Lys Phe Lys Asp Ala Val Lys Glu Tyr Phe Ala Lys Phe
   1               5                   10                  15
   Trp Glu
<210> 268
   <211> 18
   <212> PRT
   <213> Artificial
<220>
   <223> Synthetic peptide optionally bearing protecting groups.
<400> 268
   Ala Phe Asp Lys Phe Lys Glu Ala Val Lys Asp Tyr Phe Ala Lys Phe
   1               5                   10                  15
   Trp Glu
<210> 269
   <211> 18
   <212> PRT
   <213> Artificial
<220>
   <223> Synthetic peptide optionally bearing protecting groups.
<400> 269
   Ala Phe Glu Lys Phe Lys Asp Ala Val Lys Glu Tyr Phe Ala Lys Phe
   1 5 10 15
   Trp Asp
<210> 270
   <211> 18
   <212> PRT
   <213> Artificial
<220>
   <223> Synthetic peptide optionally bearing protecting groups.
<400> 270
   Ala Phe Glu Lys Phe Lys Asp Ala Val Lys Asp Tyr Phe Ala Lys Phe
   1 5 10 15
   Trp Glu
<210> 271
   <211> 18
   <212> PRT
   <213> Artificial
<220>
   <223> Synthetic peptide optionally bearing protecting groups.
<400> 271
   Val Ala Glu Lys Phe Lys Glu Ala Phe Lys Asp Tyr Phe Ala Lys Phe
   1 5 10 15
   Trp Asp
<210> 272
   <211> 18
   <212> PRT
   <213> Artificial
<220>
   <223> Synthetic peptide optionally bearing protecting groups.
<400> 272
   Val Ala Asp Lys Phe Lys Asp Ala Phe Lys Glu Tyr Phe Ala Lys Phe
   1 5 10 15
   Trp Glu
<210> 273
   <211> 18
   <212> PRT
   <213> Artificial
<220>
   <223> Synthetic peptide optionally bearing protecting groups.
<400> 273
   Val Ala Asp Lys Phe Lys Glu Ala Phe Lys Asp Tyr Phe Ala Lys Phe
   1               5                   10                  15
   Trp Glu
<210> 274
   <211> 18
   <212> PRT
   <213> Artificial
<220>
   <223> Synthetic peptide optionally bearing protecting groups.
<400> 274
   Val Ala Glu Lys Phe Lys Asp Ala Phe Lys Glu Tyr Phe Ala Lys Phe
   1               5                   10                  15
   Trp Asp
<210> 275
   <211> 18
   <212> PRT
   <213> Artificial
<220>
   <223> Synthetic peptide optionally bearing protecting groups.
<400> 275
   Val Ala Glu Lys Phe Lys Asp Ala Phe Lys Asp Tyr Phe Ala Lys Phe
   1               5                   10                  15
   Trp Glu
<210> 276
   <211> 18
   <212> PRT
   <213> Artificial
<220>
   <223> Synthetic peptide optionally bearing protecting groups.
<400> 276
   Tyr Ala Glu Lys Phe Lys Glu Ala Val Lys Asp Phe Phe Ala Lys Phe
   1 5 10 15
   Trp Asp
<210> 277
   <211> 18
   <212> PRT
   <213> Artificial
<220>
   <223> Synthetic peptide optionally bearing protecting groups.
<400> 277
   Tyr Ala Asp Lys Phe Lys Asp Ala Val Lys Glu Phe Phe Ala Lys Phe
   1 5 10 15
   Trp Glu
<210> 278
   <211> 18
   <212> PRT
   <213> Artificial
<220>
   <223> Synthetic peptide optionally bearing protecting groups.
<400> 278
   Tyr Ala Asp Lys Phe Lys Glu Ala Val Lys Asp Phe Phe Ala Lys Phe
   1 5 10 15
   Trp Glu
<210> 279
   <211> 18
   <212> PRT
   <213> Artificial
<220>
   <223> Synthetic peptide optionally bearing protecting groups.
<400> 279
   Tyr Ala Glu Lys Phe Lys Asp Ala Val Lys Glu Phe Phe Ala Lys Phe
   1 5 10 15
   Trp Asp
<210> 280
   <211> 18
   <212> PRT
   <213> Artificial
<220>
   <223> Synthetic peptide optionally bearing protecting groups.
<400> 280
   Tyr Ala Glu Lys Phe Lys Asp Ala Val Lys Asp Phe Phe Ala Lys Phe
   1 5 10 15
   Trp Glu
<210> 281
   <211> 18
   <212> PRT
   <213> Artificial
<220>
   <223> Synthetic peptide optionally bearing protecting groups.
<400> 281
   Ala Ala Glu Lys Phe Lys Glu Phe Val Lys Asp Tyr Phe Ala Lys Phe
   1 5 10 15
   Trp Asp
<210> 282
   <211> 18
   <212> PRT
   <213> Artificial
<220>
   <223> Synthetic peptide optionally bearing protecting groups.
<400> 282
   Ala Ala Asp Lys Phe Lys Asp Phe Val Lys Glu Tyr Phe Ala Lys Phe
   1 5 10 15
   Trp Glu
<210> 283
   <211> 18
   <212> PRT
   <213> Artificial
<220>
   <223> Synthetic peptide optionally bearing protecting groups.
<400> 283
   Ala Ala Asp Lys Phe Lys Glu Phe Val Lys Asp Tyr Phe Ala Lys Phe
   1 5 10 15
   Trp Glu
<210> 284
   <211> 18
   <212> PRT
   <213> Artificial
<220>
   <223> Synthetic peptide optionally bearing protecting groups.
<400> 284
   Ala Ala Glu Lys Phe Lys Asp Phe Val Lys Glu Tyr Phe Ala Lys Phe
   1               5                   10                  15
   Trp Asp
<210> 285
   <211> 18
   <212> PRT
   <213> Artificial
<220>
   <223> Synthetic peptide optionally bearing protecting groups.
<400> 285
   Ala Ala Glu Lys Phe Lys Asp Phe Val Lys Asp Tyr Phe Ala Lys Phe
   1               5                   10                  15
   Trp Glu
<210> 286
   <211> 18
   <212> PRT
   <213> Artificial
<220>
   <223> Synthetic peptide optionally bearing protecting groups.
<400> 286
   Phe Phe Glu Lys Ala Lys Glu Ala Val Lys Asp Tyr Phe Ala Lys Phe
   1               5                   10                  15
   Trp Asp
<210> 287
   <211> 18
   <212> PRT
   <213> Artificial
<220>
   <223> Synthetic peptide optionally bearing protecting groups.
<400> 287
   Phe Phe Asp Lys Ala Lys Asp Ala Val Lys Glu Tyr Phe Ala Lys Phe
   1 5 10 15
   Trp Glu
<210> 288
   <211> 18
   <212> PRT
   <213> Artificial
<220>
   <223> Synthetic peptide optionally bearing protecting groups.
<400> 288
   Phe Phe Asp Lys Ala Lys Glu Ala Val Lys Asp Tyr Phe Ala Lys Phe
   1 5 10 15
   Trp Glu
<210> 289
   <211> 18
   <212> PRT
   <213> Artificial
<220>
   <223> Synthetic peptide optionally bearing protecting groups.
<400> 289
   Phe Phe Glu Lys Ala Lys Asp Ala Val Lys Glu Tyr Phe Ala Lys Phe
   1 5 10 15
   Trp Asp
<210> 290
   <211> 18
   <212> PRT
   <213> Artificial
<220>
   <223> Synthetic peptide optionally bearing protecting groups.
<400> 290
   Phe Phe Glu Lys Ala Lys Asp Ala Val Lys Asp Tyr Phe Ala Lys Phe
   1 5 10 15
   Trp Glu
<210> 291
   <211> 18
   <212> PRT
   <213> Artificial
<220>
   <223> Synthetic peptide optionally bearing protecting groups.
<400> 291
   Phe Tyr Glu Lys Phe Lys Glu Ala Val Lys Asp Ala Phe Ala Lys Phe
   1 5 10 15
   Trp Asp
<210> 292
   <211> 18
   <212> PRT
   <213> Artificial
<220>
   <223> Synthetic peptide optionally bearing protecting groups.
<400> 292
   Phe Tyr Asp Lys Phe Lys Asp Ala Val Lys Glu Ala Phe Ala Lys Phe
   1 5 10 15
   Trp Glu
<210> 293
   <211> 18
   <212> PRT
   <213> Artificial
<220>
   <223> Synthetic peptide optionally bearing protecting groups.
<400> 293
   Phe Tyr Asp Lys Phe Lys Glu Ala Val Lys Asp Ala Phe Ala Lys Phe
   1 5 10 15
   Trp Glu
<210> 294
   <211> 18
   <212> PRT
   <213> Artificial
<220>
   <223> Synthetic peptide optionally bearing protecting groups.
<400> 294
   Phe Tyr Glu Lys Phe Lys Asp Ala Val Lys Glu Ala Phe Ala Lys Phe
   1 5 10 15
   Trp Asp
<210> 295
   <211> 18
   <212> PRT
   <213> Artificial
<220>
   <223> Synthetic peptide optionally bearing protecting groups.
<400> 295
   Phe Tyr Glu Lys Phe Lys Asp Ala Val Lys Asp Ala Phe Ala Lys Phe
   1               5                   10                  15
   Trp Glu
<210> 296
   <211> 18
   <212> PRT
   <213> Artificial
<220>
   <223> Synthetic peptide optionally bearing protecting groups.
<400> 296
   Phe Val Glu Lys Phe Lys Glu Ala Ala Lys Asp Tyr Phe Ala Lys Phe
   1               5                   10                  15
   Trp Asp
<210> 297
   <211> 18
   <212> PRT
   <213> Artificial
<220>
   <223> Synthetic peptide optionally bearing protecting groups.
<400> 297
   Phe Val Asp Lys Phe Lys Asp Ala Ala Lys Glu Tyr Phe Ala Lys Phe
   1               5                   10                  15
   Trp Glu
<210> 298
   <211> 18
   <212> PRT
   <213> Artificial
<220>
   <223> Synthetic peptide optionally bearing protecting groups.
<400> 298
   Phe Val Asp Lys Phe Lys Glu Ala Ala Lys Asp Tyr Phe Ala Lys Phe
   1 5 10 15
   Trp Glu
<210> 299
   <211> 18
   <212> PRT
   <213> Artificial
<220>
   <223> Synthetic peptide optionally bearing protecting groups.
<400> 299
   Phe Val Glu Lys Phe Lys Asp Ala Ala Lys Glu Tyr Phe Ala Lys Phe
   1 5 10 15
   Trp Asp
<210> 300
   <211> 18
   <212> PRT
   <213> Artificial
<220>
   <223> Synthetic peptide optionally bearing protecting groups.
<400> 300
   Phe Val Glu Lys Phe Lys Asp Ala Ala Lys Asp Tyr Phe Ala Lys Phe
   1 5 10 15
   Trp Glu
<210> 301
   <211> 18
   <212> PRT
   <213> Artificial
<220>
   <223> Synthetic peptide optionally bearing protecting groups.
<400> 301
   Phe Ala Glu Lys Tyr Lys Glu Ala Val Lys Asp Phe Phe Ala Lys Phe
   1 5 10 15
   Trp Asp
<210> 302
   <211> 18
   <212> PRT
   <213> Artificial
<220>
   <223> Synthetic peptide optionally bearing protecting groups.
<400> 302
   Phe Ala Asp Lys Tyr Lys Asp Ala Val Lys Glu Phe Phe Ala Lys Phe
   1 5 10 15
   Trp Glu
<210> 303
   <211> 18
   <212> PRT
   <213> Artificial
<220>
   <223> Synthetic peptide optionally bearing protecting groups.
<400> 303
   Phe Ala Asp Lys Tyr Lys Glu Ala Val Lys Asp Phe Phe Ala Lys Phe
   1 5 10 15
   Trp Glu
<210> 304
   <211> 18
   <212> PRT
   <213> Artificial
<220>
   <223> Synthetic peptide optionally bearing protecting groups.
<400> 304
   Phe Ala Glu Lys Tyr Lys Asp Ala Val Lys Glu Phe Phe Ala Lys Phe
   1 5 10 15
   Trp Asp
<210> 305
   <211> 18
   <212> PRT
   <213> Artificial
<220>
   <223> Synthetic peptide optionally bearing protecting groups.
<400> 305
   Phe Ala Glu Lys Tyr Lys Asp Ala Val Lys Asp Phe Phe Ala Lys Phe
   1 5 10 15
   Trp Glu
<210> 306
   <211> 18
   <212> PRT
   <213> Artificial
<220>
   <223> Synthetic peptide optionally bearing protecting groups.
<400> 306
   Phe Ala Glu Lys Val Lys Glu Ala Phe Lys Asp Tyr Phe Ala Lys Phe
   1               5                   10                  15
   Trp Asp
<210> 307
   <211> 18
   <212> PRT
   <213> Artificial
<220>
   <223> Synthetic peptide optionally bearing protecting groups.
<400> 307
   Phe Ala Asp Lys Val Lys Asp Ala Phe Lys Glu Tyr Phe Ala Lys Phe
   1               5                   10                  15
   Trp Glu
<210> 308
   <211> 18
   <212> PRT
   <213> Artificial
<220>
   <223> synthetic peptide optionally bearing protecting groups.
<400> 308
   Phe Ala Asp Lys Val Lys Glu Ala Phe Lys Asp Tyr Phe Ala Lys Phe
   1               5                   10                  15
   Trp Glu
<210> 309
   <211> 18
   <212> PRT
   <213> Artificial
<220>
   <223> Synthetic peptide optionally bearing protecting groups.
<400> 309
   Phe Ala Glu Lys Val Lys Asp Ala Phe Lys Glu Tyr Phe Ala Lys Phe
   1 5 10 15
   Trp Asp
<210> 310
   <211> 18
   <212> PRT
   <213> Artificial
<220>
   <223> Synthetic peptide optionally bearing protecting groups.
<400> 310
   Phe Ala Glu Lys Val Lys Asp Ala Phe Lys Asp Tyr Phe Ala Lys Phe
   1 5 10 15
   Trp Glu

<210> 311
   <211> 18
   <212> PRT
   <213> Artificial
<220>
   <223> Synthetic peptide optionally bearing protecting groups.
<400> 311
   Phe Ala Glu Lys Phe Lys Glu Tyr Val Lys Asp Ala Phe Ala Lys Phe
   1 5 10 15
   Trp Asp
<210> 312
   <211> 18
   <212> PRT
   <213> Artificial
<220>
   <223> Synthetic peptide optionally bearing protecting groups.
<400> 312
   Phe Ala Asp Lys Phe Lys Asp Tyr Val Lys Glu Ala Phe Ala Lys Phe
   1 5 10 15
   Trp Glu
<210> 313
   <211> 18
   <212> PRT
   <213> Artificial
<220>
   <223> Synthetic peptide optionally bearing protecting groups.
<400> 313
   Phe Ala Asp Lys Phe Lys Glu Tyr Val Lys Asp Ala Phe Ala Lys Phe
   1               5                   10                  15
   Trp Glu
<210> 314
   <211> 18
   <212> PRT
   <213> Artificial
<220>
   <223> Synthetic peptide optionally bearing protecting groups.
<400> 314
   Phe Ala Glu Lys Phe Lys Asp Tyr Val Lys Glu Ala Phe Ala Lys Phe
   1               5                   10                  15
   Trp Asp
<210> 315
   <211> 18
   <212> PRT
   <213> Artificial
<220>
   <223> Synthetic peptide optionally bearing protecting groups.
<400> 315
   Phe Ala Glu Lys Phe Lys Asp Tyr Val Lys Asp Ala Phe Ala Lys Phe
   1               5                   10                  15
   Trp Glu
<210> 316
   <211> 18
   <212> PRT
   <213> Artificial
<220>
   <223> Synthetic peptide optionally bearing protecting groups.
<400> 316
   Phe Ala Glu Lys Phe Lys Glu Ala Phe Lys Asp Tyr Val Ala Lys Phe
   1               5                   10                  15
   Trp Asp
<210> 317
   <211> 18
   <212> PRT
   <213> Artificial
<220>
   <223> Synthetic peptide optionally bearing protecting groups.
<400> 317
   Phe Ala Asp Lys Phe Lys Asp Ala Phe Lys Glu Tyr Val Ala Lys Phe
   1               5                   10                  15
   Trp Glu
<210> 318
   <211> 18
   <212> PRT
   <213> Artificial
<220>
   <223> Synthetic peptide optionally bearing protecting groups.
<400> 318
   Phe Ala Asp Lys Phe Lys Glu Ala Phe Lys Asp Tyr Val Ala Lys Phe
   1               5                   10                  15
   Trp Glu
<210> 319
   <211> 18
   <212> PRT
   <213> Artificial
<220>
   <223> Synthetic peptide optionally bearing protecting groups.
<400> 319
   Phe Ala Glu Lys Phe Lys Asp Ala Phe Lys Glu Tyr Val Ala Lys Phe
   1               5                   10                  15
   Trp Asp
<210> 320
   <211> 18
   <212> PRT
   <213> Artificial
<220>
   <223> Synthetic peptide optionally bearing protecting groups.
<400> 320
   Phe Ala Glu Lys Phe Lys Asp Ala Phe Lys Asp Tyr Val Ala Lys Phe
   1               5                   10                  15
   Trp Glu
<210> 321
   <211> 18
   <212> PRT
   <213> Artificial
<220>
   <223> Synthetic peptide optionally bearing protecting groups.
<400> 321
   Phe Ala Glu Lys Phe Lys Glu Ala Phe Lys Asp Tyr Phe Ala Lys Val
   1               5                   10                  15
   Trp Asp
<210> 322
   <211> 18
   <212> PRT
   <213> Artificial
<220>
   <223> Synthetic peptide optionally bearing protecting groups.
<400> 322
   Phe Ala Asp Lys Phe Lys Asp Ala Phe Lys Glu Tyr Phe Ala Lys Val
   1               5                   10                  15
   Trp Glu
<210> 323
   <211> 18
   <212> PRT
   <213> Artificial
<220>
   <223> Synthetic peptide optionally bearing protecting groups.
<400> 323
   Phe Ala Asp Lys Phe Lys Glu Ala Phe Lys Asp Tyr Phe Ala Lys Val
   1               5                   10                  15
   Trp Glu
<210> 324
   <211> 18
   <212> PRT
   <213> Artificial
<220>
   <223> Synthetic peptide optionally bearing protecting groups.
<400> 324
   Phe Ala Glu Lys Phe Lys Asp Ala Phe Lys Glu Tyr Phe Ala Lys Val
   1 5 10 15
   Trp Asp
<210> 325
   <211> 18
   <212> PRT
   <213> Artificial
<220>
   <223> Synthetic peptide optionally bearing protecting groups.
<400> 325
   Phe Ala Glu Lys Phe Lys Asp Ala Phe Lys Asp Tyr Phe Ala Lys Val
   1 5 10 15
   Trp Glu
<210> 326
   <211> 18
   <212> PRT
   <213> Artificial
<220>
   <223> Synthetic peptide optionally bearing protecting groups.
<400> 326
   Phe Ala Glu Lys Phe Lys Glu Ala Val Lys Asp Phe Phe Ala Lys Tyr
   1 5 10 15
   Trp Asp
<210> 327
   <211> 18
   <212> PRT
   <213> Artificial
<220>
   <223> Synthetic peptide optionally bearing protecting groups.
<400> 327
   Phe Ala Asp Lys Phe Lys Asp Ala Val Lys Glu Phe Phe Ala Lys Tyr
   1 5 10 15
   Trp Glu
<210> 328
   <211> 18
   <212> PRT
   <213> Artificial
<220>
   <223> Synthetic peptide optionally bearing protecting groups.
<400> 328
   Phe Ala Asp Lys Phe Lys Glu Ala Val Lys Asp Phe Phe Ala Lys Tyr
   1 5 10 15
   Trp Glu
<210> 329
   <211> 18
   <212> PRT
   <213> Artificial
<220>
   <223> Synthetic peptide optionally bearing protecting groups.
<400> 329
   Phe Ala Glu Lys Phe Lys Asp Ala Val Lys Glu Phe Phe Ala Lys Tyr
   1 5 10 15
   Trp Asp
<210> 330
   <211> 18
   <212> PRT
   <213> Artificial
<220>
   <223> Synthetic peptide optionally bearing protecting groups.
<400> 330
   Phe Ala Glu Lys Phe Lys Asp Ala Val Lys Asp Phe Phe Ala Lys Tyr
   1 5 10 15
   Trp Glu
<210> 331
   <211> 18
   <212> PRT
   <213> Artificial
<220>
   <223> Synthetic peptide optionally bearing protecting groups.
<400> 331
   Trp Ala Glu Lys Phe Phe Glu Ala Val Lys Asp Tyr Phe Ala Lys Phe
   1 5 10 15
   Lys Asp
<210> 332
   <211> 18
   <212> PRT
   <213> Artificial
<220>
   <223> Synthetic peptide optionally bearing protecting groups.
<400> 332
   Trp Ala Asp Lys Phe Phe Asp Ala Val Lys Glu Tyr Phe Ala Lys Phe
   1 5 10 15
   Lys Glu
<210> 333
   <211> 18
   <212> PRT
   <213> Artificial
<220>
   <223> Synthetic peptide optionally bearing protecting groups.
<400> 333
   Trp Ala Asp Lys Phe Phe Glu Ala Val Lys Asp Tyr Phe Ala Lys Phe
   1 5 10 15
   Lys Glu
<210> 334
   <211> 18
   <212> PRT
   <213> Artificial
<220>
   <223> Synthetic peptide optionally bearing protecting groups.
<400> 334
   Trp Ala Glu Lys Phe Phe Asp Ala Val Lys Glu Tyr Phe Ala Lys Phe
   1 5 10 15
   Lys Asp
<210> 335
   <211> 18
   <212> PRT
   <213> Artificial
<220>
   <223> Synthetic peptide optionally bearing protecting groups.
<400> 335
   Trp Ala Glu Lys Phe Phe Asp Ala Val Lys Asp Tyr Phe Ala Lys Phe
   1               5                   10                  15
   Lys Glu
<210> 336
   <211> 18
   <212> PRT
   <213> Artificial
<220>
   <223> Synthetic peptide optionally bearing protecting groups.
<400> 336
   Phe Ala Glu Lys Trp Phe Glu Ala Val Lys Asp Tyr Phe Ala Lys Phe
   1               5                   10                  15
   Lys Asp
<210> 337
   <211> 18
   <212> PRT
   <213> Artificial
<220>
   <223> Synthetic peptide optionally bearing protecting groups.
<400> 337
   Phe Ala Asp Lys Trp Phe Asp Ala Val Lys Glu Tyr Phe Ala Lys Phe
   1               5                   10                  15
   Lys Glu
<210> 338
   <211> 18
   <212> PRT
   <213> Artificial
<220>
   <223> Synthetic peptide optionally bearing protecting groups.
<400> 338
   Phe Ala Asp Lys Trp Phe Glu Ala Val Lys Asp Tyr Phe Ala Lys Phe
   1 5 10 15
   Lys Glu
<210> 339
   <211> 18
   <212> PRT
   <213> Artificial
<220>
   <223> Synthetic peptide optionally bearing protecting groups.
<400> 339
   Phe Ala Glu Lys Trp Phe Asp Ala Val Lys Glu Tyr Phe Ala Lys Phe
   1 5 10 15
   Lys Asp
<210> 340
   <211> 18
   <212> PRT
   <213> Artificial
<220>
   <223> Synthetic peptide optionally bearing protecting groups.
<400> 340
   Phe Ala Glu Lys Trp Phe Asp Ala Val Lys Asp Tyr Phe Ala Lys Phe
   1 5 10 15
   Lys Glu
<210> 341
   <211> 18
   <212> PRT
   <213> Artificial
<220>
   <223> Synthetic peptide optionally bearing protecting groups.
<400> 341
   Phe Ala Glu Lys Phe Val Glu Ala Trp Lys Asp Tyr Phe Ala Lys Phe
   1 5 10 15
   Lys Asp
<210> 342
   <211> 18
   <212> PRT
   <213> Artificial
<220>
   <223> Synthetic peptide optionally bearing protecting groups.
<400> 342
   Phe Ala Asp Lys Phe Val Asp Ala Trp Lys Glu Tyr Phe Ala Lys Phe
   1               5                   10                  15
   Lys Glu
<210> 343
   <211> 18
   <212> PRT
   <213> Artificial
<220>
   <223> Synthetic peptide optionally bearing protecting groups.
<400> 343
   Phe Ala Asp Lys Phe Val Glu Ala Trp Lys Asp Tyr Phe Ala Lys Phe
   1               5                   10                  15
   Lys Glu
<210> 344
   <211> 18
   <212> PRT
   <213> Artificial
<220>
   <223> Synthetic peptide optionally bearing protecting groups.
<400> 344
   Phe Ala Glu Lys Phe Val Asp Ala Trp Lys Glu Tyr Phe Ala Lys Phe
   1               5                   10                  15
   Lys Asp
<210> 345
   <211> 18
   <212> PRT
   <213> Artificial
<220>
   <223> Synthetic peptide optionally bearing protecting groups.
<400> 345
   Phe Ala Glu Lys Phe Val Asp Ala Trp Lys Asp Tyr Phe Ala Lys Phe
   1               5                   10                  15
   Lys Glu
<210> 346
   <211> 18
   <212> PRT
   <213> Artificial
<220>
   <223> Synthetic peptide optionally bearing protecting groups.
<400> 346
   Phe Tyr Glu Lys Phe Ala Glu Ala Val Lys Asp Trp Phe Ala Lys Phe
   1               5                   10                  15
   Lys Asp
<210> 347
   <211> 18
   <212> PRT
   <213> Artificial
<220>
   <223> Synthetic peptide optionally bearing protecting groups.
<400> 347
   Phe Tyr Asp Lys Phe Ala Asp Ala Val Lys Glu Trp Phe Ala Lys Phe
   1               5                   10                  15
   Lys Glu
<210> 348
   <211> 18
   <212> PRT
   <213> Artificial
<220>
   <223> Synthetic peptide optionally bearing protecting groups.
<400> 348
   Phe Tyr Asp Lys Phe Ala Glu Ala Val Lys Asp Trp Phe Ala Lys Phe
   1               5                   10                  15
   Lys Glu
<210> 349
   <211> 18
   <212> PRT
   <213> Artificial
<220>
   <223> Synthetic peptide optionally bearing protecting groups.
<400> 349
   Phe Tyr Glu Lys Phe Ala Asp Ala Val Lys Glu Trp Phe Ala Lys Phe
   1               5                   10                  15
   Lys Asp
<210> 350
   <211> 18
   <212> PRT
   <213> Artificial
<220>
   <223> Synthetic peptide optionally bearing protecting groups.
<400> 350
   Phe Tyr Glu Lys Phe Ala Asp Ala Val Lys Asp Trp Phe Ala Lys Phe
   1               5                   10                  15
   Lys Glu
<210> 351
   <211> 18
   <212> PRT
   <213> Artificial
<220>
   <223> Synthetic peptide optionally bearing protecting groups.
<220>
   <221> misc_feature
   <222> (1)..(1)
   <223> Xaa is Lys or Arg
<220>
   <221> misc_feature
   <222> (2)..(3)
   <223> Xaa is Leu, norLeu, Val, Ile, Trp, Phe, Tyr, beta-Nal, or alpha-Nal
<220>
   <221> misc_feature
   <222> (4)..(4)
   <223> Xaa is Lys or Arg
<220>
   <221> misc_feature
   <222> (5)..(5)
   <223> Xaa is Ala, His, Ser, Gln, Asn, or Thr
<220>
   <221> misc_feature
   <222> (6)..(7)
   <223> Xaa is Leu, norLeu, Val, Ile, Trp, Phe, Tyr, beta-Nal, or alpha-Nal
<220>
   <221> misc_feature
   <222> (8)..(8)
   <223> Xaa is Lys or Arg
<220>
   <221> misc_feature
   <222> (9)..(9)
   <223> Xaa is Lys or Arg
<220>
   <221> misc_feature
   <222> (10)..(10)
   <223> Xaa is Leu, norLeu, Val, Ile, Trp, Phe, Tyr, beta-Nal, or alpha-Nal
<220>
   <221> misc_feature
   <222> (11)..(11)
   <223> Xaa is Ala, His, Ser, Gln, Asn, or Thr
<220>
   <221> misc_feature
   <222> (12)..(12)
   <223> Xaa is Lys or Arg
<220>
   <221> misc_feature
   <222> (13)..(13)
   <223> Xaa is Lys or Arg
<220>
   <221> misc_feature
   <222> (14)..(14)
   <223> Xaa is Leu, norLeu, Val, Ile, Trp, Phe, Tyr, beta-Nal, or alpha-Nal
<220>
   <221> misc_feature
   <222> (15)..(15)
   <223> Xaa is Lys or Arg
<220>
   <221> misc_feature
   <222> (16)..(16)
   <223> Xaa is Lys or Arg
<220>
   <221> misc_feature
   <222> (17)..(17)
   <223> Xaa is Ala, His, Ser, Gln, Asn, or Thr
<220>
   <221> misc_feature
   <222> (18)..(18)
   <223> Xaa is Leu, norLeu, Val, Ile, Trp, Phe, Tyr, beta-Nal, or alpha-Nal
<400> 351
   Xaa Xaa Xaa Xaa Xaa Xaa Xaa Xaa Xaa Xaa Xaa Xaa Xaa Xaa Xaa Xaa
   1 5 10 15
   Xaa Xaa
<210> 352
   <211> 18
   <212> PRT
   <213> Artificial
<220>
   <223> Synthetic peptide optionally bearing protecting groups.
<400> 352
   Asp Trp Phe Lys His Phe Tyr Asp Lys Val Ala Glu Lys Phe Lys Glu
   1 5 10 15
   Ala Phe
<210> 353
   <211> 18
   <212> PRT
   <213> Artificial
<220>
   <223> Synthetic peptide optionally bearing protecting groups.
<400> 353
   Glu Trp Phe Lys His Phe Tyr Glu Lys Val Ala Asp Lys Phe Lys Asp
   1 5 10 15
   Ala Phe
<210> 354
   <211> 18
   <212> PRT
   <213> Artificial
<220>
   <223> Synthetic peptide optionally bearing protecting groups.
<400> 354
   Glu Trp Phe Lys His Phe Tyr Asp Lys Val Ala Glu Lys Phe Lys Glu
   1 5 10 15
   Ala Phe
<210> 355
   <211> 18
   <212> PRT
   <213> Artificial
<220>
   <223> Synthetic peptide optionally bearing protecting groups.
<400> 355
   Asp Trp Phe Lys His Phe Tyr Glu Lys Val Ala Glu Lys Phe Lys Glu
   1 5 10 15
   Ala Phe
<210> 356
   <211> 18
   <212> PRT
   <213> Artificial
<220>
   <223> Synthetic peptide optionally bearing protecting groups.
<400> 356
   Asp Trp Phe Lys His Phe Tyr Asp Lys Val Ala Asp Lys Phe Lys Glu
   1 5 10 15
   Ala Phe
<210> 357
   <211> 18
   <212> PRT
   <213> Artificial
<220>
   <223> Synthetic peptide optionally bearing protecting groups.
<400> 357
   Asp Trp Phe Lys His Phe Tyr Asp Lys Val Ala Glu Lys Phe Lys Asp
   1 5 10 15
   Ala Phe
<210> 358
   <211> 18
   <212> PRT
   <213> Artificial
<220>
   <223> Synthetic peptide optionally bearing protecting groups.
<400> 358
   Asp Trp His Lys Phe Phe Tyr Asp Lys Val Ala Glu Lys Phe Lys Glu
   1 5 10 15
   Ala Phe
<210> 359
   <211> 18
   <212> PRT
   <213> Artificial
<220>
   <223> Synthetic peptide optionally bearing protecting groups.
<400> 359
   Glu Trp His Lys Phe Phe Tyr Glu Lys Val Ala Asp Lys Phe Lys Asp
   1 5 10 15
   Ala Phe
<210> 360
   <211> 18
   <212> PRT
   <213> Artificial
<220>
   <223> Synthetic peptide optionally bearing protecting groups.
<400> 360
   Glu Trp His Lys Phe Phe Tyr Asp Lys Val Ala Glu Lys Phe Lys Glu
   1 5 10 15
   Ala Phe
<210> 361
   <211> 18
   <212> PRT
   <213> Artificial
<220>
   <223> Synthetic peptide optionally bearing protecting groups.
<400> 361
   Asp Trp His Lys Phe Phe Tyr Glu Lys Val Ala Glu Lys Phe Lys Glu
   1 5 10 15
   Ala Phe
<210> 362
   <211> 18
   <212> PRT
   <213> Artificial
<220>
   <223> Synthetic peptide optionally bearing protecting groups.
<400> 362
   Asp Trp His Lys Phe Phe Tyr Asp Lys Val Ala Asp Lys Phe Lys Glu
   1               5                   10                  15
   Ala Phe
<210> 363
   <211> 18
   <212> PRT
   <213> Artificial
<220>
   <223> Synthetic peptide optionally bearing protecting groups.
<400> 363
   Asp Trp His Lys Phe Phe Tyr Asp Lys Val Ala Glu Lys Phe Lys Asp
   1               5                   10                  15
   Ala Phe
<210> 364
   <211> 18
   <212> PRT
   <213> Artificial
<220>
   <223> Synthetic peptide optionally bearing protecting groups.
<400> 364
   Asp Trp Phe Lys Phe His Tyr Asp Lys Val Ala Glu Lys Phe Lys Glu
   1               5                   10                  15
   Ala Phe
<210> 365
   <211> 18
   <212> PRT
   <213> Artificial
<220>
   <223> Synthetic peptide optionally bearing protecting groups.
<400> 365
   Glu Trp Phe Lys Phe His Tyr Glu Lys Val Ala Asp Lys Phe Lys Asp
   1               5                   10                  15
   Ala Phe
<210> 366
   <211> 18
   <212> PRT
   <213> Artificial
<220>
   <223> Synthetic peptide optionally bearing protecting groups.
<400> 366
   Glu Trp Phe Lys Phe His Tyr Asp Lys Val Ala Glu Lys Phe Lys Glu
   1               5                   10                  15
   Ala Phe
<210> 367
   <211> 18
   <212> PRT
   <213> Artificial
<220>
   <223> Synthetic peptide optionally bearing protecting groups.
<400> 367
   Asp Trp Phe Lys Phe His Tyr Glu Lys Val Ala Glu Lys Phe Lys Glu
   1               5                   10                  15
   Ala Phe
<210> 368
   <211> 18
   <212> PRT
   <213> Artificial
<220>
   <223> Synthetic peptide optionally bearing protecting groups.
<400> 368
   Asp Trp Phe Lys Phe His Tyr Asp Lys Val Ala Asp Lys Phe Lys Glu
   1               5                   10                  15
   Ala Phe
<210> 369
   <211> 18
   <212> PRT
   <213> Artificial
<220>
   <223> Synthetic peptide optionally bearing protecting groups.
<400> 369
   Asp Trp Phe Lys Phe His Tyr Asp Lys Val Ala Glu Lys Phe Lys Asp
   1 5 10 15
   Ala Phe
<210> 370
   <211> 18
   <212> PRT
   <213> Artificial
<220>
   <223> Synthetic peptide optionally bearing protecting groups.
<400> 370
   Asp Trp Phe Lys Val Phe Tyr Asp Lys His Ala Glu Lys Phe Lys Glu
   1 5 10 15
   Ala Phe
<210> 371
   <211> 18
   <212> PRT
   <213> Artificial
<220>
   <223> Synthetic peptide optionally bearing protecting groups.
<400> 371
   Glu Trp Phe Lys Val Phe Tyr Glu Lys His Ala Asp Lys Phe Lys Asp
   1 5 10 15
   Ala Phe
<210> 372
   <211> 18
   <212> PRT
   <213> Artificial
<220>
   <223> Synthetic peptide optionally bearing protecting groups.
<400> 372
   Glu Trp Phe Lys Val Phe Tyr Asp Lys His Ala Glu Lys Phe Lys Glu
   1 5 10 15
   Ala Phe
<210> 373
   <211> 18
   <212> PRT
   <213> Artificial
<220>
   <223> Synthetic peptide optionally bearing protecting groups.
<400> 373
   Asp Trp Phe Lys Val Phe Tyr Glu Lys His Ala Glu Lys Phe Lys Glu
   1               5                   10                  15
   Ala Phe
<210> 374
   <211> 18
   <212> PRT
   <213> Artificial
<220>
   <223> Synthetic peptide optionally bearing protecting groups.
<400> 374
   Asp Trp Phe Lys Val Phe Tyr Asp Lys His Ala Asp Lys Phe Lys Glu
   1               5                   10                  15
   Ala Phe
<210> 375
   <211> 18
   <212> PRT
   <213> Artificial
<220>
   <223> Synthetic peptide optionally bearing protecting groups.
<400> 375
   Asp Trp Phe Lys Val Phe Tyr Asp Lys His Ala Glu Lys Phe Lys Asp
   1               5                   10                  15
   Ala Phe
<210> 376
   <211> 18
   <212> PRT
   <213> Artificial
<220>
   <223> Synthetic peptide optionally bearing protecting groups.
<400> 376
   Asp Trp Phe Lys Ala Phe Tyr Asp Lys Val Ala Glu Lys Phe Lys Glu
   1               5                   10                  15
   His Phe
<210> 377
   <211> 18
   <212> PRT
   <213> Artificial
<220>
   <223> Synthetic peptide optionally bearing protecting groups.
<400> 377
   Glu Trp Phe Lys Ala Phe Tyr Glu Lys Val Ala Asp Lys Phe Lys Asp
   1               5                   10                  15
   His Phe
<210> 378
   <211> 18
   <212> PRT
   <213> Artificial
<220>
   <223> Synthetic peptide optionally bearing protecting groups.
<400> 378
   Glu Trp Phe Lys Ala Phe Tyr Asp Lys Val Ala Glu Lys Phe Lys Glu
   1               5                   10                  15
   His Phe
<210> 379
   <211> 18
   <212> PRT
   <213> Artificial
<220>
   <223> Synthetic peptide optionally bearing protecting groups.
<400> 379
   Asp Trp Phe Lys Ala Phe Tyr Glu Lys Val Ala Glu Lys Phe Lys Glu
   1               5                   10                  15
   His Phe
<210> 380
   <211> 18
   <212> PRT
   <213> Artificial
<220>
   <223> Synthetic peptide optionally bearing protecting groups.
<400> 380
   Asp Trp Phe Lys Ala Phe Tyr Asp Lys Val Ala Asp Lys Phe Lys Glu
   1 5 10 15
   His Phe
<210> 381
   <211> 18
   <212> PRT
   <213> Artificial
<220>
   <223> Synthetic peptide optionally bearing protecting groups.
<400> 381
   Asp Trp Phe Lys Ala Phe Tyr Asp Lys Val Ala Glu Lys Phe Lys Asp
   1 5 10 15
   His Phe
<210> 382
   <211> 18
   <212> PRT
   <213> Artificial
<220>
   <223> Synthetic peptide optionally bearing protecting groups.
<400> 382
   Asp Trp Phe Lys Ala Phe Tyr Asp Lys Val Ala Glu Lys Phe Lys Glu
   1 5 10 15
   Phe His

<210> 383
   <211> 18
   <212> PRT
   <213> Artificial
<220>
   <223> Synthetic peptide optionally bearing protecting groups.
<400> 383
   Glu Trp Phe Lys Ala Phe Tyr Glu Lys Val Ala Asp Lys Phe Lys Asp
   1 5 10 15
   Phe His
<210> 384
   <211> 18
   <212> PRT
   <213> Artificial
<220>
   <223> Synthetic peptide optionally bearing protecting groups.
<400> 384
   Glu Trp Phe Lys Ala Phe Tyr Asp Lys Val Ala Glu Lys Phe Lys Glu
   1 5 10 15
   Phe His
<210> 385
   <211> 18
   <212> PRT
   <213> Artificial
<220>
   <223> Synthetic peptide optionally bearing protecting groups.
<400> 385
   Asp Trp Phe Lys Ala Phe Tyr Asp Lys Val Ala Glu Lys Phe Lys Glu
   1 5 10 15
   Phe His
<210> 386
   <211> 18
   <212> PRT
   <213> Artificial
<220>
   <223> Synthetic peptide optionally bearing protecting groups.
<400> 386
   Asp Trp Phe Lys Ala Phe Tyr Glu Lys Val Ala Glu Lys Phe Lys Glu
   1 5 10 15
   Phe His
<210> 387
   <211> 18
   <212> PRT
   <213> Artificial
<220>
   <223> Synthetic peptide optionally bearing protecting groups.
<400> 387
   Asp Trp Phe Lys Ala Phe Tyr Asp Lys Val Ala Glu Lys Phe Lys Glu
   1 5 10 15
   Phe His
<210> 388
   <211> 18
   <212> PRT
   <213> Artificial
<220>
   <223> Synthetic peptide optionally bearing protecting groups.
<400> 388
   Asp Trp Phe Lys Ala Phe Tyr Asp Lys Val Ala Glu Lys Phe Lys Asp
   1               5                   10                  15
   Phe His
<210> 389
   <211> 18
   <212> PRT
   <213> Artificial
<220>
   <223> Synthetic peptide optionally bearing protecting groups.
<400> 389
   Phe Ala Glu Lys Phe Lys Glu Ala Val Lys Asp Tyr Phe Ala Lys Phe
   1               5                   10                  15
   Trp Asp
<210> 390
   <211> 18
   <212> PRT
   <213> Artificial
<220>
   <223> Synthetic peptide optionally bearing protecting groups.
<400> 390
   Phe His Glu Lys Phe Lys Glu Ala Val Lys Asp Tyr Phe Ala Lys Phe
   1               5                   10                  15
   Trp Asp
<210> 391
   <211> 18
   <212> PRT
   <213> Artificial
<220>
   <223> Synthetic peptide optionally bearing protecting groups.
<400> 391
   Phe His Glu Lys Phe Lys Glu Ala Val Lys Glu Tyr Phe Ala Lys Phe
   1 5 10 15
   Trp Glu
<210> 392
   <211> 18
   <212> PRT
   <213> Artificial
<220>
   <223> Synthetic peptide optionally bearing protecting groups.
<400> 392
   Phe His Asp Lys Phe Lys Asp Ala Val Lys Asp Tyr Phe Ala Lys Phe
   1 5 10 15
   Trp Asp
<210> 393
   <211> 18
   <212> PRT
   <213> Artificial
<220>
   <223> Synthetic peptide optionally bearing protecting groups.
<400> 393
   Phe His Asp Lys Phe Lys Asp Ala Val Lys Glu Tyr Phe Ala Lys Phe
   1 5 10 15
   Trp Glu
<210> 394
   <211> 18
   <212> PRT
   <213> Artificial
<220>
   <223> Synthetic peptide optionally bearing protecting groups.
<400> 394
   Phe His Asp Lys Phe Lys Glu Ala Val Lys Asp Tyr Phe Ala Lys Phe
   1 5 10 15
   Trp Asp
<210> 395
   <211> 18
   <212> PRT
   <213> Artificial
<220>
   <223> Synthetic peptide optionally bearing protecting groups.
<400> 395
   Phe His Glu Lys Phe Lys Asp Ala Val Lys Asp Tyr Phe Ala Lys Phe
   1 5 10 15
   Trp Asp
<210> 396
   <211> 18
   <212> PRT
   <213> Artificial
<220>
   <223> Synthetic peptide optionally bearing protecting groups.
<400> 396
   Phe His Glu Lys Phe Lys Glu Ala Val Lys Glu Tyr Phe Ala Lys Phe
   1 5 10 15
   Trp Asp
<210> 397
   <211> 18
   <212> PRT
   <213> Artificial
<220>
   <223> Synthetic peptide optionally bearing protecting groups.
<400> 397
   Phe His Glu Lys Phe Lys Glu Ala Val Lys Asp Tyr Phe Ala Lys Phe
   1 5 10 15
   Trp Glu
<210> 398
   <211> 18
   <212> PRT
   <213> Artificial
<220>
   <223> Synthetic peptide optionally bearing protecting groups.
<400> 398
   His Phe Glu Lys Phe Lys Glu Ala Val Lys Asp Tyr Phe Ala Lys Phe
   1 5 10 15
   Trp Asp
<210> 399
   <211> 18
   <212> PRT
   <213> Artificial
<220>
   <223> Synthetic peptide optionally bearing protecting groups.
<400> 399
   His Phe Asp Lys Phe Lys Asp Ala Val Lys Glu Tyr Phe Ala Lys Phe
   1 5 10 15
   Trp Glu
<210> 400
   <211> 18
   <212> PRT
   <213> Artificial
<220>
   <223> Synthetic peptide optionally bearing protecting groups.
<400> 400
   His Phe Glu Lys Phe Lys Glu Ala Val Lys Glu Tyr Phe Ala Lys Phe
   1 5 10 15
   Trp Glu
<210> 401
   <211> 18
   <212> PRT
   <213> Artificial
<220>
   <223> Synthetic peptide optionally bearing protecting groups.
<400> 401
   His Phe Asp Lys Phe Lys Glu Ala Val Lys Asp Tyr Phe Ala Lys Phe
   1 5 10 15
   Trp Asp
<210> 402
   <211> 18
   <212> PRT
   <213> Artificial
<220>
   <223> Synthetic peptide optionally bearing protecting groups.
<400> 402
   His Phe Glu Lys Phe Lys Asp Ala Val Lys Asp Tyr Phe Ala Lys Phe
   1 5 10 15
   Trp Asp
<210> 403
   <211> 18
   <212> PRT
   <213> Artificial
<220>
   <223> Synthetic peptide optionally bearing protecting groups.
<400> 403
   His Phe Glu Lys Phe Lys Glu Ala Val Lys Glu Tyr Phe Ala Lys Phe
   1 5 10 15
   Trp Asp
<210> 404
   <211> 18
   <212> PRT
   <213> Artificial
<220>
   <223> Synthetic peptide optionally bearing protecting groups.
<400> 404
   His Phe Glu Lys Phe Lys Glu Ala Val Lys Asp Tyr Phe Ala Lys Phe
   1 5 10 15
   Trp Glu
<210> 405
   <211> 18
   <212> PRT
   <213> Artificial
<220>
   <223> Synthetic peptide optionally bearing protecting groups.
<400> 405
   Phe Phe Glu Lys His Lys Glu Ala Val Lys Asp Tyr Phe Ala Lys Phe
   1 5 10 15
   Trp Asp
<210> 406
   <211> 18
   <212> PRT
   <213> Artificial
<220>
   <223> Synthetic peptide optionally bearing protecting groups.
<400> 406
   Phe Phe Asp Lys His Lys Asp Ala Val Lys Glu Tyr Phe Ala Lys Phe
   1               5                   10                  15
   Trp Glu
<210> 407
   <211> 18
   <212> PRT
   <213> Artificial
<220>
   <223> Synthetic peptide optionally bearing protecting groups.
<400> 407
   Phe Phe Glu Lys His Lys Glu Ala Val Lys Glu Tyr Phe Ala Lys Phe
   1               5                   10                  15
   Trp Glu
<210> 408
   <211> 18
   <212> PRT
   <213> Artificial
<220>
   <223> Synthetic peptide optionally bearing protecting groups.
<400> 408
   Phe Phe Asp Lys His Lys Asp Ala Val Lys Asp Tyr Phe Ala Lys Phe
   1               5                   10                  15
   Trp Asp
<210> 409
   <211> 18
   <212> PRT
   <213> Artificial
<220>
   <223> Synthetic peptide optionally bearing protecting groups.
<400> 409
   Phe Phe Asp Lys His Lys Glu Ala Val Lys Asp Tyr Phe Ala Lys Phe
   1               5                   10                  15
   Trp Asp
<210> 410
   <211> 18
   <212> PRT
   <213> Artificial
<220>
   <223> Synthetic peptide optionally bearing protecting groups.
<400> 410
   Phe Phe Glu Lys His Lys Glu Ala Val Lys Glu Tyr Phe Ala Lys Phe
   1               5                   10                  15
   Trp Asp
<210> 411
   <211> 18
   <212> PRT
   <213> Artificial
<220>
   <223> Synthetic peptide optionally bearing protecting groups.
<400> 411
   Phe Phe Glu Lys His Lys Glu Ala Val Lys Asp Tyr Phe Ala Lys Phe
   1               5                   10                  15
   Trp Glu
<210> 412
   <211> 18
   <212> PRT
   <213> Artificial
<220>
   <223> Synthetic peptide optionally bearing protecting groups.
<400> 412
   Phe Val Glu Lys Phe Lys Glu Ala His Lys Asp Tyr Phe Ala Lys Phe
   1               5                   10                  15
   Trp Asp
<210> 413
   <211> 18
   <212> PRT
   <213> Artificial
<220>
   <223> Synthetic peptide optionally bearing protecting groups.
<400> 413
   Phe Val Asp Lys Phe Lys Asp Ala His Lys Glu Tyr Phe Ala Lys Phe
   1 5 10 15
   Trp Glu
<210> 414
   <211> 18
   <212> PRT
   <213> Artificial
<220>
   <223> Synthetic peptide optionally bearing protecting groups.
<400> 414
   Phe Val Glu Lys Phe Lys Glu Ala His Lys Glu Tyr Phe Ala Lys Phe
   1 5 10 15
   Trp Glu
<210> 415
   <211> 18
   <212> PRT
   <213> Artificial
<220>
   <223> Synthetic peptide optionally bearing protecting groups.
<400> 415
   Phe Val Asp Lys Phe Lys Asp Ala His Lys Asp Tyr Phe Ala Lys Phe
   1 5 10 15
   Trp Asp
<210> 416
   <211> 18
   <212> PRT
   <213> Artificial
<220>
   <223> Synthetic peptide optionally bearing protecting groups.
<400> 416
   Phe Val Asp Lys Phe Lys Glu Ala His Lys Asp Tyr Phe Ala Lys Phe
   1 5 10 15
   Trp Asp
<210> 417
   <211> 18
   <212> PRT
   <213> Artificial
<220>
   <223> Synthetic peptide optionally bearing protecting groups.
<400> 417
   Phe Val Glu Lys Phe Lys Asp Ala His Lys Asp Tyr Phe Ala Lys Phe
   1               5                   10                  15
   Trp Asp
<210> 418
   <211> 18
   <212> PRT
   <213> Artificial
<220>
   <223> Synthetic peptide optionally bearing protecting groups.
<400> 418
   Phe Val Glu Lys Phe Lys Glu Ala His Lys Glu Tyr Phe Ala Lys Phe
   1               5                   10                  15
   Trp Asp
<210> 419
   <211> 18
   <212> PRT
   <213> Artificial
<220>
   <223> Synthetic peptide optionally bearing protecting groups.
<400> 419
   Phe Val Glu Lys Phe Lys Glu Ala His Lys Asp Tyr Phe Ala Lys Phe
   1               5                   10                  15
   Trp Glu
<210> 420
   <211> 18
   <212> PRT
   <213> Artificial
<220>
   <223> Synthetic peptide optionally bearing protecting groups.
<400> 420
   Phe Ala Glu Lys Phe Lys Glu His Val Lys Asp Tyr Phe Ala Lys Phe
   1               5                   10                  15
   Trp Asp
<210> 421
   <211> 18
   <212> PRT
   <213> Artificial
<220>
   <223> Synthetic peptide optionally bearing protecting groups.
<400> 421
   Phe Ala Asp Lys Phe Lys Asp His Val Lys Glu Tyr Phe Ala Lys Phe
   1               5                   10                  15
   Trp Glu
<210> 422
   <211> 18
   <212> PRT
   <213> Artificial
<220>
   <223> Synthetic peptide optionally bearing protecting groups.
<400> 422
   Phe Ala Glu Lys Phe Lys Glu His Val Lys Glu Tyr Phe Ala Lys Phe
   1               5                   10                  15
   Trp Glu
<210> 423
   <211> 18
   <212> PRT
   <213> Artificial
<220>
   <223> Synthetic peptide optionally bearing protecting groups.
<400> 423
   Phe Ala Asp Lys Phe Lys Asp His Val Lys Asp Tyr Phe Ala Lys Phe
   1               5                   10                  15
   Trp Asp
<210> 424
   <211> 18
   <212> PRT
   <213> Artificial
<220>
   <223> Synthetic peptide optionally bearing protecting groups.
<400> 424
   Phe Ala Asp Lys Phe Lys Glu His Val Lys Asp Tyr Phe Ala Lys Phe
   1 5 10 15
   Trp Asp
<210> 425
   <211> 18
   <212> PRT
   <213> Artificial
<220>
   <223> Synthetic peptide optionally bearing protecting groups.
<400> 425
   Phe Ala Glu Lys Phe Lys Asp His Val Lys Asp Tyr Phe Ala Lys Phe
   1 5 10 15
   Trp Asp
<210> 426
   <211> 18
   <212> PRT
   <213> Artificial
<220>
   <223> Synthetic peptide optionally bearing protecting groups.
<400> 426
   Phe Ala Glu Lys Phe Lys Glu His Val Lys Glu Tyr Phe Ala Lys Phe
   1 5 10 15
   Trp Asp
<210> 427
   <211> 18
   <212> PRT
   <213> Artificial
<220>
   <223> Synthetic peptide optionally bearing protecting groups.
<400> 427
   Phe Ala Glu Lys Phe Lys Glu His Val Lys Asp Tyr Phe Ala Lys Phe
   1 5 10 15
   Trp Glu
<210> 428
   <211> 18
   <212> PRT
   <213> Artificial
<220>
   <223> Synthetic peptide optionally bearing protecting groups.
<400> 428
   Phe Ala Glu Lys Phe Lys Glu Phe Val Lys Asp Tyr His Ala Lys Phe
   1 5 10 15
   Trp Asp
<210> 429
   <211> 18
   <212> PRT
   <213> Artificial
<220>
   <223> Synthetic peptide optionally bearing protecting groups.
<400> 429
   Phe Ala Asp Lys Phe Lys Asp Phe Val Lys Glu Tyr His Ala Lys Phe
   1 5 10 15
   Trp Glu
<210> 430
   <211> 18
   <212> PRT
   <213> Artificial
<220>
   <223> Synthetic peptide optionally bearing protecting groups.
<400> 430
   Phe Ala Asp Lys Phe Lys Glu Phe Val Lys Asp Tyr His Ala Lys Phe
   1 5 10 15
   Trp Asp
<210> 431
   <211> 18
   <212> PRT
   <213> Artificial
<220>
   <223> Synthetic peptide optionally bearing protecting groups.
<400> 431
   Phe Ala Glu Lys Phe Lys Asp Phe Val Lys Asp Tyr His Ala Lys Phe
   1 5 10 15
   Trp Asp
<210> 432
   <211> 18
   <212> PRT
   <213> Artificial
<220>
   <223> Synthetic peptide optionally bearing protecting groups.
<400> 432
   Phe Ala Asp Lys Phe Lys Asp Phe Val Lys Asp Tyr His Ala Lys Phe
   1 5 10 15
   Trp Asp
<210> 433
   <211> 18
   <212> PRT
   <213> Artificial
<220>
   <223> Synthetic peptide optionally bearing protecting groups.
<400> 433
   Phe Ala Glu Lys Phe Lys Glu Phe Val Lys Glu Tyr His Ala Lys Phe
   1 5 10 15
   Trp Glu
<210> 434
   <211> 18
   <212> PRT
   <213> Artificial
<220>
   <223> synthetic peptide optionally bearing protecting groups.
<400> 434
   Phe Ala Glu Lys Phe Lys Glu Phe Val Lys Glu Tyr His Ala Lys Phe
   1 5 10 15
   Trp Asp
<210> 435
   <211> 18
   <212> PRT
   <213> Artificial
<220>
   <223> Synthetic peptide optionally bearing protecting groups.
<400> 435
   Phe Ala Glu Lys Phe Lys Glu Phe Val Lys Asp Tyr His Ala Lys Phe
   1 5 10 15
   Trp Glu
<210> 436
   <211> 18
   <212> PRT
   <213> Artificial
<220>
   <223> Synthetic peptide optionally bearing protecting groups.
<400> 436
   Phe Ala Glu Lys Phe Lys Glu Phe Val Lys Asp Tyr Phe Ala Lys His
   1 5 10 15
   Trp Asp
<210> 437
   <211> 18
   <212> PRT
   <213> Artificial
<220>
   <223> Synthetic peptide optionally bearing protecting groups.
<400> 437
   Phe Ala Asp Lys Phe Lys Asp Phe Val Lys Glu Tyr Phe Ala Lys His
   1 5 10 15
   Trp Glu
<210> 438
   <211> 18
   <212> PRT
   <213> Artificial
<220>
   <223> Synthetic peptide optionally bearing protecting groups.
<400> 438
   Phe Ala Glu Lys Phe Lys Glu Phe Val Lys Glu Tyr Phe Ala Lys His
   1 5 10 15
   Trp Glu
<210> 439
   <211> 18
   <212> PRT
   <213> Artificial
<220>
   <223> Synthetic peptide optionally bearing protecting groups.
<400> 439
   Phe Ala Asp Lys Phe Lys Asp Phe Val Lys Asp Tyr Phe Ala Lys His
   1 5 10 15
   Trp Asp
<210> 440
   <211> 18
   <212> PRT
   <213> Artificial
<220>
   <223> Synthetic peptide optionally bearing protecting groups.
<400> 440
   Phe Ala Asp Lys Phe Lys Glu Phe Val Lys Asp Tyr Phe Ala Lys His
   1 5 10 15
   Trp Asp
<210> 441
   <211> 18
   <212> PRT
   <213> Artificial
<220>
   <223> Synthetic peptide optionally bearing protecting groups.
<400> 441
   Phe Ala Glu Lys Phe Lys Asp Phe Val Lys Asp Tyr Phe Ala Lys His
   1 5 10 15
   Trp Asp
<210> 442
   <211> 18
   <212> PRT
   <213> Artificial
<220>
   <223> Synthetic peptide optionally bearing protecting groups.
<400> 442
   Phe Ala Glu Lys Phe Lys Glu Phe Val Lys Glu Tyr Phe Ala Lys His
   1 5 10 15
   Trp Asp
<210> 443
   <211> 18
   <212> PRT
   <213> Artificial
<220>
   <223> Synthetic peptide optionally bearing protecting groups.
<400> 443
   Phe Ala Glu Lys Phe Lys Glu Phe Val Lys Asp Tyr Phe Ala Lys His
   1 5 10 15
   Trp Glu
<210> 444
   <211> 18
   <212> PRT
   <213> Artificial
<220>
   <223> Synthetic peptide optionally bearing protecting groups.
<220>
   <221> misc_feature
   <222> (3)..(3)
   <223> Xaa is Nph
<220>
   <221> misc_feature
   <222> (6)..(6)
   <223> Xaa is Nph
<220>
   <221> misc_feature
   <222> (14)..(14)
   <223> Xaa is Nph
<220>
   <221> misc_feature
   <222> (18)..(18)
   <223> Xaa is Nph
<400> 444
   Asp Trp Xaa Lys Ala Xaa Tyr Asp Lys Val Ala Glu Lys Xaa Lys Glu
   1 5 10 15
   Ala Xaa
<210> 445
   <211> 18
   <212> PRT
   <213> Artificial
<220>
   <223> Synthetic peptide optionally bearing protecting groups.
<220>
   <221> misc_feature
   <222> (3)..(3)
   <223> Xaa is Nph
<220>
   <221> misc_feature
   <222> (6)..(6)
   <223> Xaa is Nph
<220>
   <221> misc_feature
   <222> (14)..(14)
   <223> Xaa is Nph
<220>
   <221> misc_feature
   <222> (18)..(18)
   <223> Xaa is Nph
<400> 445
   Glu Trp Xaa Lys Ala Xaa Tyr Glu Lys Val Ala Asp Lys Xaa Lys Asp
   1               5                   10                  15
   Ala Xaa
<210> 446
   <211> 18
   <212> PRT
   <213> Artificial
<220>
   <223> Synthetic peptide optionally bearing protecting groups.
<220>
   <221> misc_feature
   <222> (3)..(3)
   <223> Xaa is Nph
<220>
   <221> misc_feature
   <222> (6)..(6)
   <223> Xaa is Nph
<220>
   <221> misc_feature
   <222> (14)..(14)
   <223> Xaa is Nph
<220>
   <221> misc_feature
   <222> (18)..(18)
   <223> Xaa is Nph
<400> 446
   Glu Trp Xaa Lys Ala Xaa Tyr Glu Lys Val Ala Glu Lys Xaa Lys Glu
   1 5 10 15
   Ala Xaa
<210> 447
   <211> 18
   <212> PRT
   <213> Artificial
<220>
   <223> Synthetic peptide optionally bearing protecting groups.
<220>
   <221> misc_feature
   <222> (3)..(3)
   <223> Xaa is Nph
<220>
   <221> misc_feature
   <222> (6)..(6)
   <223> Xaa is Nph
<220>
   <221> misc_feature
   <222> (14)..(14)
   <223> Xaa is Nph
<220>
   <221> misc_feature
   <222> (18)..(18)
   <223> Xaa is Nph
<400> 447
   Asp Trp Xaa Lys Ala Xaa Tyr Asp Lys Val Ala Asp Lys Xaa Lys Asp
   1 5 10 15
   Ala Xaa
<210> 448
   <211> 18
   <212> PRT
   <213> Artificial
<220>
   <223> Synthetic peptide optionally bearing protecting groups.
<220>
   <221> misc_feature
   <222> (3)..(3)
   <223> Xaa is Nph
<220>
   <221> misc_feature
   <222> (6)..(6)
   <223> Xaa is Nph
<220>
   <221> misc_feature
   <222> (14)..(14)
   <223> Xaa is Nph
<220>
   <221> misc_feature
   <222> (18)..(18)
   <223> Xaa is Nph
<400> 448
   Glu Trp Xaa Lys Ala Xaa Tyr Asp Lys Val Ala Glu Lys Xaa Lys Glu
   1               5                   10                  15
   Ala Xaa
<210> 449
   <211> 18
   <212> PRT
   <213> Artificial
<220>
   <223> Synthetic peptide optionally bearing protecting groups.
<220>
   <221> misc_feature
   <222> (3)..(3)
   <223> Xaa is Nph
<220>
   <221> misc_feature
   <222> (6)..(6)
   <223> Xaa is Nph
<220>
   <221> misc_feature
   <222> (14)..(14)
   <223> Xaa is Nph
<220>
   <221> misc_feature
   <222> (18)..(18)
   <223> Xaa is Nph
<400> 449
   Asp Trp Xaa Lys Ala Xaa Tyr Glu Lys Val Ala Glu Lys Xaa Lys Glu
   1               5                   10                  15
   Ala Xaa
<210> 450
   <211> 18
   <212> PRT
   <213> Artificial
<220>
   <223> Synthetic peptide optionally bearing protecting groups.
<220>
   <221> misc_feature
   <222> (3)..(3)
   <223> Xaa is Nph
<220>
   <221> misc_feature
   <222> (6)..(6)
   <223> Xaa is Nph
<220>
   <221> misc_feature
   <222> (14)..(14)
   <223> Xaa is Nph
<220>
   <221> misc_feature
   <222> (18)..(18)
   <223> Xaa is Nph
<400> 450
   Asp Trp Xaa Lys Ala Xaa Tyr Asp Lys Val Ala Asp Lys Xaa Lys Glu
   1               5                   10                  15
   Ala Xaa

<210> 451
   <211> 18
   <212> PRT
   <213> Artificial
<220>
   <223> Synthetic peptide optionally bearing protecting groups.
<220>
   <221> misc_feature
   <222> (3)..(3)
   <223> Xaa is Nph
<220>
   <221> misc_feature
   <222> (6)..(6)
   <223> Xaa is Nph
<220>
   <221> misc_feature
   <222> (14)..(14)
   <223> Xaa is Nph
<220>
   <221> misc_feature
   <222> (18)..(18)
   <223> Xaa is Nph
<400> 451
   Asp Trp Xaa Lys Ala Xaa Tyr Asp Lys Val Ala Glu Lys Xaa Lys Asp
   1 5 10 15
   Ala Xaa
<210> 452
   <211> 18
   <212> PRT
   <213> Artificial
<220>
   <223> Synthetic peptide optionally bearing protecting groups.
<220>
   <221> misc_feature
   <222> (3)..(3)
   <223> Xaa is Nph
<220>
   <221> misc_feature
   <222> (6)..(6)
   <223> Xaa is Nph
<400> 452
   Asp Trp Xaa Lys Ala Xaa Tyr Asp Lys Val Ala Glu Lys Phe Lys Glu
   1 5 10 15
   Ala Phe
<210> 453
   <211> 18
   <212> PRT
   <213> Artificial
<220>
   <223> Synthetic peptide optionally bearing protecting groups.
<220>
   <221> misc_feature
   <222> (14)..(14)
   <223> Xaa is Nph
<220>
   <221> misc_feature
   <222> (18)..(18)
   <223> Xaa is Nph
<400> 453
   Asp Trp Phe Lys Ala Phe Tyr Asp Lys Val Ala Glu Lys Xaa Lys Glu
   1 5 10 15
   Ala Xaa
<210> 454
   <211> 18
   <212> PRT
   <213> Artificial
<220>
   <223> Synthetic peptide optionally bearing protecting groups.
<220>
   <221> misc_feature
   <222> (3)..(3)
   <223> Xaa is Nph
<400> 454
   Asp Trp Xaa Lys Ala Phe Tyr Asp Lys Val Ala Glu Lys Phe Lys Glu
   1 5 10 15
   Ala Phe
<210> 455
   <211> 18
   <212> PRT
   <213> Artificial
<220>
   <223> Synthetic peptide optionally bearing protecting groups.
<220>
   <221> misc_feature
   <222> (6)..(6)
   <223> Xaa is Nph
<400> 455
   Asp Trp Phe Lys Ala Xaa Tyr Asp Lys Val Ala Glu Lys Phe Lys Glu
   1 5 10 15
   Ala Phe
<210> 456
   <211> 18
   <212> PRT
   <213> Artificial
<220>
   <223> Synthetic peptide optionally bearing protecting groups.
<220>
   <221> misc_feature
   <222> (14)..(14)
   <223> Xaa is Nph
<400> 456
   Asp Trp Phe Lys Ala Phe Tyr Asp Lys Val Ala Glu Lys Xaa Lys Glu
   1 5 10 15
   Ala Phe
<210> 457
   <211> 18
   <212> PRT
   <213> Artificial
<220>
   <223> Synthetic peptide optionally bearing protecting groups.
<220>
   <221> misc_feature
   <222> (18)..(18)
   <223> Xaa is Nph
<400> 457
   Asp Trp Phe Lys Ala Phe Tyr Asp Lys Val Ala Glu Lys Phe Lys Glu
   1 5 10 15
   Ala Xaa
<210> 458
   <211> 18
   <212> PRT
   <213> Artificial
<220>
   <223> Synthetic peptide optionally bearing protecting groups.
<220>
   <221> misc_feature
   <222> (1)..(1)
   <223> Xaa is Nph
<220>
   <221> misc_feature
   <222> (5)..(5)
   <223> Xaa is Nph
<220>
   <221> misc_feature
   <222> (13)..(13)
   <223> Xaa is Nph
<220>
   <221> misc_feature
   <222> (16)..(16)
   <223> Xaa is Nph
<400> 458
   Xaa Ala Glu Lys Xaa Lys Glu Ala Val Lys Asp Tyr Xaa Ala Lys Xaa
   1 5 10 15
   Trp Asp
<210> 459
   <211> 18
   <212> PRT
   <213> Artificial
<220>
   <223> Synthetic peptide optionally bearing protecting groups.
<220>
   <221> misc_feature
   <222> (1)..(1)
   <223> Xaa is Nph
<220>
   <221> misc_feature
   <222> (5)..(5)
   <223> Xaa is Nph
<400> 459
   Xaa Ala Glu Lys Xaa Lys Glu Ala Val Lys Asp Tyr Phe Ala Lys Phe
   1 5 10 15
   Trp Asp
<210> 460
   <211> 18
   <212> PRT
   <213> Artificial
<220>
   <223> Synthetic peptide optionally bearing protecting groups.
<220>
   <221> misc_feature
   <222> (13)..(13)
   <223> Xaa is Nph
<220>
   <221> misc_feature
   <222> (16)..(16)
   <223> Xaa is Nph
<400> 460
   Phe Ala Glu Lys Phe Lys Glu Ala Val Lys Asp Tyr Xaa Ala Lys Xaa
   1 5 10 15
   Trp Asp
<210> 461
   <211> 18
   <212> PRT
   <213> Artificial
<220>
   <223> Synthetic peptide optionally bearing protecting groups.
<220>
   <221> misc_feature
   <222> (1)..(1)
   <223> Xaa is Nph
<400> 461
   Xaa Ala Glu Lys Phe Lys Glu Ala Val Lys Asp Tyr Phe Ala Lys Phe
   1 5 10 15
   Trp Asp
<210> 462
   <211> 18
   <212> PRT
   <213> Artificial
<220>
   <223> Synthetic peptide optionally bearing protecting groups.
<220>
   <221> misc_feature
   <222> (5)..(5)
   <223> Xaa is Nph
<400> 462
   Phe Ala Glu Lys Xaa Lys Glu Ala Val Lys Asp Tyr Phe Ala Lys Phe
   1 5 10 15
   Trp Asp
<210> 463
   <211> 18
   <212> PRT
   <213> Artificial
<220>
   <223> Synthetic peptide optionally bearing protecting groups.
<220>
   <221> misc_feature
   <222> (13)..(13)
   <223> Xaa is Nph
<400> 463
   Phe Ala Glu Lys Phe Lys Glu Ala Val Lys Asp Tyr Xaa Ala Lys Phe
   1 5 10 15
   Trp Asp
<210> 464
   <211> 18
   <212> PRT
   <213> Artificial
<220>
   <223> Synthetic peptide optionally bearing protecting groups.
<220>
   <221> misc_feature
   <222> (16)..(16)
   <223> Xaa is Nph
<400> 464
   Phe Ala Glu Lys Phe Lys Glu Ala Val Lys Asp Tyr Phe Ala Lys Xaa
   1 5 10 15
   Trp Asp
<210> 465
   <211> 18
   <212> PRT
   <213> Artificial
<220>
   <223> Synthetic peptide optionally bearing protecting groups.
<400> 465
   Phe Ala Glu Lys Phe Lys Glu Ala Val Lys Glu Tyr Phe Ala Lys Phe
   1 5 10 15
   Trp Glu
<210> 466
   <211> 18
   <212> PRT
   <213> Artificial
<220>
   <223> Synthetic peptide optionally bearing protecting groups.
<400> 466
   Phe Ala Asp Lys Phe Lys Asp Ala Val Lys Asp Tyr Phe Ala Lys Phe
   1 5 10 15
   Trp Asp
<210> 467
   <211> 18
   <212> PRT
   <213> Artificial
<220>
   <223> Synthetic peptide optionally bearing protecting groups.
<400> 467
   Phe Ala Glu Arg Phe Arg Glu Ala Val Lys Asp Tyr Phe Ala Lys Phe
   1 5 10 15
   Trp Asp
<210> 468
   <211> 18
   <212> PRT
   <213> Artificial
<220>
   <223> Synthetic peptide optionally bearing protecting groups.
<400> 468
   Phe Ala Glu Lys Phe Arg Glu Ala Val Lys Asp Tyr Phe Ala Lys Phe
   1 5 10 15
   Trp Asp
<210> 469
   <211> 18
   <212> PRT
   <213> Artificial
<220>
   <223> Synthetic peptide optionally bearing protecting groups.
<400> 469
   Phe Ala Glu Lys Phe Lys Glu Ala Val Arg Asp Tyr Phe Ala Lys Phe
   1 5 10 15
   Trp Asp
<210> 470
   <211> 18
   <212> PRT
   <213> Artificial
<220>
   <223> Synthetic peptide optionally bearing protecting groups.
<400> 470
   Phe Ala Glu Lys Phe Lys Glu Ala Val Lys Asp Tyr Phe Ala Arg Phe
   1               5                   10                  15
   Trp Asp
<210> 471
   <211> 18
   <212> PRT
   <213> Artificial
<220>
   <223> Synthetic peptide optionally bearing protecting groups.
<400> 471
   Phe Ala Glu Lys Phe Lys Glu Ala Val Lys Glu Tyr Phe Ala Lys Phe
   1               5                   10                  15
   Trp Glu
<210> 472
   <211> 18
   <212> PRT
   <213> Artificial
<220>
   <223> synthetic peptide optionally bearing protecting groups.
<400> 472
   Phe Ala Asp Lys Phe Lys Asp Ala Val Lys Asp Tyr Phe Ala Lys Phe
   1               5                   10                  15
   Trp Asp
<210> 473
   <211> 18
   <212> PRT
   <213> Artificial
<220>
   <223> Synthetic peptide optionally bearing protecting groups.
<400> 473
   Phe Ala Glu Arg Phe Arg Glu Ala Val Lys Asp Tyr Phe Ala Lys Phe
   1 5 10 15
   Trp Asp
<210> 474
   <211> 18
   <212> PRT
   <213> Artificial
<220>
   <223> Synthetic peptide optionally bearing protecting groups.
<400> 474
   Phe Ala Glu Lys Phe Arg Glu Ala Val Lys Asp Tyr Phe Ala Lys Phe
   1 5 10 15
   Trp Asp
<210> 475
   <211> 18
   <212> PRT
   <213> Artificial
<220>
   <223> Synthetic peptide optionally bearing protecting groups.
<400> 475
   Phe Ala Glu Lys Phe Lys Glu Ala Val Arg Asp Tyr Phe Ala Lys Phe
   1 5 10 15
   Trp Asp
<210> 476
   <211> 18
   <212> PRT
   <213> Artificial
<220>
   <223> Synthetic peptide optionally bearing protecting groups.
<400> 476
   Phe Ala Glu Lys Phe Lys Glu Ala Val Lys Asp Tyr Phe Ala Arg Phe
   1 5 10 15
   Trp Asp
<210> 477
   <211> 18
   <212> PRT
   <213> Artificial
<220>
   <223> Synthetic peptide optionally bearing protecting groups.
<400> 477
   Phe Ala Glu Lys Phe Lys Glu Ala Val Lys Glu Tyr Phe Ala Lys Phe
   1 5 10 15
   Trp Glu
<210> 478
   <211> 18
   <212> PRT
   <213> Artificial
<220>
   <223> Synthetic peptide optionally bearing protecting groups.
<400> 478
   Phe Ala Asp Lys Phe Lys Asp Ala Val Lys Asp Tyr Phe Ala Lys Phe
   1 5 10 15
   Trp Asp
<210> 479
   <211> 18
   <212> PRT
   <213> Artificial
<220>
   <223> Synthetic peptide optionally bearing protecting groups.
<400> 479
   Phe Ala Glu Arg Phe Arg Glu Ala Val Lys Asp Tyr Phe Ala Lys Phe
   1 5 10 15
   Trp Asp
<210> 480
   <211> 18
   <212> PRT
   <213> Artificial
<220>
   <223> Synthetic peptide optionally bearing protecting groups.
<400> 480
   Phe Ala Glu Lys Phe Arg Glu Ala Val Lys Asp Tyr Phe Ala Lys Phe
   1 5 10 15
   Trp Asp
<210> 481
   <211> 18
   <212> PRT
   <213> Artificial
<220>
   <223> Synthetic peptide optionally bearing protecting groups.
<400> 481
   Phe Ala Glu Lys Phe Lys Glu Ala Val Arg Asp Tyr Phe Ala Lys Phe
   1               5                   10                  15
   Trp Asp
<210> 482
   <211> 18
   <212> PRT
   <213> Artificial
<220>
   <223> Synthetic peptide optionally bearing protecting groups.
<400> 482
   Phe Ala Glu Lys Phe Lys Glu Ala Val Lys Asp Tyr Phe Ala Arg Phe
   1               5                   10                  15
   Trp Asp
<210> 483
   <211> 18
   <212> PRT
   <213> Artificial
<220>
   <223> Synthetic peptide optionally bearing protecting groups.
<400> 483
   Phe Ala Glu Arg Phe Arg Glu Ala Val Lys Asp Tyr Phe Ala Lys Phe
   1               5                   10                  15
   Trp Asp
<210> 484
   <211> 18
   <212> PRT
   <213> Artificial
<220>
   <223> Synthetic peptide optionally bearing protecting groups.
<400> 484
   Phe Ala Glu Lys Phe Arg Glu Ala Val Lys Asp Tyr Phe Ala Lys Phe
   1 5 10 15
   Trp Asp
<210> 485
   <211> 18
   <212> PRT
   <213> Artificial
<220>
   <223> Synthetic peptide optionally bearing protecting groups.
<400> 485
   Phe Ala Glu Lys Phe Lys Glu Ala Val Arg Asp Tyr Phe Ala Lys Phe
   1 5 10 15
   Trp Asp
<210> 486
   <211> 18
   <212> PRT
   <213> Artificial
<220>
   <223> Synthetic peptide optionally bearing protecting groups.
<400> 486
   Phe Ala Glu Lys Phe Lys Glu Ala Val Lys Asp Tyr Phe Ala Arg Phe
   1 5 10 15
   Trp Asp
<210> 487
   <211> 18
   <212> PRT
   <213> Artificial
<220>
   <223> Synthetic peptide optionally bearing protecting groups.
<400> 487
   Phe Ala Glu Lys Phe Lys Glu Ala Val Lys Glu Tyr Phe Ala Lys Phe
   1 5 10 15
   Trp Glu
<210> 488
   <211> 18
   <212> PRT
   <213> Artificial
<220>
   <223> Synthetic peptide optionally bearing protecting groups.
<400> 488
   Phe Ala Asp Lys Phe Lys Asp Ala Val Lys Asp Tyr Phe Ala Lys Phe
   1 5 10 15
   Trp Asp
<210> 489
   <211> 18
   <212> PRT
   <213> Artificial
<220>
   <223> Synthetic peptide optionally bearing protecting groups.
<400> 489
   Phe Ala Glu Arg Phe Arg Glu Ala Val Lys Asp Tyr Phe Ala Lys Phe
   1 5 10 15
   Trp Asp
<210> 490
   <211> 18
   <212> PRT
   <213> Artificial
<220>
   <223> Synthetic peptide optionally bearing protecting groups.
<400> 490
   Phe Ala Glu Lys Phe Arg Glu Ala Val Lys Asp Tyr Phe Ala Lys Phe
   1 5 10 15
   Trp Asp
<210> 491
   <211> 18
   <212> PRT
   <213> Artificial
<220>
   <223> Synthetic peptide optionally bearing protecting groups.
<400> 491
   Phe Ala Glu Lys Phe Lys Glu Ala Val Arg Asp Tyr Phe Ala Lys Phe
   1 5 10 15
   Trp Asp
<210> 492
   <211> 18
   <212> PRT
   <213> Artificial
<220>
   <223> Synthetic peptide optionally bearing protecting groups.
<400> 492
   Phe Ala Glu Lys Phe Lys Glu Ala Val Lys Asp Tyr Phe Ala Arg Phe
   1 5 10 15
   Trp Asp
<210> 493
   <211> 18
   <212> PRT
   <213> Artificial
<220>
   <223> Synthetic peptide optionally bearing protecting groups.
<400> 493
   Leu Phe Glu Lys Phe Ala Glu Ala Phe Lys Asp Tyr Val Ala Lys Trp
   1 5 10 15
   Lys Asp
<210> 494
   <211> 18
   <212> PRT
   <213> Artificial
<220>
   <223> Synthetic peptide optionally bearing protecting groups.
<400> 494
   Leu Phe Glu Arg Phe Ala Glu Ala Phe Lys Asp Tyr Val Ala Lys Trp
   1 5 10 15
   Lys Asp
<210> 495
   <211> 18
   <212> PRT
   <213> Artificial
<220>
   <223> Synthetic peptide optionally bearing protecting groups.
<400> 495
   Leu Phe Glu Lys Phe Ala Glu Ala Phe Arg Asp Tyr Val Ala Lys Trp
   1 5 10 15
   Lys Asp
<210> 496
   <211> 18
   <212> PRT
   <213> Artificial
<220>
   <223> Synthetic peptide optionally bearing protecting groups.
<400> 496
   Leu Phe Glu Lys Phe Ala Glu Ala Phe Lys Asp Tyr Val Ala Arg Trp
   1 5 10 15
   Lys Asp
<210> 497
   <211> 18
   <212> PRT
   <213> Artificial
<220>
   <223> Synthetic peptide optionally bearing protecting groups.
<400> 497
   Leu Phe Glu Lys Phe Ala Glu Ala Phe Lys Asp Tyr Val Ala Lys Trp
   1 5 10 15
   Arg Asp
<210> 498
   <211> 18
   <212> PRT
   <213> Artificial
<220>
   <223> Synthetic peptide optionally bearing protecting groups.
<400> 498
   Leu Phe Glu Lys Phe Ala Glu Ala Phe Lys Glu Tyr Val Ala Lys Trp
   1 5 10 15
   Lys Glu
<210> 499
   <211> 18
   <212> PRT
   <213> Artificial
<220>
   <223> Synthetic peptide optionally bearing protecting groups.
<400> 499
   Leu Phe Asp Lys Phe Ala Asp Ala Phe Lys Asp Tyr Val Ala Lys Trp
   1 5 10 15
   Lys Asp
<210> 500
   <211> 18
   <212> PRT
   <213> Artificial
<220>
   <223> Synthetic peptide optionally bearing protecting groups.
<400> 500
   Leu Phe Asp Lys Phe Ala Glu Ala Phe Lys Asp Tyr Val Ala Lys Trp
   1 5 10 15
   Lys Asp
<210> 501
   <211> 18
   <212> PRT
   <213> Artificial
<220>
   <223> Synthetic peptide optionally bearing protecting groups.
<400> 501
   Leu Phe Glu Lys Phe Ala Asp Ala Phe Lys Asp Tyr Val Ala Lys Trp
   1 5 10 15
   Lys Asp
<210> 502
   <211> 18
   <212> PRT
   <213> Artificial
<220>
   <223> Synthetic peptide optionally bearing protecting groups.
<400> 502
   Leu Phe Glu Lys Phe Ala Glu Ala Phe Lys Glu Tyr Val Ala Lys Trp
   1 5 10 15
   Lys Asp
<210> 503
   <211> 18
   <212> PRT
   <213> Artificial
<220>
   <223> Synthetic peptide optionally bearing protecting groups.
<400> 503
   Leu Phe Glu Lys Phe Ala Glu Ala Phe Lys Asp Tyr Val Ala Lys Trp
   1 5 10 15
   Lys Glu
<210> 504
   <211> 18
   <212> PRT
   <213> Artificial
<220>
   <223> Synthetic peptide optionally bearing protecting groups.
<400> 504
   Phe Ala Glu Lys Ala Trp Glu Phe Val Lys Asp Tyr Phe Ala Lys Leu
   1 5 10 15
   Lys Asp
<210> 505
   <211> 18
   <212> PRT
   <213> Artificial
<220>
   <223> Synthetic peptide optionally bearing protecting groups.
<400> 505
   Phe Ala Glu Arg Ala Trp Glu Phe Val Lys Asp Tyr Phe Ala Lys Leu
   1 5 10 15
   Lys Asp
<210> 506
   <211> 18
   <212> PRT
   <213> Artificial
<220>
   <223> Synthetic peptide optionally bearing protecting groups.
<400> 506
   Phe Ala Glu Lys Ala Trp Glu Phe Val Lys Asp Tyr Phe Ala Lys Leu
   1               5                   10                  15
   Lys Asp
<210> 507
   <211> 18
   <212> PRT
   <213> Artificial
<220>
   <223> Synthetic peptide optionally bearing protecting groups.
<400> 507
   Phe Ala Glu Lys Ala Trp Glu Phe Val Lys Asp Tyr Phe Ala Lys Leu
   1               5                   10                  15
   Lys Asp
<210> 508
   <211> 18
   <212> PRT
   <213> Artificial
<220>
   <223> Synthetic peptide optionally bearing protecting groups.
<400> 508
   Phe Ala Glu Lys Ala Trp Glu Phe Val Lys Asp Tyr Phe Ala Lys Leu
   1               5                   10                  15
   Arg Asp
<210> 509
   <211> 18
   <212> PRT
   <213> Artificial
<220>
   <223> Synthetic peptide optionally bearing protecting groups.
<400> 509
   Phe Ala Glu Lys Ala Trp Glu Phe Val Lys Glu Tyr Phe Ala Lys Leu
   1               5                   10                  15
   Lys Glu
<210> 510
   <211> 18
   <212> PRT
   <213> Artificial
<220>
   <223> Synthetic peptide optionally bearing protecting groups.
<400> 510
   Phe Ala Asp Lys Ala Trp Asp Phe Val Lys Asp Tyr Phe Ala Lys Leu
   1               5                   10                  15
   Lys Asp
<210> 511
   <211> 18
   <212> PRT
   <213> Artificial
<220>
   <223> Synthetic peptide optionally bearing protecting groups.
<400> 511
   Phe Ala Asp Lys Ala Trp Glu Phe Val Lys Asp Tyr Phe Ala Lys Leu
   1               5                   10                  15
   Lys Asp
<210> 512
   <211> 18
   <212> PRT
   <213> Artificial
<220>
   <223> Synthetic peptide optionally bearing protecting groups.
<400> 512
   Phe Ala Glu Lys Ala Trp Asp Phe Val Lys Asp Tyr Phe Ala Lys Leu
   1               5                   10                  15
   Lys Asp
<210> 513
   <211> 18
   <212> PRT
   <213> Artificial
<220>
   <223> Synthetic peptide optionally bearing protecting groups.
<400> 513
   Phe Ala Glu Lys Ala Trp Glu Phe Val Lys Glu Tyr Phe Ala Lys Leu
   1 5 10 15
   Lys Asp
<210> 514
   <211> 18
   <212> PRT
   <213> Artificial
<220>
   <223> Synthetic peptide optionally bearing protecting groups.
<400> 514
   Phe Ala Glu Lys Ala Trp Glu Phe Val Lys Asp Tyr Phe Ala Lys Leu
   1 5 10 15
   Lys Glu
<210> 515
   <211> 18
   <212> PRT
   <213> Artificial
<220>
   <223> Synthetic peptide optionally bearing protecting groups.
<400> 515
   Phe Phe Glu Lys Phe Lys Glu Phe Val Lys Asp Tyr Phe Ala Lys Leu
   1 5 10 15
   Trp Asp
<210> 516
   <211> 18
   <212> PRT
   <213> Artificial
<220>
   <223> Synthetic peptide optionally bearing protecting groups.
<400> 516
   Phe Phe Glu Lys Phe Lys Glu Phe Val Lys Glu Tyr Phe Ala Lys Leu
   1 5 10 15
   Trp Glu
<210> 517
   <211> 18
   <212> PRT
   <213> Artificial
<220>
   <223> Synthetic peptide optionally bearing protecting groups.
<400> 517
   Phe Phe Asp Lys Phe Lys Asp Phe Val Lys Asp Tyr Phe Ala Lys Leu
   1 5 10 15
   Trp Asp
<210> 518
   <211> 18
   <212> PRT
   <213> Artificial
<220>
   <223> Synthetic peptide optionally bearing protecting groups.
<400> 518
   Phe Phe Glu Arg Phe Lys Glu Phe Val Lys Asp Tyr Phe Ala Lys Leu
   1 5 10 15
   Trp Asp
<210> 519
   <211> 18
   <212> PRT
   <213> Artificial
<220>
   <223> Synthetic peptide optionally bearing protecting groups.
<400> 519
   Phe Phe Glu Lys Phe Arg Glu Phe Val Lys Asp Tyr Phe Ala Lys Leu
   1 5 10 15
   Trp Asp
<210> 520
   <211> 18
   <212> PRT
   <213> Artificial
<220>
   <223> Synthetic peptide optionally bearing protecting groups.

<400> 520
   Phe Phe Glu Lys Phe Lys Glu Phe Val Arg Asp Tyr Phe Ala Lys Leu
   1 5 10 15
   Trp Asp
<210> 521
   <211> 18
   <212> PRT
   <213> Artificial
<220>
   <223> Synthetic peptide optionally bearing protecting groups.
<400> 521
   Phe Phe Glu Lys Phe Lys Glu Phe Val Lys Asp Tyr Phe Ala Arg Leu
   1 5 10 15
   Trp Asp
<210> 522
   <211> 18
   <212> PRT
   <213> Artificial
<220>
   <223> Synthetic peptide optionally bearing protecting groups.
<400> 522
   Phe Phe Asp Lys Phe Lys Glu Phe Val Lys Asp Tyr Phe Ala Lys Leu
   1 5 10 15
   Trp Asp
<210> 523
   <211> 18
   <212> PRT
   <213> Artificial
<220>
   <223> Synthetic peptide optionally bearing protecting groups.
<400> 523
   Phe Phe Glu Lys Phe Lys Asp Phe Val Lys Asp Tyr Phe Ala Lys Leu
   1 5 10 15
   Trp Asp
<210> 524
   <211> 18
   <212> PRT
   <213> Artificial
<220>
   <223> Synthetic peptide optionally bearing protecting groups.
<400> 524
   Phe Phe Glu Lys Phe Lys Glu Phe Val Lys Glu Tyr Phe Ala Lys Leu
   1 5 10 15
   Trp Asp
<210> 525
   <211> 18
   <212> PRT
   <213> Artificial
<220>
   <223> Synthetic peptide optionally bearing protecting groups.
<400> 525
   Phe Phe Glu Lys Phe Lys Glu Phe Val Lys Asp Tyr Phe Ala Lys Leu
   1 5 10 15
   Trp Glu
<210> 526
   <211> 18
   <212> PRT
   <213> Artificial
<220>
   <223> Synthetic peptide optionally bearing protecting groups.
<400> 526
   Phe Leu Glu Lys Phe Lys Glu Phe Val Lys Asp Tyr Phe Ala Lys Phe
   1 5 10 15
   Trp Asp
<210> 527
   <211> 18
   <212> PRT
   <213> Artificial
<220>
   <223> Synthetic peptide optionally bearing protecting groups.
<400> 527
   Phe Leu Glu Lys Phe Lys Glu Phe Val Lys Glu Tyr Phe Ala Lys Phe
   1 5 10 15
   Trp Glu
<210> 528
   <211> 18
   <212> PRT
   <213> Artificial
<220>
   <223> Synthetic peptide optionally bearing protecting groups.
<400> 528
   Phe Leu Asp Lys Phe Lys Glu Phe Val Lys Asp Tyr Phe Ala Lys Phe
   1 5 10 15
   Trp Asp
<210> 529
   <211> 18
   <212> PRT
   <213> Artificial
<220>
   <223> Synthetic peptide optionally bearing protecting groups.
<400> 529
   Phe Leu Asp Lys Phe Lys Glu Phe Val Lys Asp Tyr Phe Ala Lys Phe
   1 5 10 15
   Trp Asp
<210> 530
   <211> 18
   <212> PRT
   <213> Artificial
<220>
   <223> Synthetic peptide optionally bearing protecting groups.
<400> 530
   Phe Leu Glu Lys Phe Lys Asp Phe Val Lys Asp Tyr Phe Ala Lys Phe
   1 5 10 15
   Trp Asp
<210> 531
   <211> 18
   <212> PRT
   <213> Artificial
<220>
   <223> Synthetic peptide optionally bearing protecting groups.
<400> 531
   Phe Leu Glu Lys Phe Lys Glu Phe Val Lys Glu Tyr Phe Ala Lys Phe
   1 5 10 15
   Trp Asp
<210> 532
   <211> 18
   <212> PRT
   <213> Artificial
<220>
   <223> Synthetic peptide optionally bearing protecting groups.
<400> 532
   Phe Leu Glu Lys Phe Lys Glu Phe Val Lys Asp Tyr Phe Ala Lys Phe
   1 5 10 15
   Trp Glu
<210> 533
   <211> 18
   <212> PRT
   <213> Artificial
<220>
   <223> Synthetic peptide optionally bearing protecting groups.
<400> 533
   Phe Leu Glu Arg Phe Lys Glu Phe Val Lys Asp Tyr Phe Ala Lys Phe
   1 5 10 15
   Trp Asp
<210> 534
   <211> 18
   <212> PRT
   <213> Artificial
<220>
   <223> Synthetic peptide optionally bearing protecting groups.
<400> 534
   Phe Leu Glu Lys Phe Arg Glu Phe Val Lys Asp Tyr Phe Ala Lys Phe
   1 5 10 15
   Trp Asp
<210> 535
   <211> 18
   <212> PRT
   <213> Artificial
<220>
   <223> Synthetic peptide optionally bearing protecting groups.
<400> 535
   Phe Leu Glu Lys Phe Lys Glu Phe Val Arg Asp Tyr Phe Ala Lys Phe
   1 5 10 15
   Trp Asp
<210> 536
   <211> 18
   <212> PRT
   <213> Artificial
<220>
   <223> Synthetic peptide optionally bearing protecting groups.
<400> 536
   Phe Leu Glu Lys Phe Lys Glu Phe Val Lys Asp Tyr Phe Ala Arg Phe
   1 5 10 15
   Trp Asp
<210> 537
   <211> 18
   <212> PRT
   <213> Artificial
<220>
   <223> Synthetic peptide optionally bearing protecting groups.
<400> 537
   Phe Phe Glu Lys Phe Lys Glu Phe Phe Lys Asp Tyr Phe Ala Lys Leu
   1 5 10 15
   Trp Asp
<210> 538
   <211> 18
   <212> PRT
   <213> Artificial
<220>
   <223> Synthetic peptide optionally bearing protecting groups.
<400> 538
   Phe Phe Glu Lys Phe Lys Glu Phe Phe Lys Glu Tyr Phe Ala Lys Leu
   1 5 10 15
   Trp Glu
<210> 539
   <211> 18
   <212> PRT
   <213> Artificial
<220>
   <223> Synthetic peptide optionally bearing protecting groups.
<400> 539
   Phe Phe Asp Lys Phe Lys Asp Phe Phe Lys Asp Tyr Phe Ala Lys Leu
   1 5 10 15
   Trp Asp
<210> 540
   <211> 18
   <212> PRT
   <213> Artificial
<220>
   <223> Synthetic peptide optionally bearing protecting groups.
<400> 540
   Phe Phe Glu Arg Phe Lys Glu Phe Phe Lys Asp Tyr Phe Ala Lys Leu
   1 5 10 15
   Trp Asp
<210> 541
   <211> 18
   <212> PRT
   <213> Artificial
<220>
   <223> Synthetic peptide optionally bearing protecting groups.
<400> 541
   Phe Phe Glu Lys Phe Arg Glu Phe Phe Lys Asp Tyr Phe Ala Lys Leu
   1 5 10 15
   Trp Asp
<210> 542
   <211> 18
   <212> PRT
   <213> Artificial
<220>
   <223> Synthetic peptide optionally bearing protecting groups.
<400> 542
   Phe Phe Glu Lys Phe Lys Glu Phe Phe Arg Asp Tyr Phe Ala Lys Leu
   1 5 10 15
   Trp Asp
<210> 543
   <211> 18
   <212> PRT
   <213> Artificial
<220>
   <223> Synthetic peptide optionally bearing protecting groups.
<400> 543
   Phe Phe Glu Arg Phe Lys Glu Phe Phe Lys Asp Tyr Phe Ala Arg Leu
   1 5 10 15
   Trp Asp
<210> 544
   <211> 18
   <212> PRT
   <213> Artificial
<220>
   <223> Synthetic peptide optionally bearing protecting groups.
<400> 544
   Phe Phe Asp Lys Phe Lys Glu Phe Phe Lys Asp Tyr Phe Ala Lys Leu
   1 5 10 15
   Trp Asp
<210> 545
   <211> 18
   <212> PRT
   <213> Artificial
<220>
   <223> Synthetic peptide optionally bearing protecting groups.
<400> 545
   Phe Phe Glu Lys Phe Lys Asp Phe Phe Lys Asp Tyr Phe Ala Lys Leu
   1 5 10 15
   Trp Asp
<210> 546
   <211> 18
   <212> PRT
   <213> Artificial
<220>
   <223> Synthetic peptide optionally bearing protecting groups.
<400> 546
   Phe Phe Glu Lys Phe Lys Glu Phe Phe Lys Glu Tyr Phe Ala Lys Leu
   1               5                   10                  15
   Trp Asp
<210> 547
   <211> 18
   <212> PRT
   <213> Artificial
<220>
   <223> Synthetic peptide optionally bearing protecting groups.
<400> 547
   Phe Phe Glu Lys Phe Lys Glu Phe Phe Lys Asp Tyr Phe Ala Lys Leu
   1               5                   10                  15
   Trp Glu
<210> 548
   <211> 18
   <212> PRT
   <213> Artificial
<220>
   <223> Synthetic peptide optionally bearing protecting groups.
<400> 548
   Phe Ala Glu Lys Phe Lys Glu Ala Val Lys Asp Tyr Phe Ala Lys Phe
   1               5                   10                  15
   Trp Asp
<210> 549
   <211> 18
   <212> PRT
   <213> Artificial
<220>
   <223> Synthetic peptide optionally bearing protecting groups.
<400> 549
   Phe Ala Glu Lys Phe Lys Glu Ala Val Lys Glu Tyr Phe Ala Lys Phe
   1               5                   10                  15
   Trp Glu
<210> 550
   <211> 18
   <212> PRT
   <213> Artificial
<220>
   <223> Synthetic peptide optionally bearing protecting groups.
<400> 550
   Phe Ala Asp Lys Phe Lys Asp Ala Val Lys Asp Tyr Phe Ala Lys Phe
   1               5                   10                  15
   Trp Asp
<210> 551
   <211> 18
   <212> PRT
   <213> Artificial
<220>
   <223> Synthetic peptide optionally bearing protecting groups.
<400> 551
   Phe Ala Glu Arg Phe Arg Glu Ala Val Lys Asp Tyr Phe Ala Lys Phe
   1               5                   10                  15
   Trp Asp
<210> 552
   <211> 18
   <212> PRT
   <213> Artificial
<220>
   <223> Synthetic peptide optionally bearing protecting groups.
<400> 552
   Phe Ala Glu Lys Phe Arg Glu Ala Val Lys Asp Tyr Phe Ala Lys Phe
   1               5                   10                  15
   Trp Asp
<210> 553
   <211> 18
   <212> PRT
   <213> Artificial
<220>
   <223> Synthetic peptide optionally bearing protecting groups.
<400> 553
   Phe Ala Glu Lys Phe Lys Glu Ala Val Arg Asp Tyr Phe Ala Lys Phe
   1 5 10 15
   Trp Asp
<210> 554
   <211> 18
   <212> PRT
   <213> Artificial
<220>
   <223> Synthetic peptide optionally bearing protecting groups.
<400> 554
   Phe Ala Glu Lys Phe Lys Glu Ala Val Lys Asp Tyr Phe Ala Arg Phe
   1 5 10 15
   Trp Asp
<210> 555
   <211> 18
   <212> PRT
   <213> Artificial
<220>
   <223> Synthetic peptide optionally bearing protecting groups.
<400> 555
   Asp Lys Trp Lys Ala Val Tyr Asp Lys Phe Ala Glu Ala Phe Lys Glu
   1 5 10 15
   Phe Phe
<210> 556
   <211> 18
   <212> PRT
   <213> Artificial
<220>
   <223> Synthetic peptide optionally bearing protecting groups.
<400> 556
   Glu Lys Trp Lys Ala Val Tyr Glu Lys Phe Ala Glu Ala Phe Lys Glu
   1 5 10 15
   Phe Phe
<210> 557
   <211> 18
   <212> PRT
   <213> Artificial
<220>
   <223> Synthetic peptide optionally bearing protecting groups.
<400> 557
   Asp Lys Trp Lys Ala Val Tyr Asp Lys Phe Ala Asp Ala Phe Lys Asp
   1 5 10 15
   Phe Phe
<210> 558
   <211> 18
   <212> PRT
   <213> Artificial .
<220>
   <223> Synthetic peptide optionally bearing protecting groups.
<400> 558
   Asp Arg Trp Lys Ala Val Tyr Asp Lys Phe Ala Glu Ala Phe Lys Glu
   1 5 10 15
   Phe Phe
<210> 559
   <211> 18
   <212> PRT
   <213> Artificial
<220>
   <223> Synthetic peptide optionally bearing protecting groups.
<400> 559
   Asp Lys Trp Arg Ala Val Tyr Asp Lys Phe Ala Glu Ala Phe Lys Glu
   1 5 10 15
   Phe Phe
<210> 560
   <211> 18
   <212> PRT
   <213> Artificial
<220>
   <223> Synthetic peptide optionally bearing protecting groups.
<400> 560
   Asp Lys Trp Lys Ala Val Tyr Asp Arg Phe Ala Glu Ala Phe Lys Glu
   1 5 10 15
   Phe Phe
<210> 561
   <211> 18
   <212> PRT
   <213> Artificial
<220>
   <223> Synthetic peptide optionally bearing protecting groups.
<400> 561
   Asp Lys Trp Lys Ala Val Tyr Asp Lys Phe Ala Glu Ala Phe Arg Glu
   1 5 10 15
   Phe Phe
<210> 562
   <211> 18
   <212> PRT
   <213> Artificial
<220>
   <223> Synthetic peptide optionally bearing protecting groups.
<400> 562
   Phe Phe Glu Lys Phe Ala Glu Ala Phe Lys Asp Tyr Val Ala Lys Trp
   1 5 10 15
   Lys Asp
<210> 563
   <211> 18
   <212> PRT
   <213> Artificial
<220>
   <223> Synthetic peptide optionally bearing protecting groups.
<400> 563
   Phe Phe Glu Lys Phe Ala Glu Ala Phe Lys Glu Tyr Val Ala Lys Trp
   1 5 10 15
   Lys Glu
<210> 564
   <211> 18
   <212> PRT
   <213> Artificial
<220>
   <223> Synthetic peptide optionally bearing protecting groups.
<400> 564
   Phe Phe Asp Lys Phe Ala Asp Ala Phe Lys Asp Tyr Val Ala Lys Trp
   1 5 10 15
   Lys Asp
<210> 565
   <211> 18
   <212> PRT
   <213> Artificial
<220>
   <223> Synthetic peptide optionally bearing protecting groups.
<400> 565
   Phe Phe Glu Arg Phe Ala Glu Ala Phe Lys Asp Tyr Val Ala Lys Trp
   1 5 10 15
   Lys Asp
<210> 566
   <211> 18
   <212> PRT
   <213> Artificial
<220>
   <223> Synthetic peptide optionally bearing protecting groups.
<400> 566
   Phe Phe Glu Arg Phe Ala Glu Ala Phe Arg Asp Tyr Val Ala Lys Trp
   1 5 10 15
   Lys Asp
<210> 567
   <211> 18
   <212> PRT
   <213> Artificial
<220>
   <223> Synthetic peptide optionally bearing protecting groups.
<400> 567
   Phe Phe Glu Lys Phe Ala Glu Ala Phe Lys Asp Tyr Val Ala Arg Trp
   1 5 10 15
   Lys Asp
<210> 568
   <211> 18
   <212> PRT
   <213> Artificial
<220>
   <223> Synthetic peptide optionally bearing protecting groups.
<400> 568
   Phe Phe Glu Arg Phe Ala Glu Ala Phe Lys Asp Tyr Val Ala Lys Trp
   1 5 10 15
   Arg Asp
<210> 569
   <211> 18
   <212> PRT
   <213> Artificial
<220>
   <223> Synthetic peptide optionally bearing protecting groups.
<400> 569
   Phe Phe Asp Lys Phe Ala Glu Ala Phe Lys Asp Tyr Val Ala Lys Trp
   1 5 10 15
   Lys Asp
<210> 570
   <211> 18
   <212> PRT
   <213> Artificial
<220>
   <223> Synthetic peptide optionally bearing protecting groups.
<400> 570
   Phe Phe Glu Lys Phe Ala Asp Ala Phe Lys Asp Tyr Val Ala Lys Trp
   1 5 10 15
   Lys Asp
<210> 571
   <211> 18
   <212> PRT
   <213> Artificial
<220>
   <223> Synthetic peptide optionally bearing protecting groups.
<400> 571
   Phe Phe Glu Arg Phe Ala Glu Ala Phe Lys Glu Tyr Val Ala Lys Trp
   1               5                   10                  15
   Lys Asp
<210> 572
   <211> 18
   <212> PRT
   <213> Artificial
<220>
   <223> Synthetic peptide optionally bearing protecting groups.
<400> 572
   Phe Phe Glu Arg Phe Ala Glu Ala Phe Lys Asp Tyr Val Ala Lys Trp
   1               5                   10                  15
   Lys Glu
<210> 573
   <211> 18
   <212> PRT
   <213> Artificial
<220>
   <223> Synthetic peptide optionally bearing protecting groups.
<400> 573
   Phe Phe Glu Lys Phe Lys Glu Phe Phe Lys Asp Tyr Phe Ala Lys Phe
   1               5                   10                  15
   Trp Asp
<210> 574
   <211> 18
   <212> PRT
   <213> Artificial
<220>
   <223> Synthetic peptide optionally bearing protecting groups.
<400> 574
   Phe Phe Asp Lys Phe Lys Asp Phe Phe Lys Asp Tyr Phe Ala Lys Phe
   1 5 10 15
   Trp Asp
<210> 575
   <211> 18
   <212> PRT
   <213> Artificial
<220>
   <223> Synthetic peptide optionally bearing protecting groups.
<400> 575
   Phe Phe Glu Lys Phe Lys Glu Phe Phe Lys Glu Tyr Phe Ala Lys Phe
   1 5 10 15
   Trp Glu
<210> 576
   <211> 18
   <212> PRT
   <213> Artificial
<220>
   <223> Synthetic peptide optionally bearing protecting groups.
<400> 576
   Phe Phe Glu Arg Phe Lys Glu Phe Phe Lys Asp Tyr Phe Ala Lys Phe
   1 5 10 15
   Trp Asp
<210> 577
   <211> 18
   <212> PRT
   <213> Artificial
<220>
   <223> Synthetic peptide optionally bearing protecting groups.
<400> 577
   Phe Phe Glu Lys Phe Arg Glu Phe Phe Lys Asp Tyr Phe Ala Lys Phe
   1 5 10 15
   Trp Asp
<210> 578
   <211> 18
   <212> PRT
   <213> Artificial
<220>
   <223> Synthetic peptide optionally bearing protecting groups.
<400> 578
   Phe Phe Glu Lys Phe Lys Glu Phe Phe Arg Asp Tyr Phe Ala Lys Phe
   1 5 10 15
   Trp Asp
<210> 579
   <211> 18
   <212> PRT
   <213> Artificial
<220>
   <223> Synthetic peptide optionally bearing protecting groups.
<400> 579
   Phe Phe Glu Lys Phe Lys Glu Phe Phe Lys Asp Tyr Phe Ala Arg Phe
   1 5 10 15
   Trp Asp
<210> 580
   <211> 18
   <212> PRT
   <213> Artificial
<220>
   <223> Synthetic peptide optionally bearing protecting groups.
<400> 580
   Phe Phe Asp Lys Phe Lys Glu Phe Phe Lys Asp Tyr Phe Ala Lys Phe
   1 5 10 15
   Trp Asp
<210> 581
   <211> 18
   <212> PRT
   <213> Artificial
<220>
   <223> Synthetic peptide optionally bearing protecting groups.
<400> 581
   Phe Phe Glu Lys Phe Lys Asp Phe Phe Lys Asp Tyr Phe Ala Lys Phe
   1 5 10 15
   Trp Asp
<210> 582
   <211> 18
   <212> PRT
   <213> Artificial
<220>
   <223> Synthetic peptide optionally bearing protecting groups.
<400> 582
   Phe Phe Glu Lys Phe Lys Glu Phe Phe Lys Glu Tyr Phe Ala Lys Phe
   1 5 10 15
   Trp Asp
<210> 583
   <211> 18
   <212> PRT
   <213> Artificial
<220>
   <223> Synthetic peptide optionally bearing protecting groups.
<400> 583
   Phe Phe Glu Lys Phe Lys Glu Phe Phe Lys Asp Tyr Phe Ala Lys Phe
   1 5 10 15
   Trp Glu
<210> 584
   <211> 18
   <212> PRT
   <213> Artificial
<220>
   <223> Synthetic peptide optionally bearing protecting groups.
<400> 584
   Asp Trp Phe Lys Ala Phe Tyr Asp Lys Val Ala Glu Lys Phe Lys Glu
   1 5 10 15
   Ala Phe
<210> 585
   <211> 18
   <212> PRT
   <213> Artificial
<220>
   <223> Synthetic peptide optionally bearing protecting groups.
<400> 585
   Asp Trp Phe Lys Ala Phe Tyr Asp Lys Val Ala Glu Lys Phe Lys Glu
   1 5 10 15
   Ala Phe
<210> 586
   <211> 18
   <212> PRT
   <213> Artificial
<220>
   <223> Synthetic peptide optionally bearing protecting groups.
<400> 586
   Asp Trp Phe Lys Ala Phe Tyr Asp Lys Val Ala Glu Lys Phe Lys Glu
   1 5 10 15
   Ala Phe
<210> 587
   <211> 18
   <212> PRT
   <213> Artificial
<220>
   <223> Synthetic peptide optionally bearing protecting groups.
<400> 587
   Asp Trp Phe Lys Ala Phe Tyr Asp Lys Val Ala Glu Lys Phe Lys Glu
   1 5 10 15
   Ala Phe
<210> 588
   <211> 18
   <212> PRT
   <213> Artificial
<220>
   <223> Synthetic peptide optionally bearing protecting groups.
<400> 588
   Asp Trp Phe Lys Ala Phe Tyr Asp Lys Val Ala Glu Lys Phe Lys Glu
   1 5 10 15
   Ala Phe
<210> 589
   <211> 18
   <212> PRT
   <213> Artificial
<220>
   <223> Synthetic peptide optionally bearing protecting groups.
<400> 589
   Asp Trp Phe Lys Ala Phe Tyr Asp Lys Val Ala Glu Lys Phe Lys Glu
   1               5                   10                  15
   Ala Phe
<210> 590
   <211> 18
   <212> PRT
   <213> Artificial
<220>
   <223> Synthetic peptide optionally bearing protecting groups.
<400> 590
   Asp Trp Phe Lys Ala Phe Tyr Asp Lys Val Ala Glu Lys Phe Lys Glu
   1               5                   10                  15
   Ala Phe
<210> 591
   <211> 18
   <212> PRT
   <213> Artificial
<220>
   <223> Synthetic peptide optionally bearing protecting groups.
<400> 591
   Asp Trp Phe Lys Ala Phe Tyr Asp Lys Val Ala Glu Lys Phe Lys Glu
   1               5                   10                  15
   Ala Phe
<210> 592
   <211> 18
   <212> PRT
   <213> Artificial
<220>
   <223> Synthetic peptide optionally bearing protecting groups.
<400> 592
   Asp Trp Phe Lys Ala Phe Tyr Asp Lys Val Ala Glu Lys Phe Lys Glu
   1               5                   10                  15
   Ala Phe
<210> 593
   <211> 18
   <212> PRT
   <213> Artificial
<220>
   <223> Synthetic peptide optionally bearing protecting groups.
<400> 593
   Asp Trp Phe Lys Ala Phe Tyr Asp Lys Val Ala Glu Lys Phe Lys Glu
   1               5                   10                  15
   Ala Phe
<210> 594
   <211> 18
   <212> PRT
   <213> Artificial
<220>
   <223> Synthetic peptide optionally bearing protecting groups.
<400> 594
   Asp Trp Phe Lys Ala Phe Tyr Asp Lys Val Ala Glu Lys Phe Lys Glu
   1               5                   10                  15
   Ala Phe
<210> 595
   <211> 18
   <212> PRT
   <213> Artificial
<220>
   <223> Synthetic peptide optionally bearing protecting groups.
<400> 595
   Asp Trp Phe Lys Ala Phe Tyr Asp Lys Val Ala Glu Lys Phe Lys Glu
   1               5                   10                  15
   Ala Phe
<210> 596
   <211> 18
   <212> PRT
   <213> Artificial
<220>
   <223> Synthetic peptide optionally bearing protecting groups.
<400> 596
   Asp Trp Phe Lys Ala Phe Tyr Asp Lys Val Ala Glu Lys Phe Lys Glu
   1               5                   10                  15
   Ala Phe

<210> 597
   <211> 18
   <212> PRT
   <213> Artificial
<220>
   <223> Synthetic peptide optionally bearing protecting groups.
<400> 597
   Asp Trp Phe Lys Ala Phe Tyr Asp Lys Val Ala Glu Lys Phe Lys Glu
   1               5                   10                  15
   Ala Phe
<210> 598
   <211> 18
   <212> PRT
   <213> Artificial
<220>
   <223> Synthetic peptide optionally bearing protecting groups.
<400> 598
   Asp Trp Phe Lys Ala Phe Tyr Asp Lys Val Ala Glu Lys Phe Lys Glu
   1               5                   10                  15
   Ala Phe
<210> 599
   <211> 18
   <212> PRT
   <213> Artificial
<220>
   <223> Synthetic peptide optionally bearing protecting groups.
<400> 599
   Asp Trp Phe Lys Ala Phe Tyr Asp Lys Val Ala Glu Lys Phe Lys Glu
   1               5                   10                  15
   Ala Phe
<210> 600
   <211> 18
   <212> PRT
   <213> Artificial
<220>
   <223> Synthetic peptide optionally bearing protecting groups.
<400> 600
   Asp Trp Phe Lys Ala Phe Tyr Asp Lys Val Ala Glu Lys Phe Lys Glu
   1 5 10 15
   Ala Phe
<210> 601
   <211> 18
   <212> PRT
   <213> Artificial
<220>
   <223> Synthetic peptide optionally bearing protecting groups.
<400> 601
   Asp Trp Phe Lys Ala Phe Tyr Asp Lys Val Ala Glu Lys Phe Lys Glu
   1 5 10 15
   Ala Phe
<210> 602
   <211> 18
   <212> PRT
   <213> Artificial
<220>
   <223> Synthetic peptide optionally bearing protecting groups.
<400> 602
   Asp Trp Phe Lys Ala Phe Tyr Asp Lys Val Ala Glu Lys Phe Lys Glu
   1 5 10 15
   Ala Phe
<210> 603
   <211> 18
   <212> PRT
   <213> Artificial
<220>
   <223> Synthetic peptide optionally bearing protecting groups.
<400> 603
   Asp Trp Phe Lys Ala Phe Tyr Asp Lys Val Ala Glu Lys Phe Lys Glu
   1               5                   10                  15
   Ala Phe
<210> 604
   <211> 18
   <212> PRT
   <213> Artificial
<220>
   <223> Synthetic peptide optionally bearing protecting groups.
<400> 604
   Asp Trp Phe Lys Ala Phe Tyr Asp Lys Val Ala Glu Lys Phe Lys Glu
   1               5                   10                  15
   Ala Phe
<210> 605
   <211> 18
   <212> PRT
   <213> Artificial
<220>
   <223> Synthetic peptide optionally bearing protecting groups.
<400> 605
   Asp Trp Phe Lys Ala Phe Tyr Asp Lys Val Ala Glu Lys Phe Lys Glu
   1               5                   10                  15
   Ala Phe
<210> 606
   <211> 18
   <212> PRT
   <213> Artificial
<220>
   <223> Synthetic peptide optionally bearing protecting groups.
<400> 606
   Asp Trp Phe Lys Ala Phe Tyr Asp Lys Val Ala Glu Lys Phe Lys Glu
   1               5                   10                  15
   Ala Phe
<210> 607
   <211> 18
   <212> PRT
   <213> Artificial
<220>
   <223> Synthetic peptide optionally bearing protecting groups.
<400> 607
   Asp Trp Phe Lys Ala Phe Tyr Asp Lys Val Ala Glu Lys Phe Lys Glu
   1               5                   10                  15
   Ala Phe
<210> 608
   <211> 18
   <212> PRT
   <213> Artificial
<220>
   <223> Synthetic peptide optionally bearing protecting groups.
<400> 608
   Asp Trp Phe Lys Ala Phe Tyr Asp Lys Val Ala Glu Lys Phe Lys Glu
   1               5                   10                  15
   Ala Phe
<210> 609
   <211> 18
   <212> PRT
   <213> Artificial
<220>
   <223> Synthetic peptide optionally bearing protecting groups.
<220>
   <221> misc_feature
   <222> (4)..(4)
   <223> Xaa is ornithine (orn).
<220>
   <221> misc_feature
   <222> (9)..(9)
   <223> Xaa is Ornithine (Orn).
<220>
   <221> misc_feature
   <222> (13)..(13)
   <223> Xaa is Ornithine (Orn).
<220>
   <221> misc_feature
   <222> (15)..(15)
   <223> Xaa is Ornithine (Orn).
<400> 609
   Asp Trp Phe Xaa Ala Phe Tyr Asp Xaa Val Ala Glu Xaa Phe Xaa Glu
   1               5                   10                  15
   Ala Phe
<210> 610
   <211> 18
   <212> PRT
   <213> Artificial
<220>
   <223> Synthetic peptide optionally bearing protecting groups.
<220>
   <221> misc_feature
   <222> (4)..(4)
   <223> Xaa can be any naturally occurring amino acid
<220>
   <221> misc_feature
   <222> (9)..(9)
   <223> Xaa can be any naturally occurring amino acid
<220>
   <221> misc_feature
   <222> (13)..(13)
   <223> Xaa can be any naturally occurring amino acid
<220>
   <221> misc_feature
   <222> (15)..(15)
   <223> Xaa can be any naturally occurring amino acid
<400> 610
   Asp Trp Phe Xaa Ala Phe Tyr Asp Xaa Val Ala Glu Xaa Phe Xaa Glu
   1               5                   10                  15
   Ala Phe
<210> 611
   <211> 18
   <212> PRT
   <213> Artificial
<220>
   <223> Synthetic peptide optionally bearing protecting groups.
<220>
   <221> misc_feature
   <222> (4)..(4)
   <223> Xaa is Ornithine (Orn).
<220>
   <221> misc_feature
   <222> (9)..(9)
   <223> Xaa is Ornithine (Orn).
<220>
   <221> misc_feature
   <222> (13)..(13)
   <223> Xaa is Ornithine (Orn).
<220>
   <221> misc_feature
   <222> (15)..(15)
   <223> Xaa is Ornithine (Orn).
<400> 611
   Asp Trp Phe Xaa Ala Phe Tyr Asp Xaa Val Ala Glu Xaa Phe Xaa Glu
   1               5                   10                  15
   Ala Phe
<210> 612
   <211> 18
   <212> PRT
   <213> Artificial
<220>
   <223> Synthetic peptide optionally bearing protecting groups.
<220>
   <221> misc_feature
   <222> (4)..(4)
   <223> Xaa is Ornithine (Orn).
<220>
   <221> misc_feature
   <222> (9)..(9)
   <223> Xaa is Ornithine (Orn).
<220>
   <221> misc_feature
   <222> (13)..(13)
   <223> Xaa is Ornithine (orn).
<220>
   <221> misc_feature
   <222> (15)..(15)
   <223> Xaa is Ornithine (Orn).
<400> 612
   Asp Trp Phe Xaa Ala Phe Tyr Asp Xaa Val Ala Glu Xaa Phe Xaa Glu
   1               5                   10                  15
   Ala Phe
<210> 613
   <211> 18
   <212> PRT
   <213> Artificial
<220>
   <223> Synthetic peptide optionally bearing protecting groups.
<220>
   <221> misc_feature
   <222> (4)..(4)
   <223> Xaa is Ornithine (Orn).
<220>
   <221> misc_feature
   <222> (9)..(9)
   <223> Xaa is Ornithine (Orn).
<220>
   <221> misc_feature
   <222> (13)..(13)
   <223> Xaa is Ornithine (Orn).
<220>
   <221> misc_feature
   <222> (15)..(15)
   <223> Xaa is Ornithine (Orn).
<400> 613
   Asp Trp Phe Xaa Ala Phe Tyr Asp Xaa Val Ala Glu Xaa Phe Xaa Glu
   1               5                   10                  15
   Ala Phe
<210> 614
   <211> 18
   <212> PRT
   <213> Artificial
<220>
   <223> Synthetic peptide optionally bearing protecting groups.
<220>
   <221> misc_feature
   <222> (4)..(4)
   <223> Xaa is Ornithine (Orn).
<220>
   <221> misc_feature
   <222> (9)..(9)
   <223> Xaa is Ornithine (Orn).
<220>
   <221> misc_feature
   <222> (13)..(13)
   <223> Xaa is Ornithine (Orn).
<220>
   <221> misc_feature
   <222> (15)..(15)
   <223> Xaa is Ornithine (Orn).
<400> 614
   Asp Trp Phe Xaa Ala Phe Tyr Asp Xaa Val Ala Glu Xaa Phe Xaa Glu
   1               5                   10                  15
   Ala Phe
<210> 615
   <211> 18
   <212> PRT
   <213> Artificial
<220>
   <223> Synthetic peptide optionally bearing protecting groups.
<220>
   <221> misc_feature
   <222> (4)..(4)
   <223> Xaa is Ornithine (Orn).
<220>
   <221> misc_feature
   <222> (9)..(9)
   <223> Xaa is Ornithine (Orn).
<220>
   <221> misc_feature
   <222> (13)..(13)
   <223> Xaa is Ornithine (Orn).
<220>
   <221> misc_feature
   <222> (15)..(15)
   <223> Xaa is Ornithine (Orn).
<400> 615
   Asp Trp Phe Xaa Ala Phe Tyr Asp Xaa Val Ala Glu Xaa Phe Xaa Glu
   1               5                   10                  15
   Ala Phe
<210> 616
   <211> 18
   <212> PRT
   <213> Artificial
<220>
   <223> Synthetic peptide optionally bearing protecting groups.
<220>
   <221> misc_feature
   <222> (4)..(4)
   <223> Xaa is Ornithine (Orn).
<220>
   <221> misc_feature
   <222> (9)..(9)
   <223> Xaa is Ornithine (Orn).
<220>
   <221> misc_feature
   <222> (13)..(13)
   <223> Xaa is Ornithine (Orn).
<220>
   <221> misc_feature
   <222> (15)..(15)
   <223> Xaa is Ornithine (Orn).
<400> 616
   Asp Trp Phe Xaa Ala Phe Tyr Asp Xaa Val Ala Glu Xaa Phe Xaa Glu
   1               5                   10                  15
   Ala Phe
<210> 617
   <211> 18
   <212> PRT
   <213> Artificial
<220>
   <223> Synthetic peptide optionally bearing protecting groups.
<220>
   <221> misc_feature
   <222> (4)..(4)
   <223> Xaa is Ornithine (Orn).
<220>
   <221> misc_feature
   <222> (9)..(9)
   <223> Xaa is Ornithine (Orn).
<220>
   <221> misc_feature
   <222> (13)..(13)
   <223> Xaa is Ornithine (Orn).
<220>
   <221> misc_feature
   <222> (15)..(15)
   <223> Xaa is Ornithine (Orn).
<400> 617
   Asp Trp Phe Xaa Ala Phe Tyr Asp Xaa Val Ala Glu Xaa Phe Xaa Glu
   1 5 10 15
   Ala Phe
<210> 618
   <211> 18
   <212> PRT
   <213> Artificial
<220>
   <223> Synthetic peptide optionally bearing protecting groups.
<220>
   <221> misc_feature
   <222> (4)..(4)
   <223> Xaa is Ornithine (Orn).
<220>
   <221> misc_feature
   <222> (9)..(9)
   <223> Xaa is Ornithine (Orn).
<220>
   <221> misc_feature
   <222> (13)..(13)
   <223> Xaa is Ornithine (Orn).
<220>
   <221> misc_feature
   <222> (15)..(115)
   <223> Xaa is Ornithine (Orn).
<400> 618
   Asp Trp Phe Xaa Ala Phe Tyr Asp Xaa Val Ala Glu Xaa Phe Xaa Glu
   1 5 10 15
   Ala Phe
<210> 619
   <211> 18
   <212> PRT
   <213> Artificial
<220>
   <223> Synthetic peptide optionally bearing protecting groups.
<220>
   <221> misc_feature
   <222> (4)..(4)
   <223> Xaa is Ornithine (Orn).
<220>
   <221> misc_feature
   <222> (9)..(9)
   <223> Xaa is Ornithine (Orn).
<220>
   <221> misc_feature
   <222> (13)..(13)
   <223> Xaa is Ornithine (Orn).
<220>
   <221> misc_feature
   <222> (15)..(15)
   <223> Xaa is Ornithine (Orn).
<400> 619
   Asp Trp Phe Xaa Ala Phe Tyr Asp Xaa Val Ala Glu Xaa Phe Xaa Glu
   1               5                   10                  15
   Ala Phe
<210> 620
   <211> 4
   <212> PRT
   <213> Artificial
<220>
   <223> Synthetic peptide optionally bearing protecting groups.
<220>
   <221> misc_feature
   <222> (1)..(1)
   <223> Xaa is hydrophobic.
<220>
   <221> misc_feature
   <222> (2)..(3)
   <223> Xaa is acidic or basic
<220>
   <221> misc_feature
   <222> (4)..(4)
   <223> Xaa is hydrophobic.
<400> 620
   Xaa Xaa Xaa Xaa
   1
<210> 621
   <211> 3
   <212> PRT
   <213> Artificial
<220>
   <223> Synthetic peptide optionally bearing protecting groups.
<220>
   <221> misc_feature
   <222> (1)..(1)
   <223> Xaa is hydrophobic
<220>
   <221> misc_feature
   <222> (2)..(2)
   <223> Xaa is acidic or basic
<220>
   <221> misc_feature
   <222> (3)..(3)
   <223> Xaa is hydrophobic
<400> 621
   Xaa Xaa Xaa
   1
<210> 622
   <211> 3
   <212> PRT
   <213> Artificial
<220>
   <223> Synthetic peptide optionally bearing protecting groups.
<400> 622
   Lys Arg Ser
   1
<210> 623
   <211> 3
   <212> PRT
   <213> Artificial
<220>
   <223> Synthetic peptide optionally bearing protecting groups.
<400> 623
   Lys Arg Thr
   1
<210> 624
   <211> 3
   <212> PRT
   <213> Artificial
<220>
   <223> Synthetic peptide optionally bearing protecting groups.
<400> 624
   Trp Arg Ile
   1
<210> 625
   <211> 3
   <212> PRT
   <213> Artificial
<220>
   <223> Synthetic peptide optionally bearing protecting groups.
<400> 625
   Trp Arg Leu
<210> 626
   <211> 3
   <212> PRT
   <213> Artificial
<220>
   <223> Synthetic peptide optionally bearing protecting groups.
<400> 626
   Phe Arg Ile
   1
<210> 627
   <211> 3
   <212> PRT
   <213> Artificial
<220>
   <223> Synthetic peptide optionally bearing protecting groups.
<400> 627
   Phe Arg Leu
   1
<210> 628
   <211> 3
   <212> PRT
   <213> Artificial
<220>
   <223> Synthetic peptide optionally bearing protecting groups.
<400> 628
   Lys Glu Ser
   1
<210> 629
   <211> 3
   <212> PRT
   <213> Artificial
<220>
   <223> Synthetic peptide optionally bearing protecting groups.
<400> 629
   Lys Glu Thr
   1
<210> 630
   <211> 3
   <212> PRT
   <213> Artificial
<220>
   <223> Synthetic peptide optionally bearing protecting groups.
<400> 630
   Lys Asp Ser
   1
<210> 631
   <211> 3
   <212> PRT
   <213> Artificial
<220>
   <223> Synthetic peptide optionally bearing protecting groups.
<400> 631
   Lys Asp Thr
   1
<210> 632
   <211> 3
   <212> PRT
   <213> Artificial
<220>
   <223> Synthetic peptide optionally bearing protecting groups.
<400> 632
   Lys Arg Ser
   1
<210> 633
   <211> 3
   <212> PRT
   <213> Artificial
<220>
   <223> Synthetic peptide optionally bearing protecting groups.
<400> 633.
   Lys Arg Thr
   1
<210> 634
   <211> 3
   <212> PRT
   <213> Artificial
<220>
   <223> Synthetic peptide optionally bearing protecting groups.
<400> 634
   Leu Glu Ser
   1
<210> 635
   <211> 3
   <212> PRT
   <213> Artificial
<220>
   <223> Synthetic peptide optionally bearing protecting groups.
<400> 635
   Leu Glu Thr
   1
<210> 636
   <211> 3
   <212> PRT
   <213> Artificial
<220>
   <223> Synthetic peptide optionally bearing protecting groups.
<400> 636
   Trp Arg Ser
   1
<210> 637
   <211> 3
   <212> PRT
   <213> Artificial
<220>
   <223> Synthetic peptide optionally bearing protecting groups.
<400> 637
   Trp Asp Ser
   1
<210> 638
   <211> 3
   <212> PRT
   <213> Artificial
<220>
   <223> Synthetic peptide optionally bearing protecting groups.
<400> 638
   Trp Glu Ser
   1
<210> 639
   <211> 3
   <212> PRT
   <213> Artificial
<220>
   <223> Synthetic peptide optionally bearing protecting groups.
<400> 639
   Trp Arg Ser
   1
<210> 640
   <211> 3
   <212> PRT
   <213> Artificial
<220>
   <223> Synthetic peptide optionally bearing protecting groups.
<400> 640
   Lys Glu Leu
   1
<210> 641
   <211> 3
   <212> PRT
   <213> Artificial
<220>
   <223> Synthetic peptide optionally bearing protecting groups.
<400> 641
   Leu Arg Ser
   1
<210> 642
   <211> 3
   <212> PRT
   <213> Artificial
<220>
   <223> Synthetic peptide optionally bearing protecting groups.
<400> 642
   Leu Asp Ser
   1
<210> 643
   <211> 3
   <212> PRT
   <213> Artificial
<220>
   <223> Synthetic peptide optionally bearing protecting groups.
<400> 643
   Leu Glu Ser
   1
<210> 644
   <211> 3
   <212> PRT
   <213> Artificial
<220>
   <223> Synthetic peptide optionally bearing protecting groups.
<400> 644
   Leu Arg Ser
   1
<210> 645
   <211> 3
   <212> PRT
   <213> Artificial
<220>
   <223> Synthetic peptide optionally bearing protecting groups.
<400> 645
   Leu Arg Thr
   1
<210> 646
   <211> 3
   <212> PRT
   <213> Artificial
<220>
   <223> Synthetic peptide optionally bearing protecting groups.
<400> 646
   Glu Asp Tyr
   1
<210> 647
   <211> 3
   <212> PRT
   <213> Artificial
<220>
   <223> Synthetic peptide optionally bearing protecting groups.
<400> 647
   Lys Arg Ser
   1
<210> 648
   <211> 3
   <212> PRT
   <213> Artificial
<220>
   <223> Synthetic peptide optionally bearing protecting groups.
<400> 648
   Trp Arg Ile
   1
<210> 649
   <211> 3
   <212> PRT
   <213> Artificial
<220>
   <223> Synthetic peptide optionally bearing protecting groups.
<400> 649
   Trp Arg Leu
   1
<210> 650
   <211> 3
   <212> PRT
   <213> Artificial
<220>
   <223> Synthetic peptide optionally bearing protecting groups.
<400> 650
   Phe Arg Ile
   1
<210> 651
   <211> 3
   <212> PRT
   <213> Artificial
<220>
   <223> Synthetic peptide optionally bearing protecting groups.
<400> 651
   Phe Arg Leu
   1
<210> 652
   <211> 3
   <212> PRT
   <213> Artificial
<220>
   <223> Synthetic peptide optionally bearing protecting groups.
<400> 652
   Trp Arg Phe
   1
<210> 653
   <211> 3
   <212> PRT
   <213> Artificial
<220>
   <223> Synthetic peptide optionally bearing protecting groups.
<400> 653
   Trp Arg Tyr
   1
<210> 654
   <211> 3
   <212> PRT
   <213> Artificial
<220>
   <223> Synthetic peptide optionally bearing protecting groups.
<400> 654
   Trp Arg Phe
   1
<210> 655
   <211> 3
   <212> PRT
   <213> Artificial
<220>
   <223> Synthetic peptide optionally bearing protecting groups.
<400> 655
   Trp Arg Tyr
   1
<210> 656
   <211> 3
   <212> PRT
   <213> Artificial
<220>
   <223> Synthetic peptide optionally bearing protecting groups.
<220>
   <221> misc_feature
   <222> (1)..(1)
   <223> Xaa is ornithine (Orn).
<400> 656
   Xaa Arg Ser
   1
<210> 657
   <211> 3
   <212> PRT
   <213> Artificial
<220>
   <223> Synthetic peptide optionally bearing protecting groups.
<400> 657
   Lys Arg Ser
   1
<210> 658
   <211> 3
   <212> PRT
   <213> Artificial
<220>
   <223> Synthetic peptide optionally bearing protecting groups.
<400> 658
   Lys Arg Thr
   1
<210> 659
   <211> 3
   <212> PRT
   <213> Artificial
<220>
   <223> Synthetic peptide optionally bearing protecting groups.
<400> 659
   Leu Asp Thr
   1
<210> 660
   <211> 3
   <212> PRT
   <213> Artificial
<220>
   <223> Synthetic peptide optionally bearing protecting groups.
<400> 660
   Leu Glu Thr
   1
<210> 661
   <211> 3
   <212> PRT
   <213> Artificial
<220>
   <223> Synthetic peptide optionally bearing protecting groups.
<400> 661
   Leu Arg Thr
   1
<210> 662
   <211> 3
   <212> PRT
   <213> Artificial
<220>
   <223> Synthetic peptide optionally bearing protecting groups.
<220>
   <221> misc_feature
   <222> (1)..(1)
   <223> Xaa is norleucine (norLeu)
<400> 662
   Xaa Arg Ser
   1
<210> 663
   <211> 3
   <212> PRT
   <213> Artificial
<220>
   <223> Synthetic peptide optionally bearing protecting groups.
<220>
   <221> misc_feature
   <222> (1)..(1)
   <223> Xaa is norleucine (norLeu)
<400> 663
   Xaa Asp Ser
   1
<210> 664
   <211> 3
   <212> PRT
   <213> Artificial
<220>
   <223> Synthetic peptide optionally bearing protecting groups.
<220>
   <221> misc_feature
   <222> (1)..(1)
   <223> Xaa is norleucine (norLeu)
<400> 664
   Xaa Glu Ser
   1
<210> 665
   <211> 3
   <212> PRT
   <213> Artificial
<220>
   <223> Synthetic peptide optionally bearing protecting groups.
<400> 665
   Lys Arg Ser
   1
<210> 666
   <211> 3
   <212> PRT
   <213> Artificial
<220>
   <223> Synthetic peptide optionally bearing protecting groups.
<400> 666
   Lys Arg Thr
   1
<210> 667
   <211> 3
   <212> PRT
   <213> Artificial
<220>
   <223> Synthetic peptide optionally bearing protecting groups.
<400> 667
   Lys Glu Ser
   1
<210> 668
   <211> 3
   <212> PRT
   <213> Artificial
<220>
   <223> Synthetic peptide optionally bearing protecting groups.

<400> 668
   Lys Glu Thr
   1
<210> 669
   <211> 3
   <212> PRT
   <213> Artificial
<220>
   <223> Synthetic peptide optionally bearing protecting groups.
<400> 669
   Lys Asp Ser
   1
<210> 670
   <211> 3
   <212> PRT
   <213> Artificial
<220>
   <223> Synthetic peptide optionally bearing protecting groups.
<400> 670
   Lys Asp Thr
   1
<210> 671
   <211> 3
   <212> PRT
   <213> Artificial
<220>
   <223> Synthetic peptide optionally bearing protecting groups.
<400> 671
   Lys Glu Leu
   1
<210> 672
   <211> 3
   <212> PRT
   <213> Artificial
<220>
   <223> Synthetic peptide optionally bearing protecting groups.
<400> 672
   Lys Arg Leu
   1
<210> 673
   <211> 3
   <212> PRT
   <213> Artificial
<220>
   <223> Synthetic peptide optionally bearing protecting groups.
<400> 673
   Lys Arg Thr
   1
<210> 674
   <211> 3
   <212> PRT
   <213> Artificial
<220>
   <223> Synthetic peptide optionally bearing protecting groups.
<400> 674
   Lys Glu Ser
   1
<210> 675
   <211> 3
   <212> PRT
   <213> Artificial
<220>
   <223> Synthetic peptide optionally bearing protecting groups.
<400> 675
   Lys Glu Thr
   1
<210> 676
   <211> 3
   <212> PRT
   <213> Artificial
<220>
   <223> Synthetic peptide optionally bearing protecting groups.
<400> 676
   Lys Asp Ser
   1
<210> 677
   <211> 3
   <212> PRT
   <213> Artificial
<220>
   <223> Synthetic peptide optionally bearing protecting groups.
<400> 677
   Lys Asp Thr
   1
<210> 678
   <211> 3
   <212> PRT
   <213> Artificial
<220>
   <223> Synthetic peptide optionally bearing protecting groups.
<400> 678
   Lys Arg Ser
   1
<210> 679
   <211> 3
   <212> PRT
   <213> Artificial
<220>
   <223> Synthetic peptide optionally bearing protecting groups.
<400> 679
   Lys Glu Leu
   1
<210> 680
   <211> 3
   <212> PRT
   <213> Artificial
<220>
   <223> Synthetic peptide optionally bearing protecting groups.
<400> 680
   Lys Asp Ser
   1
<210> 681
   <211> 3
   <212> PRT
   <213> Artificial
<220>
   <223> Synthetic peptide optionally bearing protecting groups.
<400> 681
   Lys Asp Thr
   1
<210> 682
   <211> 3
   <212> PRT
   <213> Artificial
<220>
   <223> Synthetic peptide optionally bearing protecting groups.
<400> 682
   Lys Arg Thr
   1
<210> 683
   <211> 3
   <212> PRT
   <213> Artificial
<220>
   <223> Synthetic peptide optionally bearing protecting groups.
<400> 683
   Lys Glu Leu
   1
<210> 684
   <211> 3
   <212> PRT
   <213> Artificial
<220>
   <223> Synthetic peptide optionally bearing protecting groups.
<220>
   <221> misc_feature
   <222> (1)..(1)
   <223> Xaa is norleucine (norLeu)
<400> 684
   Xaa Glu Ser
   1
<210> 685
   <211> 3
   <212> PRT
   <213> Artificial
<220>
   <223> Synthetic peptide optionally bearing protecting groups.
<220>
   <221> misc_feature
   <222> (1)..(1)
   <223> Xaa is norleucine (norLeu)
<400> 685
   Xaa Asp Ser
   1
<210> 686
   <211> 3
   <212> PRT
   <213> Artificial
<220>
   <223> Synthetic peptide optionally bearing protecting groups.
<220>
   <221> misc_feature
   <222> (1)..(1)
   <223> Xaa is norleucine (norLeu)
<400> 686
   Xaa Asp Thr
   1
<210> 687
   <211> 3
   <212> PRT
   <213> Artificial
<220>
   <223> Synthetic peptide optionally bearing protecting groups.
<220>
   <221> misc_feature
   <222> (1)..(1)
   <223> Xaa is norleucine (norLeu)
<400> 687
   Xaa Arg Thr
   1
<210> 688
   <211> 3
   <212> PRT
   <213> Artificial
<220>
   <223> Synthetic peptide optionally bearing protecting groups.
<220>
   <221> misc_feature
   <222> (1)..(1)
   <223> Xaa is norleucine (norLeu)
<400> 688
   Xaa Glu Thr
   1
<210> 689
   <211> 3
   <212> PRT
   <213> Artificial
<220>
   <223> Synthetic peptide optionally bearing protecting groups.
<400> 689
   Trp Asp Ile
   1
<210> 690
   <211> 3
   <212> PRT
   <213> Artificial
<220>
   <223> Synthetic peptide optionally bearing protecting groups.
<400> 690
   Trp Arg Ile
   1
<210> 691
   <211> 3
   <212> PRT
   <213> Artificial
<220>
   <223> Synthetic peptide optionally bearing protecting groups.
<400> 691
   Trp Glu Ile
   1
<210> 692
   <211> 3
   <212> PRT
   <213> Artificial
<220>
   <223> Synthetic peptide optionally bearing protecting groups.
<400> 692
   Trp Asp Leu
   1
<210> 693
   <211> 3
   <212> PRT
   <213> Artificial
<220>
   <223> Synthetic peptide optionally bearing protecting groups.
<400> 693
   Trp Glu Leu
   1
<210> 694
   <211> 3
   <212> PRT
   <213> Artificial
<220>
   <223> Synthetic peptide optionally bearing protecting groups.
<400> 694
   Phe Asp Ile
   1
<210> 695
   <211> 3
   <212> PRT
   <213> Artificial
<220>
   <223> Synthetic peptide optionally bearing protecting groups.
<400> 695
   Phe Asp Leu
   1
<210> 696
   <211> 3
   <212> PRT
   <213> Artificial
<220>
   <223> Synthetic peptide optionally bearing protecting groups.
<400> 696
   Phe Glu Leu
   1
<210> 697
   <211> 3
   <212> PRT
   <213> Artificial
<220>
   <223> Synthetic peptide optionally bearing protecting groups.
<400> 697
   Trp Arg Phe
   1
<210> 698
   <211> 3
   <212> PRT
   <213> Artificial
<220>
   <223> Synthetic peptide optionally bearing protecting groups.
<400> 698
   Trp Glu Phe
   1
<210> 699
   <211> 3
   <212> PRT
   <213> Artificial
<220>
   <223> Synthetic peptide optionally bearing protecting groups.
<400> 699
   Trp Asp Phe
   1
<210> 700
   <211> 3
   <212> PRT
   <213> Artificial
<220>
   <223> Synthetic peptide optionally bearing protecting groups.
<400> 700
   Trp Asp Tyr
   1
<210> 701
   <211> 3
   <212> PRT
   <213> Artificial
<220>
   <223> Synthetic peptide optionally bearing protecting groups.
<400> 701
   Trp Arg Tyr
   1
<210> 702
   <211> 3
   <212> PRT
   <213> Artificial
<220>
   <223> Synthetic peptide optionally bearing protecting groups.
<400> 702
   Trp Glu Tyr
   1
<210> 703
   <211> 3
   <212> PRT
   <213> Artificial
<220>
   <223> Synthetic peptide optionally bearing protecting groups.
<400> 703
   Trp Arg Thr
   1
<210> 704
   <211> 3
   <212> PRT
   <213> Artificial
<220>
   <223> Synthetic peptide optionally bearing protecting groups.
<400> 704
   Trp Asp Thr
   1
<210> 705
   <211> 3
   <212> PRT
   <213> Artificial
<220>
   <223> Synthetic peptide optionally bearing protecting groups.
<400> 705
   Trp Glu Thr
   1
<210> 706
   <211> 3
   <212> PRT
   <213> Artificial
<220>
   <223> Synthetic peptide optionally bearing protecting groups.
<220>
   <221> misc_feature
   <222> (3)..(3)
   <223> Xaa is norleucine (Nle)
<400> 706
   Phe Arg Xaa
   1
<210> 707
   <211> 3
   <212> PRT
   <213> Artificial
<220>
   <223> Synthetic peptide optionally bearing protecting groups.
<220>
   <221> misc_feature
   <222> (3)..(3)
   <223> Xaa is norleucine (Nle)
<400> 707
   Phe Glu Xaa
   1
<210> 708
   <211> 3
   <212> PRT
   <213> Artificial
<220>
   <223> Synthetic peptide optionally bearing protecting groups.
<220>
   <221> misc_feature
   <222> (3)..(3)
   <223> Xaa is norleucine (Nle)
<400> 708
   Phe Asp Xaa
   1
<210> 709
   <211> 3
   <212> PRT
   <213> Artificial
<220>
   <223> Synthetic peptide optionally bearing protecting groups.
<400> 709
   Glu His Tyr
   1
<210> 710
   <211> 3
   <212> PRT
   <213> Artificial
<220>
   <223> Synthetic peptide optionally bearing protecting groups.
<400> 710
   Leu His Ser
   1
<210> 711
   <211> 3
   <212> PRT
   <213> Artificial
<220>
   <223> Synthetic peptide optionally bearing protecting groups.
<400> 711
   Leu His Thr
   1
<210> 712
   <211> 3
   <212> PRT
   <213> Artificial
<220>
   <223> Synthetic peptide optionally bearing protecting groups.
<400> 712
   Lys His Ser
   1
<210> 713
   <211> 3
   <212> PRT
   <213> Artificial
<220>
   <223> Synthetic peptide optionally bearing protecting groups.
<400> 713
   Lys His Thr
   1
<210> 714
   <211> 3
   <212> PRT
   <213> Artificial
<220>
   <223> Synthetic peptide optionally bearing protecting groups.
<400> 714
   Lys His Leu
   1
<210> 715
   <211> 3
   <212> PRT
   <213> Artificial
<220>
   <223> Synthetic peptide optionally bearing protecting groups.
<400> 715
   Lys His Ser
   1
<210> 716
   <211> 3
   <212> PRT
   <213> Artificial
<220>
   <223> Synthetic peptide optionally bearing protecting groups.
<400> 716
   Lys His Thr
   1
<210> 717
   <211> 3
   <212> PRT
   <213> Artificial
<220>
   <223> Synthetic peptide optionally bearing protecting groups.
<400> 717
   Lys His Leu
   1
<210> 718
   <211> 3
   <212> PRT
   <213> Artificial
<220>
   <223> Synthetic peptide optionally bearing protecting groups.
<220>
   <221> misc_feature
   <222> (1)..(1)
   <223> Xaa is ornithine (Orn)
<400> 718
   Xaa His Ser
   1
<210> 719
   <211> 3
   <212> PRT
   <213> Artificial
<220>
   <223> Synthetic peptide optionally bearing protecting groups.
<220>
   <221> misc_feature
   <222> (1)..(1)
   <223> Xaa is ornithine (Orn)
<400> 719
   Xaa His Thr
   1
<210> 720
   <211> 3
   <212> PRT
   <213> Artificial
<220>
   <223> Synthetic peptide optionally bearing protecting groups.
<400> 720
   Phe His Ile
   1
<210> 721
   <211> 3
   <212> PRT
   <213> Artificial
<220>
   <223> Synthetic peptide optionally bearing protecting groups.
<400> 721
   Phe His Leu
   1
<210> 722
   <211> 3
   <212> PRT
   <213> Artificial
<220>
   <223> Synthetic peptide optionally bearing protecting groups.
<220>
   <221> misc_feature
   <222> (3)..(3)
   <223> Xaa is norleucine (Nle)
<400> 722
   Phe His Xaa
   1
<210> 723
   <211> 3
   <212> PRT
   <213> Artificial
<220>
   <223> Synthetic peptide optionally bearing protecting groups.
<400> 723
   Phe Lys Leu
   1
<210> 724
   <211> 3
   <212> PRT
   <213> Artificial
<220>
   <223> Synthetic peptide optionally bearing protecting groups.
<400> 724
   Trp His Ile
   1
<210> 725
   <211> 3
   <212> PRT
   <213> Artificial
<220>
   <223> Synthetic peptide optionally bearing protecting groups.
<400> 725
   Trp His Leu
   1
<210> 726
   <211> 3
   <212> PRT
   <213> Artificial
<220>
   <223> Synthetic peptide optionally bearing protecting groups.
<400> 726
   Trp His Phe
   1
<210> 727
   <211> 3
   <212> PRT
   <213> Artificial
<220>
   <223> Synthetic peptide optionally bearing protecting groups.
<400> 727
   Trp His Tyr
   1
<210> 728
   <211> 3
   <212> PRT
   <213> Artificial
<220>
   <223> Synthetic peptide optionally bearing protecting groups.
<400> 728
   Phe Lys Leu
   1
<210> 729
   <211> 3
   <212> PRT
   <213> Artificial
<220>
   <223> Synthetic peptide optionally bearing protecting groups.
<400> 729
   Lys His Ser
   1
<210> 730
   <211> 3
   <212> PRT
   <213> Artificial
<220>
   <223> Synthetic peptide optionally bearing protecting groups.
<400> 730
   Lys His Thr
   1
<210> 731
   <211> 3
   <212> PRT
   <213> Artificial
<220>
   <223> Synthetic peptide optionally bearing protecting groups.
<400> 731
   Lys His Leu
   1
<210> 732
   <211> 3
   <212> PRT
   <213> Artificial
<220>
   <223> Synthetic peptide optionally bearing protecting groups.
<400> 732
   Leu His Ser
   1
<210> 733
   <211> 3
   <212> PRT
   <213> Artificial
<220>
   <223> Synthetic peptide optionally bearing protecting groups.
<400> 733
   Leu His Thr
   1
<210> 734
   <211> 3
   <212> PRT
   <213> Artificial
<220>
   <223> Synthetic peptide optionally bearing protecting groups.
<400> 734
   Lys His Ser
   1
<210> 735
   <211> 3
   <212> PRT
   <213> Artificial
<220>
   <223> Synthetic peptide optionally bearing protecting groups.
<400> 735
   Lys His Thr
   1
<210> 736
   <211> 3
   <212> PRT
   <213> Artificial
<220>
   <223> Synthetic peptide optionally bearing protecting groups.
<400> 736
   Lys His Leu
   1
<210> 737
   <211> 3
   <212> PRT
   <213> Artificial
<220>
   <223> Synthetic peptide optionally bearing protecting groups.
<400> 737
   Lys His Ser
   1
<210> 738
   <211> 3
   <212> PRT
   <213> Artificial
<220>
   <223> Synthetic peptide optionally bearing protecting groups.
<400> 738
   Lys His Thr
   1
<210> 739
   <211> 3
   <212> PRT
   <213> Artificial
<220>
   <223> Synthetic peptide optionally bearing protecting groups.
<220>
   <221> misc_feature
   <222> (1)..(1)
   <223> Xaa is norleucine (Nle)
<400> 739
   Xaa His Ser
   1
<210> 740
   <211> 3
   <212> PRT
   <213> Artificial
<220>
   <223> Synthetic peptide optionally bearing protecting groups.
<400> 740
   Phe His Ile
   1
<210> 741
   <211> 3
   <212> PRT
   <213> Artificial
<220>
   <223> Synthetic peptide optionally bearing protecting groups.
<400> 741
   Phe His Leu
   1
<210> 742
   <211> 3
   <212> PRT
   <213> Artificial
<220>
   <223> Synthetic peptide optionally bearing protecting groups.
<220>
   <221> misc_feature
   <222> (3)..(3)
   <223> Xaa is norleucine (Nle)
<400> 742
   Phe His Xaa
   1
<210> 743
   <211> 3
   <212> PRT
   <213> Artificial
<220>
   <223> Synthetic peptide optionally bearing protecting groups.
<400> 743
   Trp His Ser
   1
<210> 744
   <211> 3
   <212> PRT
   <213> Artificial
<220>
   <223> Synthetic peptide optionally bearing protecting groups.
<400> 744
   Trp His Ile
   1
<210> 745
   <211> 3
   <212> PRT
   <213> Artificial
<220>
   <223> Synthetic peptide optionally bearing protecting groups.
<400> 745
   Trp His Leu
   1
<210> 746
   <211> 3
   <212> PRT
   <213> Artificial
<220>
   <223> Synthetic peptide optionally bearing protecting groups.
<400> 746
   Trp His Phe
   1
<210> 747
   <211> 3
   <212> PRT
   <213> Artificial
<220>
   <223> Synthetic peptide optionally bearing protecting groups.
<400> 747
   Trp His Tyr
   1
<210> 748
   <211> 3
   <212> PRT
   <213> Artificial
<220>
   <223> Synthetic peptide optionally bearing protecting groups.
<400> 748
   Trp His Thr
   1
<210> 749
   <211> 3
   <212> PRT
   <213> Artificial
<220>
   <223> Synthetic peptide optionally bearing protecting groups.
<400> 749
   Lys His Ser
   1
<210> 750
   <211> 3
   <212> PRT
   <213> Artificial
<220>
   <223> Synthetic peptide optionally bearing protecting groups.
<400> 750
   Lys His Thr
   1
<210> 751
   <211> 4
   <212> PRT
   <213> Artificial
<220>
   <223> Synthetic peptide optionally bearing protecting groups.
<400> 751
   Lys Arg Asp Ser
   1
<210> 752
   <211> 4
   <212> PRT
   <213> Artificial
<220>
   <223> Synthetic peptide optionally bearing protecting groups.
<400> 752
   Lys Arg Asp Thr
   1
<210> 753
   <211> 4
   <212> PRT
   <213> Artificial
<220>
   <223> Synthetic peptide optionally bearing protecting groups.
<400> 753
   Trp Arg Asp Ile
   1
<210> 754
   <211> 4
   <212> PRT
   <213> Artificial
<220>
   <223> Synthetic peptide optionally bearing protecting groups.
<400> 754
   Trp Arg Asp Leu
   1
<210> 755
   <211> 4
   <212> PRT
   <213> Artificial
<220>
   <223> Synthetic peptide optionally bearing protecting groups.
<400> 755
   Phe Arg Asp Leu
   1
<210> 756
   <211> 4
   <212> PRT
   <213> Artificial
<220>
   <223> Synthetic peptide optionally bearing protecting groups.
<400> 756
   Phe Arg Asp Ile
   1
<210> 757
   <211> 4
   <212> PRT
   <213> Artificial
<220>
   <223> Synthetic peptide optionally bearing protecting groups.
<220>
   <221> misc_feature
   <222> (4)..(4)
   <223> Xaa is norleucine (Nle)
<400> 757
   Phe Arg Asp Xaa
   1
<210> 758
   <211> 4
   <212> PRT
   <213> Artificial
<220>
   <223> Synthetic peptide optionally bearing protecting groups.
<220>
   <221> misc_feature
   <222> (4)..(4)
   <223> Xaa is norleucine (Nle) -
<400> 758
   Phe Arg Glu Xaa
   1
<210> 759
   <211> 4
   <212> PRT
   <213> Artificial
<220>
   <223> Synthetic peptide optionally bearing protecting groups.
<400> 759
   Phe Arg Glu Ile
   1
<210> 760
   <211> 4
   <212> PRT
   <213> Artificial
<220>
   <223> Synthetic peptide optionally bearing protecting groups.

<400> 760
   Phe Asp Arg Ile
   1
<210> 761
   <211> 4
   <212> PRT
   <213> Artificial
<220>
   <223> Synthetic peptide optionally bearing protecting groups.
<400> 761
   Phe Glu Arg Ile
   1
<210> 762
   <211> 4
   <212> PRT
   <213> Artificial
<220>
   <223> Synthetic peptide optionally bearing protecting groups.
<400> 762
   Phe Asp Arg Leu
   1
<210> 763
   <211> 4
   <212> PRT
   <213> Artificial
<220>
   <223> Synthetic peptide optionally bearing protecting groups.
<400> 763
   Phe Arg Glu Leu
   1
<210> 764
   <211> 4
   <212> PRT
   <213> Artificial
<220>
   <223> Synthetic peptide optionally bearing protecting groups.
<400> 764
   Phe Glu Arg Leu
   1
<210> 765
   <211> 4
   <212> PRT
   <213> Artificial
<220>
   <223> Synthetic peptide optionally bearing protecting groups.
<220>
   <221> misc_feature
   <222> (4)..(4)
   <223> Xaa is norleucine (Nle)
<400> 765
   Phe Asp Arg Xaa
   1
<210> 766
   <211> 4
   <212> PRT
   <213> Artificial
<220>
   <223> Synthetic peptide optionally bearing protecting groups.
<220>
   <221> misc_feature
   <222> (4)..(4)
   <223> Xaa is norleucine (Nle)
<400> 766
   Phe Glu Arg Xaa
   1
<210> 767
   <211> 4
   <212> PRT
   <213> Artificial
<220>
   <223> Synthetic peptide optionally bearing protecting groups.
<400> 767
   Lys Glu Arg Ser
   1
<210> 768
   <211> 4
   <212> PRT
   <213> Artificial
<220>
   <223> Synthetic peptide optionally bearing protecting groups.
<400> 768
   Lys Glu Arg Thr
   1
<210> 769
   <211> 4
   <212> PRT
   <213> Artificial
<220>
   <223> Synthetic peptide optionally bearing protecting groups.
<400> 769
   Lys Asp Arg Ser
   1
<210> 770
   <211> 4
   <212> PRT
   <213> Artificial
<220>
   <223> Synthetic peptide optionally bearing protecting groups.
<400> 770
   Lys Asp Arg Thr
   1
<210> 771
   <211> 4
   <212> PRT
   <213> Artificial
<220>
   <223> Synthetic peptide optionally bearing protecting groups.
<400> 771
   Lys Arg Glu Ser
   1
<210> 772
   <211> 4
   <212> PRT
   <213> Artificial
<220>
   <223> Synthetic peptide optionally bearing protecting groups.
<400> 772
   Lys Arg Glu Thr
   1
<210> 773
   <211> 4
   <212> PRT
   <213> Artificial
<220>
   <223> Synthetic peptide optionally bearing protecting groups.
<400> 773
   Leu Glu Arg Ser
   1
<210> 774
   <211> 4
   <212> PRT
   <213> Artificial
<220>
   <223> Synthetic peptide optionally bearing protecting groups.
<400> 774
   Leu Glu Arg Thr
   1
<210> 775
   <211> 4
   <212> PRT
   <213> Artificial
<220>
   <223> Synthetic peptide optionally bearing protecting groups.
<400> 775
   Trp Arg Asp Ser
   1
<210> 776
   <211> 4
   <212> PRT
   <213> Artificial
<220>
   <223> Synthetic peptide optionally bearing protecting groups.
<400> 776
   Trp Asp Arg Ser
   1
<210> 777
   <211> 4
   <212> PRT
   <213> Artificial
<220>
   <223> Synthetic peptide optionally bearing protecting groups.
<400> 777
   Trp Glu Arg Ser
   1
<210> 778
   <211> 4
   <212> PRT
   <213> Artificial
<220>
   <223> Synthetic peptide optionally bearing protecting groups.
<400> 778
   Trp Arg Glu Ser
   1
<210> 779
   <211> 4
   <212> PRT
   <213> Artificial
<220>
   <223> Synthetic peptide optionally bearing protecting groups.
<400> 779
   Lys Glu Arg Leu
   1
<210> 780
   <211> 4
   <212> PRT
   <213> Artificial
<220>
   <223> Synthetic peptide optionally bearing protecting groups.
<400> 780
   Leu Arg Asp Ser
   1
<210> 781
   <211> 4
   <212> PRT
   <213> Artificial
<220>
   <223> Synthetic peptide optionally bearing protecting groups.
<400> 781
   Leu Asp Arg Ser
   1
<210> 782
   <211> 4
   <212> PRT
   <213> Artificial
<220>
   <223> Synthetic peptide optionally bearing protecting groups.
<400> 782
   Leu Glu Arg Ser
   1
<210> 783
   <211> 4
   <212> PRT
   <213> Artificial
<220>
   <223> Synthetic peptide optionally bearing protecting groups.
<400> 783
   Leu Arg Glu Ser
   1
<210> 784
   <211> 4
   <212> PRT
   <213> Artificial
<220>
   <223> Synthetic peptide optionally bearing protecting groups.
<400> 784
   Leu Arg Asp Thr
   1
<210> 785
   <211> 4
   <212> PRT
   <213> Artificial
<220>
   <223> Synthetic peptide optionally bearing protecting groups.
<400> 785
   Glu Asp Arg Tyr
   1
<210> 786
   <211> 4
   <212> PRT
   <213> Artificial
<220>
   <223> Synthetic peptide optionally bearing protecting groups.
<400> 786
   Lys Arg Asp Ser
   1
<210> 787
   <211> 4
   <212> PRT
   <213> Artificial
<220>
   <223> Synthetic peptide optionally bearing protecting groups.
<400> 787
   Trp Arg Asp Ile
   1
<210> 788
   <211> 4
   <212> PRT
   <213> Artificial
<220>
   <223> Synthetic peptide optionally bearing protecting groups.
<400> 788
   Trp Arg Asp Leu
   1
<210> 789
   <211> 4
   <212> PRT
   <213> Artificial
<220>
   <223> Synthetic peptide optionally bearing protecting groups.
<400> 789
   Phe Arg Asp Ile
   1
<210> 790
   <211> 4
   <212> PRT
   <213> Artificial
<220>
   <223> Synthetic peptide optionally bearing protecting groups.
<400> 790
   Phe Arg Asp Leu
   1
<210> 791
   <211> 4
   <212> PRT
   <213> Artificial
<220>
   <223> Synthetic peptide optionally bearing protecting groups.
<400> 791
   Trp Arg Asp Phe
   1
<210> 792
   <211> 4
   <212> PRT
   <213> Artificial
<220>
   <223> Synthetic peptide optionally bearing protecting groups.
<400> 792
   Trp Arg Asp Tyr
   1
<210> 793
   <211> 4
   <212> PRT
   <213> Artificial
<220>
   <223> Synthetic peptide optionally bearing protecting groups.
<400> 793
   Trp Arg Asp Phe
   1
<210> 794
   <211> 4
   <212> PRT
   <213> Artificial
<220>
   <223> Synthetic peptide optionally bearing protecting groups.
<400> 794
   Trp Arg Asp Tyr
   1
<210> 795
   <211> 4
   <212> PRT
   <213> Artificial
<220>
   <223> Synthetic peptide optionally bearing protecting groups.
<220>
   <221> misc_feature
   <222> (1)..(1)
   <223> Xaa is ornithine (Orn)
<400> 795
   Xaa Arg Glu Ser
   1
<210> 796
   <211> 4
   <212> PRT
   <213> Artificial
<220>
   <223> Synthetic peptide optionally bearing protecting groups.
<400> 796
   Lys Arg Asp Ser
   1
<210> 797
   <211> 4
   <212> PRT
   <213> Artificial
<220>
   <223> Synthetic peptide optionally bearing protecting groups.
<400> 797
   Lys Arg Asp Thr
   1
<210> 798
   <211> 4
   <212> PRT
   <213> Artificial
<220>
   <223> Synthetic peptide optionally bearing protecting groups.
<400> 798
   Leu Asp Arg Thr
   1
<210> 799
   <211> 4
   <212> PRT
   <213> Artificial
<220>
   <223> Synthetic peptide optionally bearing protecting groups.
<400> 799
   Leu Glu Arg Thr
   1
<210> 800
   <211> 4
   <212> PRT
   <213> Artificial
<220>
   <223> Synthetic peptide optionally bearing protecting groups.
<400> 800
   Leu Arg Glu Thr
   1
<210> 801
   <211> 4
   <212> PRT
   <213> Artificial
<220>
   <223> Synthetic peptide optionally bearing protecting groups.
<220>
   <221> misc_feature
   <222> (1)..(1)
   <223> Xaa is norleucine (Nle)
<400> 801
   Xaa Arg Asp Ser
   1
<210> 802
   <211> 4
   <212> PRT
   <213> Artificial
<220>
   <223> Synthetic peptide optionally bearing protecting groups.
<220>
   <221> misc_feature
   <222> (1)..(1)
   <223> Xaa is norleucine (Nle)
<400> 802
   Xaa Asp Arg Ser
   1
<210> 803
   <211> 4
   <212> PRT
   <213> Artificial
<220>
   <223> Synthetic peptide optionally bearing protecting groups.
<220>
   <221> misc_feature
   <222> (1) .. (1)
   <223> Xaa is norleucine (Nle)
<400> 803
   Xaa Glu Arg Ser
   1
<210> 804
   <211> 4
   <212> PRT
   <213> Artificial
<220>
   <223> Synthetic peptide optionally bearing protecting groups.
<220>
   <221> misc_feature
   <222> (1)..(1)
   <223> Xaa is norleucine (Nle)
<400> 804
   Xaa Arg Glu Ser
   1
<210> 805
   <211> 4
   <212> PRT
   <213> Artificial
<220>
   <223> Synthetic peptide optionally bearing protecting groups.
<400> 805
   Lys Arg Asp Ser
   1
<210> 806
   <211> 4
   <212> PRT
   <213> Artificial
<220>
   <223> Synthetic peptide optionally bearing protecting groups.
<400> 806
   Lys Arg Asp Thr
   1
<210> 807
   <211> 4
   <212> PRT
   <213> Artificial
<220>
   <223> Synthetic peptide optionally bearing protecting groups.
<400> 807
   Lys Glu Arg Ser
   1
<210> 808
   <211> 4
   <212> PRT
   <213> Artificial
<220>
   <223> Synthetic peptide optionally bearing protecting groups.
<400> 808
   Lys Glu Arg Thr
   1
<210> 809
   <211> 4
   <212> PRT
   <213> Artificial
<220>
   <223> Synthetic peptide optionally bearing protecting groups.
<400> 809
   Lys Asp Arg Ser
   1
<210> 810
   <211> 4
   <212> PRT
   <213> Artificial
<220>
   <223> Synthetic peptide optionally bearing protecting groups.
<400> 810
   Lys Asp Arg Thr
   1
<210> 811
   <211> 4
   <212> PRT
   <213> Artificial
<220>
   <223> Synthetic peptide optionally bearing protecting groups.
<400> 811
   Lys Arg Glu Ser
   1
<210> 812
   <211> 4
   <212> PRT
   <213> Artificial
<220>
   <223> Synthetic peptide optionally bearing protecting groups.
<400> 812
   Lys Arg Glu Thr
   1
<210> 813
   <211> 4
   <212> PRT
   <213> Artificial
<220>
   <223> Synthetic peptide optionally bearing protecting groups.
<400> 813
   Lys Glu Arg Leu
   1
<210> 814
   <211> 4
   <212> PRT
   <213> Artificial
<220>
   <223> Synthetic peptide optionally bearing protecting groups.
<400> 814
   Lys Arg Glu Leu
   1
<210> 815
   <211> 4
   <212> PRT
   <213> Artificial
<220>
   <223> Synthetic peptide optionally bearing protecting groups.
<400> 815
   Lys Arg Asp Thr
   1
<210> 816
   <211> 4
   <212> PRT
   <213> Artificial
<220>
   <223> Synthetic peptide optionally bearing protecting groups.
<400> 816
   Lys Glu Arg Ser
   1
<210> 817
   <211> 4
   <212> PRT
   <213> Artificial
<220>
   <223> Synthetic peptide optionally bearing protecting groups.
<400> 817
   Lys Glu Arg Thr
   1
<210> 818
   <211> 4
   <212> PRT
   <213> Artificial
<220>
   <223> Synthetic peptide optionally bearing protecting groups.
<400> 818
   Lys Asp Arg Ser
   1
<210> 819
   <211> 4
   <212> PRT
   <213> Artificial
<220>
   <223> Synthetic peptide optionally bearing protecting groups.
<400> 819
   Lys Asp Arg Thr
   1
<210> 820
   <211> 4
   <212> PRT
   <213> Artificial
<220>
   <223> Synthetic peptide optionally bearing protecting groups.
<400> 820
   Lys Arg Glu Ser
   1
<210> 821
   <211> 4
   <212> PRT
   <213> Artificial
<220>
   <223> Synthetic peptide optionally bearing protecting groups.
<400> 821
   Lys Arg Glu Thr
   1
<210> 822
   <211> 4
   <212> PRT
   <213> Artificial
<220>
   <223> Synthetic peptide optionally bearing protecting groups.
<400> 822
   Lys Glu Arg Leu
   1
<210> 823
   <211> 4
   <212> PRT
   <213> Artificial
<220>
   <223> Synthetic peptide optionally bearing protecting groups.
<400> 823
   Lys Arg Asp Ser
   1
<210> 824
   <211> 4
   <212> PRT
   <213> Artificial
<220>
   <223> Synthetic peptide optionally bearing protecting groups.
<400> 824
   Lys Arg Asp Thr
   1
<210> 825
   <211> 4
   <212> PRT
   <213> Artificial
<220>
   <223> Synthetic peptide optionally bearing protecting groups.
<400> 825
   Lys Glu Arg Ser
   1
<210> 826
   <211> 4
   <212> PRT
   <213> Artificial
<220>
   <223> Synthetic peptide optionally bearing protecting groups.
<400> 826
   Lys Glu Arg Thr
   1
<210> 827
   <211> 4
   <212> PRT
   <213> Artificial
<220>
   <223> Synthetic peptide optionally bearing protecting groups.
<400> 827
   Lys Asp Arg Ser
   1
<210> 828
   <211> 4
   <212> PRT
   <213> Artificial
<220>
   <223> Synthetic peptide optionally bearing protecting groups.
<400> 828
   Lys Asp Arg Thr
   1
<210> 829
   <211> 4
   <212> PRT
   <213> Artificial
<220>
   <223> Synthetic peptide optionally bearing protecting groups.
<400> 829
   Lys Arg Glu Ser
   1
<210> 830
   <211> 4
   <212> PRT
   <213> Artificial
<220>
   <223> Synthetic peptide optionally bearing protecting groups.
<400> 830
   Lys Arg Glu Thr
   1
<210> 831
   <211> 4
   <212> PRT
   <213> Artificial
<220>
   <223> Synthetic peptide optionally bearing protecting groups.
<400> 831
   Lys Glu Arg Leu
   1
<210> 832
   <211> 4
   <212> PRT
   <213> Artificial
<220>
   <223> Synthetic peptide optionally bearing protecting groups.
<220>
   <221> misc_feature
   <222> (1)..(1)
   <223> Xaa is ornithine (Orn)
<400> 832
   Xaa Arg Glu Ser
   1
<210> 833
   <211> 4
   <212> PRT
   <213> Artificial
<220>
   <223> Synthetic peptide optionally bearing protecting groups.
<220>
   <221> misc_feature
   <222> (1)..(1)
   <223> Xaa is ornithine (Orn)
<400> 833
   Xaa Glu Arg Ser
   1
<210> 834
   <211> 4
   <212> PRT
   <213> Artificial
<220>
   <223> Synthetic peptide optionally bearing protecting groups.
<220>
   <221> misc_feature
   <222> (1)..(1)
   <223> Xaa is ornithine (Orn)
<400> 834
   Xaa Arg Asp Ser
   1
<210> 835
   <211> 4
   <212> PRT
   <213> Artificial
<220>
   <223> Synthetic peptide optionally bearing protecting groups.
<220>
   <221> misc_feature
   <222> (1)..(1)
   <223> Xaa is ornithine (Orn)
<400> 835
   Xaa Asp Arg Ser
   1
<210> 836
   <211> 4
   <212> PRT
   <213> Artificial
<220>
   <223> Synthetic peptide optionally bearing protecting groups.
<220>
   <221> misc_feature
   <222> (1)..(1)
   <223> Xaa is ornithine (Orn)
<400> 836
   Xaa Asp Arg Thr
   1
<210> 837
   <211> 4
   <212> PRT
   <213> Artificial
<220>
   <223> Synthetic peptide optionally bearing protecting groups.
<220>
   <221> misc_feature
   <222> (1)..(1)
   <223> Xaa is ornithine (Orn)
<400> 837
   Xaa Arg Asp Thr
   1
<210> 838
   <211> 4
   <212> PRT
   <213> Artificial
<220>
   <223> Synthetic peptide optionally bearing protecting groups.
<220>
   <221> misc_feature
   <222> (1)..(1)
   <223> Xaa is ornithine (Orn)
<400> 838
   Xaa Glu Arg Thr
   1
<210> 839
   <211> 4
   <212> PRT
   <213> Artificial
<220>
   <223> Synthetic peptide optionally bearing protecting groups.
<220>
   <221> misc_feature
   <222> (1)..(1)
   <223> Xaa is ornithine (Orn)
<400> 839
   Xaa Arg Glu Thr
   1
<210> 840
   <211> 4
   <212> PRT
   <213> Artificial
<220>
   <223> Synthetic peptide optionally bearing protecting groups.
<400> 840
   Trp Asp Arg Ile
   1
<210> 841
   <211> 4
   <212> PRT
   <213> Artificial
<220>
   <223> Synthetic peptide optionally bearing protecting groups.
<400> 841
   Trp Arg Glu Ile
   1
<210> 842
   <211> 4
   <212> PRT
   <213> Artificial
<220>
   <223> Synthetic peptide optionally bearing protecting groups.
<400> 842
   Trp Glu Arg Ile
   1
<210> 843
   <211> 4
   <212> PRT
   <213> Artificial
<220>
   <223> Synthetic peptide optionally bearing protecting groups.
<400> 843
   Trp Asp Arg Leu
   1
<210> 844
   <211> 4
   <212> PRT
   <213> Artificial
<220>
   <223> Synthetic peptide optionally bearing protecting groups.
<400> 844
   Trp Arg Glu Leu
   1
<210> 845
   <211> 4
   <212> PRT
   <213> Artificial
<220>
   <223> Synthetic peptide optionally bearing protecting groups.
<400> 845
   Trp Glu Arg Leu
   1
<210> 846
   <211> 4
   <212> PRT
   <213> Artificial
<220>
   <223> Synthetic peptide optionally bearing protecting groups.
<400> 846
   Phe Asp Arg Ile
   1
<210> 847
   <211> 4
   <212> PRT
   <213> Artificial
<220>
   <223> Synthetic peptide optionally bearing protecting groups.
<400> 847
   Phe Arg Glu Ile
   1
<210> 848
   <211> 4
   <212> PRT
   <213> Artificial
<220>
   <223> Synthetic peptide optionally bearing protecting groups.
<400> 848
   Phe Glu Arg Ile
   1
<210> 849
   <211> 4
   <212> PRT
   <213> Artificial
<220>
   <223> Synthetic peptide optionally bearing protecting groups.
<400> 849
   Phe Asp Arg Leu
   1
<210> 850
   <211> 4
   <212> PRT
   <213> Artificial
<220>
   <223> Synthetic peptide optionally bearing protecting groups.
<400> 850
   Phe Arg Glu Leu
   1
<210> 851
   <211> 4
   <212> PRT
   <213> Artificial
<220>
   <223> Synthetic peptide optionally bearing protecting groups.
<400> 851
   Phe Glu Arg Leu
   1
<210> 852
   <211> 4
   <212> PRT
   <213> Artificial
<220>
   <223> Synthetic peptide optionally bearing protecting groups.

<400> 852
   Trp Arg Asp Phe
   1
<210> 853
   <211> 4
   <212> PRT
   <213> Artificial
<220>
   <223> Synthetic peptide optionally bearing protecting groups.
<400> 853
   Trp Arg Glu Phe
   1
<210> 854
   <211> 4
   <212> PRT
   <213> Artificial
<220>
   <223> Synthetic peptide optionally bearing protecting groups.
<400> 854
   Trp Glu Arg Phe
   1
<210> 855
   <211> 4
   <212> PRT
   <213> Artificial
<220>
   <223> Synthetic peptide optionally bearing protecting groups.
<400> 855
   Trp Asp Arg Tyr
   1
<210> 856
   <211> 4
   <212> PRT
   <213> Artificial
<220>
   <223> Synthetic peptide optionally bearing protecting groups.
<400> 856
   Trp Arg Glu Tyr
   1
<210> 857
   <211> 4
   <212> PRT
   <213> Artificial
<220>
   <223> Synthetic peptide optionally bearing protecting groups.
<400> 857
   Trp Glu Arg Tyr
   1
<210> 858
   <211> 4
   <212> PRT
   <213> Artificial
<220>
   <223> Synthetic peptide optionally bearing protecting groups.
<400> 858
   Trp Arg Asp Thr
   1
<210> 859
   <211> 4
   <212> PRT
   <213> Artificial
<220>
   <223> Synthetic peptide optionally bearing protecting groups.
<400> 859
   Trp Asp Arg Thr
   1
<210> 860
   <211> 4
   <212> PRT
   <213> Artificial
<220>
   <223> Synthetic peptide optionally bearing protecting groups.
<400> 860
   Trp Arg Glu Thr
   1
<210> 861
   <211> 4
   <212> PRT
   <213> Artificial
<220>
   <223> Synthetic peptide optionally bearing protecting groups.
<400> 861
   Trp Glu Arg Thr
   1
<210> 862
   <211> 4
   <212> PRT
   <213> Artificial
<220>
   <223> Synthetic peptide optionally bearing protecting groups.
<220>
   <221> misc_feature
   <222> (4)..(4)
   <223> Xaa is norleucine (Nle)
<400> 862
   Phe Arg Asp Xaa
   1
<210> 863
   <211> 4
   <212> PRT
   <213> Artificial
<220>
   <223> Synthetic peptide optionally bearing protecting groups.
<220>
   <221> misc_feature
   <222> (4)..(4)
   <223> Xaa is norleucine (Nle)
<400> 863
   Phe Arg Glu Xaa
   1
<210> 864
   <211> 4
   <212> PRT
   <213> Artificial
<220>
   <223> Synthetic peptide optionally bearing protecting groups.
<400> 864
   Phe Lys Asp Leu
   1
<210> 865
   <211> 4
   <212> PRT
   <213> Artificial
<220>
   <223> Synthetic peptide optionally bearing protecting groups.
<400> 865
   Phe Asp Lys Leu
   1
<210> 866
   <211> 4
   <212> PRT
   <213> Artificial
<220>
   <223> Synthetic peptide optionally bearing protecting groups.
<400> 866
   Phe Lys Glu Leu
   1
<210> 867
   <211> 4
   <212> PRT
   <213> Artificial
<220>
   <223> Synthetic peptide optionally bearing protecting groups.
<400> 867
   Phe Glu Lys Leu
   1
<210> 868
   <211> 4
   <212> PRT
   <213> Artificial
<220>
   <223> Synthetic peptide optionally bearing protecting groups.
<400> 868
   Phe Lys Asp Ile
   1
<210> 869
   <211> 4
   <212> PRT
   <213> Artificial
<220>
   <223> Synthetic peptide optionally bearing protecting groups.
<400> 869
   Phe Asp Lys Ile
   1
<210> 870
   <211> 4
   <212> PRT
   <213> Artificial
<220>
   <223> Synthetic peptide optionally bearing protecting groups.
<400> 870
   Phe Lys Glu Ile
   1
<210> 871
   <211> 4
   <212> PRT
   <213> Artificial
<220>
   <223> Synthetic peptide optionally bearing protecting groups.
<400> 871
   Phe Glu Lys Ile
   1
<210> 872
   <211> 4
   <212> PRT
   <213> Artificial
<220>
   <223> Synthetic peptide optionally bearing protecting groups.
<220>
   <221> misc_feature
   <222> (4)..(4)
   <223> Xaa is norleucine (Nle)
<400> 872
   Phe Lys Asp Xaa
   1
<210> 873
   <211> 4
   <212> PRT
   <213> Artificial
<220>
   <223> Synthetic peptide optionally bearing protecting groups.
<220>
   <221> misc_feature
   <222> (4)..(4)
   <223> Xaa is norleucine (Nle)
<400> 873
   Phe Asp Lys Xaa
   1
<210> 874
   <211> 4
   <212> PRT
   <213> Artificial
<220>
   <223> Synthetic peptide optionally bearing protecting groups.
<220>
   <221> misc_feature
   <222> (4)..(4)
   <223> Xaa is norleucine (Nle)
<400> 874
   Phe Lys Glu Xaa
   1
<210> 875
   <211> 4
   <212> PRT
   <213> Artificial
<220>
   <223> Synthetic peptide optionally bearing protecting groups.
<220>
   <221> misc_feature
   <222> (4)..(4)
   <223> Xaa is norleucine (Nle)
<400> 875
   Phe Glu Lys Xaa
   1
<210> 876
   <211> 4
   <212> PRT
   <213> Artificial
<220>
   <223> Synthetic peptide optionally bearing protecting groups.
<400> 876
   Phe His Asp Leu
   1
<210> 877
   <211> 4
   <212> PRT
   <213> Artificial
<220>
   <223> Synthetic peptide optionally bearing protecting groups.
<400> 877
   Phe Asp His Leu
   1
<210> 878
   <211> 4
   <212> PRT
   <213> Artificial
<220>
   <223> Synthetic peptide optionally bearing protecting groups.
<400> 878
   Phe His Glu Leu
   1
<210> 879
   <211> 4
   <212> PRT
   <213> Artificial
<220>
   <223> Synthetic peptide optionally bearing protecting groups.
<400> 879
   Phe Glu His Leu
   1
<210> 880
   <211> 4
   <212> PRT
   <213> Artificial
<220>
   <223> Synthetic peptide optionally bearing protecting groups.
<400> 880
   Phe His Asp Ile
   1
<210> 881
   <211> 4
   <212> PRT
   <213> Artificial
<220>
   <223> Synthetic peptide optionally bearing protecting groups.
<400> 881
   Phe Asp His Ile
   1
<210> 882
   <211> 4
   <212> PRT
   <213> Artificial
<220>
   <223> Synthetic peptide optionally bearing protecting groups.
<400> 882
   Phe His Glu Ile
   1
<210> 883
   <211> 4
   <212> PRT
   <213> Artificial
<220>
   <223> Synthetic peptide optionally bearing protecting groups.
<400> 883
   Phe Glu His Ile
   1
<210> 884
   <211> 4
   <212> PRT
   <213> Artificial
<220>
   <223> Synthetic peptide optionally bearing protecting groups.
<220>
   <221> misc_feature
   <222> (4)..(4)
   <223> Xaa is norleucine (Nle)
<400> 884
   Phe His Asp Xaa
   1
<210> 885
   <211> 4
   <212> PRT
   <213> Artificial
<220>
   <223> Synthetic peptide optionally bearing protecting groups.
<220>
   <221> misc_feature
   <222> (4)..(4)
   <223> Xaa is norleucine (Nle)
<400> 885
   Phe Asp His Xaa
   1
<210> 886
   <211> 4
   <212> PRT
   <213> Artificial
<220>
   <223> Synthetic peptide optionally bearing protecting groups.
<220>
   <221> misc_feature
   <222> (4)..(4)
   <223> Xaa is norleucine (Nle)
<400> 886
   Phe His Glu Xaa
   1
<210> 887
   <211> 4
   <212> PRT
   <213> Artificial
<220>
   <223> Synthetic peptide optionally bearing protecting groups.
<220>
   <221> misc_feature
   <222> (4)..(4)
   <223> Xaa is norleucine (Nle)
<400> 887
   Phe Glu His Xaa
   1
<210> 888
   <211> 4
   <212> PRT
   <213> Artificial
<220>
   <223> Synthetic peptide optionally bearing protecting groups.
<400> 888
   Lys Lys Asp Ser
   1
<210> 889
   <211> 4
   <212> PRT
   <213> Artificial
<220>
   <223> Synthetic peptide optionally bearing protecting groups.
<400> 889
   Lys Asp Lys Ser
   1
<210> 890
   <211> 4
   <212> PRT
   <213> Artificial
<220>
   <223> Synthetic peptide optionally bearing protecting groups.
<400> 890
   Lys Lys Glu Ser
   1
<210> 891
   <211> 4
   <212> PRT
   <213> Artificial
<220>
   <223> Synthetic peptide optionally bearing protecting groups.
<400> 891
   Lys Glu Lys Ser
   1
<210> 892
   <211> 4
   <212> PRT
   <213> Artificial
<220>
   <223> Synthetic peptide optionally bearing protecting groups.
<400> 892
   Lys His Asp Ser
   1
<210> 893
   <211> 4
   <212> PRT
   <213> Artificial
<220>
   <223> Synthetic peptide optionally bearing protecting groups.
<400> 893
   Lys Asp His Ser
   1
<210> 894
   <211> 4
   <212> PRT
   <213> Artificial
<220>
   <223> Synthetic peptide optionally bearing protecting groups.
<400> 894
   Lys His Glu Ser
   1
<210> 895
   <211> 4
   <212> PRT
   <213> Artificial
<220>
   <223> Synthetic peptide optionally bearing protecting groups.
<400> 895
   Lys Glu His Ser
   1
<210> 896
   <211> 4
   <212> PRT
   <213> Artificial
<220>
   <223> Synthetic peptide optionally bearing protecting groups.
<400> 896
   Lys Leu Arg Ser
   1
<210> 897
   <211> 4
   <212> PRT
   <213> Artificial
<220>
   <223> Synthetic peptide optionally bearing protecting groups.
<400> 897
   Lys Arg Leu Ser
   1
<210> 898
   <211> 4
   <212> PRT
   <213> Artificial
<220>
   <223> Synthetic peptide optionally bearing protecting groups.
<400> 898
   Lys Leu Arg Thr
   1
<210> 899
   <211> 4
   <212> PRT
   <213> Artificial
<220>
   <223> Synthetic peptide optionally bearing protecting groups.
<400> 899
   Lys Arg Leu Thr
   1
<210> 900
   <211> 4
   <212> PRT
   <213> Artificial
<220>
   <223> Synthetic peptide optionally bearing protecting groups.
<400> 900
   Lys Glu Leu Ser
   1
<210> 901
   <211> 4
   <212> PRT
   <213> Artificial
<220>
   <223> Synthetic peptide optionally bearing protecting groups.
<400> 901
   Lys Leu Glu Ser
   1
<210> 902
   <211> 4
   <212> PRT
   <213> Artificial
<220>
   <223> Synthetic peptide optionally bearing protecting groups.
<400> 902
   Lys Glu Leu Thr
   1
<210> 903
   <211> 4
   <212> PRT
   <213> Artificial
<220>
   <223> Synthetic peptide optionally bearing protecting groups.
<400> 903
   Lys Leu Glu Thr
   1
<210> 904
   <211> 4
   <212> PRT
   <213> Artificial
<220>
   <223> Synthetic peptide optionally bearing protecting groups.
<400> 904
   Lys Leu Arg Ser
   1
<210> 905
   <211> 4
   <212> PRT
   <213> Artificial
<220>
   <223> Synthetic peptide optionally bearing protecting groups.
<400> 905
   Lys Leu Arg Thr
   1
<210> 906
   <211> 4
   <212> PRT
   <213> Artificial
<220>
   <223> Synthetic peptide optionally bearing protecting groups.
<400> 906
   Lys Glu Leu Ser
   1
<210> 907
   <211> 4
   <212> PRT
   <213> Artificial
<220>
   <223> Synthetic peptide optionally bearing protecting groups.
<400> 907
   Lys Glu Leu Thr
   1
<210> 908
   <211> 4
   <212> PRT
   <213> Artificial
<220>
   <223> Synthetic peptide optionally bearing protecting groups.
<400> 908
   Lys Glu Ile Thr
   1
<210> 909
   <211> 4
   <212> PRT
   <213> Artificial
<220>
   <223> Synthetic peptide optionally bearing protecting groups.
<400> 909
   Lys Leu Arg Ser
   1
<210> 910
   <211> 4
   <212> PRT
   <213> Artificial
<220>
   <223> Synthetic peptide optionally bearing protecting groups.
<400> 910
   Lys Leu Arg Thr
   1
<210> 911
   <211> 4
   <212> PRT
   <213> Artificial
<220>
   <223> Synthetic peptide optionally bearing protecting groups.
<400> 911
   Lys Glu Leu Ser
   1
<210> 912
   <211> 4
   <212> PRT
   <213> Artificial
<220>
   <223> Synthetic peptide optionally bearing protecting groups.
<400> 912
   Lys Glu Leu Thr
   1
<210> 913
   <211> 4
   <212> PRT
   <213> Artificial
<220>
   <223> Synthetic peptide optionally bearing protecting groups.
<400> 913
   Lys Leu Arg Ser
   1
<210> 914
   <211> 4
   <212> PRT
   <213> Artificial
<220>
   <223> Synthetic peptide optionally bearing protecting groups.
<400> 914
   Lys Arg Phe Thr
   1
<210> 915
   <211> 4
   <212> PRT
   <213> Artificial
<220>
   <223> Synthetic peptide optionally bearing protecting groups.
<400> 915
   Lys Leu Arg Thr
   1
<210> 916
   <211> 4
   <212> PRT
   <213> Artificial
<220>
   <223> Synthetic peptide optionally bearing protecting groups.
<400> 916
   Lys Glu Ile Thr
   1
<210> 917
   <211> 4
   <212> PRT
   <213> Artificial
<220>
   <223> Synthetic peptide optionally bearing protecting groups.
<400> 917
   Lys Glu Val Thr
   1
<210> 918
   <211> 4
   <212> PRT
   <213> Artificial
<220>
   <223> Synthetic peptide optionally bearing protecting groups.
<400> 918
   Lys Glu Ala Thr
   1
<210> 919
   <211> 4
   <212> PRT
   <213> Artificial
<220>
   <223> Synthetic peptide optionally bearing protecting groups.
<400> 919
   Lys Glu Gly Thr
   1
<210> 920
   <211> 4
   <212> PRT
   <213> Artificial
<220>
   <223> Synthetic peptide optionally bearing protecting groups.
<400> 920
   Lys Glu Leu Ser
   1
<210> 921
   <211> 4
   <212> PRT
   <213> Artificial
<220>
   <223> synthetic peptide optionally bearing protecting groups.
<400> 921
   Lys Glu Leu Thr
   1
<210> 922
   <211> 4
   <212> PRT
   <213> Artificial
<220>
   <223> Synthetic peptide optionally bearing protecting groups.
<400> 922
   Lys Arg Trp Tyr
   1
<210> 923
   <211> 4
   <212> PRT
   <213> Artificial
<220>
   <223> Synthetic peptide optionally bearing protecting groups.
<400> 923
   Lys Trp Arg Tyr
   1
<210> 924
   <211> 4
   <212> PRT
   <213> Artificial
<220>
   <223> Synthetic peptide optionally bearing protecting groups.
<400> 924
   Lys Arg Tyr Trp
   1
<210> 925
   <211> 4
   <212> PRT
   <213> Artificial
<220>
   <223> Synthetic peptide optionally bearing protecting groups.
<400> 925
   Lys Tyr Arg Trp
   1
<210> 926
   <211> 5
   <212> PRT
   <213> Artificial
<220>
   <223> Synthetic peptide optionally bearing protecting groups.
<400> 926
   Lys Arg Tyr Trp Thr
   1 5
<210> 927
   <211> 4
   <212> PRT
   <213> Artificial
<220>
   <223> Synthetic peptide optionally bearing protecting groups.
<400> 927
   Lys Arg Tyr Thr
   1
<210> 928
   <211> 4
   <212> PRT
   <213> Artificial
<220>
   <223> Synthetic peptide optionally bearing protecting groups.
<400> 928
   Lys Arg Trp Thr
   1
<210> 929
   <211> 4
   <212> PRT
   <213> Artificial
<220>
   <223> Synthetic peptide optionally bearing protecting groups.
<400> 929
   Lys Arg Trp Tyr
   1
<210> 930
   <211> 4
   <212> PRT
   <213> Artificial
<220>
   <223> Synthetic peptide optionally bearing protecting groups.
<400> 930
   Lys Arg Tyr Trp
   1
<210> 931
   <211> 5
   <212> PRT
   <213> Artificial
<220>
   <223> Synthetic peptide optionally bearing protecting groups.
<400> 931
   Lys Arg Tyr Trp Thr
   1 5
<210> 932
   <211> 4
   <212> PRT
   <213> Artificial
<220>
   <223> Synthetic peptide optionally bearing protecting groups.
<400> 932
   Lys Arg Tyr Thr
   1
<210> 933
   <211> 4
   <212> PRT
   <213> Artificial
<220>
   <223> synthetic peptide optionally bearing protecting groups.
<400> 933
   Lys Arg Trp Thr
   1
<210> 934
   <211> 4
   <212> PRT
   <213> Artificial
<220>
   <223> Synthetic peptide optionally bearing protecting groups.
<400> 934
   Lys Arg Trp Tyr
   1
<210> 935
   <211> 4
   <212> PRT
   <213> Artificial
<220>
   <223> Synthetic peptide optionally bearing protecting groups.
<400> 935
   Lys Arg Tyr Trp
   1
<210> 936
   <211> 5
   <212> PRT
   <213> Artificial
<220>
   <223> Synthetic peptide optionally bearing protecting groups.
<400> 936
   Lys Arg Tyr Trp Thr
   1 5
<210> 937
   <211> 4
   <212> PRT
   <213> Artificial
<220>
   <223> Synthetic peptide optionally bearing protecting groups.
<400> 937
   Lys Arg Tyr Thr
   1
<210> 938
   <211> 4
   <212> PRT
   <213> Artificial
<220>
   <223> Synthetic peptide optionally bearing protecting groups.
<400> 938
   Lys Arg Trp Thr
   1
<210> 939
   <211> 4
   <212> PRT
   <213> Artificial
<220>
   <223> Synthetic peptide optionally bearing protecting groups.
<400> 939
   Glu Lys Arg Tyr
   1
<210> 940
   <211> 4
   <212> PRT
   <213> Artificial
<220>
   <223> Synthetic peptide optionally bearing protecting groups.
<400> 940
   Lys Arg Trp Tyr
   1
<210> 941
   <211> 4
   <212> PRT
   <213> Artificial
<220>
   <223> synthetic peptide optionally bearing protecting groups.
<400> 941
   Lys Arg Tyr Trp
   1
<210> 942
   <211> 5
   <212> PRT
   <213> Artificial
<220>
   <223> Synthetic peptide optionally bearing protecting groups.
<400> 942
   Lys Arg Tyr Trp Thr
   1               5
<210> 943
   <211> 4
   <212> PRT
   <213> Artificial
<220>
   <223> Synthetic peptide optionally bearing protecting groups.
<400> 943
   Lys Arg Tyr Thr
   1
<210> 944
   <211> 4
   <212> PRT
   <213> Artificial
<220>
   <223> Synthetic peptide optionally bearing protecting groups.
<400> 944
   Lys Arg Phe Thr
   1
<210> 945
   <211> 4
   <212> PRT
   <213> Artificial
<220>
   <223> Synthetic peptide optionally bearing protecting groups.
<400> 945
   Lys Arg Trp Thr
   1
<210> 946
   <211> 5
   <212> PRT
   <213> Artificial
<220>
   <223> Synthetic peptide optionally bearing protecting groups.
<400> 946
   Lys Phe Trp Phe Ser
   1 5

<210> 947
   <211> 5
   <212> PRT
   <213> Artificial
<220>
   <223> Synthetic peptide optionally bearing protecting groups.
<400> 947
   Lys Phe Trp Phe Thr
   1 5
<210> 948
   <211> 5
   <212> PRT
   <213> Artificial
<220>
   <223> Synthetic peptide optionally bearing protecting groups.
<400> 948
   Lys Phe Tyr Phe Ser
   1               5
<210> 949
   <211> 5
   <212> PRT
   <213> Artificial
<220>
   <223> Synthetic peptide optionally bearing protecting groups.
<400> 949
   Lys Phe Tyr Phe Thr
   1               5
<210> 950
   <211> 5
   <212> PRT
   <213> Artificial
<220>
   <223> Synthetic peptide optionally bearing protecting groups.
<400> 950
   Lys Phe His Phe Ser
   1               5
<210> 951
   <211> 5
   <212> PRT
   <213> Artificial
<220>
   <223> Synthetic peptide optionally bearing protecting groups.
<400> 951
   Lys Phe His Phe Thr
   1               5
<210> 952
   <211> 6
   <212> PRT
   <213> Artificial
<220>
   <223> Synthetic peptide optionally bearing protecting groups.
<400> 952
   Lys Val Phe Phe Tyr Ser
   1 5
<210> 953
   <211> 5
   <212> PRT
   <213> Artificial
<220>
   <223> Synthetic peptide optionally bearing protecting groups.
<400> 953
   Lys Phe Trp Phe Ser
   1 5
<210> 954
   <211> 5
   <212> PRT
   <213> Artificial
<220>
   <223> Synthetic peptide optionally bearing protecting groups.
<400> 954
   Lys Phe Trp Phe Thr
   1 5
<210> 955
   <211> 5
   <212> PRT
   <213> Artificial
<220>
   <223> Synthetic peptide optionally bearing protecting groups.
<400> 955
   Lys Phe Tyr Phe Ser
   1 5
<210> 956
   <211> 5
   <212> PRT
   <213> Artificial
<220>
   <223> Synthetic peptide optionally bearing protecting groups.
<400> 956
   Lys Phe Tyr Phe Thr
   1 5
<210> 957
   <211> 5
   <212> PRT
   <213> Artificial
<220>
   <223> Synthetic peptide optionally bearing protecting groups.
<400> 957
   Lys Phe His Phe Ser
   1 5
<210> 958
   <211> 5
   <212> PRT
   <213> Artificial
<220>
   <223> Synthetic peptide optionally bearing protecting groups.
<400> 958
   Lys Phe His Phe Thr
   1 5
<210> 959
   <211> 5
   <212> PRT
   <213> Artificial
<220>
   <223> Synthetic peptide optionally bearing protecting groups.
<400> 959
   Leu Phe Trp Phe Thr
   1 5
<210> 960
   <211> 5
   <212> PRT
   <213> Artificial
<220>
   <223> Synthetic peptide optionally bearing protecting groups.
<400> 960
   Leu Phe Trp Phe Ser
   1 5
<210> 961
   <211> 22
   <212> PRT
   <213> Artificial
<220>
   <223> Synthetic peptide optionally bearing protecting groups.
<400> 961
   Leu Leu Glu Gln Leu Asn Glu Gln Phe Asn Trp Val Ser Arg Leu Ala
   1 5 10 15
   Asn Leu Thr Gln Gly Glu
   20
<210> 962
   <211> 18
   <212> PRT
   <213> Artificial
<220>
   <223> Synthetic peptide optionally bearing protecting groups.
<400> 962
   Leu Leu Glu Gln Leu Asn Glu Gln Phe Asn Trp Val Ser Arg Leu Ala
   1 5 10 15
   Asn Leu
<210> 963
   <211> 25
   <212> PRT
   <213> Artificial
<220>
   <223> Synthetic peptide optionally bearing protecting groups.
<400> 963
   Asn Glu Leu Gln Glu Met Ser Asn Gln Gly Ser Lys Tyr Val Asn Lys
   1 5 10 15
   Glu Ile Gln Asn Ala Val Asn Gly Val
   20 25
<210> 964
   <211> 21
   <212> PRT
   <213> Artificial
<220>
   <223> Synthetic peptide optionally bearing protecting groups.
<400> 964
   Ile Gln Asn Ala Val Asn Gly Val Lys Gln Ile Lys Thr Leu Ile Glu
   1 5 10 15
   Lys Thr Asn Glu Glu
   20
<210> 965
   <211> 32
   <212> PRT
   <213> Artificial
<220>
   <223> Synthetic peptide optionally bearing protecting groups.
<400> 965
   Arg Lys Thr Leu Leu Ser Asn Leu Glu Glu Ala Lys Lys Lys Lys Glu
   1 5 10 15
   Asp Ala Leu Asn Glu Thr Arg Glu Ser Glu Thr Lys Leu Lys Glu Leu
   20 25 30
<210> 966
   <211> 16
   <212> PRT
   <213> Artificial
<220>
   <223> Synthetic peptide optionally bearing protecting groups.
<400> 966
   Pro Gly Val Cys Asn Glu Thr Met Met Ala Leu Trp Glu Glu Cys Lys
   1 5 10 15
<210> 967
   <211> 16
   <212> PRT
   <213> Artificial
<220>
   <223> Synthetic peptide optionally bearing protecting groups.
<400> 967
   Pro Cys Leu Lys Gln Thr Cys Met Lys Phe Tyr Ala Arg Val Cys Arg
   1 5 10 15
<210> 968
   <211> 19
   <212> PRT
   <213> Artificial
<220>
   <223> Synthetic peptide optionally bearing protecting groups.
<400> 968
   Glu Cys Lys Pro Cys Leu Lys Gln Thr Cys Met Lys Phe Tyr Ala Arg
   1               5                   10                  15
   Val Cys Arg
<210> 969
   <211> 10
   <212> PRT
   <213> Artificial
<220>
   <223> Synthetic peptide optionally bearing protecting groups.
<400> 969
   Leu Val Gly Arg Gln Leu Glu Glu Phe Leu
   1               5                   10
<210> 970
   <211> 12
   <212> PRT
   <213> Artificial
<220>
   <223> Synthetic peptide optionally bearing protecting groups.
<400> 970
   Met Asn Gly Asp Arg Ile Asp Ser Leu Leu Glu Asn
   1               5                   10
<210> 971
   <211> 11
   <212> PRT
   <213> Artificial
<220>
   <223> Synthetic peptide optionally bearing protecting groups.
<400> 971
   Gln Gln Thr His Met Leu Asp Val Met Gln Asp
   1               5                   10
<210> 972
   <211> 14
   <212> PRT
   <213> Artificial
<220>
   <223> Synthetic peptide optionally bearing protecting groups.
<400> 972
   Phe Ser Arg Ala Ser Ser Ile Ile Asp Glu Leu Phe Gln Asp
   1 5 10
<210> 973
   <211> 15
   <212> PRT
   <213> Artificial
<220>
   <223> Synthetic peptide optionally bearing protecting groups.
<400> 973
   Pro Phe Leu Glu Met Ile His Glu Ala Gln Gln Ala Met Asp Ile
   1 5 10 15
<210> 974
   <211> 11
   <212> PRT
   <213> Artificial
<220>
   <223> Synthetic peptide optionally bearing protecting groups.
<400> 974
   Pro Thr Glu Phe Ile Arg Glu Gly Asp Asp Asp
   1 5 10
<210> 975
   <211> 15
   <212> PRT
   <213> Artificial
<220>
   <223> Synthetic peptide optionally bearing protecting groups.
<400> 975
   Arg Met Lys Asp Gln Cys Asp Lys Cys Arg Glu Ile Leu Ser Val
   1 5 10 15
<210> 976
   <211> 32
   <212> PRT
   <213> Artificial
<220>
   <223> Synthetic peptide optionally bearing protecting groups.
<400> 976
   Pro Ser Gln Ala Lys Leu Arg Arg Glu Leu Asp Glu Ser Leu Gln Val
   1               5                   10                  15
   Ala Glu Arg Leu Thr Arg Lys Tyr Asn Glu Leu Leu Lys Ser Tyr Gln
               20                  25                  30
<210> 977
   <211> 22
   <212> PRT
   <213> Artificial
<220>
   <223> Synthetic peptide optionally bearing protecting groups.
<400> 977
   Leu Leu Glu Gln Leu Asn Glu Gln Phe Asn Trp Val Ser Arg Leu Ala
   1               5                   10                  15
   Asn Leu Thr Glu Gly Glu
               20
<210> 978
   <211> 11
   <212> PRT
   <213> Artificial
<220>
   <223> Synthetic peptide optionally bearing protecting groups.
<400> 978
   Asp Gln Tyr Tyr Leu Arg Val Thr Thr Val Ala
   1               5                   10
<210> 979
   <211> 14
   <212> PRT
   <213> Artificial
<220>
   <223> Synthetic peptide optionally bearing protecting groups.
<400> 979
   Pro Ser Gly Val Thr Glu Val Val Val Lys Leu Phe Asp Ser
   1               5                   10
<210> 980
   <211> 21
   <212> PRT
   <213> Artificial
<220>
   <223> Synthetic peptide optionally bearing protecting groups.
<400> 980
   Pro Lys Phe Met Glu Thr Val Ala Glu Lys Ala Leu Gln Glu Tyr Arg
   1               5                   10                  15
   Lys Lys His Arg Glu
               20
<210> 981
   <211> 26
   <212> PRT
   <213> Artificial
<220>
   <223> Synthetic peptide optionally bearing protecting groups.
<400> 981
   Trp Asp Arg Val Lys Asp Leu Ala Thr Val Tyr Val Asp Val Leu Lys
   1               5                   10                  15
   Asp Ser Gly Arg Asp Tyr Val Ser Gln Phe
               20                  25
<210> 982
   <211> 25
   <212> PRT
   <213> Artificial
<220>
   <223> Synthetic peptide optionally bearing protecting groups.
<400> 982
   Val Ala Thr Val Met Trp Asp Tyr Phe Ser Gln Leu Ser Asn Asn Ala
   1               5                   10                  15
   Lys Glu Ala Val Glu His Leu Gln Lys
               20                  25
<210> 983
   <211> 27
   <212> PRT
   <213> Artificial
<220>
   <223> Synthetic peptide optionally bearing protecting groups.
<400> 983
   Arg Trp Glu Leu Ala Leu Gly Arg Phe Trp Asp Tyr Leu Arg Trp Val
   1               5                   10                  15
   Gln Thr Leu Ser Glu Gln Val Gln Glu Glu Leu
               20                  25
<210> 984
   <211> 35
   <212> PRT
   <213> Artificial
<220>
   <223> Synthetic peptide optionally bearing protecting groups.
<400> 984
   Leu Ser Ser Gln Val Thr Gln Glu Leu Arg Ala Leu Met Asp Glu Thr
   1               5                   10                  15
   Met Lys Glu Leu Lys Glu Leu Lys Ala Tyr Lys Ser Glu Leu Glu Glu
               20                  25                  30
   Gln Leu Thr
           35
<210> 985
   <211> 26
   <212> PRT
   <213> Artificial
<220>
   <223> Synthetic peptide optionally bearing protecting groups.
<400> 985
   Ala Arg Leu Ser Lys Glu Leu Gln Ala Ala Gln Ala Arg Leu Gly Ala
   1               5                   10                  15
   Asp Met Glu Asp Val Cys Gly Arg Leu Val
               20                  25
<210> 986
   <211> 26
   <212> PRT
   <213> Artificial
<220>
   <223> synthetic peptide optionally bearing protecting groups.
<400> 986
   Val Arg Leu Ala Ser His Leu Arg Lys Leu Arg Lys Arg Leu Leu Arg
   1 5 10 15
   Asp Ala Asp Asp Leu Gln Lys Arg Leu Ala
   20 25
<210> 987
   <211> 19
   <212> PRT
   <213> Artificial
<220>
   <223> Synthetic peptide optionally bearing protecting groups.
<400> 987
   Pro Leu Val Glu Asp Met Gln Arg Gln Trp Ala Gly Leu Val Glu Lys
   1 5 10 15
   Val Gln Ala
<210> 988
   <211> 17
   <212> PRT
   <213> Artificial
<220>
   <223> Synthetic peptide optionally bearing protecting groups.
<400> 988
   Met Ser Thr Tyr Thr Gly Ile Phe Thr Asp Gln Val Leu Ser Val Leu
   1 5 10 15
   Lys
<210> 989
   <211> 22
   <212> PRT
   <213> Artificial
<220>
   <223> Synthetic peptide optionally bearing protecting groups.
<400> 989
   Leu Leu Ser Phe Met Gln Gly Tyr Met Lys His Ala Thr Lys Thr Ala
   1 5 10 15
   Lys Asp Ala Leu Ser Ser
   20
<210> 990
   <211> 17
   <212> PRT
   <213> Artificial
<220>
   <223> Synthetic peptide optionally bearing protecting groups.
<400> 990
   Lys Trp Ile Tyr His Leu Thr Glu Gly Ser Thr Asp Leu Arg Thr Glu
   1 5 10 15
   Gly
<210> 991
   <211> 17
   <212> PRT
   <213> Artificial
<220>
   <223> Synthetic peptide optionally bearing protecting groups.
<400> 991
   Lys Trp Phe Tyr His Leu Thr Glu Gly Ser Thr Asp Leu Arg Thr Glu
   1 5 10 15
   Gly
<210> 992
   <211> 17
   <212> PRT
   <213> Artificial
<220>
   <223> Synthetic peptide optionally bearing protecting groups.
<400> 992
   Lys Trp Leu Tyr His Leu Thr Glu Gly Ser Thr Asp Leu Arg Thr Glu
   1 5 10 15
   Gly
<210> 993
   <211> 17
   <212> PRT
   <213> Artificial
<220>
   <223> Synthetic peptide optionally bearing protecting groups.
<400> 993
   Lys Trp Val Tyr His Leu Thr Glu Gly Ser Thr Asp Leu Arg Thr Glu
   1 5 10 15
   Gly
<210> 994
   <211> 17
   <212> PRT
   <213> Artificial
<220>
   <223> Synthetic peptide optionally bearing protecting groups.
<400> 994
   Lys Tyr Ile Trp His Leu Thr Glu Gly Ser Thr Asp Leu Arg Thr Glu
   1 5 10 15
   Gly
<210> 995
   <211> 17
   <212> PRT
   <213> Artificial
<220>
   <223> Synthetic peptide optionally bearing protecting groups.
<400> 995
   Lys Trp Ile Tyr His Phe Thr Glu Gly Ser Thr Asp Leu Arg Thr Glu
   1 5 10 15
   Gly
<210> 996
   <211> 17
   <212> PRT
   <213> Artificial
<220>
   <223> Synthetic peptide optionally bearing protecting groups.
<400> 996
   Lys Trp Phe Tyr His Ile Thr Glu Gly Ser Thr Asp Leu Arg Thr Glu
   1 5 10 15
   Gly
<210> 997
   <211> 17
   <212> PRT
   <213> Artificial
<220>
   <223> synthetic peptide optionally bearing protecting groups.
<400> 997
   Lys Trp Leu Tyr His Val Thr Glu Gly Ser Thr Asp Leu Arg Thr Glu
   1               5                   10                  15
   Gly
<210> 998
   <211> 17
   <212> PRT
   <213> Artificial
<220>
   <223> Synthetic peptide optionally bearing protecting groups.
<400> 998
   Lys Trp Val Tyr His Tyr Thr Glu Gly Ser Thr Asp Leu Arg Thr Glu
   1               5                   10                  15
   Gly
<210> 999
   <211> 17
   <212> PRT
   <213> Artificial
<220>
   <223> Synthetic peptide optionally bearing protecting groups.
<400> 999
   Lys Tyr Ile Trp His Phe Thr Glu Gly Ser Thr Asp Leu Arg Thr Glu
   1               5                   10                  15
   Gly
<210> 1000
   <211> 17
   <212> PRT
   <213> Artificial
<220>
   <223> Synthetic peptide optionally bearing protecting groups.
<400> 1000
   Lys Tyr Ile Trp His Ile Thr Glu Gly Ser Thr Asp Leu Arg Thr Glu
   1               5                   10                  15
   Gly
<210> 1001
   <211> 17
   <212> PRT
   <213> Artificial
<220>
   <223> Synthetic peptide optionally bearing protecting groups.
<400> 1001
   Lys Tyr Ile Trp His Val Thr Glu Gly Ser Thr Asp Leu Arg Thr Glu
   1               5                   10                  15
   Gly
<210> 1002
   <211> 17
   <212> PRT
   <213> Artificial
<220>
   <223> Synthetic peptide optionally bearing protecting groups.
<400> 1002
   Lys Tyr Ile Trp His Tyr Thr Glu Gly Ser Thr Asp Leu Arg Thr Glu
   1               5                   10                  15
   Gly
<210> 1003
   <211> 17
   <212> PRT
   <213> Artificial
<220>
   <223> Synthetic peptide optionally bearing protecting groups.
<400> 1003
   Lys Phe Ile Trp His Leu Thr Glu Gly Ser Thr Asp Leu Arg Thr Glu
   1               5                   10                  15
   Gly
<210> 1004
   <211> 17
   <212> PRT
   <213> Artificial
<220>
   <223> Synthetic peptide optionally bearing protecting groups.
<400> 1004
   Lys Leu Ile Trp His Leu Thr Glu Gly Ser Thr Asp Leu Arg Thr Glu
   1               5                   10                  15
   Gly
<210> 1005
   <211> 17
   <212> PRT
   <213> Artificial
<220>
   <223> Synthetic peptide optionally bearing protecting groups.
<400> 1005
   Lys Ile Ile Trp His Leu Thr Glu Gly Ser Thr Asp Leu Arg Thr Glu
   1               5                   10                  15
   Gly
<210> 1006
   <211> 17
   <212> PRT
   <213> Artificial
<220>
   <223> Synthetic peptide optionally bearing protecting groups.
<400> 1006
   Lys Tyr Ile Trp Phe Leu Thr Glu Gly Ser Thr Asp Leu Arg Thr Glu
   1               5                   10                  15
   Gly
<210> 1007
   <211> 17
   <212> PRT
   <213> Artificial
<220>
   <223> Synthetic peptide optionally bearing protecting groups.
<400> 1007
   Lys Trp Ile Tyr Phe Leu Thr Glu Gly Ser Thr Asp Leu Arg Thr Glu
   1               5                   10                  15
   Gly
<210> 1008
   <211> 17
   <212> PRT
   <213> Artificial
<220>
   <223> Synthetic peptide optionally bearing protecting groups.
<400> 1008
   Lys Trp Ile Tyr Leu Leu Thr Glu Gly Ser Thr Asp Leu Arg Thr Glu
   1               5                   10                  15
   Gly
<210> 1009
   <211> 17
   <212> PRT
   <213> Artificial
<220>
   <223> Synthetic peptide optionally bearing protecting groups.
<400> 1009
   Lys Trp Ile Tyr His Phe Thr Glu Gly Ser Thr Asp Leu Arg Thr Glu
   1               5                   10                  15
   Gly
<210> 1010
   <211> 17
   <212> PRT
   <213> Artificial
<220>
   <223> Synthetic peptide optionally bearing protecting groups.
<400> 1010
   Lys Trp Ile Tyr His Tyr Thr Glu Gly Ser Thr Asp Leu Arg Thr Glu
   1               5                   10                  15
   Gly
<210> 1011
   <211> 17
   <212> PRT
   <213> Artificial
<220>
   <223> Synthetic peptide optionally bearing protecting groups.
<400> 1011
   Lys Trp Ile Tyr His Ile Thr Glu Gly Ser Thr Asp Leu Arg Thr Glu
   1 5 10 15
   Gly
<210> 1012
   <211> 17
   <212> PRT
   <213> Artificial
<220>
   <223> Synthetic peptide optionally bearing protecting groups.
<400> 1012
   Lys Trp Ile Tyr His Leu Ser Glu Gly Ser Thr Asp Leu Arg Thr Glu
   1 5 10 15
   Gly
<210> 1013
   <211> 17
   <212> PRT
   <213> Artificial
<220>
   <223> Synthetic peptide optionally bearing protecting groups.
<400> 1013
   Lys Trp Ile Tyr His Leu Thr Asp Gly Ser Thr Asp Leu Arg Thr Glu
   1 5 10 15
   Gly
<210> 1014
   <211> 17
   <212> PRT
   <213> Artificial
<220>
   <223> Synthetic peptide optionally bearing protecting groups.
<400> 1014
   Lys Trp Ile Tyr His Leu Thr Glu Gly Thr Ser Asp Leu Arg Thr Glu
   1 5 10 15
   Gly
<210> 1015
   <211> 17
   <212> PRT
   <213> Artificial
<220>
   <223> Synthetic peptide optionally bearing protecting groups.
<400> 1015
   Lys Trp Ile Tyr His Leu Thr Glu Gly Ser Thr Glu Leu Arg Thr Glu
   1               5                   10                  15
   Gly
<210> 1016
   <211> 17
   <212> PRT
   <213> Artificial
<220>
   <223> Synthetic peptide optionally bearing protecting groups.
<400> 1016
   Lys Trp Ile Tyr His Leu Thr Glu Gly Ser Thr Asp Phe Arg Thr Glu
   1               5                   10                  15
   Gly
<210> 1017
   <211> 17
   <212> PRT
   <213> Artificial
<220>
   <223> Synthetic peptide optionally bearing protecting groups.
<400> 1017
   Lys Trp Ile Tyr His Leu Thr Glu Gly Ser Thr Asp Tyr Arg Thr Glu
   1               5                   10                  15
   Gly
<210> 1018
   <211> 17
   <212> PRT
   <213> Artificial
<220>
   <223> Synthetic peptide optionally bearing protecting groups.
<400> 1018
   Lys Trp Ile Tyr His Leu Thr Glu Gly Ser Thr Asp Ile Arg Thr Glu
   1               5                   10                  15
   Gly
<210> 1019
   <211> 17
   <212> PRT
   <213> Artificial
<220>
   <223> Synthetic peptide optionally bearing protecting groups.
<400> 1019
   Lys Trp Ile Tyr His Leu Thr Glu Gly Ser Thr Asp Val Arg Thr Glu
   1 5 10 15
   Gly
<210> 1020
   <211> 17
   <212> PRT
   <213> Artificial
<220>
   <223> Synthetic peptide optionally bearing protecting groups.
<400> 1020
   Lys Trp Ile Tyr His Leu Thr Glu Gly Ser Thr Asp Leu Lys Thr Glu
   1 5 10 15
   Gly
<210> 1021
   <211> 17
   <212> PRT
   <213> Artificial
<220>
   <223> Synthetic peptide optionally bearing protecting groups.
<400> 1021
   Lys Trp Ile Tyr His Leu Thr Glu Gly Ser Thr Asp Leu Arg Ser Glu
   1 5 10 15
   Gly
<210> 1022
   <211> 17
   <212> PRT
   <213> Artificial
<220>
   <223> Synthetic peptide optionally bearing protecting groups.
<400> 1022
   Lys Trp Ile Tyr His Leu Thr Glu Gly Ser Thr Asp Leu Arg Thr Asp
   1 5 10 15
   Gly
<210> 1023
   <211> 17
   <212> PRT
   <213> Artificial
<220>
   <223> Synthetic peptide optionally bearing protecting groups.
<400> 1023
   Lys Trp Ile Tyr His Leu Thr Glu Gly Ser Thr Asp Ile Lys Thr Glu
   1 5 10 15
   Gly
<210> 1024
   <211> 17
   <212> PRT
   <213> Artificial
<220>
   <223> Synthetic peptide optionally bearing protecting groups.
<400> 1024
   Lys Trp Ile Tyr His Leu Thr Glu Gly Ser Thr Asp Ile Arg Ser Glu
   1 5 10 15
   Gly
<210> 1025
   <211> 17
   <212> PRT
   <213> Artificial
<220>
   <223> Synthetic peptide optionally bearing protecting groups.
<400> 1025
   Lys Trp Ile Tyr His Leu Thr Glu Gly Ser Thr Asp Ile Lys Ser Glu
   1 5 10 15
   Gly
<210> 1026
   <211> 17
   <212> PRT
   <213> Artificial
<220>
   <223> Synthetic peptide optionally bearing protecting groups.
<400> 1026
   Lys Trp Ile Tyr His Leu Thr Glu Gly Ser Thr Asp Ile Lys Ser Asp
   1               5                   10                  15
   Gly
<210> 1027
   <211> 17
   <212> PRT
   <213> Artificial
<220>
   <223> Synthetic peptide optionally bearing protecting groups.
<400> 1027
   Arg Trp Ile Tyr His Leu Thr Glu Gly Ser Thr Asp Leu Arg Thr Glu
   1               5                   10                  15
   Gly
<210> 1028
   <211> 17
   <212> PRT
   <213> Artificial
<220>
   <223> Synthetic peptide optionally bearing protecting groups.
<400> 1028
   Arg Tyr Ile Trp His Leu Thr Glu Gly Ser Thr Asp Ile Arg Thr Glu
   1               5                   10                  15
   Gly

<210> 1029
   <211> 17
   <212> PRT
   <213> Artificial
<220>
   <223> Synthetic peptide optionally bearing protecting groups.
<400> 1029
   Arg Trp Ile Tyr His Leu Thr Glu Gly Ser Thr Asp Ile Arg Thr Asp
   1               5                   10                  15
   Gly
<210> 1030
   <211> 17
   <212> PRT
   <213> Artificial
<220>
   <223> Synthetic peptide optionally bearing protecting groups.
<400> 1030
   Arg Trp Ile Phe His Leu Thr Glu Gly Ser Thr Asp Ile Arg Thr Glu
   1 5 10 15
   Gly
<210> 1031
   <211> 17
   <212> PRT
   <213> Artificial
<220>
   <223> Synthetic peptide optionally bearing protecting groups.
<400> 1031
   Arg Trp Ile Tyr His Leu Thr Glu Gly Ser Thr Asp Leu Lys Thr Glu
   1 5 10 15
   Gly
<210> 1032
   <211> 17
   <212> PRT
   <213> Artificial
<220>
   <223> Synthetic peptide optionally bearing protecting groups.
<400> 1032
   Arg Trp Ile Tyr His Leu Thr Asp Gly Ser Thr Asp Ile Arg Thr Glu
   1 5 10 15
   Gly
<210> 1033
   <211> 17
   <212> PRT
   <213> Artificial
<220>
   <223> Synthetic peptide optionally bearing protecting groups.
<400> 1033
   Arg Trp Ile Tyr His Leu Thr Asp Gly Ser Thr Asp Leu Arg Thr Glu
   1 5 10 15
   Gly
<210> 1034
   <211> 17
   <212> PRT
   <213> Artificial
<220>
   <223> Synthetic peptide optionally bearing protecting groups.
<400> 1034
   Arg Trp Ile Tyr Phe Leu Thr Glu Gly Ser Thr Asp Ile Arg Thr Glu
   1 5 10 15
   Gly
<210> 1035
   <211> 17
   <212> PRT
   <213> Artificial
<220>
   <223> Synthetic peptide optionally bearing protecting groups.
<400> 1035
   Arg Trp Ile Tyr Phe Leu Thr Glu Gly Ser Thr Asp Leu Arg Thr Glu
   1 5 10 15
   Gly
<210> 1036
   <211> 17
   <212> PRT
   <213> Artificial
<220>
   <223> Synthetic peptide optionally bearing protecting groups.
<400> 1036
   Lys Trp Phe Tyr His Leu Thr Glu Gly Ser Thr Asp Phe Arg Thr Glu
   1 5 10 15
   Gly
<210> 1037
   <211> 17
   <212> PRT
   <213> Artificial
<220>
   <223> Synthetic peptide optionally bearing protecting groups.
<400> 1037
   Arg Trp Phe Tyr His Leu Thr Glu Gly Ser Thr Asp Leu Arg Thr Glu
   1 5 10 15
   Gly
<210> 1038
   <211> 17
   <212> PRT
   <213> Artificial
<220>
   <223> Synthetic peptide optionally bearing protecting groups.
<400> 1038
   Lys Trp Ile Phe His Leu Thr Glu Gly Ser Thr Asp Ile Arg Thr Asp
   1 5 10 15
   Gly
<210> 1039
   <211> 17
   <212> PRT
   <213> Artificial
<220>
   <223> Synthetic peptide optionally bearing protecting groups.
<400> 1039
   Arg Trp Ile Tyr His Leu Thr Glu Gly Ser Thr Asp Ile Arg Thr Asp
   1 5 10 15
   Gly
<210> 1040
   <211> 17
   <212> PRT
   <213> Artificial
<220>
   <223> Synthetic peptide optionally bearing protecting groups.
<400> 1040
   Arg Trp Ile Tyr His Leu Thr Glu Gly Ser Thr Asp Leu Arg Thr Asp
   1 5 10 15
   Gly
<210> 1041
   <211> 17
   <212> PRT
   <213> Artificial
<220>
   <223> Synthetic peptide optionally bearing protecting groups.
<400> 1041
   Lys Trp Ile Tyr His Leu Thr Glu Gly Ser Thr Asp Ile Lys Thr Glu
   1               5                   10                  15
   Gly
<210> 1042
   <211> 17
   <212> PRT
   <213> Artificial
<220>
   <223> Synthetic peptide optionally bearing protecting groups.
<400> 1042
   Lys Trp Ile Tyr His Leu Thr Glu Gly Ser Thr Asp Ile Lys Thr Asp
   1               5                   10                  15
   Gly
<210> 1043
   <211> 17
   <212> PRT
   <213> Artificial
<220>
   <223> Synthetic peptide optionally bearing protecting groups.
<400> 1043
   Lys Trp Ile Tyr His Leu Thr Glu Gly Ser Thr Asp Phe Lys Thr Glu
   1               5                   10                  15
   Gly
<210> 1044
   <211> 17
   <212> PRT
   <213> Artificial
<220>
   <223> Synthetic peptide optionally bearing protecting groups.
<400> 1044
   Lys Trp Ile Tyr His Leu Thr Glu Gly Ser Thr Asp Tyr Lys Thr Glu
   1 5 10 15
   Gly
<210> 1045
   <211> 17
   <212> PRT
   <213> Artificial
<220>
   <223> Synthetic peptide optionally bearing protecting groups.
<400> 1045
   Lys Trp Ile Tyr His Leu Thr Glu Gly Ser Thr Asp Ile Arg Thr Glu
   1 5 10 15
   Gly
<210> 1046
   <211> 17
   <212> PRT
   <213> Artificial
<220>
   <223> Synthetic peptide optionally bearing protecting groups.
<400> 1046
   Lys Trp Phe Tyr His Phe Thr Glu Gly Ser Thr Asp Leu Arg Thr Glu
   1 5 10 15
   Gly
<210> 1047
   <211> 17
   <212> PRT
   <213> Artificial
<220>
   <223> Synthetic peptide optionally bearing protecting groups.
<400> 1047
   Arg Trp Phe Tyr His Phe Thr Glu Gly Ser Thr Asp Leu Arg Thr Glu
   1 5 10 15
   Gly
<210> 1048
   <211> 17
   <212> PRT
   <213> Artificial
<220>
   <223> Synthetic peptide optionally bearing protecting groups.
<400> 1048
   Lys Trp Phe Tyr His Phe Thr Glu Gly Ser Thr Asp Phe Arg Thr Glu
   1 5 10 15
   Gly
<210> 1049
   <211> 17
   <212> PRT
   <213> Artificial
<220>
   <223> Synthetic peptide optionally bearing protecting groups.
<400> 1049
   Lys Trp Phe Tyr His Phe Thr Asp Gly Ser Thr Asp Ile Arg Thr Glu
   1 5 10 15
   Gly
<210> 1050
   <211> 17
   <212> PRT
   <213> Artificial
<220>
   <223> Synthetic peptide optionally bearing protecting groups.
<400> 1050
   Arg Trp Phe Tyr His Phe Thr Glu Gly Ser Thr Asp Leu Arg Thr Glu
   1 5 10 15
   Gly
<210> 1051
   <211> 17
   <212> PRT
   <213> Artificial
<220>
   <223> Synthetic peptide optionally bearing protecting groups.
<400> 1051
   Arg Trp Phe Tyr His Phe Thr Glu Gly Ser Thr Asp Phe Arg Thr Glu
   1 5 10 15
   Gly
<210> 1052
   <211> 17
   <212> PRT
   <213> Artificial
<220>
   <223> Synthetic peptide optionally bearing protecting groups.
<400> 1052
   Arg Trp Phe Tyr His Phe Thr Glu Gly Ser Thr Asp Phe Arg Thr Asp
   1 5 10 15
   Gly
<210> 1053
   <211> 19
   <212> PRT
   <213> Artificial
<220>
   <223> Synthetic peptide optionally bearing protecting groups.
<400> 1053
   Glu Lys Cys Val Glu Glu Phe Lys Ser Leu Thr Ser Cys Leu Asp Ser
   1 5 10 15
   Lys Ala Phe
<210> 1054
   <211> 19
   <212> PRT
   <213> Artificial
<220>
   <223> Synthetic peptide optionally bearing protecting groups.
<400> 1054
   Asp Lys Cys Val Glu Glu Phe Lys Ser Leu Thr Ser Cys Leu Asp Ser
   1 5 10 15
   Lys Ala Phe
<210> 1055
   <211> 19
   <212> PRT
   <213> Artificial
<220>
   <223> Synthetic peptide optionally bearing protecting groups.
<400> 1055
   Glu Lys Cys Val Asp Glu Phe Lys Ser Leu Thr Ser Cys Leu Asp Ser
   1 5 10 15
   Lys Ala Phe
<210> 1056
   <211> 19
   <212> PRT
   <213> Artificial
<220>
   <223> Synthetic peptide optionally bearing protecting groups.
<400> 1056
   Glu Lys Cys Val Glu Asp Phe Lys Ser Leu Thr Ser Cys Leu Asp Ser
   1 5 10 15
   Lys Ala Phe
<210> 1057
   <211> 19
   <212> PRT
   <213> Artificial
<220>
   <223> Synthetic peptide optionally bearing protecting groups.
<400> 1057
   Glu Arg Cys Val Glu Glu Phe Lys Ser Leu Thr Ser Cys Leu Asp Ser
   1 5 10 15
   Lys Ala Phe
<210> 1058
   <211> 19
   <212> PRT
   <213> Artificial
<220>
   <223> Synthetic peptide optionally bearing protecting groups.
<400> 1058
   Asp Lys Cys Val Asp Asp Phe Lys Ser Leu Thr Ser Cys Leu Asp Ser
   1 5 10 15
   Lys Ala Phe
<210> 1059
   <211> 19
   <212> PRT
   <213> Artificial
<220>
   <223> Synthetic peptide optionally bearing protecting groups.
<400> 1059
   Asp Arg Cys Val Glu Glu Phe Lys Ser Leu Thr Ser Cys Leu Asp Ser
   1 5 10 15
   Lys Ala Phe
<210> 1060
   <211> 19
   <212> PRT
   <213> Artificial
<220>
   <223> Synthetic peptide optionally bearing protecting groups.
<400> 1060
   Glu Arg Cys Val Asp Asp Phe Lys Ser Leu Thr Ser Cys Leu Asp Ser
   1 5 10 15
   Lys Ala Phe
<210> 1061
   <211> 19
   <212> PRT
   <213> Artificial
<220>
   <223> Synthetic peptide optionally bearing protecting groups.
<400> 1061
   Glu Lys Cys Val Glu Glu Phe Lys Ser Phe Thr Ser Cys Leu Asp Ser
   1 5 10 15
   Lys Ala Phe
<210> 1062
   <211> 19
   <212> PRT
   <213> Artificial
<220>
   <223> Synthetic peptide optionally bearing protecting groups.
<400> 1062
   Glu Lys Cys Val Glu Glu Phe Lys Ser Ile Thr Ser Cys Leu Asp Ser
   1 5 10 15
   Lys Ala Phe
<210> 1063
   <211> 19
   <212> PRT
   <213> Artificial
<220>
   <223> Synthetic peptide optionally bearing protecting groups.
<400> 1063
   Glu Lys Cys Val Glu Glu Phe Lys Ser Val Thr Ser Cys Leu Asp Ser
   1 5 10 15
   Lys Ala Phe
<210> 1064
   <211> 19
   <212> PRT
   <213> Artificial
<220>
   <223> Synthetic peptide optionally bearing protecting groups.
<400> 1064
   Glu Arg Cys Val Glu Glu Phe Lys Ser Tyr Thr Ser Cys Leu Asp Ser
   1 5 10 15
   Lys Ala Phe
<210> 1065
   <211> 19
   <212> PRT
   <213> Artificial
<220>
   <223> Synthetic peptide optionally bearing protecting groups.
<400> 1065
   Glu Arg Cys Val Glu Glu Phe Lys Ser Phe Thr Ser Cys Leu Asp Ser
   1 5 10 15
   Lys Ala Phe
<210> 1066
   <211> 19
   <212> PRT
   <213> Artificial
<220>
   <223> Synthetic peptide optionally bearing protecting groups.
<400> 1066
   Glu Arg Cys Val Glu Glu Phe Lys Ser Ile Thr Ser Cys Leu Asp Ser
   1 5 10 15
   Lys Ala Phe
<210> 1067
   <211> 19
   <212> PRT
   <213> Artificial
<220>
   <223> Synthetic peptide optionally bearing protecting groups.
<400> 1067
   Glu Arg Cys Val Glu Glu Phe Lys Ser Val Thr Ser Cys Leu Asp Ser
   1 5 10 15
   Lys Ala Phe
<210> 1068
   <211> 19
   <212> PRT
   <213> Artificial
<220>
   <223> Synthetic peptide optionally bearing protecting groups.
<400> 1068
   Glu Arg Cys Val Glu Glu Phe Lys Ser Tyr Thr Ser Cys Leu Asp Ser
   1 5 10 15
   Lys Ala Phe
<210> 1069
   <211> 19
   <212> PRT
   <213> Artificial
<220>
   <223> Synthetic peptide optionally bearing protecting groups.
<400> 1069
   Glu Lys Cys Val Glu Glu Phe Lys Ser Phe Thr Thr Cys Leu Asp Ser
   1 5 10 15
   Lys Ala Phe
<210> 1070
   <211> 19
   <212> PRT
   <213> Artificial
<220>
   <223> Synthetic peptide optionally bearing protecting groups.
<400> 1070
   Glu Lys Cys Val Glu Glu Phe Lys Ser Ile Ser Ser Cys Leu Asp Ser
   1               5                   10                  15
   Lys Ala Phe
<210> 1071
   <211> 19
   <212> PRT
   <213> Artificial
<220>
   <223> Synthetic peptide optionally bearing protecting groups.
<400> 1071
   Glu Lys Cys Val Glu Glu Phe Lys Ser Val Ser Thr Cys Leu Asp Ser
   1               5                   10                  15
   Lys Ala Phe
<210> 1072
   <211> 19
   <212> PRT
   <213> Artificial
<220>
   <223> Synthetic peptide optionally bearing protecting groups.
<400> 1072
   Glu Lys Cys Val Glu Glu Phe Lys Ser Tyr Thr Ser Cys Leu Asp Ser
   1               5                   10                  15
   Lys Ala Phe
<210> 1073
   <211> 19
   <212> PRT
   <213> Artificial
<220>
   <223> Synthetic peptide optionally bearing protecting groups.
<400> 1073
   Glu Lys Cys Val Glu Glu Phe Lys Ser Phe Thr Thr Cys Leu Asp Ser
   1               5                   10                  15
   Lys Ala Phe
<210> 1074
   <211> 19
   <212> PRT
   <213> Artificial
<220>
   <223> Synthetic peptide optionally bearing protecting groups.
<400> 1074
   Glu Lys Cys Val Glu Glu Phe Lys Ser Phe Ser Ser Cys Leu Asp Ser
   1 5 10 15
   Lys Ala Phe
<210> 1075
   <211> 19
   <212> PRT
   <213> Artificial
<220>
   <223> Synthetic peptide optionally bearing protecting groups.
<400> 1075
   Glu Lys Cys Val Glu Glu Phe Lys Ser Phe Thr Ser Cys Leu Asp Ser
   1 5 10 15
   Lys Ala Phe
<210> 1076
   <211> 19
   <212> PRT
   <213> Artificial
<220>
   <223> Synthetic peptide optionally bearing protecting groups.
<400> 1076
   Glu Lys Cys Val Glu Glu Phe Lys Ser Phe Thr Ser Cys Leu Asp Ser
   1 5 10 15
   Lys Ala Phe
<210> 1077
   <211> 19
   <212> PRT
   <213> Artificial
<220>
   <223> Synthetic peptide optionally bearing protecting groups.
<400> 1077
   Glu Lys Cys Val Glu Glu Phe Lys Ser Phe Thr Ser Cys Leu Asp Ser
   1               5                   10                  15
   Lys Ala Phe
<210> 1078
   <211> 19
   <212> PRT
   <213> Artificial
<220>
   <223> Synthetic peptide optionally bearing protecting groups.
<400> 1078
   Glu Lys Cys Val Glu Glu Phe Lys Ser Phe Thr Ser Cys Phe Asp Ser
   1               5                   10                  15
   Lys Ala Phe
<210> 1079
   <211> 19
   <212> PRT
   <213> Artificial
<220>
   <223> Synthetic peptide optionally bearing protecting groups.
<400> 1079
   Glu Lys Cys Val Glu Glu Phe Lys Ser Phe Thr Ser Cys Phe Glu Ser
   1               5                   10                  15
   Lys Ala Phe
<210> 1080
   <211> 19
   <212> PRT
   <213> Artificial
<220>
   <223> Synthetic peptide optionally bearing protecting groups.
<400> 1080
   Glu Lys Cys Val Glu Glu Phe Lys Ser Phe Thr Ser Cys Leu Glu Ser
   1               5                   10                  15
   Lys Ala Phe
<210> 1081
   <211> 19
   <212> PRT
   <213> Artificial
<220>
   <223> Synthetic peptide optionally bearing protecting groups.
<400> 1081
   Glu Lys Cys Val Glu Glu Phe Lys Ser Phe Thr Ser Cys Ile Asp Ser
   1 5 10 15
   Lys Ala Phe
<210> 1082
   <211> 19
   <212> PRT
   <213> Artificial
<220>
   <223> Synthetic peptide optionally bearing protecting groups.
<400> 1082
   Glu Lys Cys Val Glu Glu Leu Lys Ser Phe Thr Ser Cys Phe Asp Ser
   1 5 10 15
   Lys Ala Phe
<210> 1083
   <211> 19
   <212> PRT
   <213> Artificial
<220>
   <223> Synthetic peptide optionally bearing protecting groups.
<400> 1083
   Asp Lys Cys Val Glu Glu Phe Lys Ser Phe Thr Ser Cys Phe Asp Ser
   1 5 10 15
   Lys Ala Phe
<210> 1084
   <211> 19
   <212> PRT
   <213> Artificial
<220>
   <223> Synthetic peptide optionally bearing protecting groups.
<400> 1084
   Asp Lys Cys Val Glu Glu Phe Lys Ser Phe Thr Ser Cys Phe Glu Ser
   1 5 10 15
   Lys Ala Phe
<210> 1085
   <211> 19
   <212> PRT
   <213> Artificial
<220>
   <223> Synthetic peptide optionally bearing protecting groups.
<400> 1085
   Glu Arg Cys Val Glu Glu Phe Lys Ser Phe Thr Ser Cys Phe Asp Ser
   1 5 10 15
   Lys Ala Phe
<210> 1086
   <211> 19
   <212> PRT
   <213> Artificial
<220>
   <223> Synthetic peptide optionally bearing protecting groups.
<400> 1086
   Glu Lys Cys Phe Glu Glu Phe Lys Ser Phe Thr Ser Cys Phe Asp Ser
   1 5 10 15
   Lys Ala Phe
<210> 1087
   <211> 19
   <212> PRT
   <213> Artificial
<220>
   <223> Synthetic peptide optionally bearing protecting groups.
<400> 1087
   Glu Lys Cys Phe Glu Glu Phe Lys Ser Phe Thr Ser Cys Phe Glu Ser
   1 5 10 15
   Lys Ala Phe
<210> 1088
   <211> 19
   <212> PRT
   <213> Artificial
<220>
   <223> Synthetic peptide optionally bearing protecting groups.
<400> 1088
   Glu Lys Cys Val Glu Glu Phe Lys Ser Phe Ser Ser Cys Phe Glu Ser
   1 5 10 15
   Lys Ala Phe
<210> 1089
   <211> 19
   <212> PRT
   <213> Artificial
<220>
   <223> Synthetic peptide optionally bearing protecting groups.
<400> 1089
   Glu Lys Cys Val Glu Glu Phe Lys Ser Phe Gln Ser Cys Phe Asp Ser
   1 5 10 15
   Lys Ala Phe
<210> 1090
   <211> 19
   <212> PRT
   <213> Artificial
<220>
   <223> Synthetic peptide optionally bearing protecting groups.
<400> 1090
   Glu Lys Cys Phe Glu Glu Phe Lys Ser Phe Gln Ser Cys Phe Asp Ser
   1 5 10 15
   Lys Ala Phe
<210> 1091
   <211> 19
   <212> PRT
   <213> Artificial
<220>
   <223> Synthetic peptide optionally bearing protecting groups.
<400> 1091
   Glu Lys Cys Val Glu Glu Phe Lys Gln Phe Thr Ser Cys Phe Asp Ser
   1 5 10 15
   Lys Ala Phe
<210> 1092
   <211> 19
   <212> PRT
   <213> Artificial
<220>
   <223> Synthetic peptide optionally bearing protecting groups.
<400> 1092
   Glu Lys Cys Val Glu Glu Phe Lys Gln Leu Thr Ser Cys Leu Asp Ser
   1 5 10 15
   Lys Ala Phe
<210> 1093
   <211> 19
   <212> PRT
   <213> Artificial
<220>
   <223> Synthetic peptide optionally bearing protecting groups.
<400> 1093
   Glu Lys Cys Phe Glu Glu Phe Lys Ser Phe Gln Ser Cys Leu Asp Ser
   1 5 10 15
   Lys Ala Phe
<210> 1094
   <211> 19
   <212> PRT
   <213> Artificial
<220>
   <223> Synthetic peptide optionally bearing protecting groups.
<400> 1094
   Glu Lys Cys Val Glu Glu Phe Lys Gln Phe Thr Ser Cys Phe Asp Ser
   1 5 10 15
   Lys Ala Phe
<210> 1095
   <211> 19
   <212> PRT
   <213> Artificial
<220>
   <223> Synthetic peptide optionally bearing protecting groups.
<400> 1095
   Glu Lys Cys Val Glu Glu Phe Lys Ser Phe Thr Ser Cys Phe Glu Ser
   1 5 10 15
   Lys Ala Phe
<210> 1096
   <211> 19
   <212> PRT
   <213> Artificial
<220>
   <223> Synthetic peptide optionally bearing protecting groups.
<400> 1096
   Glu Arg Cys Phe Glu Glu Phe Lys Ser Phe Thr Ser Cys Phe Asp Ser
   1 5 10 15
   Lys Ala Phe
<210> 1097
   <211> 19
   <212> PRT
   <213> Artificial
<220>
   <223> Synthetic peptide optionally bearing protecting groups.
<400> 1097
   Asp Lys Cys Phe Glu Glu Phe Lys Ser Phe Thr Ser Cys Phe Asp Ser
   1 5 10 15
   Lys Ala Phe
<210> 1098
   <211> 19
   <212> PRT
   <213> Artificial
<220>
   <223> Synthetic peptide optionally bearing protecting groups.
<400> 1098
   Glu Arg Cys Val Glu Glu Phe Lys Ser Leu Thr Ser Cys Leu Glu Ser
   1 5 10 15
   Lys Ala Phe
<210> 1099
   <211> 19
   <212> PRT
   <213> Artificial
<220>
   <223> Synthetic peptide optionally bearing protecting groups.
<400> 1099
   Glu Lys Cys Val Glu Glu Phe Lys Ser Leu Thr Ser Cys Leu Asp Ser
   1 5 10 15
   Lys Phe Phe
<210> 1100
   <211> 19
   <212> PRT
   <213> Artificial
<220>
   <223> Synthetic peptide optionally bearing protecting groups.
<400> 1100
   Glu Lys Cys Phe Glu Glu Phe Lys Ser Phe Thr Ser Cys Phe Asp Ser
   1 5 10 15
   Lys Phe Phe
<210> 1101
   <211> 19
   <212> PRT
   <213> Artificial
<220>
   <223> Synthetic peptide optionally bearing protecting groups.
<400> 1101
   Asp Lys Cys Phe Glu Glu Phe Lys Ser Phe Thr Ser Cys Leu Asp Ser
   1 5 10 15
   Lys Phe Phe
<210> 1102
   <211> 19
   <212> PRT
   <213> Artificial
<220>
   <223> Synthetic peptide optionally bearing protecting groups.
<400> 1102
   Asp Lys Cys Phe Glu Glu Phe Lys Ser Phe Thr Ser Cys Leu Glu Ser
   1 5 10 15
   Lys Phe Phe
<210> 1103
   <211> 19
   <212> PRT
   <213> Artificial
<220>
   <223> Synthetic peptide optionally bearing protecting groups.
<400> 1103
   Asp Lys Cys Phe Glu Glu Leu Lys Ser Phe Thr Ser Cys Leu Asp Ser
   1               5                   10                  15
   Lys Phe Phe
<210> 1104
   <211> 19
   <212> PRT
   <213> Artificial
<220>
   <223> Synthetic peptide optionally bearing protecting groups.
<400> 1104
   Glu Arg Cys Phe Glu Glu Phe Lys Ser Phe Thr Ser Cys Leu Asp Ser
   1               5                   10                  15
   Lys Phe Phe
<210> 1105
   <211> 19
   <212> PRT
   <213> Artificial
<220>
   <223> Synthetic peptide optionally bearing protecting groups.
<400> 1105
   Glu Lys Ala Val Glu Glu Phe Lys Ser Phe Thr Ser Cys Leu Asp Ser
   1               5                   10                  15
   Lys Ala Phe

<210> 1106
   <211> 19
   <212> PRT
   <213> Artificial
<220>
   <223> Synthetic peptide optionally bearing protecting groups.
<400> 1106
   Asp Lys Ala Val Glu Glu Phe Lys Ser Phe Thr Ser Cys Leu Asp Ser
   1 5 10 15
   Lys Phe Phe
<210> 1107
   <211> 19
   <212> PRT
   <213> Artificial
<220>
   <223> Synthetic peptide optionally bearing protecting groups.
<400> 1107
   Glu Lys Ala Val Glu Glu Phe Lys Ser Phe Thr Ser Ala Leu Asp Ser
   1 5 10 15
   Lys Ala Phe
<210> 1108
   <211> 19
   <212> PRT
   <213> Artificial
<220>
   <223> Synthetic peptide optionally bearing protecting groups.
<400> 1108
   Asp Lys Ala Val Glu Glu Phe Lys Ser Phe Thr Ser Ala Leu Asp Ser
   1 5 10 15
   Lys Ala Phe
<210> 1109
   <211> 19
   <212> PRT
   <213> Artificial
<220>
   <223> Synthetic peptide optionally bearing protecting groups.
<400> 1109
   Asp Arg Ala Phe Glu Glu Phe Lys Ser Phe Thr Ser Cys Leu Asp Ser
   1 5 10 15
   Lys Phe Phe
<210> 1110
   <211> 19
   <212> PRT
   <213> Artificial
<220>
   <223> Synthetic peptide optionally bearing protecting groups.
<400> 1110
   Asp Arg Ala Phe Glu Glu Phe Lys Ser Phe Thr Ser Ala Leu Asp Ser
   1               5                   10                  15
   Lys Phe Phe
<210> 1111
   <211> 19
   <212> PRT
   <213> Artificial
<220>
   <223> Synthetic peptide optionally bearing protecting groups.
<400> 1111
   Asp Lys Cys Phe Glu Glu Phe Lys Ser Phe Thr Ser Cys Phe Glu Ser
   1               5                   10                  15
   Lys Phe Phe
<210> 1112
   <211> 19
   <212> PRT
   <213> Artificial
<220>
   <223> Synthetic peptide optionally bearing protecting groups.
<400> 1112
   Glu Lys Cys Tyr Glu Glu Phe Lys Ser Phe Thr Ser Cys Leu Asp Ser
   1               5                   10                  15
   Lys Phe Phe
<210> 1113
   <211> 19
   <212> PRT
   <213> Artificial
<220>
   <223> Synthetic peptide optionally bearing protecting groups.
<400> 1113
   Asp Lys Cys Trp Glu Glu Phe Lys Ser Phe Thr Ser Cys Leu Asp Ser
   1               5                   10                  15
   Lys Phe Phe
<210> 1114
   <211> 19
   <212> PRT
   <213> Artificial
<220>
   <223> Synthetic peptide optionally bearing protecting groups.
<400> 1114
   Glu Lys Cys Phe Glu Glu Phe Lys Ser Tyr Thr Ser Cys Leu Asp Ser
   1               5                   10                  15
   Lys Phe Phe
<210> 1115
   <211> 19
   <212> PRT
   <213> Artificial
<220>
   <223> Synthetic peptide optionally bearing protecting groups.
<400> 1115
   Glu Lys Cys Phe Glu Glu Phe Lys Ser Trp Thr Ser Cys Leu Asp Ser
   1               5                   10                  15
   Lys Phe Phe
<210> 1116
   <211> 19
   <212> PRT
   <213> Artificial
<220>
   <223> Synthetic peptide optionally bearing protecting groups.
<400> 1116
   Glu Lys Cys Val Glu Glu Phe Lys Ser Trp Thr Ser Cys Leu Asp Ser
   1               5                   10                  15
   Lys Ala Phe
<210> 1117
   <211> 19
   <212> PRT
   <213> Artificial
<220>
   <223> Synthetic peptide optionally bearing protecting groups.
<400> 1117
   Asp Lys Cys Phe Glu Glu Phe Lys Ser Trp Thr Ser Cys Leu Asp Ser
   1               5                   10                  15
   Lys Ala Phe
<210> 1118
   <211> 18
   <212> PRT
   <213> Artificial
<220>
   <223> Synthetic peptide optionally bearing protecting groups.
<400> 1118
   Asp Val Trp Lys Ala Ala Tyr Asp Lys Phe Ala Glu Lys Phe Lys Glu
   1               5                   10                  15
   Phe Phe
<210> 1119
   <211> 18
   <212> PRT
   <213> Artificial
<220>
   <223> Synthetic peptide optionally bearing protecting groups.
<400> 1119
   Asp Val Trp Lys Ala Phe Tyr Asp Lys Phe Ala Glu Lys Phe Lys Glu
   1               5                   10                  15
   Ala Phe
<210> 1120
   <211> 18
   <212> PRT
   <213> Artificial
<220>
   <223> Synthetic peptide optionally bearing protecting groups.
<400> 1120
   Asp Phe Trp Lys Ala Phe Tyr Asp Lys Val Ala Glu Lys Phe Lys Glu
   1               5                   10                  15
   Ala Phe
<210> 1121
   <211> 3
   <212> PRT
   <213> Artificial
<220>
   <223> Synthetic peptide optionally bearing protecting groups.
<400> 1121
   Arg Phe Lys
   1
<210> 1122
   <211> 3
   <212> PRT
   <213> Artificial
<220>
   <223> Synthetic peptide optionally bearing protecting groups.
<400> 1122
   Arg Phe Lys
   1
<210> 1123
   <211> 3
   <212> PRT
   <213> Artificial
<220>
   <223> Synthetic peptide optionally bearing protecting groups.
<400> 1123
   Arg Glu Leu
   1
<210> 1124
   <211> 7
   <212> PRT
   <213> Artificial
<220>
   <223> Synthetic peptide optionally bearing protecting groups.
<400> 1124
   Leu Ala Glu Tyr His Ala Lys
   1               5
<210> 1125
   <211> 7
   <212> PRT
   <213> Artificial
<220>
   <223> Synthetic peptide optionally bearing protecting groups.
<400> 1125
   Leu Ala Glu Tyr His Ala Lys
   1 5
<210> 1126
   <211> 7
   <212> PRT
   <213> Artificial
<220>
   <223> Synthetic peptide linker.
<400> 1126
   Gly Gly Gly Gly Ser Ser Ser
   1 5
<210> 1127
   <211> 45
   <212> PRT
   <213> Artificial
<220>
   <223> Synthetic peptide optionally bearing protecting groups.
<400> 1127
<210> 1128
   <211> 41
   <212> PRT
   <213> Artificial
<220>
   <223> Synthetic peptide optionally bearing protecting groups.
<400> 1128
   Leu Leu Glu Gln Leu Asn Glu Gln Phe Asn Trp Val Ser Arg Leu Ala
   1 5 10 15
   Asn Leu Thr Gln Gly Glu Pro Asp Trp Phe Lys Ala Phe Tyr Asp Lys
   20 25 30
   Val Ala Glu Lys Phe Lys Glu Ala Phe
   35 40
<210> 1129
   <211> 22
   <212> PRT
   <213> Artificial
<220>
   <223> Synthetic peptide optionally bearing protecting groups.
<400> 1129
   Leu Leu Glu Gln Leu Asn Glu Gln Phe Asn Trp Val Ser Arg Leu Ala
   1 5 10 15
   Asn Leu Thr Gln Gly Glu
   20
<210> 1130
   <211> 10
   <212> PRT
   <213> Artificial
<220>
   <223> Synthetic peptide optionally bearing protecting groups.
<400> 1130
   Leu Val Gly Arg Gln Leu Glu Glu Phe Leu
   1 5 10
<210> 1131
   <211> 22
   <212> PRT
   <213> Artificial
<220>
   <223> Synthetic peptide optionally bearing protecting groups.
<400> 1131
   Leu Leu Glu Gln Leu Asn Glu Gln Phe Asn Trp Val Ser Arg Leu Ala
   1 5 10 15
   Asn Leu Thr Gln Gly Glu
   20
<210> 1132
   <211> 14
   <212> PRT
   <213> Artificial
<220>
   <223> Synthetic peptide optionally bearing protecting groups.
<400> 1132
   Pro Ser Gly Val Thr Glu Val Val Val Lys Leu Phe Asp Ser
   1 5 10
<210> 1133
   <211> 11
   <212> PRT
   <213> Artificial
<220>
   <223> Synthetic peptide optionally bearing protecting groups.
<400> 1133
   Gln Gln Thr His Met Leu Asp Val Met Gln Asp
   1 5 10
<210> 1134
   <211> 6
   <212> PRT
   <213> Artificial
<220>
   <223> Synthetic peptide optionally bearing protecting groups.
<400> 1134
   Lys Phe Lys Glu Ala Phe
   1 5
<210> 1135
   <211> 6
   <212> PRT
   <213> Artificial
<220>
   <223> Synthetic peptide optionally bearing protecting groups.
<400> 1135
   Tyr Asp Lys Val Ala Glu
   1 5
<210> 1136
   <211> 6
   <212> PRT
   <213> Artificial
<220>
   <223> Synthetic peptide optionally bearing protecting groups.
<400> 1136
   Asp Trp Phe Lys Ala Phe
   1 5
<210> 1137
   <211> 6
   <212> PRT
   <213> Artificial
<220>
   <223> Synthetic peptide optionally bearing protecting groups.
<400> 1137
   Lys Phe Lys Glu Ala Phe
   1               5
<210> 1138
   <211> 6
   <212> PRT
   <213> Artificial
<220>
   <223> Synthetic peptide optionally bearing protecting groups.
<400> 1138
   Lys Phe Lys Glu Ala Phe
   1               5
<210> 1139
   <211> 11
   <212> PRT
   <213> Artificial
<220>
   <223> Synthetic peptide optionally bearing protecting groups.
<400> 1139
   Tyr Asp Lys Val Ala Glu Lys Phe Lys Glu Phe
   1               5                   10
<210> 1140
   <211> 18
   <212> PRT
   <213> Artificial
<220>
   <223> Synthetic peptide optionally bearing protecting groups.
<400> 1140
   Asp Trp Phe Lys Ala Phe Tyr Asp Lys Val Ala Glu Lys Phe Lys Glu
   1               5                   10                  15
   Ala Phe
<210> 1141
   <211> 6
   <212> PRT
   <213> Artificial
<220>
   <223> Synthetic peptide optionally bearing protecting groups.
<400> 1141
   Asp Trp Phe Lys Ala Phe
   1 5
<210> 1142
   <211> 6
   <212> PRT
   <213> Artificial
<220>
   <223> Synthetic peptide optionally bearing protecting groups.
<400> 1142
   Tyr Asp Lys Val Ala Glu
   1 5
<210> 1143
   <211> 6
   <212> PRT
   <213> Artificial
<220>
   <223> Synthetic peptide optionally bearing protecting groups.
<400> 1143
   Lys Phe Lys Glu Ala Phe
   1 5
<210> 1144
   <211> 6
   <212> PRT
   <213> Artificial
<220>
   <223> Synthetic peptide optionally bearing protecting groups.
<400> 1144
   Asp Trp Phe Lys Ala Phe
   1 5
<210> 1145
   <211> 6
   <212> PRT
   <213> Artificial
<220>
   <223> Synthetic peptide optionally bearing protecting groups.
<400> 1145
   Tyr Asp Lys Val Ala Glu
   1 5
<210> 1146
   <211> 6
   <212> PRT
   <213> Artificial
<220>
   <223> Synthetic peptide optionally bearing protecting groups.
<400> 1146
   Lys Phe Lys Glu Ala Phe
   1               5

## Description

### FIELD OF THE INVENTION

This invention relates to the field of atherosclerosis and other conditions characterized by inflammation and/or the formation of various oxidized species. In particular, this invention pertains to the identification of classes of active agents that are orally administrable and that ameliorate one or more symptoms of conditions characterized by an inflammatory response and/or the formation of various oxidized species.

### BACKGROUND OF THE INVENTION

The introduction of statins (*e.g*., Mevacor®, Lipitor®, etc.) has reduced mortality from heart attack and stroke by about one-third. However, heart attack and stroke remain the major cause of death and disability, particularly in the United States and in Western European countries. Heart attack and stroke are the result of a chronic inflammatory condition, which is called atherosclerosis.

Several causative factors are implicated in the development of cardiovascular disease including hereditary predisposition to the disease, gender, lifestyle factors such as smoking and diet, age, hypertension, and hyperlipidemia, including hypercholesterolemia. Several of these factors, particularly hyperlipidemia and hypercholesteremia (high blood cholesterol concentrations) provide a significant risk factor associated with atherosclerosis.

Cholesterol is present in the blood as free and esterified cholesterol within lipoprotein particles, commonly known as chylomicrons, very low density lipoproteins (VLDLs), low density lipoproteins (LDLs), and high density lipoproteins (HDLs). Concentration of total cholesterol in the blood is influenced by (1) absorption of cholesterol from the digestive tract, (2) synthesis of cholesterol from dietary constituents such as carbohydrates, proteins, fats and ethanol, and (3) removal of cholesterol from blood by tissues, especially the liver, and subsequent conversion of the cholesterol to bile acids, steroid hormones, and biliary cholesterol.

Maintenance of blood cholesterol concentrations is influenced by both genetic and environmental factors. Genetic factors include concentration of rate-limiting enzymes in cholesterol biosynthesis, concentration of receptors for low density lipoproteins in the liver, concentration of rate-limiting enzymes for conversion of cholesterols bile acids, rates of synthesis and secretion of lipoproteins and gender of person. Environmental factors influencing the hemostasis of blood cholesterol concentration in humans include dietary composition, incidence of smoking, physical activity, and use of a variety of pharmaceutical agents. Dietary variables include the amount and type of fat (saturated and polyunsaturated fatty acids), the amount of cholesterol, amount and type of fiber, and perhaps the amounts of vitamins such as vitamin C and D and minerals such as calcium.

Low density lipoprotein (LDL) oxidation has been strongly implicated in the pathogenesis of atherosclerosis. High density lipoprotein (HDL) has been found to be capable of protecting against LDL oxidation, but in some instances has been found to accelerate LDL oxidation. Important initiating factors in atherosclerosis include the production of LDL-derived oxidized phospholipids.

Normal HDL has the capacity to prevent the formation of these oxidized phospholipids and also to inactivate these oxidized phospholipids once they have formed. However, under some circumstances HDL can be converted from an anti-inflammatory molecule to a pro-inflammatory molecule that actually promotes the formation of these oxidized phospholipids.

It has been suggested that HDL and LDL function as part of the innate immune system (Navab et al. (2001) Arterioscler. Thromb. Vasc. Biol., 21: 481-488). The generation of anti-inflammatory HDL has been achieved using class A amphipathic helical peptides that mimic the major protein of HDL, apolipoprotein A-I (apo A-I) *(see, e.g.,* WO 02/15923).
US2003/171277, US2004/266671 and WO2004/034977 disclose the peptide "6F" as defined herein.

### SUMMARY OF THE INVENTION

This invention provides a "D" or "L" peptide comprising the amino acid sequence D-W-L-K-A-F-Y-D-K-F-F-E-K-F-K-E-F-F (SEQ ID NO:7), or the retro form of said sequence, for use in a method for the treatment of cancer. The amino acid sequence of said peptide may consist of the sequence D-W-L-K-A-F-Y-D-K-F-F-E-K-F-K-E-F-F (SEQ ID NO:7). The peptide may comprise all L amino acids, or may comprise all D amino acids. In certain embodiments the peptide(s) further comprise a protecting group coupled to the amino or carboxyl terminus. In certain embodiments the peptides comprise a first protecting group coupled to the amino terminus and a second protecting group coupled to the carboxyl terminus. In certain embodiments the protecting groups can be independently selected from the group consisting of acetyl, amide, and 3 to 20 carbon alkyl groups, Fmoc, Tboc, 9-fluoreneacetyl group, 1-fluorenecarboxylic group, 9-florenecarboxylic group, 9-fluorenone-1-carboxylic group, benzyloxycarbonyl, Xanthyl (Xan), Trityl (Trt), 4-methyltrityl (Mtt), 4-methoxytrityl (Mmt), 4-methoxy-2,3,6-trimethyl-benzenesulphonyl (Mtr), Mesitylene-2-sulphonyl (Mts), 4,4-dimethoxybenzhydryl (Mbh),Tosyl (Tos), 2,2,5,7,8-pentamethyl chroman-6-sulphonyl (Pmc), 4-methylbenzyl (MeBzl), 4-methoxybenzyl (MeOBzl), Benzyloxy (BzlO), Benzyl (Bzl), Benzoyl (Bz), 3-nitro-2-pyridinesulphenyl (Npys), 1-(4,4-dimentyl-2,6-diaxocyclohexylidene)ethyl (Dde), 2,6-dichlorobenzyl (2,6-DiCl-Bzl), 2-chlorobenzyloxycarbonyl (2-Cl-Z), 2-bromobenzyloxycarbonyl (2-Br-Z), Benzyloxymethyl (Bom), t-butoxycarbonyl (Boc), cyclohexyloxy (cHxO),t-butoxymethyl (Bum), t-butoxy (tBuO), t-Butyl (tBu), Acetyl (Ac), and Trifluoroacetyl (TFA).

In certain embodiments the peptide comprises a protecting group coupled to the amino terminal and the amino terminal protecting group is a protecting group selected from the group consisting of acetyl, propeonyl, and a 3 to 20 carbon alkyl. In certain embodiments the peptide comprises a protecting group coupled to the carboxyl terminal and the carboxyl terminal protecting group is an amide. In certain embodiments the peptide comprises: a first protecting group coupled to the amino terminus where the protecting group is a protecting group selected from the group consisting of acetyl, propeonyl, and a 3 to 20 carbon alkyl; and a second protecting group coupled to the carboxyl terminal and the carboxyl terminal protecting group is an amide.

The peptide(s) can, optionally, be mixed/combined with a pharmacologically acceptable excipient. In certain embodiments the excipient is an excipient suitable for oral administration to a mammal.

The cancer may be myeloma/multiple myeloma, ovarian cancer, breast cancer, colon cancer or bone cancer.

### Definitions.

The term "treat" when used with reference to treating, e.g. a pathology or disease refers to the mitigation and/or elimination of one or more symptoms of that pathology or disease, and/or a reduction in the rate of onset or severity of one or more symptoms of that pathology or disease, and/or the prevention of that pathology or disease.

The terms "isolated", "purified", or "biologically pure" when referring to an isolated polypeptide refer to material that is substantially or essentially free from components that normally accompany it as found in its native state. With respect to nucleic acids and/or polypeptides the term can refer to nucleic acids or polypeptides that are no longer flanked by the sequences typically flanking them in nature. Chemically synthesized polypeptides are "isolated" because they are not found in a native state (e.g. in blood, serum, *etc.).* In certain embodiments, the term "isolated" indicates that the polypeptide is not found in nature.

The terms "polypeptide", "peptide" and "protein" are used interchangeably herein to refer to a polymer of amino acid residues. The terms apply to amino acid polymers in which one or more amino acid residues is an artificial chemical analogue of a corresponding naturally occurring amino acid, as well as to naturally occurring amino acid polymers.

The term "an amphipathic helical peptide" refers to a peptide comprising at least one amphipathic helix (amphipathic helical domain). Certain amphipathic helical peptides of this invention can comprise two or more (*e.g.,* 3, 4, 5, *etc*.) amphipathic helices.

The term " class A amphipathic helix" refers to a protein structure that forms an α-helix producing a segregation of a polar and nonpolar faces with the positively charged residues residing at the polar-nonpolar interface and the negatively charged residues residing at the center of the polar face (*see, e.g*., Segrest et al. (1990) Proteins: Structure, Function, and Genetics 8: 103-117).

"Apolipoprotein J" (apo J) is known by a variety of names including clusterin, TRPM2, GP80, and SP 40 *(see, e.g.,* Fritz (1995) Pp 112 In: Clusterin: Role in Vertebrate Development, Function, and Adaptation (Harmony JAK Ed.), R.G. Landes, Georgetown, TX,). It was first described as a heterodimeric glycoprotein and a component of the secreted proteins of cultured rat Sertoli cells (*see, e.g.,* Kissinger et al. (1982) Biol. Reprod.; 27: 233240). The translated product is a single-chain precursor protein that undergoes intracellular cleavage into a disulfide-linked 34kDa α subunit and a 47 kDa β subunit (*see, e.g.,* Collard and Griswold (1987) Biochem., 26: 3297-3303). It has been associated with cellular injury, lipid transport, apoptosis and it may be involved in clearance of cellular debris caused by cell injury or death. Clusterin has been shown to bind to a variety of molecules with high affinity including lipids, peptides, and proteins and the hydrophobic probe 1-anilino-8-naphthalenesulfonate (Bailey et al. (2001) Biochem., 40: 11828-11840).

The class G amphipathic helix is found in globular proteins, and thus, the name class G. The feature of this class of amphipathic helix is that it possesses a random distribution of positively charged and negatively charged residues on the polar face with a narrow nonpolar face. Because of the narrow nonpolar face this class does not readily associate with phospholipid *(see, e.g.,* Segrest et al. (1990) Proteins: Structure, Function, and Genetics. 8: 103-117; Erratum (1991) Proteins: Structure, Function and Genetics, 9: 79). Several exchangeable apolipoproteins possess similar but not identical characteristics to the G amphipathic helix. Similar to the class G amphipathic helix, this other class possesses a random distribution of positively and negatively charged residues on the polar face. However, in contrast to the class G amphipathic helix which has a narrow nonpolar face, this class has a wide nonpolar face that allows this class to readily bind phospholipid and the class is termed G* to differentiate it from the G class of amphipathic helix (*see, e.g.,* Segrest et al. (1992) J. Lipid Res., 33: 141-166; Anantharamaiah et al. (1993) Pp. 109-142 In: The Amphipathic Helix, Epand, R.M. Ed CRC Press, Boca Raton, Florida). Computer programs to identify and classify amphipathic helical domains have been described by Jones et al. (1992) J. Lipid Res. 33: 287-296) and include, but are not limited to the helical wheel program (WHEEL or WHEEL/SNORKEL), helical net program (HELNET, HELNET/SNORKEL, HELNET/Angle), program for addition of helical wheels (COMBO or COMBO/SNORKEL), program for addition of helical nets (COMNET, COMNET/SNORKEL, COMBO/SELECT, COMBO/NET), consensus wheel program (CONSENSUS, CONSENSUS/SNORKEL), and the like.

The term "enantiomeric amino acids" refers to amino acids that can exist in at least two forms that are nonsuperimposable mirror images of each other. Most amino acids (except glycine) are enantiomeric and exist in a so-called L-form (L amino acid) or D-form (D amino acid). Most naturally occurring amino acids are "L" amino acids. The terms "D amino acid" and "L amino acid" are used to refer to absolute configuration of the amino acid, rather than a particular direction of rotation of plane-polarized light. The usage herein is consistent with standard usage by those of skill in the art. Amino acids are designated herein using standard 1-letter or three-letter codes, *e.g*. as designated in Standard ST.25 in the Handbook On Industrial Property Information and Documentation.

The term "protecting group" refers to a chemical group that, when attached to a functional group in an amino acid (*e.g*. a side chain, an alpha amino group, an alpha carboxyl group, *etc*.) blocks or masks the properties of that functional group. Preferred amino-terminal protecting groups include, but are not limited to acetyl, or amino groups. Other amino-terminal protecting groups include, but are not limited to alkyl chains as in fatty acids, propeonyl, formyl and others. Preferred carboxyl terminal protecting groups include, but are not limited to groups that form amides or esters.

The phrase "protect a phospholipid from oxidation by an oxidizing agent" refers to the ability of a compound to reduce the rate of oxidation of a phospholipid (or the amount of oxidized phospholipid produced) when that phospholipid is contacted with an oxidizing agent (*e.g.* hydrogen peroxide, 13-(S)-HPODE, 15-(S)-HPETE, HPODE, HPETE, HODE, HETE, *etc*.).

The terms "low density lipoprotein" or "LDL" is defined in accordance with common usage of those of skill in the art. Generally, LDL refers to the lipid-protein complex which when isolated by ultracentrifugation is found in the density range d = 1.019 to d = 1.063.

The terms "high density lipoprotein" or "HDL" is defined in accordance with common usage of those of skill in the art. Generally "HDL" refers to a lipid-protein complex which when isolated by ultracentrifugation is found in the density range of d = 1.063 to d = 1.21.

The term "Group I HDL" refers to a high density lipoprotein or components thereof (*e.g.* apo A-I, paraoxonase, platelet activating factor acetylhydrolase, *etc*.) that reduce oxidized lipids (*e.g*. in low density lipoproteins) or that protect oxidized lipids from oxidation by oxidizing agents.

The term "Group II HDL" refers to an HDL that offers reduced activity or no activity in protecting lipids from oxidation or in repairing (*e.g*. reducing) oxidized lipids.

The term "HDL component" refers to a component (*e.g*. molecules) that comprises a high density lipoprotein (HDL). Assays for HDL that protect lipids from oxidation or that repair (*e.g*. reduce oxidized lipids) also include assays for components of HDL (*e.g.* apo A-I, paraoxonase, platelet activating factor acetylhydrolase, *etc*.) that display such activity.

The term "human apo A-I peptide" refers to a full-length human apo A-I peptide or to a fragment or domain thereof comprising a class A amphipathic helix.

A "monocytic reaction" as used herein refers to monocyte activity characteristic of the "inflammatory response" associated with atherosclerotic plaque formation. The monocytic reaction is characterized by monocyte adhesion to cells of the vascular wall (*e.g*. cells of the vascular endothelium), and/or chemotaxis into the subendothelial space, and/or differentiation of monocytes into macrophages.

The term "absence of change" when referring to the amount of oxidized phospholipid refers to the lack of a detectable change, more preferably the lack of a statistically significant change (*e.g*. at least at the 85%, preferably at least at the 90%, more preferably at least at the 95%, and most preferably at least at the 98% or 99% confidence level). The absence of a detectable change can also refer to assays in which oxidized phospholipid level changes, but not as much as in the absence of the protein(s) described herein or with reference to other positive or negative controls.

The following abbreviations may be used herein: PAPC: L-α-1-palmitoyl-2-arachidonoyl-*sn*-glycero-3-phosphocholine; POVPC: 1-palmitoyl-2-(5-oxovaleryl)-*sn-*glycero-3-phosphocholine; PGPC: 1-palmitoyl-2-glutaryl-*sn*-glycero-3-phosphocholine; PEIPC: 1-palmitoyl-2-(5,6-epoxyisoprostane E₂)-*sn*-glycero-3-phosphocholine; ChC18:2: cholesteryl linoleate; ChC18:2-OOH: cholesteryl linoleate hydroperoxide; DMPC: 1,2-ditetradecanoyl-rac-glycerol-3-phosphocholine; PON: paraoxonase; HPF: Standardized high power field; PAPC: L-α-1-palmitoyl-2-arachidonoyl-*sn*-glycero-3-phosphocholine; BL/6: C57BL/6J; C3H:C3H/HeJ.

The term "conservative substitution" is used in reference to proteins or peptides to reflect amino acid substitutions that do not substantially alter the activity (specificity (*e.g*. for lipoproteins)) or binding affinity (*e.g*. for lipids or lipoproteins)) of the molecule. Typically conservative amino acid substitutions involve substitution one amino acid for another amino acid with similar chemical properties (*e.g*. charge or hydrophobicity). The following six groups each contain amino acids that are typical conservative substitutions for one another: 1) Alanine (A), Serine (S), Threonine (T); 2) Aspartic acid (D), Glutamic acid (E); 3) Asparagine (N), Glutamine (Q); 4) Arginine (R), Lysine (K); 5) Isoleucine (I), Leucine (L), Methionine (M), Valine (V); and 6) Phenylalanine (F), Tyrosine (Y), Tryptophan (W).

The terms "identical" or percent "identity," in the context of two or more nucleic acids or polypeptide sequences, refer to two or more sequences or subsequences that are the same or have a specified percentage of amino acid residues or nucleotides that are the same, when compared and aligned for maximum correspondence, as measured using one of the following sequence comparison algorithms or by visual inspection. With respect to the peptides of this invention sequence identity is determined over the full length of the peptide.

For sequence comparison, typically one sequence acts as a reference sequence, to which test sequences are compared. When using a sequence comparison algorithm, test and reference sequences are input into a computer, subsequence coordinates are designated, if necessary, and sequence algorithm program parameters are designated. The sequence comparison algorithm then calculates the percent sequence identity for the test sequence(s) relative to the reference sequence, based on the designated program parameters.

Optimal alignment of sequences for comparison can be conducted, *e.g*., by the local homology algorithm of Smith & Waterman, Adv. Appl. Math. 2:482 (1981), by the homology alignment algorithm ofNeedleman & Wunsch, J. Mol. Biol. 48:443 (1970), by the search for similarity method of Pearson & Lipman (1988) Proc. Natl. Acad. Sci. USA 85:2444, by computerized implementations of these algorithms (GAP, BESTFIT, FASTA, and TFASTA in the Wisconsin Genetics Software Package, Genetics Computer Group, 575 Science Dr., Madison, WI), or by visual inspection (*see generally* Ausubel *et al., supra*).

One example of a useful algorithm is PILEUP. PILEUP creates a multiple sequence alignment from a group of related sequences using progressive, pairwise alignments to show relationship and percent sequence identity. It also plots a tree or dendogram showing the clustering relationships used to create the alignment. PILEUP uses a simplification of the progressive alignment method of Feng & Doolittle (1987) J. Mol. Evol. 35:351-360. The method used is similar to the method described by Higgins & Sharp (1989) CABIOS 5: 151-153. The program can align up to 300 sequences, each of a maximum length of 5,000 nucleotides or amino acids. The multiple alignment procedure begins with the pairwise alignment of the two most similar sequences, producing a cluster of two aligned sequences. This cluster is then aligned to the next most related sequence or cluster of aligned sequences. Two clusters of sequences are aligned by a simple extension of the pairwise alignment of two individual sequences. The final alignment is achieved by a series of progressive, pairwise alignments. The program is run by designating specific sequences and their amino acid or nucleotide coordinates for regions of sequence comparison and by designating the program parameters. For example, a reference sequence can be compared to other test sequences to determine the percent sequence identity relationship using the following parameters: default gap weight (3.00), default gap length weight (0.10), and weighted end gaps.

Another example of algorithm that is suitable for determining percent sequence identity and sequence similarity is the BLAST algorithm, which is described in Altschul et al. (1990) J. Mol. Biol. 215: 403-410. Software for performing BLAST analyses is publicly available through the National Center for Biotechnology Information (http://www.ncbi.nlm.nih.gov/). This algorithm involves first identifying high scoring sequence pairs (HSPs) by identifying short words of length W in the query sequence, which either match or satisfy some positive-valued threshold score T when aligned with a word of the same length in a database sequence. T is referred to as the neighborhood word score threshold (Altschul *et al, supra*). These initial neighborhood word hits act as seeds for initiating searches to find longer HSPs containing them. The word hits are then extended in both directions along each sequence for as far as the cumulative alignment score can be increased. Cumulative scores are calculated using, for nucleotide sequences, the parameters M (reward score for a pair of matching residues; always > 0) and N (penalty score for mismatching residues; always < 0). For amino acid sequences, a scoring matrix is used to calculate the cumulative score. Extension of the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T, and X determine the sensitivity and speed of the alignment. The BLASTN program (for nucleotide sequences) uses as defaults a wordlength (W) of 11, an expectation (E) of 10, M=5, N=-4, and a comparison of both strands. For amino acid sequences, the BLASTP program uses as defaults a wordlength (W) of 3, an expectation (E) of 10, and the BLOSUM62 scoring matrix (*see* Henikoff & Henikoff (1989) Proc. Natl. Acad. Sci. USA 89:10915).

In addition to calculating percent sequence identity, the BLAST algorithm also performs a statistical analysis of the similarity between two sequences (*see, e.g.,* Karlin & Altschul (1993) Proc. Natl. Acad. Sci. USA ,90: 5873-5787). One measure of similarity provided by the BLAST algorithm is the smallest sum probability (P(N)), which provides an indication of the probability by which a match between two nucleotide or amino acid sequences would occur by chance. For example, a nucleic acid is considered similar to a reference sequence if the smallest sum probability in a comparison of the test nucleic acid to the reference nucleic acid is less than about 0.1, more preferably less than about 0.01, and most preferably less than about 0.001.

The phrase "in conjunction with" when used in reference to the use of one or more drugs in conjunction with one or more active agents described herein indicates that the drug(s) and the active agent(s) are administered so that there is at least some chronological overlap in their physiological activity on the organism. Thus the drug(s) and active agent(s) can be administered simultaneously and/or sequentially. In sequential administration there may even be some substantial delay (*e.g*., minutes or even hours or days) before administration of the second moiety as long as the first administered drug/agent has exerted some physiological alteration on the organism when the second administered agent is administered or becomes active in the organism.

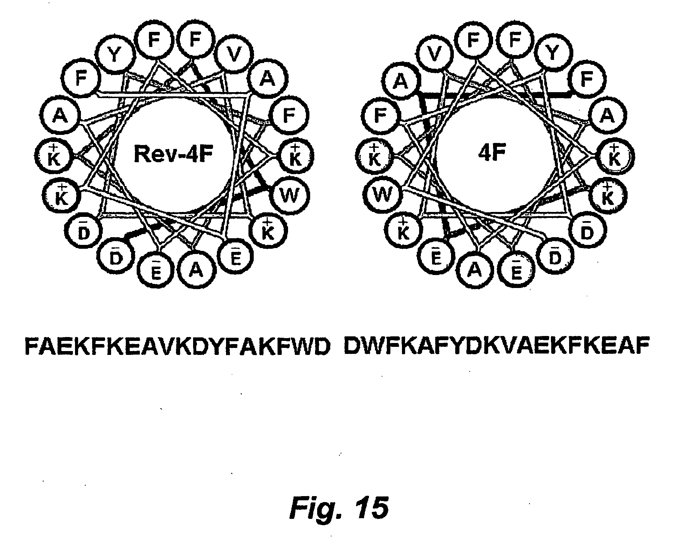

The phrases "adjacent to each other in a helical wheel diagram of a peptide" or "contiguous in a helical wheel diagram of a peptide" when referring to residues in a helical peptide indicates that in the helical wheel representation the residuces appear adjacent or contiguous even though they may not be adjacent or contiguous in the linear peptide. Thus, for example, the residues "A, E, K, W, K, and F" are contiguous in the helical wheel diagrams shown in Figure 15 even though these residues are not contiguous in the linear peptide.

### BRIEF DESCRIPTION OF THE DRAWINGS

Figure 1 shows a comparison of the effect of D4F (Navab, et al. (2002) Circulation, 105: 290-292) and apo-J peptide 336 made from D amino acids (D-J336*) on the prevention of LDL-induced monocyte chemotactic activity *in vitro* in a co-incubation experiment. The data are mean ±SD of the number of migrated monocytes in nine high power fields in quadruple cultures. (D-J336 = Ac-LLEQLNEQFNWVSRLANLTQGE-NH₂, SEQ ID NO:1).
Figure 2 illustrates the prevention of LDL-induced monocyte chemotactic activity by pre-treatment of artery wall cells with D-J336 as compared to D-4F. The data are mean ±SD of the number of migrated monocytes in nine high power fields in quadruple cultures.
Figure 3 illustrates he effect of apo J peptide mimetics on HDL protective capacity in LDL receptor null mice. The values are the mean ± SD of the number of migrated monocytes in 9 high power fields from each of quadruple assay wells.
Figure 4 illustrates protection against LDL-induced monocyte chemotactic activity by HDL from apo E null mice given oral peptides. The values are the mean ± SD of the number of migrated monocytes in 9 high power fields from each of quadruple assay wells. Asterisks indicate significant difference (p<0.05) as compared to No Peptide mHDL.
Figure 5 illustrates the effect of oral apo A-1 peptide mimetic and apoJ peptide on LDL susceptibility to oxidation. The values are the mean ± SD of the number of migrated monocytes in 9 high power fields from each of quadruple assay wells. Asterisks indicate significant difference (p<0.05) as compared to No Peptide LDL.
Figure 6 illustrates the effect of oral apoA-1 peptide mimetic and apoJ peptide on HDL protective capacity. The values are the mean ± SD of the number of migrated monocytes in 9 high power fields from each of quadruple assay wells. Asterisks indicate significant difference (p<0.05) as compared to No Peptide mHDL.
Figure 7 illustrates the effect of oral apoA-1 peptide mimetic and apoJ peptide on plasma paraoxonase activity. The values are the mean ± SD of readings from quadruple plasma aliquots. Asterisks indicate significant differences (p<0.05) as compared to No Peptide control plasma.
Figure 8 shows the effect of oral G* peptides on HDL protective capacity in apoE-/- mice . The values are the mean ± SD of readings from quadruple plasma aliquots. Asterisks indicate significant differences (p<0.05) as compared to no peptide control plasma.
Figure 9 shows the effect of Oral G* peptide, 146-156, on HDL protective capacity in ApoE-/- mice.

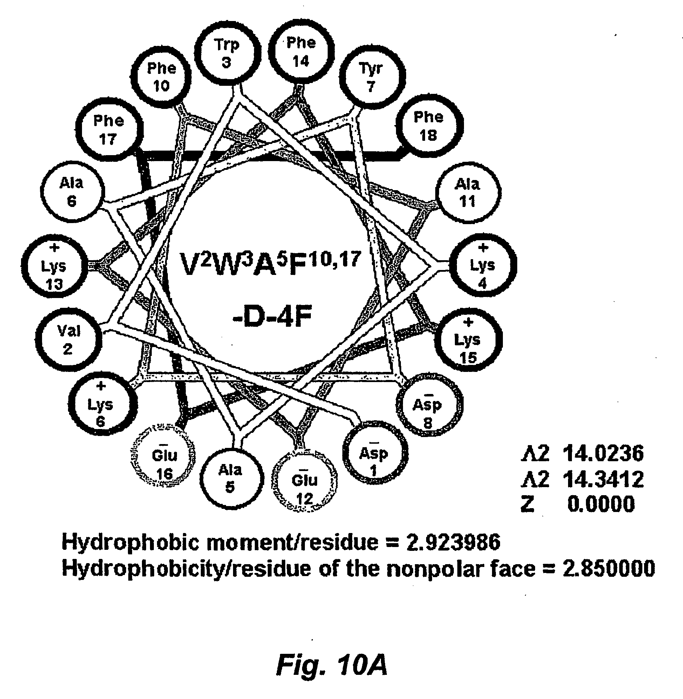

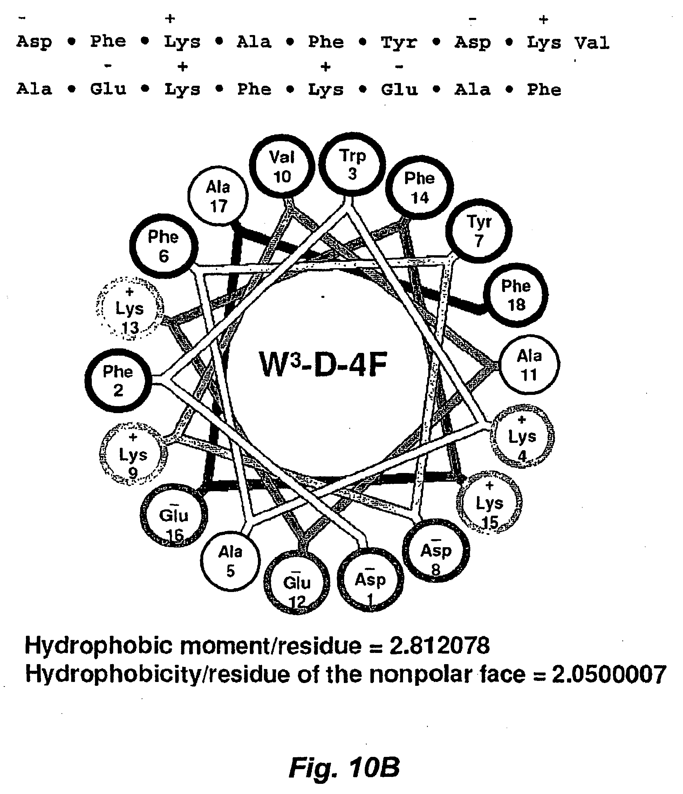

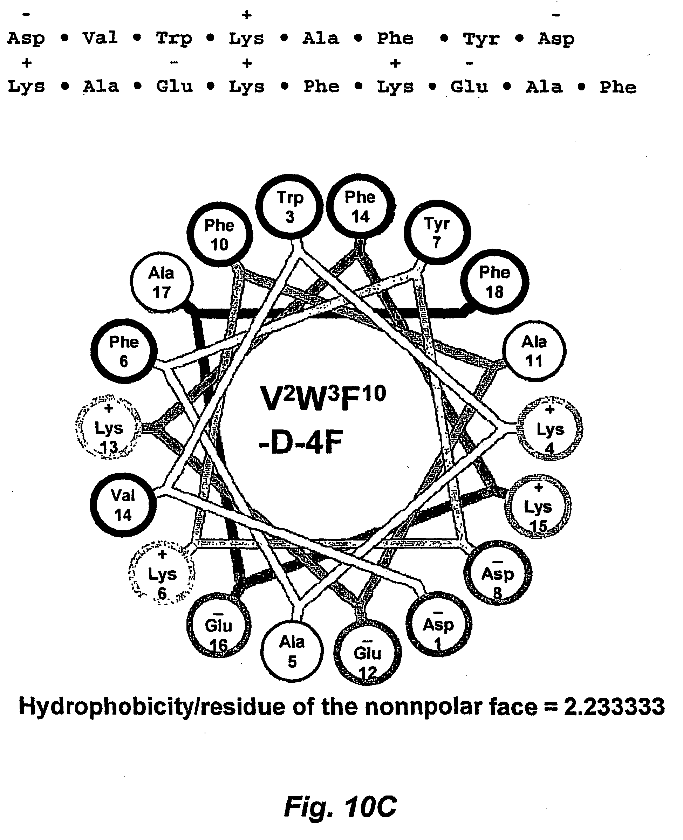

Figures 10A through 10C illustrate helical wheel diagrams of certain peptides. Figure 10A: V²W³A⁵F^{10,17}-D-4F; Figure 10B: W³-D-4F; Figure 10C: V²W³F¹⁰-D-4F:

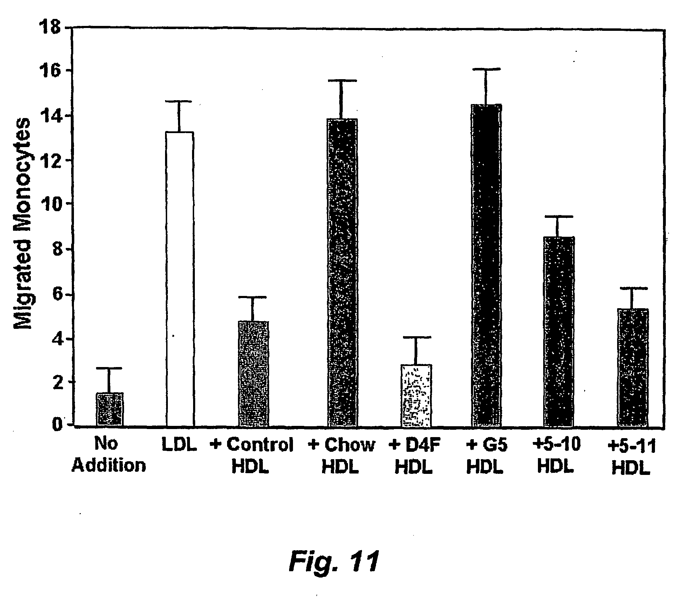

Figure 11 A standard human LDL (LDL) was added to human artery wall cocultures without (No Addition) or with human HDL (+Control HDL) or with mouse HDL from apoE null mice given Chow overnight (+Chow HDL), or given D-4F in the chow overnight (+D4F HDL) or given G5-D-4F in the chow overnight (+G5 HDL), or given G5,10-D-4F in the chow overnight (+5-10 HDL), or given G5,11-D-4F in the chow overnight (+5-11 HDL) and the resulting monocyte chemotactic activity determined as previously described (Navab et al. (2002) Circulation, 105: 290-292).

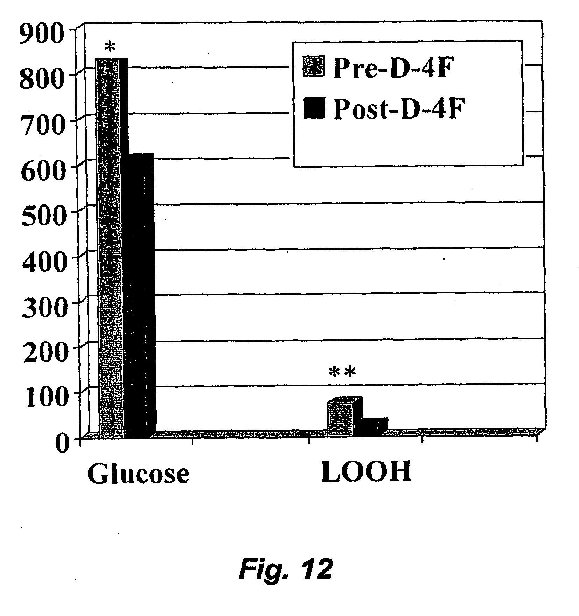

Figure 12 shows that peptides of this invention are effective in mitigating symptoms of diabetes (*e.g*., blood glucose). Obese Zucker rats 26 weeks of age were bled and then treated with daily intraperitoneal injections of D-4F (5.0 mg/kg/day). After 10 days the rats were bled again plasma glucose and lipid hydroperoxides (LOOH) were determined. *p=0.027; ** p=0.0017.

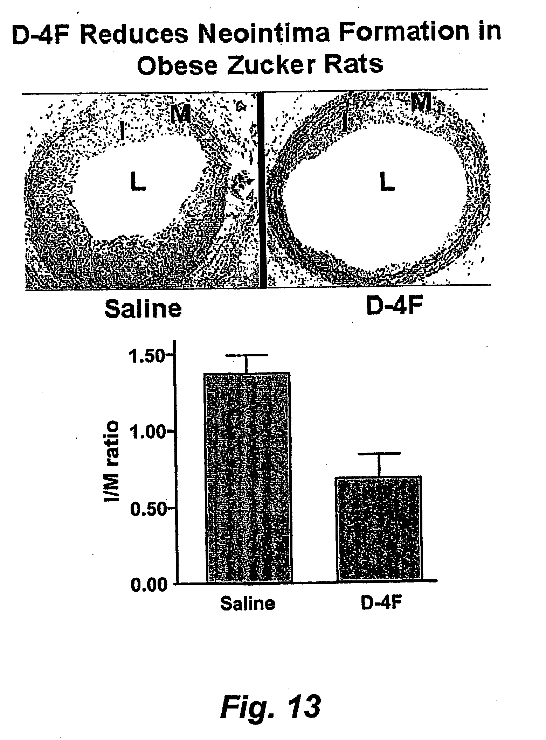

Figure 13. Sixteen week old Obese Zucker Rats were injected with D-4F (5 mg/kg/daily) for 1 week at which time they underwent balloon injury of the common carotid artery. Two weeks later the rats were sacrificed and the intimal media ratio determined.

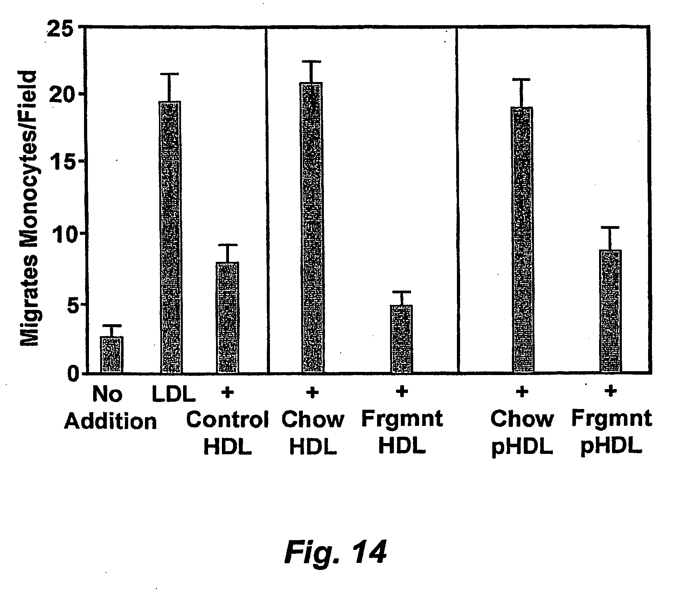

Figure 14 demonstrates that the product of the solution phase synthesis scheme is very biologically active in producing HDL and pre-beta HDL that inhibit LDL-induced monocyte chemotaxis in apo E null mice. ApoE null mice were fed 5 micrograms of the D-4F synthesized as described above (Frgmnt) or the mice were given the same amount of mouse chow without D-4F (Chow). Twelve hours after the feeding was started, the mice were bled and their plasma was fractionated on FPLC. LDL (100 micrograms LDL-cholesterol) was added to cocultures of human artery wall cells alone (LDL) or with a control human HDL (Control HDL) or with HDL (50 micrograms HDL-cholesterol) or post-HDL (pHDL; prebeta HDL) from mice that did (Frgmnt) or did not (Chow) receive the D-4F and the monocyte chemotactic activity produced was determined.
Figure 15 illustrates a helical wheel representation of 4F and reverse (retro) 4F. Reverse-4F is a mirror image of 4F with the relative positions of the amino acids to each other and to the hydrophilic and hydrophobic faces being identical.
Figure 16 shows a comparison of the HDL inflammatory index of D-4F versus reverse D-4F.

### DETAILED DESCRIPTION

### I. Methods of treatment.

The active agents (*e.g*. peptides, small organic molecules, amino acid pairs, *etc*.) described herein are effective for mitigating one or more symptoms and/or reducing the rate of onset and/or severity of cancer. Without being bound to a particular theory, it is believed that the peptides bind the "seeding molecules" required for the formation of pro-inflammatory oxidized phospholipids such as Ox-PAPC, POVPC, PGPC, and PEIPC.

In addition, since many inflammatory conditions and/or other pathologies are mediated at least in part by oxidized lipids, we believe that the peptides of this invention are effective in ameliorating conditions that are characterized by the formation of biologically active oxidized lipids. In addition, there are a number of other conditions for which the active agents described herein appear to be efficacious.

A number of pathologies for which the active agents described herein appear to be a palliative and/or a preventative are described below.

### A) Atherosclerosis and associated pathologies.

We discovered that normal HDL inhibits three steps in the formation of mildly oxidized LDL. In particular, we demonstrated that treating human LDL *in vitro* with apo A-I or an apo A-I mimetic peptide (37pA) removed seeding molecules from the LDL that included HPODE and HPETE. These seeding molecules were required for cocultures of human artery wall cells to be able to oxidize LDL and for the LDL to induce the artery wall cells to produce monocyte chemotactic activity. We also demonstrated that after injection of apo A-I into mice or infusion into humans, the LDL isolated from the mice or human volunteers after injection/infusion of apo A-I was resistant to oxidation by human artery wall cells and did not induce monocyte chemotactic activity in the artery wall cell cocultures.

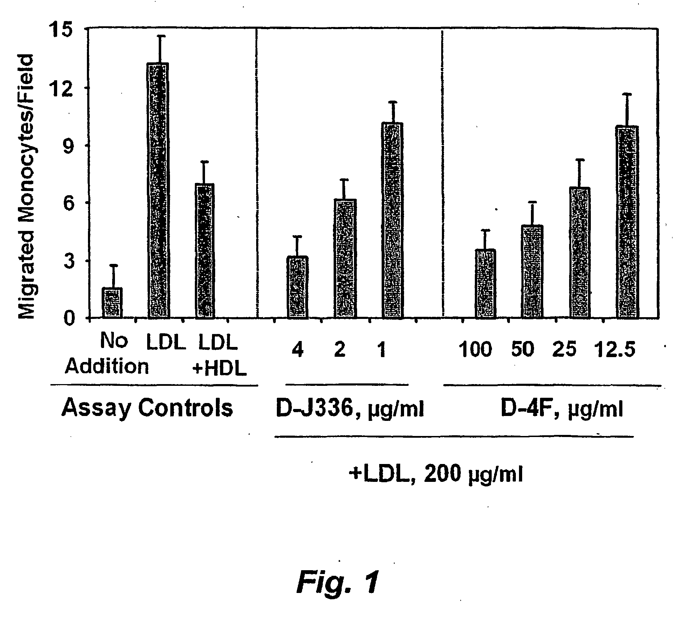

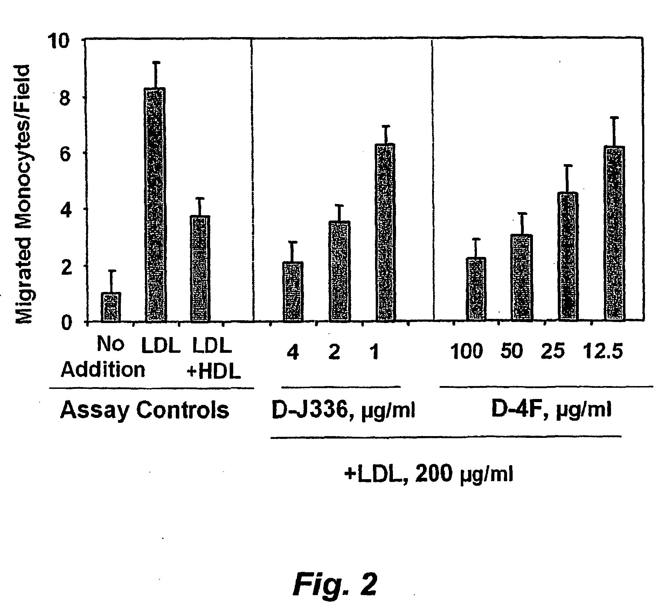

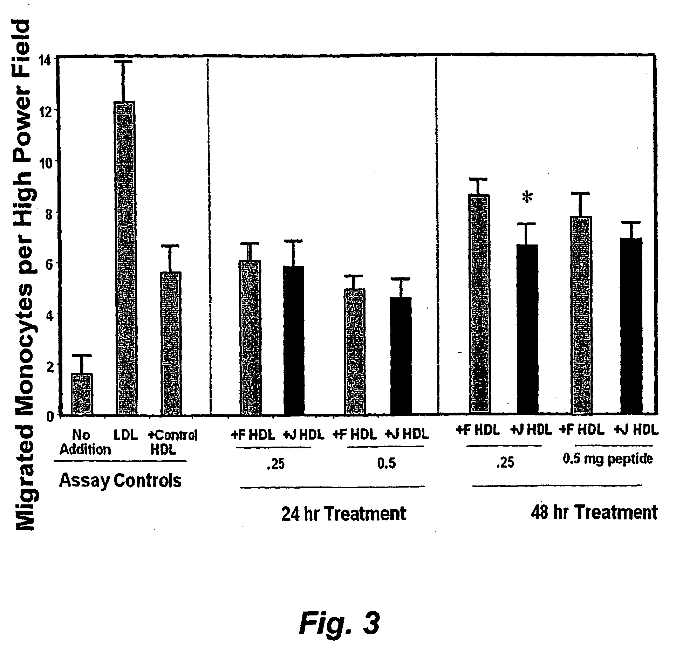

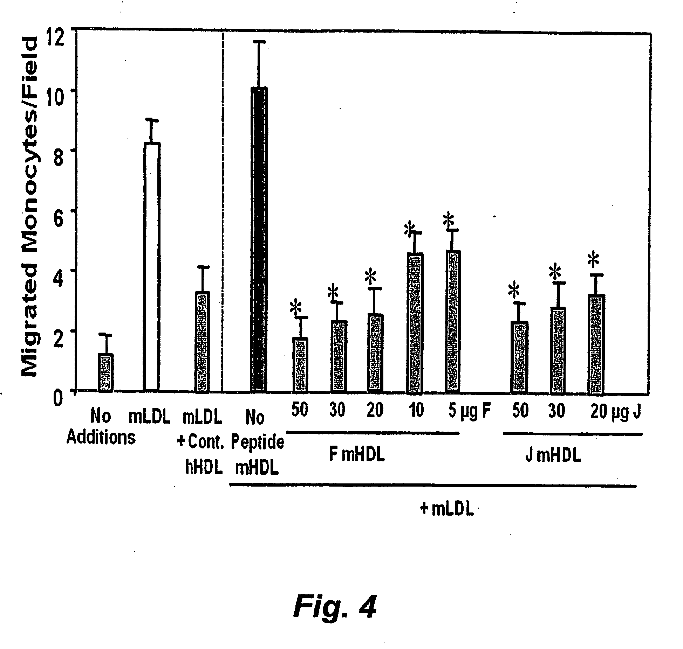

The protective function of various active agents of this invention is illustrated in the parent applications (09/645,454, filed August 24, 2000, 09/896,841, filed June 29, 2001, and WO 02/15923 (PCT/US01/26497), filed June 29, 2001, *see, e.g.,* Figures 1-5 in WO 02/15923. Figure 1, panels A, B, C, and D in WO 02/15923 show the association of ¹⁴C-D-5F with blood components in an ApoE null mouse. It is also demonstrated that HDL from mice that were fed an atherogenic diet and injected with PBS failed to inhibit the oxidation of human LDL and failed to inhibit LDL-induced monocyte chemotactic activity in human artery wall coculures. In contrast, HDL from mice fed an atherogenic diet and injected daily with peptides described herein was as effective in inhibiting human LDL oxidation and preventing LDL-induced monocyte chemotactic activity in the cocultures as was normal human HDL (Figures 2A and 2B in WO 02/15923). In addition, LDL taken from mice fed the atherogenic diet and injected daily with PBS was more readily oxidized and more readily induced monocyte chemotactic activity than LDL taken from mice fed the same diet but injected with 20 µg daily of peptide 5F. The D peptide did not appear to be immunogenic (Figure 4 in WO 02/15923).

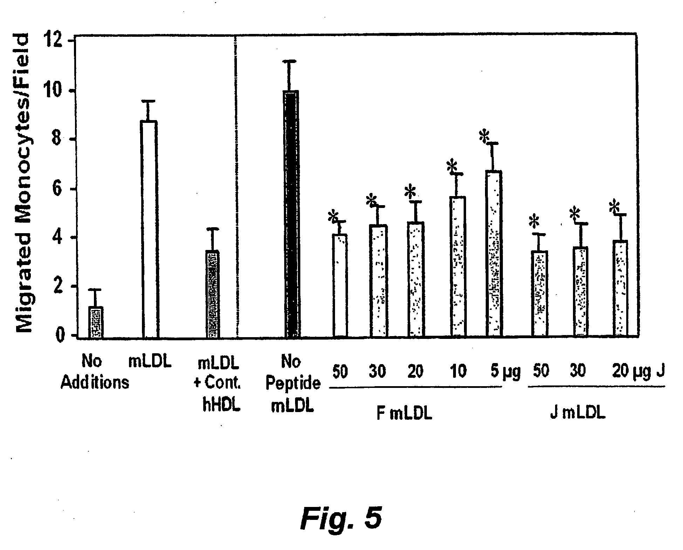

The *in vitro* responses of human artery wall cells to HDL and LDL from mice fed the atherogenic diet and injected with a peptide according to this invention are consistent with the protective action shown by such peptides *in vivo.* Despite, similar levels oftotal cholesterol, LDL-cholesterol, IDL+VLDL-cholesterol, and lower HDL-cholesterol as a percent of total cholesterol, the animals fed the atherogenic diet and injected with the peptide had significantly lower lesion scores (Figure 5 in WO 02/15923). The peptides of this invention thus prevented progression of atherosclerotic lesions in mice fed an atherogenic diet.

It is also noted that c-reactive protein, a marker for inflammation, is elevated in congestive heart failure. Also, in congestive heart failure there is an accumulation of reactive oxygen species and vasomotion abnormalities.

### B) Arteriole/vascular indications.

Vessels smaller than even the smallest arteries (i.e., arterioles) thicken, become dysfunctional and cause end organ damage to tissues as diverse as the brain and the kidney. The active agents described herein can function to improve areteriole structure and function and/or to slow the rate and/or severity of arteriole dysfunction. Without being bound to a particular theory, it is believed that arteriole dysfunction is a causal factor in various brain and kidney disorders.

### C) Pulmonary indications.

The agents described herein are also suitable for treatment of a variety of pulmonary indications. These include, but are not limited to chronic obstructive pulmonary disease (COPD), emphysema, pulmonary disease, asthma, idiopathic pulmonary fibrosis, and the like.

### D) Mitigation of a symptom or condition associated with coronary calcification and osteoporosis.

Vascular calcification and osteoporosis often co-exist in the same subjects (Ouchi et al. (1993) Ann NY Acad Sci., 676: 297-307; Boukhris and Becker ('1972) JAMA, 219: 1307-1311; Banks et al. (1994) Eur J Clin Invest., 24: 813-817; Laroche et al. (1994) Clin Rheumatol., 13: 611-614; Broulik and Kapitola (1993) Endocr Regul., 27: 57-60; Frye et al. (1992) Bone Mine., 19: 185-194; Barengolts et al. (1998) Calcif Tissue Int., 62: 209-213; Burnett and Vasikaran (2002) Ann Clin Biochem., 39: 203-210. Parhami et al. (1997) Arterioscl Thromb Vasc Biol., 17: 680-687, demonstrated that mildly oxidized LDL (MM-LDL) and the biologically active lipids in MM-LDL [*i.e*. oxidized 1-palmitoyl-2-arachidonoyl-*sn*-glycero-3-phosphorylcholine) (Ox-PAPC)], as well as the isoprostane, 8-iso prostaglandin E₂, but not the unoxidized phospholipid (PAPC) or isoprostane 8-iso progstaglandin F_{2α} induced alkaline phosphatase activity and osteoblastic differentiation of calcifying vascular cells (CVCs) in vitro, but inhibited the differentiation of MC3T3-E1 bone cells.

The osteon resembles the artery wall in that the osteon is centered on an endothelial cell-lined lumen surrounded by a subendothelial space containing matrix and fibroblast-like cells, which is in turn surrounded by preosteoblasts and osteoblasts occupying a position analogous to smooth muscle cells in the artery wall (*Id*.). Trabecular bone osteoblasts also interface with bone marrow subendothelial spaces (*Id*.). Parhami *et al.* postulated that lipoproteins could cross the endothelium of bone arteries and be deposited in the subendothelial space where they could undergo oxidation as in coronary arteries (*Id*.). Based on their *in vitro* data they predicted that LDL oxidation in the subendothelial space of bone arteries and in bone marrow would lead to reduced osteoblastic differentiation and mineralization which would contribute to osteoporosis (*Id*.). Their hypothesis further predicted that LDL levels would be positively correlated with osteoporosis as they are with coronary calcification (Pohle et al. (2001) Circulation, 104: 1927-1932), but HDL levels would be negatively correlated with osteoporosis (Parhami et al. (1997) Arterioscl Thromb Vasc Biol., 17: 680-687).

*In vitro,* the osteoblastic differentiation of the marrow stromal cell line M2-10B4 was inhibited by MM-LDL but not native LDL (Parhami et al. (1999) J Bone Miner Res., 14: 2067-2078). When marrow stromal cells from atherosclerosis susceptible C57BL/6 (BL6) mice fed a low fat chow diet were cultured there was robust osteogenic differentiation *(Id.).* In contrast, when the marrow stromal cells taken from the mice after a high fat, atherogenic diet were cultured they did not undergo osteogenic differentiation *(Id.).* This observation is particularly important since it provides a possible explanation for the decreased osteogenic potential of marrow stromal cells in the development of osteoporosis (Nuttall and Gimble (2000) Bone, 27: 177-184). *In vivo* the decrease in osteogenic potential is accompanied by an increase in adipogenesis in osteoporotic bone (*Id*.).

It was found that adding D-4F to the drinking water of apoE null mice for 6 weeks dramatically increased trabecular bone mineral density and it is believed that the other active agents described herein will act similarly.

Our data indicate that osteoporosis can be regarded as an "atherosclerosis of bone". It appears to be a result of the action of oxidized lipids. HDL destroys these oxidized lipids and promotes osteoblastic differentiation. Our data indicate that administering active agent (s) described herein to a mammal (*e.g*., in the drinking water of apoE null mice) dramatically increases trabecular bone in just a matter of weeks.

This indicates that the active agents, described herein are useful for mitigation one or more symptoms of osteoporosis (*e.g*., for inhibiting decalcification) or for inducing recalcification of osteoporotic bone. The active agents are also useful as prophylactics to prevent the onset of symptom(s) of osteoporosis in a mammal (*e.g*., a patient at risk for osteoporosis).

We believe similar mechanisms are a cause of coronary calcification, *e.g*., calcific aortic stenosis.

### E) Inflammatory and Autoimmune Indications.

Chronic inflammatory and/or autoimmune conditions are also characterized by the formation of a number of reactive oxygen species and are amenable to treatment using one or more of the active agents described herein. Thus, without being bound to a particular theory, we also believe the active agents described herein are useful, prophylactically or therapeutically, to mitigate the onset and/or more or more symptoms of a variety of other conditions including, but not limited to rheumatoid arthritis, lupus erythematous, polyarteritis nodosa, polymyalgia rheumatica, scleroderma, multiple sclerosis, and the like.

The active agents are useful in mitigating one or more symptoms caused by, or associated with, an inflammatory response in these conditions.

Also, the active agents are useful in mitigating one or more symptoms caused by or associated with an inflammatory response associated with AIDS.

### F) Infections/trauma/transplants.

We have observed that a consequence of influenza infection and other infections is the diminution in paraoxonase and platelet activating acetylhydrolase activity in the HDL. Without being bound by a particular theory, we believe that, as a result of the loss of these HDL enzymatic activities and also as a result of the association of pro-oxidant proteins with HDL during the acute phase response, HDL is no longer able to prevent LDL oxidation and is no longer able to prevent the LDL-induced production of monocyte chemotactic activity by endothelial cells.

We observed that in a subject injected with very low dosages of certain agents of this invention (e.g., 20 micrograms for mice) daily after infection with the influenza A virus paraoxonase levels did not fall and the biologically active oxidized phospholipids were not generated beyond background. This indicates that 4F, D4F (and/or other agents of this invention) can be administered (*e.g*. orally or by injection) to patients (including, for example with known coronary artery disease during influenza infection or other events that can generate an acute phase inflammatory response, *e.g*. due to viral infection, bacterial infection, trauma, transplant, various autoimmune conditions, *etc.)* and thus we can prevent by this short term treatment the increased incidence of heart attack and stroke associated with pathologies that generate such inflammatory states.

In addition, by restoring and/or maintaining paroxonase levels and/or monocyte activity, the agent(s) of this invention are useful in the treatment of infection (*e.g*., viral infection, bacterial infection, fungal infection) and/or the inflammatory pathologies associated with infection (*e.g*. meningitis) and/or trauma.

Because of the combined anti-inflammatory activity and anti-infective activity, the agents described herein are also useful in the treatment of a wound or other trauma, mitigating adverse effects associated with organ or tissue transplant, and/or organ or tissue transplant rejection, and/or implanted prostheses, and/or transplant atherosclerosis, and/or biofilm formation. In addition, we believe that L-4F, D-4F, and/or other agents described herein are also useful in mitigating the effects of spinal cord injuries.

### G) Diabetes and associated conditions.

Various active agents described herein have also been observed to show efficacy in reducing and/or preventing one or more symptoms associated with diabetes.

### H) Cancer.

NFκB is a transcription factor that is normally activated in response to proinflammatory cytokines and that regulates the expression of more than 200 genes. Many tumor cell lines show constitutive activation of NFκB signaling. Various studies of mouse models of intestinal, and mammary tumors conclude that activation of the NFκB pathway enhances tumor development and may act primarily in the late stages of tumorigenesis (*see, e.g.,* (2004) Cell 118: 285; (2004) J. Clin. Invest., 114: 569). Inhibition of NFκB signaling suppressed tumor development. Without being bound to a particular theory, mechanisms for this suppression are believed to include an increase in tumor cell apoptosis, reduced expression of tumor cell growth factors supplied by surrounding stromal cells, and/or abrogation of a tumor cell dedifferentiation program that is critical for tumor invasion/metastasis.

Without being bound by a particular theory, it is believed the administration of one or more active agents described herein will inhibit expression and/or secretion, and/or activity of NFκB. Thus, in certain embodiments, this invention provides methods of treating a pathology characterized by elevated NFκB by administering one or more active agents described herein. Thus, In various embodiments this invention contemplates inhibiting NFκB activation associated with cancer by administering one ore more active agents described herein, optionally in combination with appropriate cancer therapeutics.

### I) Biochemical Activity.

The active agent(s) described herein have been shown to exhibit a number of specific biological activities. Thus, for example, they increase heme oxygenase 1, they increase extracellular superoxide dismutase, they reduce or prevent the association of myeloperoxidase with apoA-I, they reduce or prevent the nitrosylation of tyrosine in apoA-I, they render HDL Anti-inflammatory or more anti-inflammatory, and they increase the formation cycling of pre-β HDL, they promote reverse cholesterol transport, in particular, reverse cholesterol transport from macrophages, and they synergize the activity of statins. The active agents described herein can thus be administered to a mammal to promote any of these activities, *e.g*. to treat a condition/pathology whose severity, and/or likelihood of onset is reduced by one or more of these activities.

### J) Mitigation of a symptom of atherosclerosis associated with an acute inflammatory response.

The active agents are also useful in a number of contexts. For example, we have observed that cardiovascular complications (*e.g*., atherosclerosis, stroke, *etc*.) frequently accompany or follow the onset of an acute phase inflammatory response, *e.g*., such as that associated with a recurrent inflammatory disease, a viral infection (*e.g*., influenza), a bacterial infection, a fungal infection, an organ transplant, a wound or other trauma, and so forth.

### K) Other indications.

In various embodiments it is contemplated that the active agents described herein are useful in the treatment (*e.g*. mitigation and/or prevention) of corneal ulcers, endothelial sloughing, Crohn's disease, acute and chronic dermatitis (including, but not limited to eczema and/or psoriasis), macular degeneration, neuropathy, scleroderma, and ulcerative colitis.

A summary of indications/conditions for which the active agents have been shown to be effective and/or are believed to be effective is shown in Table 1.

**Table 1. Summary of conditions in which the active agents (e.g., D-4F) have been shown to be or are believed to be effective, in accordance with the invention.**

| cancers | |
|---|---|
| | myeloma/multiple myeloma |
| | ovarian cancer |
| | breast cancer |
| | colon cancer |
| | bone cancer |

It is noted that the conditions listed in Table 1 are intended to be illustrative and not limiting.

### L) Administration.

Typically the active agent(s) will be administered to a mammal (*e.g*.,. a human) in need thereof. Such a mammal will typically include a mammal (*e.g*. a human) having or at risk for one or more of the pathologies described herein.

The active agent(s) can be administered, as described herein, according to any of a number of standard methods including, but not limited to injection, suppository, nasal spray, time-release implant, transdermal patch, and the like. In one particularly preferred embodiment, the peptide(s) are administered orally (*e.g*. as a syrup, capsule, or tablet).

The methods involve the administration of a single active agent of this invention or the administration of two or more different active agents. The active agents can be provided as monomers (*e.g*., in separate or combined formulations), or in dimeric, oligomeric or polymeric forms. In certain embodiments, the multimeric forms may comprise associated monomers (*e.g*., ionically or hydrophobically linked) while certain other multimeric forms comprise covalently linked monomers (directly linked or through a linker).

While the invention is described with respect to use in humans, it is also suitable for animal, *e.g*. veterinary use. Thus certain preferred organisms include, but are not limited to humans, non-human primates, canines, equines, felines, porcines, ungulates, largomorphs, and the like.

The methods of this invention are not limited to humans or non-human animals showing one or more symptom(s) of the pathologies described herein, but are also useful in a prophylactic context. Thus, the active agents of this invention can be administered to organisms to prevent the onset/development of one or more symptoms of the pathologies described herein (*e.g*., atherosclerosis, stroke, *etc*.). Particularly preferred subjects in this context are subjects showing one or more risk factors for the pathology. Thus, for example, in the case of atherosclerosis risk factors include family history, hypertension, obesity, high alcohol consumption, smoking, high blood cholesterol, high blood triglycerides, elevated blood LDL, VLDL, IDL, or low HDL, diabetes, or a family history of diabetes, high blood lipids, heart attack, angina or stroke, *etc..*

### II. Active Agents.

A wide variety of active agents are suitable for the treatment of one or more of the indications discussed above. These agents include, but are not limited to class A amphipathic helical peptides, class A amphipathic helical peptide mimetics of apoA-I having aromatic or aliphatic residues in the non-polar face, small peptides including penta-peptides, tetrapeptides, tripeptides, dipeptides and pairs of amino acids, Apo-J (G* peptides), and peptide mimetics, *e.g*., as described below.

### A) Class A amphipathic helical peptides.

In certain embodiments, the activate agents for use in the method of this invention include class A amphipathic helical peptides, e.g. as described in U.S. Patent 6,664,230, and PCT Publications WO 02/15923 and WO 2004/034977. It was discovered that peptides comprising a class A amphipathic helix ("class A peptides"), in addition to being capable of mitigating one or more symptoms of atherosclerosis are also useful in the treatment of one or more of the other indications described herein.

Class A peptides are characterized by formation of an α-helix that produces a segregation of polar and non-polar residues thereby forming a polar and a nonpolar face with the positively charged residues residing at the polar-nonpolar interface and the negatively charged residues residing at the center of the polar face (*see, e.g.,* Anantharamaiah (1986) Meth. Enzymol, 128: 626-668). It is noted that the fourth exon of apo A-I, when folded into 3.667 residues/turn produces a class A amphipathic helical structure.

One class A peptide, designated 18A *(see, e.g.,* Anantharamaiah (1986) Meth. Enzymol, 128: 626-668) was modified as described herein to produce peptides orally administrable and highly effective at inhibiting or preventing one or more symptoms of atherosclerosis and/or other indications described herein.. Without being bound by a particular theory, it is believed that the peptides of this invention may act *in vivo* may by picking up seeding molecule(s) that mitigate oxidation of LDL.

We determined that increasing the number of Phe residues on the hydrophobic face of 18A would theoretically increase lipid affinity as determined by the computation described by Palgunachari et al. (1996) Arteriosclerosis, Thrombosis, & Vascular Biol. 16: 328-338. Theoretically, a systematic substitution of residues in the nonpolar face of 18A with Phe could yield six peptides. Peptides with an additional 2, 3 and 4 Phe would have theoretical lipid affinity (λ) values of 13, 14 and 15 units, respectively. However, the λ values jumped four units if the additional Phe were increased from 4 to 5 (to 19 λ units). Increasing to 6 or 7 Phe would produce a less dramatic increase (to 20 and 21 λ units, respectively).

A number of these class A peptides were made including, the peptide designated 4F, D4F, 5F, and D5F, and the like. Various class A peptides inhibited lesion development in atherosclerosis-susceptible mice. In addition, the peptides show varying, but significant degrees of efficacy in mitigating one or more symptoms of the various pathologies described herein.

**Table 2. Illustrative class A amphipathic helical peptide for use in this invention.**

| Peptide Name | Amino Acid Sequence | SEQ ID NO. |
|---|---|---|
| 6F | Ac-D-W-L-K-A-F-Y-D-K-F-F-E-K-F-K-E-F-F-NH₂ | 7 |

While the peptide of Table 2 is illustrated with an acetyl group protecting the amino terminus and an amide group protecting the carboxyl terminus, any of these protecting groups may be eliminated and/or substituted with another protecting group as described herein. In particularly preferred embodiments, the peptides comprise one or more D-form amino acids as described herein. In certain embodiments, every amino acid (*e.g*., every enantiomeric amino acid) of the peptide of Table 2 is a D-form amino acid.

Longer peptides are also suitable. Such longer peptides may entirely form a class A amphipathic helix, or the class A amphipathic helix (helices) can form one or more domains of the peptide. In addition, this invention contemplates multimeric versions of the peptides (*e.g*., concatamers). Thus, for example, the peptides illustrated herein can be coupled together (directly or through a linker (*e.g*., a carbon linker, or one or more amino acids) with one or more intervening amino acids).

It will also be appreciated in addition to the peptide sequence expressly illustrated herein, this invention also contemplates retro and retro-inverso forms of the peptide. In retro form, the direction of the sequence is reversed. In inverse forms, the chirality of the constituent amino acids is reversed (*i.e*., L form amino acids become D form amino acids and D form amino acids become L form amino acids). In the retroinverso form, both the order and the chirality of the amino acids is reversed. Thus, for example, a retro form of the 4F peptide (DWFKAFYDKVAEKFKEAF, SEQ ID NO:5), where the amino terminus is at the aspartate (D) and the carboxyl terminus is at the phenylalanine (F), has the same sequence, but the amino terminus is at the phenylalanine and the carboxy terminus is at the aspartate (i.e., FAEKFKEAVKDYFAKFWD, SEQ ID NO:104). Where the 4F peptide comprises all L amino acids, the retro-inverso form will have the sequence shown above (SEQ ID NO:104) and comprise all D form amino acids. As illustrated in the helical wheel diagrams of Figure 15, 4F and retroinverso (Rev-4F) are mirror images of each other with identical segregation of the polar and nonpolar faces with the positively charged residues residing at the polar-nonpolar interface and the negatively charged residues residing at the center of the polar face. These mirror images of the same polymer of amino acids are identical in terms of the segregation of the polar and nonpolar faces with the positively charged residues residing at the polar-nonpolar interface and the negatively charged residues residing at the center of the polar face. Thus, 4F and Rev-4F are enantiomers of each other. For a discussion of retro- and retro-inverso peptides *see, e.g*., Chorev and Goodman, (1995) TibTech, 13: 439-445.

Where reference is made to a sequence and orientation is not expressly indicated, the sequence can be viewed as representing the amino acid sequence in the amino to carboxyl orientation, the retro form (*i.e*., the amino acid sequence in the carboxyl to amino orientation), the retro form where L amino acids are replaced with D amino acids or D amino acids are replaced with L amino acids, and the retro-inverso form where both the order is reversed and the amino acid chirality is reversed.

### B) Blocking groups and D residues.

While the various peptides and/or amino acid pairs described herein may be shown with no protecting groups, in certain embodiments (e.g. particularly for oral administration), they can bear one, two, three, four, or more protecting groups. The protecting groups can be coupled to the C- and/or N-terminus of the peptide(s) and/or to one or more internal residues comprising the peptide(s) (*e.g*., one or more R-groups on the constituent amino acids can be blocked). Thus, for example, in certain embodiments, any of the peptides described herein can bear, *e.g*. an acetyl group protecting the amino terminus and/or an amide group protecting the carboxyl terminus.

Without being bound by a particular theory, it was a discovery of this invention that blockage, particularly of the amino and/or carboxyl termini of the subject peptides of this invention greatly improves oral delivery and significantly increases serum half-life.

A wide number of protecting groups are suitable for this purpose. Such groups include, but are not limited to acetyl, amide, and alkyl groups with acetyl and alkyl groups being particularly preferred for N-terminal protection and amide groups being preferred for carboxyl terminal protection. In certain particularly preferred embodiments, the protecting groups include, but are not limited to alkyl chains as in fatty acids, propeonyl, formyl, and others. Particularly preferred carboxyl protecting groups include amides, esters, and ether-forming protecting groups. In one preferred embodiment, an acetyl group is used to protect the amino terminus and an amide group is used to protect the carboxyl terminus. These blocking groups enhance the helix-forming tendencies of the peptides. Certain particularly preferred blocking groups include alkyl groups of various lengths, e.g. groups having the formula: CH₃-(CH₂)ₙ-CO- where n ranges from about 1 to about 20, preferably from about 1 to about 16 or 18, more preferably from about 3 to about 13, and most preferably from about 3 to about 10.

In certain particularly preferred embodiments, the protecting groups include, but are not limited to alkyl chains as in fatty acids, propeonyl, formyl, and others. Particularly preferred carboxyl protecting groups include amides, esters, and ether-forming protecting groups. In one preferred embodiment, an acetyl group is used to protect the amino terminus and an amide group is used to protect the carboxyl terminus. These blocking groups enhance the helix-forming tendencies of the peptides. Certain particularly preferred blocking groups include alkyl groups of various lengths, *e.g*. groups having the formula: CH₃-(CH₂)ₙ-CO- where n ranges from about 3 to about 20, preferably from about 3 to about 16, more preferably from about 3 to about 13, and most preferably from about 3 to about 10.

Other protecting groups include, but are not limited to Fmoc, t-butoxycarbonyl (*t*-BOC), 9-fluoreneacetyl group, 1-fluorenecarboxylic group, 9-florenecarboxylic group, 9-fluorenone-1-carboxylic group, benzyloxycarbonyl, Xanthyl (Xan), Trityl (Trt), 4-methyltrityl (Mtt), 4-methoxytrityl (Mmt), 4-methoxy-2,3,6-trimethyl-benzenesulphonyl (Mtr), Mesitylene-2-sulphonyl (Mts), 4,4-dimethoxybenzhydryl (Mbh),Tosyl (Tos), 2,2,5,7,8-pentamethyl chroman-6-sulphonyl (Pmc), 4-methylbenzyl (MeBzl), 4-methoxybenzyl (MeOBzl), Benzyloxy (BzlO), Benzyl (Bzl), Benzoyl (Bz), 3-nitro-2-pyridinesulphenyl (Npys), 1-(4,4-dimentyl-2,6-diaxocyclohexylidene)ethyl (Dde), 2,6-dichlorobenzyl (2,6-DiCl-Bzl), 2-chlorobenzyloxycarbonyl (2-Cl-Z), 2-bromobenzyloxycarbonyl (2-Br-Z), Benzyloxymethyl (Bom), cyclohexyloxy (cHxO),t-butoxymethyl (Bum), t-butoxy (tBuO), t-Butyl (tBu), Acetyl (Ac), and Trifluoroacetyl (TFA).

Protecting/blocking groups are well known to those of skill as are methods of coupling such groups to the appropriate residue(s) comprising the peptides of this invention (*see, e.g.,* Greene et al., (1991) Protective Groups in Organic Synthesis, 2nd ed., John Wiley & Sons, Inc. Somerset, N.J.). In one preferred embodiment, for example, acetylation is accomplished during the synthesis when the peptide is on the resin using acetic anhydride. Amide protection can be achieved by the selection of a proper resin for the synthesis. During the synthesis of the peptides described herein in the examples, rink amide resin was used. After the completion of the synthesis, the semipermanent protecting groups on acidic bifunctional amino acids such as Asp and Glu and basic amino acid Lys, hydroxyl of Tyr are all simultaneously removed. The peptides released from such a resin using acidic treatment comes out with the n-terminal protected as acetyl and the carboxyl protected as NH₂ and with the simultaneous removal of all of the other protecting groups.

In certain particularly preferred embodiments, the peptides comprise one or more D-form (dextro rather than levo) amino acids as described herein. In certain embodiments at least two enantiomeric amino acids, more preferably at least 4 enantiomeric amino acids and most preferably at least 8 or 10 enantiomeric amino acids are "D" form amino acids. In certain embodiments every other, ore even every amino acid (e.g. every enantiomeric amino acid) of the peptides described herein is a D-form amino acid.

In certain embodiments at least 50% of the enantiomeric amino acids are "D" form, more preferably at least 80% of the enantiomeric amino acids are "D" form, and most preferably at least 90% or even all of the enantiomeric amino acids are "D" form amino acids.

### III. Peptide preparation.

The peptides used in this invention can be chemically synthesized using standard chemical peptide synthesis techniques or, particularly where the peptide does not comprise "D" amino acid residues, can be recombinantly expressed. In certain embodiments, even peptides comprising "D" amino acid residues are recombinantly expressed. Where the polypeptides are recombinantly expressed, a host organism (*e.g*. bacteria, plant, fungal cells, *etc.)* in cultured in an environment where one or more of the amino acids is provided to the organism exclusively in a D form. Recombinantly expressed peptides in such a system then incorporate those D amino acids.

In preferred embodiments the peptides are chemically synthesized by any of a number of fluid or solid phase peptide synthesis techniques known to those of skill in the art. Solid phase synthesis in which the C-terminal amino acid of the sequence is attached to an insoluble support followed by sequential addition of the remaining amino acids in the sequence is a preferred method for the chemical synthesis of the polypeptides of this invention. Techniques for solid phase synthesis are well known to those of skill in the art and are described, for example, by Barany and Merrifield (1963) Solid-Phase Peptide Synthesis; pp. 3-284 in The Peptides: Analysis, Synthesis, Biology. Vol. 2: Special Methods in Peptide Synthesis, Part A.; Merrifield et al. (1963) J. Am. Chem. Soc., 85: 2149-2156, and Stewart et al. (1984) Solid Phase Peptide Synthesis, 2nd ed. Pierce Chem. Co., Rockford, Ill.

In certain embodiments, the peptides are synthesized by the solid phase peptide synthesis procedure using a benzhyderylamine resin (Beckman Bioproducts, 0.59 mmol of NH₂/g of resin) as the solid support. The COOH terminal amino acid (*e.g., t-*butylcarbonyl-Phe) is attached to the solid support through a 4-(oxymethyl)phenacetyl group. This is a more stable linkage than the conventional benzyl ester linkage, yet the finished peptide can still be cleaved by hydrogenation. Transfer hydrogenation using formic acid as the hydrogen donor is used for this purpose. Detailed protocols used for peptide synthesis and analysis of synthesized peptides are described in a miniprint supplement accompanying Anantharamaiah et al. (1985) J. Biol. Chem., 260(16): 10248-10255.

It is noted that in the chemical synthesis of peptides, particularly peptides comprising D amino acids, the synthesis usually produces a number of truncated peptides in addition to the desired full-length product. The purification process (*e.g*. HPLC) typically results in the loss of a significant amount of the full-length product.

It was a discovery of this invention that, in the synthesis of a D peptide (*e.g*. D-4), in order to prevent loss in purifying the longest form one can dialyze and use the mixture and thereby eliminate the last HPLC purification. Such a mixture loses about 50% of the potency of the highly purified product (*e.g*. per wt of protein product), but the mixture contains about 6 times more peptide and thus greater total activity.

### IV. Pharmaceutical formulations and devices.

### A) Pharmaceutical formulations.

In order to carry out the methods of the invention, one or more active agents of this invention are administered, *e.g*. to an individual diagnosed as having one or more symptoms of atherosclerosis, or as being at risk for atherosclerosis and or the various other pathologies described herein. The active agent(s) can be administered in the "native" form or, if desired, in the form of salts, esters, amides, prodrugs, derivatives, and the like, provided the salt, ester, amide, prodrug or derivative is suitable pharmacologically, *i.e*., effective in the present method. Salts, esters, amides, prodrugs and other derivatives of the active agents can be prepared using standard procedures known to those skilled in the art of synthetic organic chemistry and described, for example, by March (1992) Advanced Organic Chemistry; Reactions, Mechanisms and Structure, 4th Ed. N.Y. Wiley-Interscience.

For example, acid addition salts are prepared from the free base using conventional methodology, that typically involves reaction with a suitable acid. Generally, the base form of the drug is dissolved in a polar organic solvent such as methanol or ethanol and the acid is added thereto. The resulting salt either precipitates or can be brought out of solution by addition of a less polar solvent. Suitable acids for preparing acid addition salts include both organic acids, *e.g*., acetic acid, propionic acid, glycolic acid, pyruvic acid, oxalic acid, malic acid, malonic acid, succinic acid, maleic acid, fumaric acid, tartaric acid, citric acid, benzoic acid, cinnamic acid, mandelic acid, methanesulfonic acid, ethanesulfonic acid, p-toluenesulfonic acid, salicylic acid, and the like, as well as inorganic acids, *e.g*., hydrochloric acid, hydrobromic acid, sulfuric acid, nitric acid, phosphoric acid, and the like. An acid addition salt may be reconverted to the free base by treatment with a suitable base. Particularly preferred acid addition salts of the active agents herein are halide salts, such as may be prepared using hydrochloric or hydrobromic acids. Conversely, preparation of basic salts of the active agents of this invention are prepared in a similar manner using a pharmaceutically acceptable base such as sodium hydroxide, potassium hydroxide, ammonium hydroxide, calcium hydroxide, trimethylamine, or the like. Particularly preferred basic salts include alkali metal salts, *e.g*., the sodium salt, and copper salts.

Preparation of esters typically involves functionalization of hydroxyl and/or carboxyl groups which may be present within the molecular structure of the drug. The esters are typically acyl-substituted derivatives of free alcohol groups, *i.e*., moieties that are derived from carboxylic acids of the formula RCOOH where R is alky, and preferably is lower alkyl. Esters can be reconverted to the free acids, if desired, by using conventional hydrogenolysis or hydrolysis procedures.

Amides and prodrugs can also be prepared using techniques known to those skilled in the art or described in the pertinent literature. For example, amides may be prepared from esters, using suitable amine reactants, or they may be prepared from an anhydride or an acid chloride by reaction with ammonia or a lower alkyl amine. Prodrugs are typically prepared by covalent attachment of a moiety that results in a compound that is therapeutically inactive until modified by an individual's metabolic system.

The active agents identified herein are useful for parenteral, topical, oral, nasal (or otherwise inhaled), rectal, or local administration, such as by aerosol or transdermally, for prophylactic and/or therapeutic treatment of one or more of the pathologies/indications described herein (*e.g*., atherosclerosis and/or symptoms thereof). The pharmaceutical compositions can be administered in a variety of unit dosage forms depending upon the method of administration. Suitable unit dosage forms, include, but are not limited to powders, tablets, pills, capsules, lozenges, suppositories, patches, nasal sprays, injectibles, implantable sustained-release formulations, lipid complexes, *etc.*

The active agents of this invention are typically combined with a pharmaceutically acceptable carrier (excipient) to form a pharmacological composition. Pharmaceutically acceptable carriers can contain one or more physiologically acceptable compound(s) that act, for example, to stabilize the composition or to increase or decrease the absorption of the active agent(s). Physiologically acceptable compounds can include, for example, carbohydrates, such as glucose, sucrose, or dextrans, antioxidants, such as ascorbic acid or glutathione, chelating agents, low molecular weight proteins, protection and uptake enhancers such as lipids, compositions that reduce the clearance or hydrolysis of the active agents, or excipients or other stabilizers and/or buffers.

Other physiologically acceptable compounds include wetting agents, emulsifying agents, dispersing agents or preservatives that are particularly useful for preventing the growth or action of microorganisms. Various preservatives are well known and include, for example, phenol and ascorbic acid. One skilled in the art would appreciate that the choice of pharmaceutically acceptable carrier(s), including a physiologically acceptable compound depends, for example, on the route of administration of the active agent(s) and on the particular physio-chemical characteristics of the active agent(s).

The excipients are preferably sterile and generally free of undesirable matter. These compositions may be sterilized by conventional, well-known sterilization techniques.

In therapeutic applications, the compositions of this invention are administered to a patient suffering from one or more symptoms of the one or more pathologies described herein, or at risk for one or more of the pathologies described herein in an amount sufficient to prevent and/or cure and/or or at least partially prevent or arrest the disease and/or its complications. An amount adequate to accomplish this is defined as a "therapeutically effective dose." Amounts effective for this use will depend upon the severity of the disease and the general state of the patient's health. Single or multiple administrations of the compositions may be administered depending on the dosage and frequency as required and tolerated by the patient. In any event, the composition should provide a sufficient quantity of the active agents of the formulations of this invention to effectively treat (ameliorate one or more symptoms) the patient.

The concentration of active agent(s) can vary widely, and will be selected primarily based on fluid volumes, viscosities, body weight and the like in accordance with the particular mode of administration selected and the patient's needs. Concentrations, however, will typically be selected to provide dosages ranging from about 0.1 or 1 mg/kg/day to about 50 mg/kg/day and sometimes higher. Typical dosages range from about 3 mg/kg/day to about 3.5 mg/kg/day, preferably from about 3.5 mg/kg/day to about 7.2 mg/kg/day, more preferably from about 7.2 mg/kg/day to about 11.0 mg/kg/day, and most preferably from about 11.0 mg/kg/day to about 15.0 mg/kg/day. In certain preferred embodiments, dosages range from about 10 mg/kg/day to about 50 mg/kg/day. In certain embodiments, dosages range from about 20 mg to about 50 mg given orally twice daily. It will be appreciated that such dosages may be varied to optimize a therapeutic regimen in a particular subject or group of subjects.

In certain preferred embodiments, the active agents of this invention are administered orally (e.g. via a tablet) or as an injectable in accordance with standard methods well known to those of skill in the art. In other preferred embodiments, the peptides, may also be delivered through the skin using conventional transdermal drug delivery systems, i.e., transdermal "patches" wherein the active agent(s) are typically contained within a laminated structure that serves as a drug delivery device to be affixed to the skin. In such a structure, the drug composition is typically contained in a layer, or "reservoir," underlying an upper backing layer. It will be appreciated that the term "reservoir" in this context refers to a quantity of "active ingredient(s)" that is ultimately available for delivery to the surface of the skin. Thus, for example, the "reservoir" may include the active ingredient(s) in an adhesive on a backing layer of the patch, or in any of a variety of different matrix formulations known to those of skill in the art. The patch may contain a single reservoir, or it may contain multiple reservoirs.

In one embodiment, the reservoir comprises a polymeric matrix of a pharmaceutically acceptable contact adhesive material that serves to affix the system to the skin during drug delivery. Examples of suitable skin contact adhesive materials include, but are not limited to, polyethylenes, polysiloxanes, polyisobutylenes, polyacrylates, polyurethanes, and the like. Alternatively, the drug-containing reservoir and skin contact adhesive are present as separate and distinct layers, with the adhesive underlying the reservoir which, in this case, may be either a polymeric matrix as described above, or it may be a liquid or hydrogel reservoir, or may take some other form. The backing layer in these laminates, which serves as the upper surface of the device, preferably functions as a primary structural element of the "patch" and provides the device with much of its flexibility. The material selected for the backing layer is preferably substantially impermeable to the active agent(s) and any other materials that are present.

Other preferred formulations for topical drug delivery include, but are not limited to, ointments and creams. Ointments are semisolid preparations which are typically based on petrolatum or other petroleum derivatives. Creams containing the selected active agent, are typically viscous liquid or semisolid emulsions, often either oil-in-water or water-in-oil. Cream bases are typically water-washable, and contain an oil phase, an emulsifier and an aqueous phase. The oil phase, also sometimes called the "internal" phase, is generally comprised of petrolatum and a fatty alcohol such as cetyl or stearyl alcohol; the aqueous phase usually, although not necessarily, exceeds the oil phase in volume, and generally contains a humectant. The emulsifier in a cream formulation is generally a nonionic, anionic, cationic or amphoteric surfactant. The specific ointment or cream base to be used, as will be appreciated by those skilled in the art, is one that will provide for optimum drug delivery. As with other carriers or vehicles, an ointment base should be inert, stable, nonirritating and nonsensitizing.

Unlike typical peptide formulations, the peptides of this invention comprising D-form amino acids can be administered, even orally, without protection against proteolysis by stomach acid, *etc*. Nevertheless, in certain embodiments, peptide delivery can be enhanced by the use of protective excipients. This is typically accomplished either by complexing the polypeptide with a composition to render it resistant to acidic and enzymatic hydrolysis or by packaging the polypeptide in an appropriately resistant carrier such as a liposome. Means of protecting polypeptides for oral delivery are well known in the art (see, *e.g*., U.S. Patent 5,391,377 describing lipid compositions for oral delivery of therapeutic agents).

Elevated serum half-life can be maintained by the use of sustained-release protein "packaging" systems. Such sustained release systems are well known to those of skill in the art. In one preferred embodiment, the ProLease biodegradable microsphere delivery system for proteins and peptides (Tracy (1998) Biotechnol. Prog., 14: 108; Johnson et al. (1996) Nature Med. 2: 795; Herbert et al. (1998), Pharmaceut. Res. 15, 357) a dry powder composed of biodegradable polymeric microspheres containing the active agent in a polymer matrix that can be compounded as a dry formulation with or without other agents.

The ProLease microsphere fabrication process was specifically designed to achieve a high encapsulation efficiency while maintaining integrity of the active agent. The process consists of (i) preparation of freeze-dried drug particles from bulk by spray freeze-drying the drug solution with stabilizing excipients, (ii) preparation of a drug-polymer suspension followed by sonication or homogenization to reduce the drug particle size, (iii) production of frozen drug-polymer microspheres by atomization into liquid nitrogen, (iv) extraction of the polymer solvent with ethanol, and (v) filtration and vacuum drying to produce the final dry-powder product. The resulting powder contains the solid form of the active agents, which is homogeneously and rigidly dispersed within porous polymer particles. The polymer most commonly used in the process, poly(lactide-co-glycolide) (PLG), is both biocompatible and biodegradable.

Encapsulation can be achieved at low temperatures (*e.g*., -40°C). During encapsulation, the protein is maintained in the solid state in the absence of water, thus minimizing water-induced conformational mobility of the protein, preventing protein degradation reactions that include water as a reactant, and avoiding organic-aqueous interfaces where proteins may undergo denaturation. A preferred process uses solvents in which most proteins are insoluble, thus yielding high encapsulation efficiencies (*e.g*., greater than 95%).

In another embodiment, one or more components of the solution can be provided as a "concentrate", *e.g*., in a storage container (*e.g*., in a premeasured volume) ready for dilution, or in a soluble capsule ready for addition to a volume of water.

The foregoing formulations and administration methods are intended to be illustrative and not limiting. It will be appreciated that, using the teaching provided herein, other suitable formulations and modes of administration can be readily devised.

### B) Lipid-based formulations.

In certain embodiments, the active agents of this invention are administered in conjunction with one or more lipids. The lipids can be formulated as an excipient to protect and/or enhance transport/uptake of the active agents or they can be administered separately.

Without being bound by a particular theory, it was discovered of this invention that administration (*e.g*. oral administration) of certain phospholipids can significantly increase HDL/LDL ratios. In addition, it is believed that certain medium-length phospholipids are transported by a process different than that involved in general lipid transport. Thus, co-administration of certain medium-length phospholipids with the active agents of this invention confer a number of advantages: They protect the active agents from digestion or hydrolysis, they improve uptake, and they improve HDL/LDL ratios.

The lipids can be formed into liposomes that encapsulate the active agents of this invention and/or they can be complexed/admixed with the active agents and/or they can be covalently coupled to the active agents. Methods of making liposomes and encapsulating reagents are well known to those of skill in the art (*see, e.g.,* Martin and Papahadjopoulos (1982) J. Biol. Chem., 257: 286-288; Papahadjopoulos et al. (1991) Proc. Natl. Acad. Sci. USA, 88: 11460-11464; Huang et al. (1992) Cancer Res., 52:6774-6781; Lasic et al. (1992) FEBS Lett., 312: 255-258., and the like).

Preferred phospholipids for use in these methods have fatty acids ranging from about 4 carbons to about 24 carbons in the sn-1 and sn-2 positions. In certain preferred embodiments, the fatty acids are saturated. In other preferred embodiments, the fatty acids can be unsaturated. Various preferred fatty acids are illustrated in Table 3.

**Table 3. Preferred fatty acids in the sn-1 and/or sn-2 position of the preferred phospholipids for administration of active agents described herein.**

| Carbon No. | Common Name | IUPAC Name |
|---|---|---|
| 3:0 | Propionoyl | Trianoic |
| 4:0 | Butanoyl | Tetranoic |
| 5:0 | Pentanoyl | Pentanoic |
| 6:0 | Caproyl | Hexanoic |
| 7:0 | Heptanoyl | Heptanoic |
| 8:0 | Capryloyl | Octanoic |
| 9:0 | Nonanoyl | Nonanoic |
| 10:0 | Capryl | Decanoic |
| 11:0 | Undcanoyl | Undecanoic |
| 12:0 | Lauroyl | Dodecanoic |
| 13:0 | Tridecanoyl | Tridecanoic |
| 14:0 | Myristoyl | Tetradecanoic |
| 15:0 | Pentadecanoyl | Pentadecanoic |
| 16:0 | Palmitoyl | Hexadecanoic |
| 17:0 | Heptadecanoyl | Heptadecanoic |
| 18:0 | Stearoyl | Octadecanoic |
| 19:0 | Nonadecanoyl | Nonadecanoic |
| 20:0 | Arachidoyl | Eicosanoic |
| 21:0 | Heniecosanoyl | Heniecosanoic |
| 22:0 | Behenoyl | Docosanoic |
| 23:0 | Trucisanoyl | Trocosanoic |
| 24:0 | Lignoceroyl | Tetracosanoic |
| 14:1 | Myristoleoyl (9-cis) | |
| 14:1 | Myristelaidoyl (9-trans) | |
| 16:1 | Palmitoleoyl (9-cis) | |
| 16:1 | Palmitelaidoyl (9-trans) | |

The fatty acids in these positions can be the same or different. Particularly preferred phospholipids have phosphorylcholine at the sn-3 position.

### C) Specialized delivery/devices.

### 1. Drug-eluting stents.

Restenosis, the reclosure of a previously stenosed and subsequently dilated peripheral or coronary vessel occurs at a significant rate (*e.g*., 20-50% for these procedures) and is dependent on a number of clinical and morphological variables. Restenosis may begin shortly following an angioplasty procedure, but usually ceases at the end of approximately six (6) months.

A recent technology that has been developed to address the problem of restenosis in intravascular stents. Stents are typically devices that are permanently implanted (expanded) in coronary and peripheral vessels. The goal of these stents is to provide a long-term "scaffolding" or support for the diseased (stenosed) vessels. The theory being, if the vessel is supported from the inside, it will not close down or restenose.

Known stent designs include, but are not limited to monofilament wire coil stents (*see, e.g.,* U.S. Patent 4,969,458); welded metal cages (*see*, *e.g.,* U.S. Patents 4,733,665 and 4,776,337), thin-walled metal cylinders with axial slots formed around the circumference (*see*, *e.g.,* U.S. Patents 4,733,665, 4,739,762, 4,776,337, and the like). Known construction materials for use in stents include, but are not limited to polymers, organic fabrics and biocompatible metals, such as, stainless steel, gold, silver, tantalum, titanium, and shape memory alloys such as Nitinol.

To further prevent restenosis, stents can be covered and/or impregnated with one or more pharmaceutical, *e.g*., in controlled release formulations to inhibit cell proliferation associated with rest enosis. Most commonly such "drug-eluting" stents are designed to deliver various cancer drugs (cytotoxins).

However, because of their activity in mitigating inflammatory responses, reducing and/or eliminated oxidized lipids and/or other oxidized species, inhibiting macrophage chemotactic activity and the like, the active agents described herein are well suited to prevent restenosis. Thus, in certain embodiments, this invention contemplates stents having one or more of the active agents described herein coated on the surface and/or retained within cavities or microcavities in the surface of the stent.

In certain embodiments the active agents are contained within biocompatible matrices (*e.g*. biocompatible polymers such as urethane, silicone, and the like). Suitable biocompatible materials are described, for example, in U.S. Patent Publications 20050084515, 200500791991, 20050070996, and the like. In various embodiments the polymers include, but are not limited to silicone-urethane copolymer, a polyurethane, a phenoxy, ethylene vinyl acetate, polycaprolactone, poly(lactide-co-glycolide), polylactide, polysulfone, elastin, fibrin, collagen, chondroitin sulfate, a biocompatible polymer, a biostable polymer, a biodegradable polymer.

Thus, in certain embodiments this invention provides a stent for delivering drugs to a vessel in a body. The stent typically comprises stent framework including a plurality of reservoirs formed therein. The reservoirs typically include an active agent and/or active agent-containing polymer positioned in the reservoir and/or coated on the surface of the stent. In various embodiments the stent is a metallic base or a polymeric base. Certain preferred stent materials include, but are not limited to stainless steel, nitinol, tantalum, MP35N alloy, platinum, titanium, a suitable biocompatible alloy, a suitable biocompatible polymer, and/or a combination thereof.

In various embodiments where the stent comprises pores (*e.g*. reservoirs), the pores can include micropores (*e.g*., having a diameter that ranges from about 10 to about 50 µm, preferably about 20 µm or less). In various embodiments the micropores have a depth in the range of about 10 µm to about 50 µm. In various embodiments the micropores extend through the stent framework having an opening on an interior surface of the stent and an opening on an exterior surface of the stent. In certain embodiments the stent can, optionally comprise a cap layer disposed on the interior surface of the stent framework, the cap layer covering at least a portion of the through-holes and providing a barrier characteristic to control an elution rate of the active agent(s) in the polymer from the interior surface of the stent framework. In various embodiments the reservoirs comprise channels along an exterior surface of the stent framework. The stent can optionally have multiple layers of polymer where different layers of polymer carry different active agent(s) and/or other drugs.

In certain embodiments the stent comprises: an adhesion layer positioned between the stent framework and the polymer. Suitable adhesion layers include, but are not limited to a polyurethane, a phenoxy, poly(lactide-co-glycolide)- , polylactide, polysulfone, polycaprolactone, an adhesion promoter, and/or a combination thereof.

In addition to stents, the active agents can be coated on or contained within essentially any implantable medical device configured for implantation in a extravascular and/or intravascular location.

Also provided are methods of manufacturing a drug-polymer stent, comprising. The methods involve providing a stent framework; cutting a plurality of reservoirs in the stent framework, *e.g*., using a high power laser; applying one or more of the active agents and/or a drug polymer to at least one reservoir; drying the drug polymer; applying a polymer layer to the dried drug polymer; and drying the polymer layer. The active agent(s) and/or polymer(s) can be applied by any convenient method including but not limited to spraying, dipping, painting, brushing and dispensing.

Also provided are methods of treating a vascular condition and/or a condition characterized by an inflammatory response and/or a condition characterized by the formation of oxidized reactive species. The methods typically involve positioning a stent or other implantable device as described above within the body (e.g. within a vessel of a body) and eluting at least active agent from at least one surface of the implant.

### 2. Impregnated grafts and transplants.

Vascular grafts can be classified as either biological or synthetic. There are two commonly used types of biological grafts. An autograft is one taken from another site in the patient. In peripheral vascular surgery by far the most commonly used such graft is the long saphenous vein. This can be used *in situ* with the valves surgically destroyed with an intraluminal cutting valvutome.

Alternatively, the vein can be removed and reversed but this typically produces a discrepancy between the anastomotic size of the artery and vein. In thoracic surgery the use of internal mammary artery for coronary artery bypass surgery is another example of an autograft. An allograft is one taken from another animal of the same species. Externally supported umbilical vein is rarely used but is an example of such a graft.

Synthetic grafts are most commonly made from Dacron or polytetrafluroethylene (PTFE). Dacron grafts are frequently used in aortic and aorto-iliac surgery. Below the inguinal ligament the results of all synthetic grafts are inferior to those obtained with the use of vein grafts. Suitable vein is not always available and in this situation PTFE is typically used. It can be used in conjunction with vein as a composite graft. Neointimal hyperplasia at the distal anastomosis can be reduced by the incorporation of a segment of vein as either a Millar Cuff or Taylor Patch to improve the long-term patency of the grafts.

The commonest complications associated with the use of vascular grafts include Graft occlusion, Graft infection, true and false aneurysms at the site of anastomosis, distal embolization, and erosion in to adjacent structures - e.g. Aorto-enteric fistulae. Many of these conditions are associated with an inflammatory response, macrophage migration into the site, and/or the formation of reactive oxygen species (e.g., oxidized lipids). To reduce such complications, the graft (synthetic or biological can be soaked, or otherwise coated, with one or more of the active agents described herein.

In addition, it is contemplated that other implantable tissues or materials can similarly be impregnated or coated with one or more active agents of this invention. Thus, for example, in certain embodiments this invention contemplates the use of impregnated sutures to minimize inflammation and/or infection and/or tissue rejection.

### 3. Subcutaneous matrices.

In certain embodiments, one or more active agents described herein are administered alone or in combination with other therapeutics as described herein in implantable (*e.g*., subcutaneous) matrices.

A major problem with standard drug dosing is that typical delivery of drugs results in a quick burst of medication at the time of dosing, followed by a rapid loss of the drug from the body. Most of the side effects of a drug occur during the burst phase of its release into the bloodstream. Secondly, the time the drug is in the bloodstream at therapeutic levels is very short, most is used and cleared during the short burst.

Drugs (*e.g*., the active agents described herein) imbedded in various matrix materials for sustained release provides some solution to these problems. Drugs embedded, for example, in polymer beads or in polymer wafers have several advantages. First, most systems allow slow release of the drug, thus creating a continuous dosing of the body with small levels of drug. This typically prevents side effects associated with high burst levels of normal injected or pill based drugs. Secondly, since these polymers can be made to release over hours to months, the therapeutic span of the drug is markedly increased. Often, by mixing different ratios of the same polymer components, polymers of different degradation rates can be made, allowing remarkable flexibility depending on the agent being used. A long rate of drug release is beneficial for people who might have trouble staying on regular dosage, such as the elderly, but is also an ease of use improvement that everyone can appreciate. Most polymers can be made to degrade and be cleared by the body over time, so they will not remain in the body after the therapeutic interval.

Another advantage of polymer based drug delivery is that the polymers often can stabilize or solubilize proteins, peptides, and other large molecules that would otherwise be unusable as medications. Finally, many drug/polymer mixes can be placed directly in the disease area, allowing specific targeting of the medication where it is needed without losing drug to the "first pass" effect. This is certainly effective for treating the brain, which is often deprived of medicines that can't penetrate the blood/brain barrier.

A number of implantable matrix (sustained release) systems are know to those of skill and can readily be adapted for use with one or more of the active agents described herein. Suitable sustained release systems include, but are not limited to Re-Gel®, SQ2Gel®, and Oligosphere® by MacroMed, ProLease® and Medisorb® by Alkermes, Paclimer® and Gliadel® Wafer by Guilford pharmaceuticals, the Duros implant by Alza, acoustic bioSpheres by Point Biomedical, the Intelsite capsule by Scintipharma, Inc., and the like.

### 4. Other "specialty delivery systems".

Other "specialty" delivery systems include, but are not limited to lipid based oral mist that allows absorption of drugs across the oral mucosa, developed by Generex Biotechnology, the oral transmucosal system (OTS^{™}) by Anesta Corp., the inhalable dry powder and PulmoSpheres technology by Inhale Therapeutics, the AERx® Pulmonary Drug Delivery System by Aradigm, the AIR mechanism by Alkermes, and the like.

Another approach to delivery developed by Alkermes is a system targeted for elderly and pediatric use, two populations for which taking pills is often difficult is known as Drug Sipping Technology (DST). The medication is placed in a drinking straw device, prevented from falling out by filters on either end of it. The patient merely has to drink clear liquid (water, juice, soda) through the straw. The drug dissolves in the liquid as it is pulled through and is ingested by the patient. The filter rises to the top of the straw when all of the medication is taken. This method has the advantage in that it is easy to use, the liquid often masks the medication's taste, and the drug is pre-dissolved for more efficient absorption.

It is noted that these uses and delivery systems are intended to be illustrative and not limiting. Using the teachings provided herein, other uses and delivery systems will be known to those of skill in the art.

### V. Additional pharmacologically active agents.

### Combined active agents

In various embodiments, the use of combinations of two or more active agents described is contemplated in the treatment of the various pathologies/indications described herein. The use of combinations of active agents can alter pharmacological activity, bioavailability, and the like.

### EXAMPLES

The following examples are offered to illustrate, but not to limit the claimed invention.

### Example 1

### Use of ApoJ-Related Peptides to Mediate Symptoms of Atherosclerosis

### Prevention of LDL-induced monocyte chemotactic activity

Figure 1 illustrates a comparison of the effect of D-4F (Anantharamaiah et al. (2002) Circulation, 105: 290-292) with the effect of an apoJ peptide made from D amino acids (D-J336, Ac-L-L-E-Q-L-N-E-Q-F-N-W-V-S-R-L-A-N-L-T-Q-G-E-NH₂, SEQ ID NO:1129)) on the prevention of LDL-induced monocyte chemotactic activity *in vitro* in a co-incubation. Human aortic endothelial cells were incubated with medium alone (no addition), with control human LDL (200 µg protein/ml) or control human LDL + control human HDL (350 µg HDL protein/ml). D-J336 or D-4F was added to other wells in a concentration range as indicated plus control human LDL (200 µg protein/ml). Following overnight incubation, the supernatants were assayed for monocyte chemotactic activity. As shown in Figure 1, the *in vitro* concentration of the apoJ variant peptide that prevents LDL-induced monocyte chemotactic activity by human artery wall cells is 10 to 25 times less than the concentration required for the D-4F peptide.

### Prevention of LDL-induced monocyte chemotactic activity by Pre-Treatment of artery wall cells with D-J336

Figure 2 illustrates a comparison of the effect of D-4F with the effect of D-J336 on the prevention of LDL induced monocyte chemotactic activity in a pre-incubation. Human aortic endothelial cells were pre- incubated with D-J336 or D-4F at 4, 2, and 1 µg/ml for DJ336 or 100, 50, 25, and 12.5 µg/ml for D-4F for 6 hrs. The cultures were then washed and were incubated with medium alone (no addition), or with control human LDL (200 µg protein/ml), or with control human LDL + control human HDL (350 µg HDL protein/ml) as assay controls. The wells that were pre-treated with peptides received the control human LDL at 200 µg protein/ml. Following overnight incubation, the supernatants were assayed for monocyte chemotactic activity.

As illustrated in Figure 2, the ApoJ variant peptide was 10-25 times more potent in preventing LDL oxidation by artery wall cells *in vitro.*

### The effect of apo J peptide mimetics on HDL protective capacity in LDL receptor null mice.

D-4F designated as F, or the apoJ peptide made from D amino acids (D-J336, designated as J) was added to the drinking water of LDL receptor null mice (4 per group) at 0.25 or 0.5 mg per ml of drinking water. After 24- or 48-hrs blood was collected from the mice and their HDL was isolated and tested for its ability to protect against LDL-induced monocyte chemotactic activity. Assay controls included culture wells that received no lipoproteins (no addition), or control human LDL alone (designated as LDL, 200 µg cholesterol/ml), or control LDL + control human HDL (designated as + HDL, 350 µg HDL cholesterol). For testing the mouse HDL, the control LDL was added together with mouse HDL (+F HDL or +J HDL) to artery wall cell cultures. The mouse HDL was added at 100 µg cholesterol/ml respectively. After treatment with either D-4F or D-J336 the mouse HDL at 100 µg/ml was as active as 350 µg/ml of control human HDL in preventing the control LDL from inducing the artery wall cells to produce monocyte chemotactic activity.. The reason for the discrepancy between the relative doses required for the D-J336 peptide relative to D-4F *in vitro* and *in vivo* may be related to the solubility of the peptides in water and we believe that when measures are taken to achieve equal solubility the D-J peptides will be much more active *in vivo* as they are *in vitro.*

### Protection against LDL-induced monocyte chemotactic activity by HDL from apo E null mice given oral peptides.

Figure 4 illustrates the effect of oral apoA-1 peptide mimetic and apoJ peptide on HDL protective capacity. ApoE null mice (4 per group) were provided with D-4F (designated as F) at 50, 30, 20, 10, 5 µg per ml of drinking water or apoJ peptide (designated as J) at 50, 30 or 20 µg per ml of drinking water. After 24 hrs blood was collected, plasma fractionated by FPLC and fractions containing LDL (designated as mLDL for murine LDL) and fractions containing HDL (designated as mHDL) were separately pooled and HDL protective capacity against LDL oxidation as determined by LDL-induced monocyte chemotactic activity was determined. For the assay controls the culture wells received no lipoproteins (no additions), mLDL alone (at 200 µg cholesterol/ml), or mLDL + standard normal human HDL (designated as Cont. h HDL, at 350 µg HDL cholesterol/ml).

For testing the murine HDL, mLDL together with murine HDL (+F mHDL or +J mHDL) were added to artery wall cell cultures. The HDL from the mice that did not receive any peptide in their drinking water is designated as no peptide mHDL. The murine HDL was used at 100 µg cholesterol/ml. After receiving D-4F or D-J336 the murine HDL at 100 µg/ml was as active as 350 µg/ml of normal human HDL. As shown in Figure 4, when added to the drinking water the D-J peptide was as potent as D-4F in enhancing HDL protective capacity in apo E null mice.

### Ability of LDL obtained from apoE null mice given oral peptides to induce monocyte chemotactic activity.

Figure 5 illustrates the effect of oral apo A-1 peptide mimetic and apoJ peptide on LDL susceptibility to oxidation. ApoE null mice (4 per group) were provided, in their drinking water, with D-4F (designated as F) at 50, 30, 20, 10, 5 µg per ml of drinking water or the apoJ peptide (D-J336 made from D amino acids and designated as J) at 50, 30 or 20 µg per ml of drinking water. After 24 hrs blood was collected from the mice shown in Figure 4, plasma fractionated by FPLC and fractions containing LDL (designated as mLDL for murine LDL) were pooled and LDL susceptibility to oxidation as determined by induction of monocyte chemotactic activity was determined. For the assay controls the culture wells received no lipoproteins (no additions), mLDL alone (at 200 µg cholesterol/ml), or mLDL + standard normal human HDL (designated as Cont. h HDL, 350 µg HDL cholesterol).

Murine LDL, mLDL, from mice that received the D-4F (F mLDL) or those that received the apoJ peptide (J mLDL) were added to artery wall cell cultures. LDL from mice that did not receive any peptide in their drinking water is designated as No peptide LDL.

As shown in Figure 5, when added to the drinking water, D-J336 was slightly more potent than D-4F in rendering the LDL from apo E null mice resistant to oxidation by human artery wall cells as determined by the induction of monocyte chemotactic activity.

### Protection against phospholipid oxidation and induction of monocyte chemotactic activity by HDL obtained from apo E null mice given oral peptides.

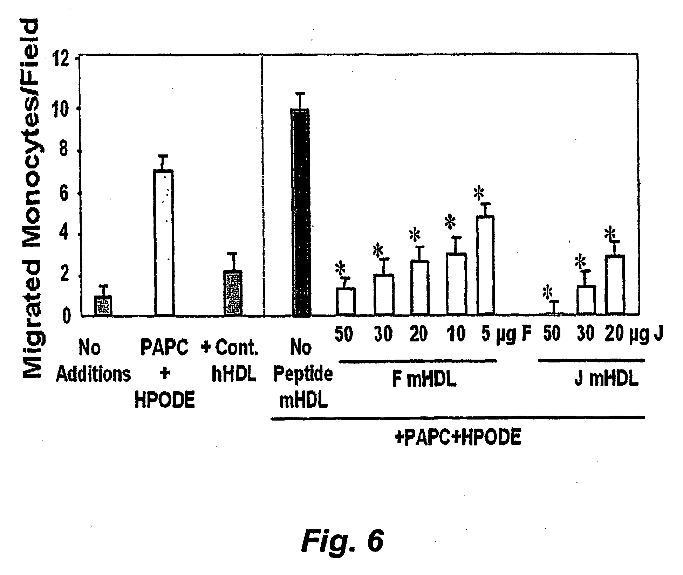

Figure 6 illustrates the effect of oral apoA-1 peptide mimetic and apoJ peptide on HDL protective capacity. ApoE null mice (4 per group) were provided with D-4F (designated as F) at 50, 30, 20, 10, 5 µg per ml of drinking water or apoJ peptide (D-J336 made from D amino acids and designated as J) at 50, 30 or 20 µg per ml of drinking water. After 24 hrs blood was collected, plasma fractionated by FPLC and fractions containing HDL (designated as mHDL) were pooled and HDL protective capacity against PAPC oxidation as determined by the induction of monocyte chemotactic activity was determined. For the assay controls the culture wells received no lipoproteins (no additions), the phospholipid PAPC at 20 µg /ml + HPODE, at 1.0 µg/ml, or PAPC+HPODE plus standard normal human HDL (at 350 µg HDL cholesterol/ml and designated as +Cont. h HDL).

For testing the murine HDL, PAPC+HPODE together with murine HDL (+F mHDL or +J mHDL) were added to artery wall cell cultures. The HDL from mice that did not receive any peptide in their drinking water is designated as "no peptide mHDL". The murine HDL was used at 100 µg cholesterol/ml.

The data show in Figure 6 indicate that, when added to the drinking water, D-J336 was as potent as D-4F in causing HDL to inhibit the oxidation of a phospholipid PAPC by the oxidant HPODE in a human artery wall co-culture as measured by the generation of monocyte chemotactic activity

### Effect of oral apoA-1 peptide mimetic and apoJ peptide on plasma paraoxonase activity in mice.

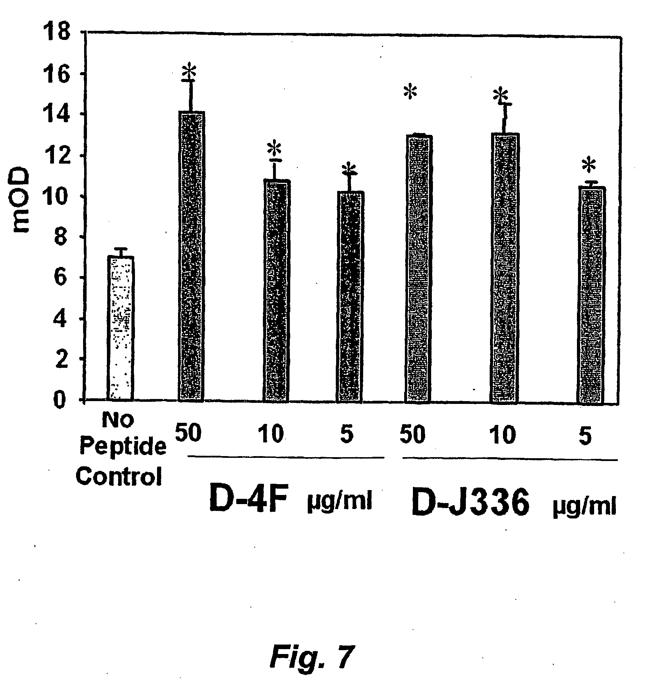

Figure 7 shows the effect of oral apoA-1 peptide mimetic and apoJ peptide on plasma paraoxonase activity in mice. ApoE null mice (4 per group) were provided with D-4F designated as F at 50, 10, 5 or 0 µg per ml of drinking water or apoJ peptide (D-J336 made from D amino acids and designated as J) at 50, 10 or 5 µg per ml of drinking water. After 24 hrs blood was collected and plasma was assayed for PON1 activity. These data demonstrate that, when added to the drinking water, D-J336 was at least as potent as D-4F in increasing the paraoxonase activity of apo E null mice.

### Example 2

### Oral G* Peptides Increase HDL Protective Capacity in Apo E Deficient Mice.

Female, 4 month old apoE deficient mice (n=4 per group) were treated with G* peptides having the following amino acid sequences. Peptide 113-122 = Ac-L V G R Q L E E F L-NH₂ (SEQ ID NO:1130), Peptide 336-357 = Ac-LLEQLNEQFN W V S R L A N L T Q G E-NH2 (SEQ ID NO:1131) and Peptide 377-390 = Ac-P S G V T E V V V K L F D S-NH₂ (SEQ ID NO:1132).

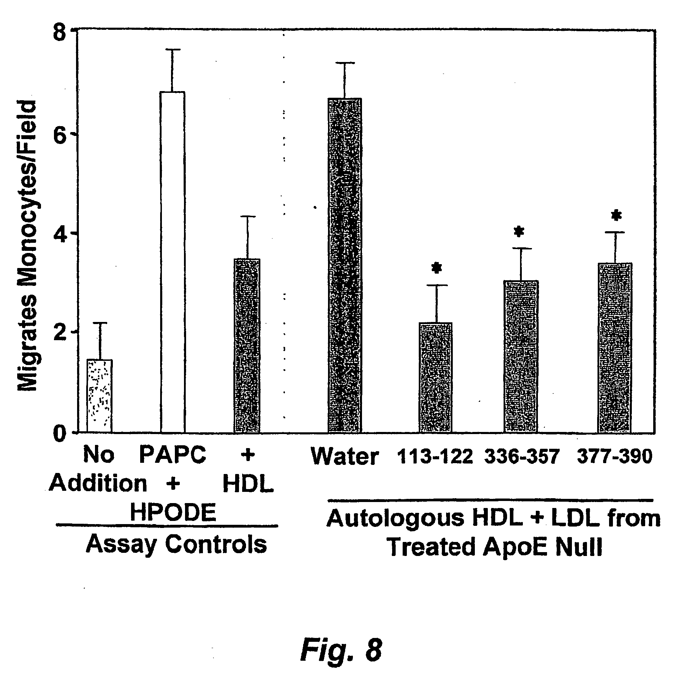

Each mouse received 200 µg of the peptide by stomach tube. Four hours later blood was obtained, plasma separated, lipoproteins fractionated and HDL (at 25 µg per ml) was assayed for protective capacity against the oxidation of LDL (at 100 µg per ml) in cultures of human artery wall cells. The data are shown in Figure 8. The peptide afforded significant HDL-protective capacity in the mice.

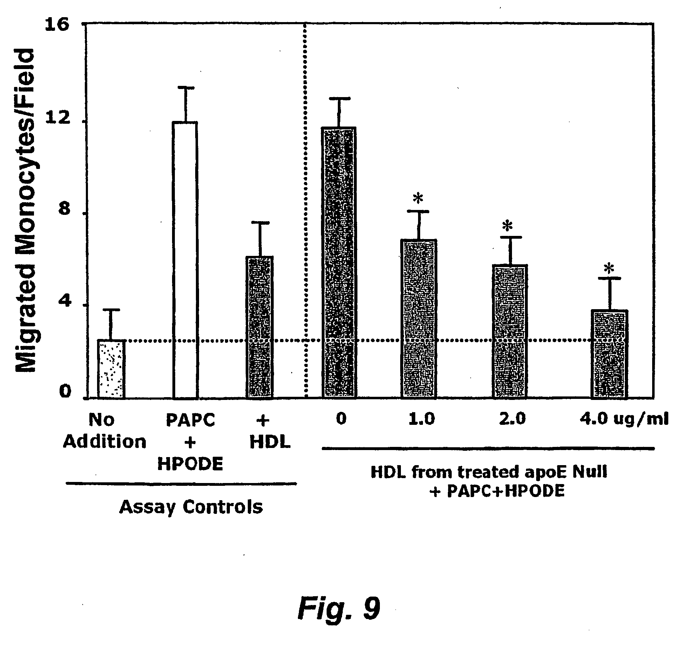

In another experiment, female, 4 month old apoE deficient mice (n=4 per group) were treated with the 11 amino acid G* peptide 146-156 with the sequence: Ac-Q Q T H M L D V M Q D-NH₂. (SEQ ID NO:1133). The mice received the peptide in their drinking water at the indicated concentrations (see Figure 9). Following eighteen hrs, blood was obtained, plasma separated, lipoproteins fractionated and HDL (at 50 µg cholesterol per ml) was assayed for protective capacity against the oxidation of PAPC (at 25 µg per ml) + HPODE (at 1.0 µg per ml) in cultures of human artery wall cells. Assay controls included No additions, PAPC+ HPODE and PAPC + HPODE plus Control HDL (designated as +HDL). The data are mean+/- SD of the number of migrated monocytes in nine high power fields in triplicate cultures. Asterisks indicate significance at the level of p<0.05 vs. the water control (0 µg/ml).

### Example 3

### Solution Phase Chemistry for Peptide Synthesis

In certain embodiments, a solution-phase synthesis chemistry provides a more economical means of synthesizing peptides of this invention.

Prior to this invention synthesis was typically performed using an all-solid phase synthesis chemistry. The solid phase synthesis of peptides of less than 9 amino acids is much more economical than the solid phase synthesis of peptides of more than 9 amino acids. Synthesis of peptides of more than 9 amino acids results in a significant loss of material due to the physical dissociation of the elongating amino acid chain from the resin. The solid phase synthesis of peptides containing less than 9 amino acids is much more economical because the there is relatively little loss of the elongating chain from the resin.

In certain embodiments, the solution phase synthesis functions by converting the synthesis of the 18 amino acid apoA-I mimetic peptide, 4F (and other related peptides) from an all solid phase synthesis to either an all solution phase synthesis or to a combination of solid phase synthesis of three chains each containing, e.g., 6 amino acids followed by the assembly of the three chains in solution. This provides a much more economical overall synthesis. This procedure is readily modified where the peptides are not 18 amino acids in length. Thus, for example, a 15 mer can be synthesized by solid phase synthesis of three 5 mers followed by assembly of the three chains in solution. A 14 mer can be synthesized by the solid phase synthesis of two 5 mers and one 4 mer followed by assembly of these chains in solution, and so forth.

### A) Summary of synthesis protocol.

An scheme for the synthesis of the peptide D4F (Ac-D-W-F-K-A-F-Y-D-K-V-A-E-K-F-K-E-A-F-NH₂, (SEQ ID NO:5) is illustrated in Table 2. (The scheme and yields for the synthesis are shown in Table 4.

**Table 4. Illustrative solution phase synthesis scheme.**

| **Methods Used for D4F Synthesis** | | | | | | |
|---|---|---|---|---|---|---|
| **Synthesis** | **Resin** | **Fmoc Amino Acid** | **Coupling Reagent** | **Final Wt. of Resin (gms)** | **Wt. of Crude Peptide (gms)** | **Wt. of Pure Peptide (mg)** |
| | | | | | **Yield (%)** | **Yield ((%)** |
| Stepwise Solid Phase | Rink Amide (1 mmole) 1.8 gms | 6 Equiv | HBTU/ HOBT | 4 | 2.0 | 500 |
| | | | | | 86 | 25 |
| Stepwise Solid Phase | Rink Amide (1 mmole) 1.8 gms | 2 Equiv | DIC/HOBT | 3.9 | 2.0 | 450 |
| | | | | | 86 | 22.5 |
| Fragment coupling (6+6+6) | Rink Amide (1 mmole) 1.8 gms* | | HBTU/ HOBT | 3.3 | 1.0 | 100 |
| | | | | | 43 | 10 |
| **Synthesis of D4F Fragments** | | | Fragment 1 (2HN-KFKEAF (SEQ ID NO:1134) on rink amide resin (K and E are properly protected) | | | |
| Fragment 2 6 residues stepwise Solid Phase | Cl-TrT-Resin (5 mmol) 6.5 gms | 6 Equiv | HBTU/ HOBT | 11 | 2.2 crude protected | |
| | | | | | 36 | |
| | | | Fmoc-Y(But)-D(But)-K(Boc)-V-A-E(But)-COOH (SEQ ID NO:1135) | | | |
| Fragment 2 6 residues stepwise Solid Phase | Cl-TrT-Resin (5 mmol) 6.5 gms | 6 Equiv | HBTU/ HOBT | 10 | 1.8 crude protected | |
| | | | | | 32 | |
| | | | Ac-D(But)-W-F-K(Boc)-A-F-COOH (SEQ ID NO:1136) | | | |

| **Synthesis by solution phase using fragments produced by the solid phase method.** | | | | | | |
|---|---|---|---|---|---|---|
| Fragment 1. | Wang resin. C-terminal hexapeptide (subjected to ammonolysis). Yield quantitative. NH2-K(Boc)-F-K(Boc)-E(But)-A-F-Wang resin (SEQ ID NO:1137) | | | | | |
| | NH2-K(Boc)-F-K(Boc)-E(But)-A-F-CO-NH2 (SEQ ID NO:1138) | | | | | |
| Fragment 2 from above was coupled to fragment 1 in DMF using DIC/HOBT. | | | | | | |
| | Fmoc-Y(But)-D(But)-K(Bpc)-V-A-E(But)-K(Boc)-F-K(Boc)-E(But)-F-Co-NH2 (SEQ ID NO:1139) 12 residue peptide was characterized as free peptide after removing protecting groups. Yield was 50% | | | | | |
| Fmoc from the above- 12 rtesidue was removed by piperidine in DMF (20%. After drying the peptide was copled to Fragment 3 using DCl/HOBT in DMF. | | | | | | |
| | Ac-D(But)-W-F-K(Boc)-A-F-Y(But)-D(but)-K(Boc)-V-A-E(But)-K(Boc)-F-K(Boc)-E(But)-A-FCO-NH2 (SEQ ID NO:1140) | | | | | |
| | Protected peptide yield was quantitative. | | | | | |
| | Protecting groups removed using mixture of TFA (80%), phenol (5%), thioanisole (5%). water )5%), triisopropylsilane (TIS, 5%), stirred for 90 min. | | | | | |
| | Precipitated by ether and purified by C-4 HPLC column. Yield 25% | | | | | |

### B) Details of synthesis protocol.

### 1. Fragment condensation procedure to synthesize D-4F

Fragments synthesized for fragment condensation on solid phase are:
Fragment 1: Ac-D(OBut)-W-F-K(εBoc)-A-F- COOH (SEQ ID NO:1141);
Fragment 2:Fmoc-Y(OBut)-D(OBut)-K(εBoc)-V-A-E(OBut)-COOH (SEQ ID NO:1142); and
Fragment 3 Fmoc-K(εBoc)F-K(εBoc)-E(OBut)-A-F- Rink amide resin (SEQ ID NO:1143).

Fragment 1 was left on the resin to obtain final peptide amide after TFA treatment.

To synthesize fragment 1: Fmoc-Phe (1.2 equivalents) was added to chlorotrityl resin (Nova Biochem, 1.3 mMol/g substitution, 5 mMol or 6.5 g was used) in presence of six equivalents of DIEA in DMF:dichloromethane (1:1)) and stirred for 4h. Excess of functionality on the resin was capped with methanol in presence of dichloromethane and DIEA. After the removal of Fmoc- Fmoc amino acid derivatives (2 equivalents) were added using HOBt/HBTU reagents as described above. Final Fmoc-D(OBut)-W-F-K(εBoc)-A-F Chlorotrityl resin was treated with Fmoc deblocking agent and acetylated with 6 equivalents of acetic anhydride in presence of diisoprolylethyl amine. The resulting Ac-D(OBut)-W-F-K(εBoc)-A-F -resin was treated with a mixture of triflouroethanol-acetic acid-dichloromethane (2:2:6, 10ml/g of resin) for 4h at room temperature. After removal of the resin by filtration, the solvent was removed by aziotropic distillation with n-hexane under vacuum. The residue (1.8g) was determined by mass spectral analysis to be Ac-D(OBut)-W-F-K(εBoc)-A-F -COOH (SEQ ID NO:1144).

Fragment 2, Fmoc-Y(OBut)-D(OBut)-K(εBoc)-V-A-E(OBut)-COOH (SEQ ID NO:1145), was obtained using the procedure described for Fragment 1. Final yield was 2.2g.

Fragment 3. 0.9g (0.5mmol) of Rink amide resin (Nova Biochem) was used to obtain fragment Rink amide resin was treated with 20% pipetidine in dichloromethane for 5 min once and 15 min the second time (Fmoc deblocking reagents). 1. 2equivalents of Fmoc-Phe was condensed using condensing agents HOBt/HBTU (2 equivalents in presence of few drops of diisopropylethyl amine) (amino acid condensation). Deblocking and condensation of the rest of the amino acids were continued to obtain the of Fmoc- K(εBoc)F-K(εBoc)-E(OBut)-A-F -rink amide resin (SEQ ID NO:11146). Fmoc was cleaved and the peptide resin K(εBoc)F-K(εBoc)-E(OBut)-A-F- rink amide resin (SEQ ID NO:1146) was used for fragment condensation as described below.

Fragment 2 in DMF was added to Fragment 3 (1.2 equivalents) using HOBt-HBTU procedure in presence of DIEA overnight. After washing the resin with DMF and deblocking Fmoc- Fragment 1 (1.2 equivalents) was added to the dodecapeptide resin using HOBt-HBTU procedure overnight.

The final peptide resin (3.3g) was treated with a mixture of TFA-Phenol-triisopropylsilane-thioanisole-water (80:5:5:5) for 1.5h (10 ml of the reagent/g of the resin). The resin was filtered off and the solution was diluted with10 volumes of ether. Precipitated peptide was isolated by centrifugation and washed twice with ether. 1g of the crude peptide was subjected to HPLC purification to obtain 100 mg of the peptide.

### 2. Characterization of peptide.

The peptide was identified by mass spectral and analytical HPLC methods.

As shown in Figure 14 the product of the solution phase synthesis scheme is very biologically active in producing HDL and pre-beta HDL that inhibit LDL-induced monocyte chemotaxis in apo E null mice. ApoE null mice were fed 5 micrograms of the D-4F synthesized as described above (Frgmnt) or the mice were given the same amount of mouse chow without D-4F (Chow). Twelve hours after the feeding was started, the mice were bled and their plasma was fractionated on FPLC. LDL (100 micrograms LDL-cholesterol) was added to cocultures of human artery wall cells alone (LDL) or with a control human HDL (Control HDL) or with HDL (50 micrograms HDL-cholesterol) or post-HDL (pHDL; prebeta HDL) from mice that did (Frgmnt) or did not (Chow) receive the D-4F and the monocyte chemotactic activity produced was determined

### Example 4 Comparison of D-4F and Reverse (retro-) D-4F Activity.

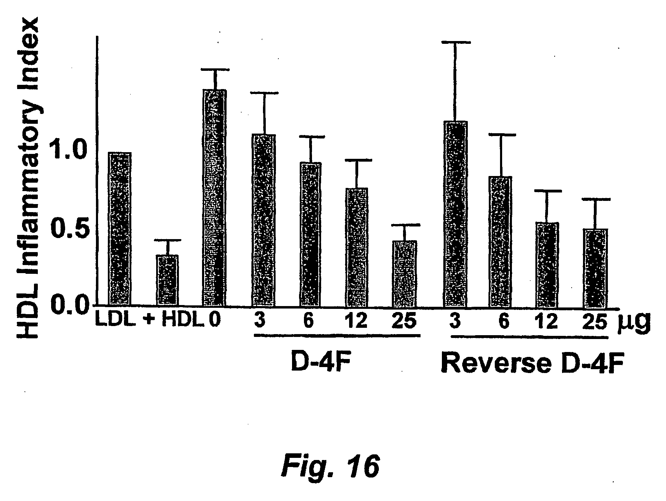

As shown in Figure 16, the biological activities of D-4F and reverse RD-4F are not significantly different. Female apoE null mice were administered by stomach tube 0, 3, 6, 12, or 25 micrograms of D-4F or Reverse D-4F in 100 microliters of water. Blood was obtained 7 hours later and the plasma was fractionated by FPLC. A standard control human LDL was added to human artery wall cells at a concentration of 100 micrograms of LDL-cholesterol/mL (LDL). The resulting monocyte chemotactic activity was normalized to 1.0. The same LDL at the same concentration was added to the human artery wall cells together with HDL at 50 micrograms HDL-cholesterol/mL from a normal human (hHDL) or from the apoE null mice that received the dose of D-4F or Reverse D-4F shown on the X-axis. The resulting monocyte chemotactic activity was normalized to that of the LDL added without HDL. The resulting value is the HDL Inflammatory Index. The results shown are the Mean ± S.D. for the data from three separate experiments.

## Claims

1. A "D" or "L" peptide comprising the amino acid sequence D-W-L-K-A-F-Y-D-K-F-F-E-K-F-K-E-F-F (SEQ ID NO: 7), or the retro form of said sequence F-F-E-K-F-K-E-F-F-K-D-Y-F-A-K-LW-D (SEQ ID NO:537), for use in a method for the treatment of cancer.

2. The peptide for use in claim 1, wherein the amino acid sequence of said peptide consists of the sequence D-W-L-K-A-F-Y-D-K-F-F-E-K-F-K-E-F-F (SEQ ID NO:7).

3. The peptide for use according to any one of claims 1-2, wherein said peptide comprises all "L" amino acids.

4. The peptide for use according to any one of claims 1-2, wherein said peptide comprises all "D" amino acids.

5. The peptide for use according to any one of claims 1-4, wherein said peptide further comprises a protecting group coupled to the amino or carboxyl terminus.

6. The peptide for use according to any one of claims 1-4, wherein said peptide comprises a first protecting group coupled to the amino terminus and a second protecting group coupled to the carboxyl terminus.

7. The peptide for use in claim 6, wherein the first protecting group and the second protecting group are independently selected from the group consisting of acetyl, amide, and 3 to 20 carbon alkyl groups, Fmoc, Tboc, 9-fluoreneacetyl group, 1- fluorenecarboxylic group, 9-fluorenecarboxylic group, 9-fluore-none- -carboxylic group, benzyloxycarbonyl, Xanthyl (Xan), Trityl (Trt), 4-methyltrityl (Mtt), 4-methoxytrityl (Mmt), 4-methoxy-2,3,6-trimethyl-benzenesulphonyl(Mtr), Mesitylene-2-sulphonyl (Mts), 4,4-dimethoxybenzhydryl (Mbh),Tosyl (Tos), 2,2,5,7,8-pentamethyl chroman-6-sulphonyl (Pmc), 4-methylbenzyl (MeBzl), 4-methoxybenzyl (MeOBzl), Benzyloxy (Bz.lO), Benzyl (Bzl), Benzoyl (Bz),3-nitro-2-pyridinesulphenyl(Npys), 1-(4,4-dimethyl-2,6-dioxocyclohexylidene)ethyl (Dde), 2,6-dichlorobcnzyl (2,6-DiCl-Bzl), 2-chlorobenzyloxycarbonyl (2-Cl-Z), 2-bromobenzyloxycarbonyl (2-Br-Z), Benzyloxymethyl (Bom), t-butoxycarbonyl (Boc), cyclohexyloxy (cHxO),t-butoxymethyl(Bum), t-butoxy (tBuO), t-Butyl (tBu), Acetyl (Ac), and Trifluoroacetyl (TFA).

8. The peptide for use in claim 6, wherein said first protecting group is selected from the group consisting of acetyl, propionyl, and a 3 to 20 carbon alkyl.

9. The peptide for use in claim 6, wherein said first protecting group is an acetyl and said second protecting group is an amide.

10. The peptide for use according to any one of claims 1-9, wherein the peptide is mixed with a pharmacologically acceptable excipient.

11. The peptide for use in claim 10, wherein said peptide is mixed with a pharmacologically acceptable excipient suitable for oral administration to a mammal.

12. The peptide for use in claim 10, wherein said peptide is mixed with a pharmacologically acceptable excipient suitable for injection to a mammal.

13. The peptide for use in any preceding claim, wherein said cancer is selected from the group consisting of myeloma/multiple myeloma, ovarian cancer, breast cancer, colon cancer, and bone cancer.

## Patentansprüche

1. "D"- oder "L"-Peptid, umfassend die Aminosäuresequenz D-W-L-K-A-F-Y-D-K-F-F-E-K-F-K-E-F-F (SEQ ID NO: 7) oder die Retroform der Sequenz F-F-E-K-F-K-E-F-F-K-D-Y-F-A-K-L-W-D (Sequenz ID NO: 537) zur Verwendung in einem Verfahren zur Behandlung von Krebs.

2. Peptid zur Verwendung in Anspruch 1, wobei die Aminosäuresequenz des Peptids aus der Sequenz D-W-L-K-A-F-Y-D-K-F-F-E-K-F-K-E-F-F (SEQ ID NO: 7) besteht.

3. Peptid zur Verwendung gemäß einem der Ansprüche 1-2, wobei das Peptid alle "L"-Aminosäuren umfasst.

4. Peptid zur Verwendung gemäß einem der Ansprüche 1-2, wobei das Peptid alle "D"-Aminosäuren umfasst.

5. Peptid zur Verwendung gemäß einem der Ansprüche 1-4, wobei das Peptid weiterhin eine Schutzgruppe umfasst, die mit dem Amino- oder Carboxy-Terminus gekoppelt ist.

6. Peptid zur Verwendung gemäß einem der Ansprüche 1-4, wobei das Peptid eine erste Schutzgruppe umfasst, die mit dem Amino-Terminus gekoppelt ist, und eine zweite Schutzgruppe, die mit dem Carboxy-Terminus gekoppelt ist.

7. Peptid zur Verwendung in Anspruch 6, wobei die erste Schutzgruppe und die zweite Schutzgruppe unabhängig ausgewählt sind aus der Gruppe, bestehend aus Acetyl-, Amid- und 3 bis 20 C-Alkylgruppen, Fmoc, t-Boc, 9-Fluorenacetyl-Gruppe, 1-Fluorencarbonsäure-Gruppe, 9-Fluorencarbonsäure-Gruppe, 9-Fluorenon-1-carbonsäure-Gruppe, Benzyloxycarbonyl, Xanthyl (Xan), Trityl (Trt), 4-Methyltrityl (Mtt), 4-Methoxytrityl (Mmt), 4-Methoxy-2,3,6-trimethylbenzolsulfonyl (Mtr), Mesitylen-2-sulfonyl (Mts), 4,4-Dimethoxybenzhydryl (Mbh), Tosyl (Tos), 2,2,5,7,8-Pentamethyl-chroman-6-sulfonyl (Pmc), 4-Methylbenzyl (MeBzl), 4-Methoxybenzyl (Me0Bzl), Benzyloxy (BzlO), Benzyl (Bzl), Benzoyl (Bz), 3-Nitro-2-pyridinsulfenyl (Npys), 1-(4,4-Dimethyl-2,6-dioxocyclohexyliden)ethyl (Dde), 2,6-Dichlorbenzyl-(2,6-DiCI-Bzl), 2-Chlorbenzyloxycarbonyl (2-Cl-Z), 2-Brombenzyloxycarbonyl (2-Br-Z), Benzyloxymethyl (Bom), t-Butoxycarbonyl (Boc), Cyclohexyloxy (cHxO), t-Butoxymethyl (Bum), t-Butoxy (tBuO), t-Butyl (tBu), Acetyl (Ac) und Trifluoracetyl (TFA).

8. Peptid zur Verwendung in Anspruch 6, wobei die erste Schutzgruppe aus der Gruppe, bestehend aus Acetyl, Propionyl und 3 bis 20 C-Alkyl, ausgewählt ist.

9. Peptid zur Verwendung in Anspruch 6, wobei die erste Schutzgruppe ein Acetyl und die zweite Schutzgruppe ein Amid ist.

10. Peptid zur Verwendung gemäß einem der Ansprüche 1-9, wobei das Peptid mit einem pharmakologisch verträglichen Hilfsstoff gemischt ist.

11. Peptid zur Verwendung in Anspruch 10, wobei das Peptid mit einem pharmakologisch verträglichen Hilfsstoff gemischt ist, der zur oralen Verabreichung an ein Säugetier geeignet ist.

12. Peptid zur Verwendung in Anspruch 10, wobei das Peptid mit einem pharmakologisch verträglichen Hilfsstoff gemischt ist, der zur Injektion in ein Säugetier geeignet ist.

13. Peptid zur Verwendung in einem der vorangehenden Ansprüche, wobei der Krebs aus der Gruppe, bestehend aus Myelom/Multiples Myelom, Eierstockkrebs, Brustkrebs, Darmkrebs und Knochenkrebs, ausgewählt ist.

## Revendications

1. Peptide de type "D" ou "L" comprenant la séquence d'acides aminés D-W-L-K-A-F-Y-D-K-F-F-E-K-F-K-E-F-F (SÉQ ID N° : 7) ou la forme inverse de ladite séquence F-F-E-K-F-K-E-F-F-K-D-Y-F-A-K-L-W-D (SÉQ ID N° : 537) pour leur utilisation dans une méthode de traitement du cancer.

2. Peptide pour son utilisation dans la revendication 1, dans lequel la séquence d'acides aminés dudit peptide consiste en la séquence D-W-L-K-A-F-Y-D-K-F-F-E-K-F-K-E-F-F (SÉQ ID N° : 7).

3. Peptide pour son utilisation selon l'une quelconque des revendications 1 à 2, dans lequel ledit peptide comprend tous les acides aminés "L".

4. Peptide pour son utilisation selon l'une quelconque des revendications 1 à 2, dans lequel ledit peptide comprend tous les acides aminés "D".

5. Peptide pour son utilisation selon l'une quelconque des revendications 1 à 4, dans lequel ledit peptide comprend en outre un groupe protecteur couplé à la terminaison amino ou carboxyle.

6. Peptide pour son utilisation selon l'une quelconque des revendications 1 à 4, dans lequel ledit peptide comprend un premier groupe protecteur couplé à la terminaison amino et un deuxième groupe protecteur couplé à la terminaison carboxyle.

7. Peptide pour son utilisation dans la revendication 6, dans lequel le premier groupe protecteur et le deuxième groupe protecteur sont choisis indépendamment dans le groupe consistant en un groupe acétyle, un amide et un groupe alkyle en C₃ à C₂₀, Fmoc, Tboc, un groupe 9-fluorène-acétyle, un groupe 1-fluorène-carboxylique, un groupe 9-fluorène-carboxylique, un groupe 9-fluorénone-1-carboxylique, les groupes benzyloxy-carbonyle, xanthyle (Xan), trityl (Trt), 4-méthyltrityle (Mtt), 4-méthoxytrityle (Mmt), 4-méthoxy-2,3,6-triméthyl-benzènesulfonyle (Mtr), mésitylène -2-sulfonyle (Mts), 4,4-diméthoxybenz-hydryle (Mbh), tosyle (Tos), 2,2,5,7,8-penta-méthyl-chroman-6-sulfonyle (Pmc), 4-méthylbenzyle (MeBzl), 4-méthoxybenzyle (MeOBzl), benzyloxy (BzlO) , benzyle (Bzl), benzoyle (Bz), 3-nitro-2-pyridinesulfényle (Npys), 1-(4,4-diméthyl-2,6-dioxo-cyclohexylidène)éthyle (Dde), 2,6-dichlorobenzyle (2,6-DiCl-Bzl), 2-chlorobenzyloxycarbonyle (2-Cl-Z) , 2-bromobenzyloxycarbonyle (2-Br-Z), benzyloxy-méthyle (Bom), t-butoxycarbonyle (Boc), cyclohexyl-oxy (cHxO), t-butoxyméthyl (Bum), t-butoxy (tBuO), t-butyle (tBu), acétyle (Ac) et trifluoroacétyle (TFA).

8. Peptide pour son utilisation dans la revendication 6, dans lequel ledit premier groupe protecteur est choisi dans le groupe consistant en groupes acétyle, propionyle et alkyle en C₃ à C₂₀.

9. Peptide pour son utilisation dans la revendication 6, dans lequel ledit premier groupe protecteur est un groupe acétyle et ledit deuxième groupe protecteur est un amide.

10. Peptide pour son utilisation selon l'une quelconque des revendications 1 à 9, dans lequel le peptide est mélangé avec un excipient pharmacologiquement acceptable.

11. Peptide pour son utilisation dans la revendication 10, dans lequel ledit peptide est mélangé avec un excipient pharmacologiquement acceptable approprié pour l'administration orale à un mammifère.

12. Peptide pour son utilisation dans la revendication 10, dans lequel ledit peptide est mélangé avec un excipient pharmacologiquement acceptable approprié pour l'injection à un mammifère.

13. Peptide pour son utilisation dans l'une quelconque des revendications précédentes, dans lequel ledit cancer est choisi dans le groupe consistant en un myélome/myélome multiple, un cancer de l'ovaire, un cancer du sein, un cancer du côlon et un cancer des os.
